# EUROPEAN PATENT APPLICATION

(11) **EP 4 582 102 A1**
(43) Date of publication of application: **09.07.2025**
(21) Application number: 23860449.0
(22) Date of filing: 30.08.2023
(51) Int. Cl.: A61K 45/00, A61K 31/437, A61K 31/4745, A61K 31/7068, A61K 31/7105, A61K 45/06, A61K 48/00, A61P 35/00, A61P 43/00, C07K 16/18, C12N 15/113, C12Q 1/02, C12Q 1/6886, G01N 33/15, G01N 33/50

(54) **THERAPEUTIC AGENT AND THERAPEUTIC METHOD WHICH ARE FOR CANCER PATIENTS EXHIBITING RB1 FUNCTIONAL DETERIORATION AND WHICH INVOLVE CONCOMITANT USE OF MYT1 INHIBITOR AND CHEMOTHERAPEUTIC AGENT**

(30) Priority: 31.08.2022 JP 2022138548; 28.02.2023 JP 2023029739
(71) Applicant: CHUGAI SEIYAKU KABUSHIKI KAISHA, Tokyo 115-8543 (JP)
(72) Inventor: OHTE Yuki, Yokohama City, Kanagawa 244-8602 (JP); SAKAMOTO Hiroshi, Yokohama City, Kanagawa 244-8602 (JP); SUYAMA Eigo, Yokohama City, Kanagawa 244-8602 (JP); OGASAWARA Kiyomoto, Yokohama City, Kanagawa 244-8602 (JP); SOGA Mayumi, Yokohama City, Kanagawa 244-8602 (JP); GOTO Takahiro, Yokohama City, Kanagawa 244-8602 (JP)
(74) Representative: Vossius & Partner Patentanwälte Rechtsanwälte mbB
(86) International application number: PCT/JP2023/031636
(87) International publication number: WO 2024/048687

(57) **Abstract**

The present invention provides a pharmaceutical composition comprising, in combination with a chemotherapeutic agent, an MYT1 inhibitor as an active ingredient for treatment or prevention of cancer of a patient with a decrease in RB1 function.

## Description

### [Technical Field]

The present invention relates to a therapeutic agent for cancer of a patient with a decrease in RB1 function using an MYT1 inhibitor and a chemotherapeutic agent in combination, and a treatment method thereof.

### [Background Art]

In recent years, rapid advances in genome sequencing techniques enable genome information including gene mutation inherent in cancer cells to be deciphered. Examples of gene mutation inherent in cancer cells include EGFR gene mutation, BRAF gene mutation, and gain-of-function genes such as ALK fusion genes and ROS1 fusion genes. For example, the ALK fusion gene is a gain-of-function gene formed by infusing an ALK gene encoding a receptor tyrosine kinase and a gene encoding a protein having a multimerization function, such as EML4, through inversion or translocation of chromosomes. In these circumstances, drug discovery research aimed at selectively inhibiting the function of the protein of cancer cells with inherent gene mutation has been conducted in anticancer agent development (see Non Patent Literatures 1 to 3). The treatment method targeting cancer cells having these gene mutations is expected to be a treatment method having high selectivity to cancer cells and a high effect.

On the other hand, gene mutations found in cancer cells of humans include not only gain-of-function, but also loss-of-function mutations. Drug discovery targeting the gene itself of the loss-of-function gene mutation is difficult, and a therapeutic strategy different from that of cancer having a gain-of-function gene mutation is required.

Examples of a few successful cases that achieved specific targeting of cancer cells having a loss-of-function mutation include a treatment with a PARP inhibitor for BRCA1/2 deficient tumor (see Non Patent Literature 4). However, therapeutic strategies for specifically targeting cancer cells having other loss-of-function mutations have not been developed yet.

The loss of function of the RB1 gene has been considered to be a cause or a promoting factor of retinoblastoma. However, in recent years, a decrease in function such as loss-of-function mutation or suppression of expression of the RB1 gene is reported to be observed in many human cancers, and involvement in the occurrence and progression of a tumor is attracting attention in a wide variety of cancers. The RB1 protein is considered to be involved in the biology of various cancers, such as cell cycle, inflammation, metabolism, autophagy, apoptosis, differentiation, aging, DNA repair, and stability of genomes. It is presumed that there is an important relationship between such a functional suppression and the occurrence and progression of cancer, and researches are conducted (see Non Patent Literature 5).

Actually, it is reported that the deficiency of RB1 protein and Aurora kinase A are in the relationship of synthetic lethality in small cell lung cancer and triple negative breast cancer (a type in which all of the estrogen receptor, progesterone receptor, and HER2 protein are negative) (see Non Patent Literature 6) as well as the deficiency of RB1 protein and CHK1 or PLK1 are in the relationship of synthetic lethality in triple negative breast cancer (see Non Patent Literature 7).

It is also reported that an in vivo antitumor effect is expected by using RP-6306 and gemcitabine in combination in NIH:OVCAR-3 cells in which CCNE1 is amplified (see Patent Literature 1, Non Patent Literature 10).

However, a therapeutic strategy specifically targeting cancer cells with a decrease in RB1 function is not sufficient yet.

### [Citation List]

### [Patent Literature]

[Patent Literature 1] WO 2021/195781

### [Non Patent Literature]

[Non Patent Literature 1] Makoto Maemondo et al. NEJM 2010 Jun 24; 362(25):2380-2388.
[Non Patent Literature 2] Paul B. Chapman et al. NEJM 2011 Jun 30; 364(26):2507-2516.
[Non Patent Literature 3] D. Ross Camidge et al. J. Thorac. Oncol. 2019 Jul; 14(7):1233-1243.
[Non Patent Literature 4] Kathleen Moore et al. NEJM 2018 Dec 27; 379(26):2495-2505.
[Non Patent Literature 5] Letian Zhang et al. Annu Rev Cancer Biol. 2022 April Vol.6:201-221.
[Non Patent Literature 6] Xueqian Gong et al. Cancer Discov. 2019 Feb;9(2):248-263.
[Non Patent Literature 7] Agnieszka K. et al. Cell Rep. 2018 Jan 30;22(5):1185-1199.
[Non Patent Literature 8] Patricia Jaaks et al. Nature 2022 Mar;603(7899):166-173.
[Non Patent Literature 9] Alina Malyutina et al. PLoS Comput Biol. 2019 May 20;15(5):e1006752.
[Non Patent Literature 10] David Gallo et al. Nature 2022 Apr;604(7907):749-756.
[Non Patent Literature 11] Sarah E. Taylor et al. Cancer Res. 2019 Aug; 79(16):4242-4257.
[Non Patent Literature 12] Paola Indovina et al. Oncotarget 2015 Jul;6(20): 17873-17890.
[Non Patent Literature 13] Kenta Kurayoshi et al. DOI: 10.5772/intechopen.72125.

### [Summary of Invention]

### [Technical Problem]

Thus, an object of the present invention is to provide a new method for treating or preventing cancer with a decrease in RB1 function.

### [Solution to Problem]

The present invention provides, for example, the following [A-1] to [A-73], [B-1] to [B-71], [C-1] to [C-72], [D-1] to [D-72], [E-1] to [E-72], [F-1] to [F-72], [G-1] to [G-32], [H-1] to [H-72], [1-1] to [1-72], [J-1] to [J-72], and [K-1] to [K-72].

[A-1]
A pharmaceutical composition comprising, in combination with a chemotherapeutic agent, an MYT1 inhibitor as an active ingredient for treatment or prevention of cancer of a cancer patient in which RB1 gene mutation positivity, a decrease in expression of an RB1 gene or protein, or positive expression of hyperphosphorylated RB1 protein is detected.

[A-1.1]
A pharmaceutical composition comprising, in combination with a chemotherapeutic agent, an MYT1 inhibitor as an active ingredient for treatment or prevention of cancer of a cancer patient in which RB1 gene mutation positivity or a decrease in expression of an RB1 gene or protein is detected.

[A-1.2]
A pharmaceutical composition comprising, in combination with a chemotherapeutic agent, an MYT1 inhibitor as an active ingredient for treatment or prevention of cancer of a cancer patient in which positive expression of hyperphosphorylated RB1 protein is detected.

[A-2]
A pharmaceutical composition comprising, in combination with an MYT1 inhibitor, a chemotherapeutic agent as an active ingredient for treatment or prevention of cancer of a cancer patient in which RB1 gene mutation positivity, a decrease in expression of an RB1 gene or protein, or positive expression of hyperphosphorylated RB1 protein is detected.

[A-2.1]
A pharmaceutical composition comprising, in combination with an MYT1 inhibitor, a chemotherapeutic agent as an active ingredient for treatment or prevention of cancer of a cancer patient in which RB1 gene mutation positivity or a decrease in expression of an RB1 gene or protein is detected.

[A-2.2]
A pharmaceutical composition comprising, in combination with an MYT1 inhibitor, a chemotherapeutic agent as an active ingredient for treatment or prevention of cancer of a cancer patient in which positive expression of hyperphosphorylated RB1 protein is detected.

[A-3]
The pharmaceutical composition according to [A-1] or [A-2], wherein the RB1 gene mutation comprises a mutation causing insertion, substitution, deletion, and/or addition of at least one amino acid residue to wild-type RB1 protein.

[A-4]
The pharmaceutical composition according to any one of [A-1] to [A-3], wherein the RB 1 gene mutation is a nonsense mutation, a frameshift mutation, a splice site mutation, or a homozygous or heterozygous deletion.

[A-5]
The pharmaceutical composition according to any one of [A-1] to [A-4], wherein the RB 1 gene mutation is a mutation decreasing a function of RB1.

[A-6] The pharmaceutical composition according to any one of [A-1] to [A-4], wherein the RB 1 gene mutation is a human RB1 gene mutation.

[A-7]
The pharmaceutical composition according to [A-6], wherein the human RB1 gene mutation is a mutation causing at least one of the following (1) to (5):
(1) a codon corresponding to a serine residue (S) at position 82 of an amino acid sequence of SEQ ID No: 2 is replaced with a stop codon,
(2) a codon corresponding to an arginine residue (R) at position 467 of the amino acid sequence of SEQ ID No: 2 is replaced with a stop codon,
(3) in a codon corresponding to an amino acid residue at position 182, at least one base is inserted or deleted, a new reading frame starting from an isoleucine residue (I) is formed, and a third reading frame therefrom is a stop codon,
(4) a glutamic acid residue (E) at position 837 of the amino acid sequence of SEQ ID No: 2 is replaced with a lysine residue (K), and a part of the RB1 gene is homozygously deleted, and
(5) a glycine residue (G) at position 449 of the amino acid sequence of SEQ ID No: 2 is replaced with a glutamic acid residue (E), and a part of the RB1 gene is homozygously deleted.

[A-8]
The pharmaceutical composition according to [A-1] or [A-2], wherein the decrease in expression of an RB1 gene or protein comprises a decrease in gene expression through methylation of an RB1 gene or a micro RNA.

[A-8.1]
The pharmaceutical composition according to any one of [A-1] to [A-7], wherein the hyperphosphorylated RB1 protein is RB1 protein having 3 or more phosphorylated amino acid residues in the amino acid sequence of the RB1 protein.

[A-8.2]
The pharmaceutical composition according to any one of [A-1] to [A-7], wherein the hyperphosphorylated RB1 protein is RB1 protein having 4 or more phosphorylated amino acid residues in the amino acid sequence of the RB1 protein.

[A-8.3]
The pharmaceutical composition according to any one of [A-1] to [A-7], wherein the hyperphosphorylated RB1 protein is RB1 protein having 8 or more phosphorylated amino acid residues in the amino acid sequence of the RB1 protein.

[A-8.4]
The pharmaceutical composition according to any one of [A-1] to [A-7], wherein the hyperphosphorylated RB1 protein is RB1 protein having 15 or more phosphorylated amino acid residues in the amino acid sequence of the RB1 protein.

[A-8.5]
The pharmaceutical composition according to any one of [A-1] to [A-7], wherein the hyperphosphorylated RB1 protein is hyperphosphorylated human RB1 protein.

[A-8.6]
The pharmaceutical composition according to [A-8.5], wherein the hyperphosphorylated human RB1 protein is RB1 protein in which at least one amino acid residue selected from the group consisting of a threonine residue (T) at position 826, a threonine residue (T) at position 823, a threonine residue (T) at position 821, a serine residue (S) at position 816, a tyrosine residue (Y) at position 813, a serine residue (S) at position 811, a serine residue (S) at position 807, a tyrosine residue (Y) at position 805, a serine residue (S) at position 780, a threonine residue (T) at position 625, a threonine residue (T) at position 601, a threonine residue (T) at position 373, a serine residue (S) at position 360, a threonine residue (T) at position 356, a serine residue (S) at position 249, and a serine residue (S) at position 37 of SEQ ID No: 2 is phosphorylated.

[A-8.7]
The pharmaceutical composition according to [A-8.5], wherein the hyperphosphorylated human RB1 protein is RB1 protein in which at least one amino acid residue selected from the group consisting of a threonine residue (T) at position 826, a threonine residue (T) at position 821, a serine residue (S) at position 811, a serine residue (S) at position 807, a serine residue (S) at position 780, a threonine residue (T) at position 373, and a threonine residue (T) at position 356 of SEQ ID No: 2 is phosphorylated.

[A-8.8]
The pharmaceutical composition according to [A-8.5], wherein the hyperphosphorylated human RB1 protein is RB1 protein in which at least one amino acid residue selected from the group consisting of a threonine residue (T) at position 826, a threonine residue (T) at position 821, a serine residue (S) at position 811, and a serine residue (S) at position 807 of SEQ ID No: 2 is phosphorylated.

[A-8.9]
The pharmaceutical composition according to [A-8.5], wherein the hyperphosphorylated human RB1 protein is RB1 protein in which at least two amino acid residues selected from the group consisting of a threonine residue (T) at position 826, a threonine residue (T) at position 821, and a serine residue (S) at position 811 and/or a serine residue (S) at position 807 of SEQ ID No: 2 are phosphorylated.

[A-8.10]
The pharmaceutical composition according to [A-8.5], wherein the hyperphosphorylated human RB1 protein is RB1 protein in which a threonine residue (T) at position 826 and a threonine residue (T) at position 821 of SEQ ID No: 2 are phosphorylated, and a serine residue (S) at position 811 and/or a serine residue (S) at position 807 are/is phosphorylated.

[A-9]
The pharmaceutical composition according to any one of [A-1] to [A-8], wherein the MYT1 inhibitor is at least one selected from the group consisting of a low molecular compound, a polypeptide, and a polynucleotide.

[A-10]
The pharmaceutical composition according to [A-9], wherein the MYT1 inhibitor is a low molecular compound.

[A-11]
The pharmaceutical composition according to [A-10], wherein the low molecular compound is a compound having a molecular weight of 2000 g/mol or less.

[A-12]
The pharmaceutical composition according to [A-10], wherein the low molecular compound is a compound having a molecular weight of 1000 g/mol or less.

[A-13] The pharmaceutical composition according to [A-9], wherein the MYT1 inhibitor is a polypeptide.

[A-14]
The pharmaceutical composition according to [A-13], wherein the polypeptide comprises an antibody.

[A-15]
The pharmaceutical composition according to [A-13], wherein the polypeptide is an anti-MYT1 antibody.

[A-16]
The pharmaceutical composition according to [A-9], wherein the MYT1 inhibitor is a polynucleotide.

[A-17]
The pharmaceutical composition according to [A-16], wherein the polynucleotide is at least one selected from the group consisting of a ribozyme, an antisense molecule, an inhibitor oligonucleotide, an aptamer, a micro RNA, and a small interfering RNA (siRNA).

[A-18]
The pharmaceutical composition according to [A-16], wherein the polynucleotide is at least one selected from the group consisting of an antisense nucleic acid, a micro RNA, and a small interfering RNA (siRNA).

[A-19]
The pharmaceutical composition according to any one of [A-1] to [A-12], wherein the MYT1 inhibitor is a compound represented by the formula (1): 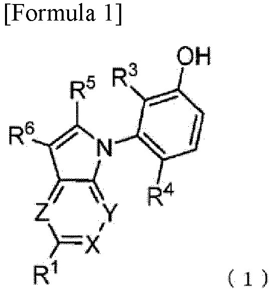
wherein each of X, Y, and Z is independently N or CR²;
each of R¹ and R² is independently a hydrogen atom, optionally substituted C₁₋₆ alkyl, optionally substituted C₂₋₆ alkenyl, optionally substituted C₂₋₆ alkynyl, optionally substituted C₃₋₈ cycloalkyl, optionally substituted C₃₋₈ cycloalkenyl, optionally substituted C₂₋₉ heterocyclyl, optionally substituted C₂₋₉ heterocyclyl C₁₋₆ alkyl, optionally substituted C₆₋₁₀ aryl, optionally substituted C₁₋₉ heteroaryl, optionally substituted C₁₋₉ heteroaryl C₁₋₆ alkyl, a halogen atom, cyano, -N(R⁷)₂, -OR⁷, -C(O)N(R⁸)₂, -SO₂N(R⁸)₂, -SO₂R^{7A}, or -Q-R^{7B}, or R¹ form optionally substituted C₃₋₆ alkylene together with one R² adjacent to R¹;
each of R³ and R⁴ is independently optionally substituted C₁₋₆ alkyl or a halogen atom;
R⁵ is a hydrogen atom or -N(R⁷)₂;
R⁶ is -C(O)NH(R⁸), -C(O)R^{7A}, or -SO₂R^{7A}
R⁷ is each independently a hydrogen atom, optionally substituted C₁₋₆ alkyl, optionally substituted C₆₋₁₀ aryl C₁₋₆ alkyl, optionally substituted C₃₋₈ cycloalkyl, optionally substituted C₆₋₁₀ aryl, optionally substituted C₂₋₉ heterocyclyl, optionally substituted C₁₋₉ heteroaryl, optionally substituted C₁₋₉ heteroaryl C₁₋₆ alkyl, or -SO₂R^{7A}, or two R⁷ form optionally substituted C₂₋₉ heterocyclyl in combination with an atom in contact with both of them;
R^{7A} is each independently optionally substituted C₁₋₆ alkyl, optionally substituted C₃₋₈ cycloalkyl, or optionally substituted C₆₋₁₀ aryl;
R^{7B} is each independently hydroxyl, optionally substituted C₁₋₆ alkyl, optionally substituted C₆₋₁₀ aryl, optionally substituted C₂₋₉ heterocyclyl, optionally substituted C₁₋₉ heteroaryl, -N(R⁷)₂, -C(O)N(R⁸)₂, -SO₂N(R⁸)₂, -SO₂R^{7A}, or optionally substituted alkoxy;
R⁸ is each independently a hydrogen atom, optionally substituted C₁₋₆ alkyl, optionally substituted C₂₋₆ alkoxyalkyl, optionally substituted C₆₋₁₀ aryl C₁₋₆ alkyl, optionally substituted C₆₋₁₀ aryl, optionally substituted C₃₋₈ cycloalkyl, or optionally substituted C₁₋₉ heteroaryl, or two R⁸ form optionally substituted C₂₋₉ heterocyclyl in combination with an atom in contact with both of them; and
Q is optionally substituted C₁₋₆ alkylene, optionally substituted C₂₋₆ alkenylene, optionally substituted C₂₋₆ alkynylene, optionally substituted C₃₋₈ cycloalkylene, optionally substituted C₃₋₈ cycloalkenylene, optionally substituted C₆₋₁₀ arylene, optionally substituted C₂₋₉ heterocyclylene, or optionally substituted C₁₋₉ heteroarylene,
or a salt thereof, or a solvate thereof.

[A-20]
The pharmaceutical composition according to [A-19], wherein the compound represented by the formula (1) comprises an atropisomer represented by the formula (1A) in a larger amount than each atropisomer: 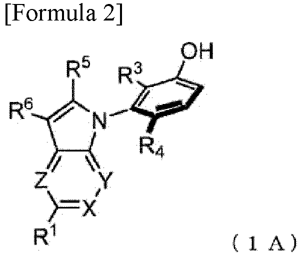 wherein X, Y, Z, R¹, R³, R⁴, R⁵, and R⁶ are as defined in the above [A-19].

[A-21]
The pharmaceutical composition according to any one of [A-1] to [A-20], wherein the MYT1 inhibitor is a compound represented by the formula (2): 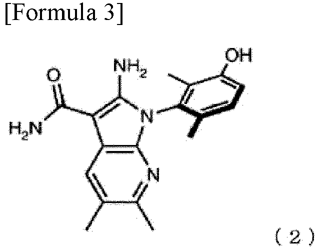 or a salt thereof, or a solvate thereof.

[A-21.5]
The pharmaceutical composition according to any one of [A-1] to [A-18], wherein the MYT1 inhibitor is a compound represented by the formula (3): 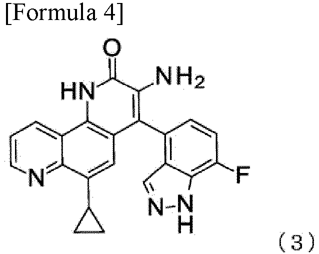 or a salt thereof, or a solvate thereof.

[A-22]
The pharmaceutical composition according to any one of [A-1] to [A-21.5], wherein the chemotherapeutic agent is at least one selected from the group consisting of an antimetabolite, an anticancer antibiotic, a mitosis inhibitor, a topoisomerase inhibitor, a platinating agent, an alkylating agent, and an antibody-drug conjugate.

[A-23]
The pharmaceutical composition according to [A-22], wherein the chemotherapeutic agent is an antimetabolite.

[A-24]
The pharmaceutical composition according to [A-22] or [A-23], wherein the antimetabolite is at least one selected from the group consisting of a purine antimetabolite, a pyrimidine antimetabolite, a folic acid antimetabolite, and a ribonucleotide reductase inhibitor.

[A-25]
The pharmaceutical composition according to [A-22], wherein the antimetabolite is a purine antimetabolite.

[A-26]
The pharmaceutical composition according to [A-24] or [A-25], wherein the purine antimetabolite is at least one selected from the group consisting of 6-thioguanine, 6-mercaptopurine, azathioprine, fludarabine, pentostatin, cladribine, clofarabine, and nelarabine.

[A-27]
The pharmaceutical composition according to [A-22], wherein the antimetabolite is a pyrimidine antimetabolite.

[A-28]
The pharmaceutical composition according to [A-24] or [A-27], wherein the pyrimidine antimetabolite is at least one selected from the group consisting of gemcitabine, cytarabine, fluorouracil, capecitabine, tegafur, azacitidine, trifluridine, and floxuridine.

[A-29]
The pharmaceutical composition according to [A-28], wherein the pyrimidine antimetabolite is gemcitabine.

[A-30] The pharmaceutical composition according to [A-22], wherein the antimetabolite is a folic acid antimetabolite.

[A-31]
The pharmaceutical composition according to [A-24] or [A-30], wherein the folic acid antimetabolite is at least one selected from the group consisting of pemetrexed and methotrexate.

[A-32]
The pharmaceutical composition according to [A-31], wherein the folic acid antimetabolite is pemetrexed.

[A-33]
The pharmaceutical composition according to [A-22], wherein the antimetabolite is a ribonucleotide reductase inhibitor.

[A-34]
The pharmaceutical composition according to [A-24] or [A-33], wherein the ribonucleotide reductase inhibitor is hydroxyurea.

[A-35]
The pharmaceutical composition according to [A-22], wherein the chemotherapeutic agent is an anticancer antibiotic.

[A-36]
The pharmaceutical composition according to [A-22] or [A-35], wherein the anticancer antibiotic is at least one selected from the group consisting of bleomycin, actinomycin D, doxorubicin, daunorubicin, idarubicin, mitomycin, mitoxantrone, epirubicin, aclarubicin, and valrubicin.

[A-37]
The pharmaceutical composition according to [A-32], wherein the chemotherapeutic agent is a mitosis inhibitor.

[A-38] The pharmaceutical composition according to [A-22] or [A-37], wherein the mitosis inhibitor is at least one selected from the group consisting of vinca alkaloid and a microtubule inhibitor.

[A-39]
The pharmaceutical composition according to [A-38], wherein the mitosis inhibitor is vinca alkaloid.

[A-40]
The pharmaceutical composition according to [A-38] or [A-39], wherein the vinca alkaloid is at least one selected from the group consisting of vincristine, vinblastine, and vinorelbine.

[A-41]
The pharmaceutical composition according to [A-38], wherein the mitosis inhibitor is a microtubule inhibitor.

[A-42]
The pharmaceutical composition according to [A-38] or [A-41], wherein the microtubule inhibitor is at least one selected from the group consisting of docetaxel, paclitaxel, eribulin, ixabepilone, and epothilone.

[A-43]
The pharmaceutical composition according to [A-22], wherein the chemotherapeutic agent is a topoisomerase inhibitor.

[A-44]
The pharmaceutical composition according to [A-22] or [A-43], wherein the topoisomerase inhibitor is at least one selected from the group consisting of topotecan, irinotecan, DXd, etoposide, and teniposide.

[A-45]
The pharmaceutical composition according to [A-44], wherein the topoisomerase inhibitor is at least one selected from the group consisting of irinotecan and DXd.

[A-46]
The pharmaceutical composition according to [A-22], wherein the chemotherapeutic agent is a platinating agent.

[A-47]
The pharmaceutical composition according to [A-22] or [A-46], wherein the platinating agent is at least one selected from the group consisting of cisplatin, carboplatin, and oxaliplatin.

[A-48]
The pharmaceutical composition according to [A-47], wherein the platinating agent is carboplatin.

[A-49]
The pharmaceutical composition according to [A-22], wherein the chemotherapeutic agent is an alkylating agent.

[A-50]
The pharmaceutical composition according to [A-22] or [A-49], wherein the alkylating agent is at least one selected from the group consisting of cyclophosphamide, ifosfamide, chlorambucil, melphalan, temozolomide, carmustine, lomustine, streptozocin, busulfan, procarbazine, dacarbazine, nimustine, ranimustine, bendamustine, altretamine, thiotepa, and mechlorethamine.

[A-51]
The pharmaceutical composition according to [A-22], wherein the chemotherapeutic agent is an antibody-drug conjugate.

[A-52]
The pharmaceutical composition according to [A-22] or [A-51], wherein the antibody-drug conjugate is at least one selected from the group consisting of trastuzumab deruxtecan, sacituzumab govitecan, tisotumab vedotin, enfortumab vedotin, trastuzumab emtansine, loncastuximab tesirine, moxetumomab pasudotox, belantamab mafodotin, polatuzumab vedotin, inotuzumab ozogamicin, brentuximab vedotin, and gemtuzumab ozogamicin.

[A-53] The pharmaceutical composition according to [A-52], wherein the antibody-drug conjugate is at least one selected from the group consisting of trastuzumab deruxtecan and sacituzumab govitecan.

[A-54]
The pharmaceutical composition according to any one of [A-1] to [A-8], wherein the MYT1 inhibitor is a small interfering RNA (siRNA), and the chemotherapeutic agent is at least one selected from the group consisting of gemcitabine, pemetrexed, irinotecan, carboplatin, and trastuzumab deruxtecan.

[A-55]
The pharmaceutical composition according to any one of [A-1] to [A-8], wherein the MYT1 inhibitor is a small interfering RNA (siRNA), and the chemotherapeutic agent is gemcitabine.

[A-56]
The pharmaceutical composition according to any one of [A-1] to [A-8], wherein the MYT1 inhibitor is a small interfering RNA (siRNA), and the chemotherapeutic agent is fluorouracil.

[A-57]
The pharmaceutical composition according to any one of [A-1] to [A-8], wherein the MYT1 inhibitor is a small interfering RNA (siRNA), and the chemotherapeutic agent is pemetrexed.

[A-58]
The pharmaceutical composition according to any one of [A-1] to [A-8], wherein the MYT1 inhibitor is a small interfering RNA (siRNA), and the chemotherapeutic agent is etoposide.

[A-59]
The pharmaceutical composition according to any one of [A-1] to [A-8], wherein the MYT1 inhibitor is a small interfering RNA (siRNA), and the chemotherapeutic agent is irinotecan.

[A-60]
The pharmaceutical composition according to any one of [A-1] to [A-8], wherein the MYT1 inhibitor is a small interfering RNA (siRNA), and the chemotherapeutic agent is carboplatin.

[A-61]
The pharmaceutical composition according to any one of [A-1] to [A-8], wherein the MYT1 inhibitor is a small interfering RNA (siRNA), and the chemotherapeutic agent is trastuzumab deruxtecan.

[A-61.1]
The pharmaceutical composition according to any one of [A-1] to [A-8], wherein the MYT1 inhibitor is a compound represented by the formula (2) or a salt thereof, or a solvate thereof, and the chemotherapeutic agent is at least one selected from the group consisting of pemetrexed, gemcitabine, carboplatin, irinotecan, sacituzumab govitecan, DXd, and trastuzumab deruxtecan.

[A-61.11]
The pharmaceutical composition according to any one of [A-1] to [A-8], wherein the MYT1 inhibitor is a compound represented by the formula (2) or a salt thereof, or a solvate thereof, and the chemotherapeutic agent is pemetrexed.

[A-61.12]
The pharmaceutical composition according to any one of [A-1] to [A-8], wherein the MYT1 inhibitor is a compound represented by the formula (2) or a salt thereof, or a solvate thereof, and the chemotherapeutic agent is gemcitabine.

[A-61.13]
The pharmaceutical composition according to any one of [A-1] to [A-8], wherein the MYT1 inhibitor is a compound represented by the formula (2) or a salt thereof, or a solvate thereof, and the chemotherapeutic agent is carboplatin.

[A-61.14]
The pharmaceutical composition according to any one of [A-1] to [A-8], wherein the MYT1 inhibitor is a compound represented by the formula (2) or a salt thereof, or a solvate thereof, and the chemotherapeutic agent is irinotecan.

[A-61.15]
The pharmaceutical composition according to any one of [A-1] to [A-8], wherein the MYT1 inhibitor is a compound represented by the formula (2) or a salt thereof, or a solvate thereof, and the chemotherapeutic agent is sacituzumab govitecan.

[A-61.16]
The pharmaceutical composition according to any one of [A-1] to [A-8], wherein the MYT1 inhibitor is a compound represented by the formula (2) or a salt thereof, or a solvate thereof, and the chemotherapeutic agent is DXd.

[A-61.17]
The pharmaceutical composition according to any one of [A-1] to [A-8], wherein the MYT1 inhibitor is a compound represented by the formula (2) or a salt thereof, or a solvate thereof, and the chemotherapeutic agent is trastuzumab deruxtecan.

[A-61.2]
The pharmaceutical composition according to any one of [A-1] to [A-8], wherein the MYT1 inhibitor is a compound represented by the formula (3) or a salt thereof, or a solvate thereof, and the chemotherapeutic agent is at least one selected from the group consisting of pemetrexed, gemcitabine, carboplatin, irinotecan, sacituzumab govitecan, DXd, and trastuzumab deruxtecan.

[A-61.21]
The pharmaceutical composition according to any one of [A-1] to [A-8], wherein the MYT1 inhibitor is a compound represented by the formula (3) or a salt thereof, or a solvate thereof, and the chemotherapeutic agent is pemetrexed.

[A-61.22]
The pharmaceutical composition according to any one of [A-1] to [A-8], wherein the MYT1 inhibitor is a compound represented by the formula (3) or a salt thereof, or a solvate thereof, and the chemotherapeutic agent is gemcitabine.

[A-61.23]
The pharmaceutical composition according to any one of [A-1] to [A-8], wherein the MYT1 inhibitor is a compound represented by the formula (3) or a salt thereof, or a solvate thereof, and the chemotherapeutic agent is carboplatin.

[A-61.24]
The pharmaceutical composition according to any one of [A-1] to [A-8], wherein the MYT1 inhibitor is a compound represented by the formula (3) or a salt thereof, or a solvate thereof, and the chemotherapeutic agent is irinotecan.

[A-61.25]
The pharmaceutical composition according to any one of [A-1] to [A-8], wherein the MYT1 inhibitor is a compound represented by the formula (3) or a salt thereof, or a solvate thereof, and the chemotherapeutic agent is sacituzumab govitecan.

[A-61.26]
The pharmaceutical composition according to any one of [A-1] to [A-8], wherein the MYT1 inhibitor is a compound represented by the formula (3) or a salt thereof, or a solvate thereof, and the chemotherapeutic agent is DXd.

[A-61.27]
The pharmaceutical composition according to any one of [A-1] to [A-8], wherein the MYT1 inhibitor is a compound represented by the formula (3) or a salt thereof, or a solvate thereof, and the chemotherapeutic agent is trastuzumab deruxtecan.

[A-62]
The pharmaceutical composition according to any one of [A-1] to [A-61], wherein the MYT1 inhibitor and the chemotherapeutic agent are simultaneously or separately administered.

[A-63]
The pharmaceutical composition according to any one of [A-1] to [A-61], wherein the MYT1 inhibitor and the chemotherapeutic agent are administered as a combination drug.

[A-64]
The pharmaceutical composition according to any one of [A-1] to [A-63], wherein the cancer is solid cancer or blood cancer.

[A-65]
The pharmaceutical composition according to any one of [A-1] to [A-63], wherein the cancer is at least one selected from the group consisting of lung cancer, breast cancer, esophageal cancer, stomach cancer, bowel cancer, uterine cancer, ovarian cancer, pancreatic cancer, bladder cancer, thyroid cancer, skin cancer, head and neck cancer, kidney cancer, liver cancer, prostate cancer, adenoid cystic cancer, retinoblastoma, cerebral tumor, leukemia, malignant lymphoma, and multiple myeloma.

[A-66]
The pharmaceutical composition according to [A-65], wherein the cancer is at least one selected from the group consisting of lung cancer, breast cancer, bladder cancer, and ovarian cancer.

[A-67]
The pharmaceutical composition according to [A-65], wherein the above cancer is at least one selected from the group consisting of lung cancer, breast cancer, and bladder cancer.

[A-68]
The pharmaceutical composition according to any one of [A-1] to [A-67], wherein the RB1 gene mutation positivity, or the decrease in expression of an RB1 gene or protein is detected in a cancer patient-derived biological sample.

[A-68.1]
The method according to any one of [A-1] to [A-67], wherein the positive expression of hyperphosphorylated RB1 protein is detected in a cancer patient-derived biological sample.

[A-69]
The pharmaceutical composition according to [A-68], wherein the cancer patient-derived biological sample is cancer cells.

[A-70]
The pharmaceutical composition according to any one of [A-1] to [A-69], wherein the decrease in expression of an RB1 gene or protein is a decrease based on an expression level in a healthy individual-derived biological sample or a non-cancer tissue derived from the cancer patient.

[A-70.1]
The pharmaceutical composition according to any one of [A-1] to [A-69], wherein the positive expression of hyperphosphorylated RB1 protein is determined from an increase in expression of the hyperphosphorylated RB1 protein based on an expression level in a healthy individual-derived biological sample or a non-cancer tissue derived from the cancer patient.

[A-71]
The pharmaceutical composition according to any one of [A-1] to [A-70], wherein the patient is a patient in which amplification of a copy number of a CCNE1 gene is not detected.

[A-72]
The pharmaceutical composition according to any one of [A-1] to [A-71], wherein the above patient is not a mouse implanted with OVCAR3.

[A-73]
The pharmaceutical composition according to any one of [A-1] to [A-72], wherein the patient is human.

[B-1]
A method for treating or preventing cancer of a cancer patient in which RB1 gene mutation positivity, a decrease in expression of an RB1 gene or protein, or positive expression of hyperphosphorylated RB1 protein is detected,
the method comprising administering a combination of a chemotherapeutic agent and an MYT1 inhibitor to the cancer patient.

[B-1.1]
A method for treating or preventing cancer of a cancer patient in which RB1 gene mutation positivity or a decrease in expression of an RB1 gene or protein is detected,
the method comprising administering a combination of a chemotherapeutic agent and an MYT1 inhibitor to the cancer patient.

[B-1.2]
A method for treating or preventing cancer of a cancer patient in which positive expression of hyperphosphorylated RB1 protein is detected,
the method comprising administering a combination of a chemotherapeutic agent and an MYT1 inhibitor to the cancer patient.

[B-2]
The method according to [B-1], further comprising detecting or allowing a third person to detect the presence or absence of an RB1 gene mutation, the presence or absence of a decrease in expression of an RB1 gene or protein, or the presence or absence of the hyperphosphorylated RB1 protein, in a cancer patient-derived biological sample.

[B-2.1]
The method according to [B-1], further comprising detecting or allowing a third person to detect the presence or absence of RB1 gene mutation or the presence or absence of a decrease in expression of an RB1 gene or protein, in a cancer patient-derived biological sample.

[B-3]
The method according to [B-2], wherein the cancer patient-derived biological sample is cancer cells.

[B-2.2]
The method according to [B-1], further comprising detecting or allowing a third person to detect the presence or absence of the hyperphosphorylated RB1 protein in a cancer patient-derived biological sample.

[B-4]
The method according to any one of [B-1] to [B-3], wherein the RB1 gene mutation comprises a mutation causing insertion, substitution, deletion, and/or addition of at least one amino acid residue to wild-type RB1 protein.

[B-5]
The method according to any one of [B-1] to [B-4], wherein the RB1 gene mutation is a nonsense mutation, a frameshift mutation, a splice site mutation, or a homozygous or heterozygous deletion.

[B-6]
The method according to any one of [B-1] to [B-5], wherein the RB1 gene mutation is a mutation decreasing a function of RB1.

[B-7]
The method according to any one of [B-1] to [B-6], wherein the RB1 gene mutation is a human RB1 gene mutation.

[B-8]
The method according to [B-7], wherein the human RB1 gene mutation is at least one of the following (1) to (5):
(1) a codon corresponding to a serine residue (S) at position 82 of an amino acid sequence of SEQ ID No: 2 is replaced with a stop codon,
(2) a codon corresponding to an arginine residue (R) at position 467 of the amino acid sequence of SEQ ID No: 2 is replaced with a stop codon,
(3) in a codon corresponding to an amino acid residue at position 182, at least one base is inserted or deleted, a new reading frame starting from an isoleucine residue (I) is formed, and a third reading frame therefrom is a stop codon,
(4) a glutamic acid residue (E) at position 837 of the amino acid sequence of SEQ ID No: 2 is replaced with a lysine residue (K), and a part of the RB1 gene is homozygously deleted, and
(5) a glycine residue (G) at position 449 of the amino acid sequence of SEQ ID No: 2 is replaced with a glutamic acid residue (E), and a part of the RB1 gene is homozygously deleted.

[B-8]
The method according to any one of [B-1] to [B-7], wherein the decrease in expression of an RB1 gene or protein comprises a decrease in gene expression through methylation of an RB1 gene or a micro RNA.

[B-8.1]
The method according to any one of [B-1] to [B-7], wherein the hyperphosphorylated RB1 protein is RB1 protein having 3 or more phosphorylated amino acid residues in the amino acid sequence of the RB1 protein.

[B-8.2]
The method according to any one of [B-1] to [B-7], wherein the hyperphosphorylated RB1 protein is RB1 protein having 4 or more phosphorylated amino acid residues in the amino acid sequence of the RB1 protein.

[B-8.3]
The method according to any one of [B-1] to [B-7], wherein the hyperphosphorylated RB1 protein is RB1 protein having 8 or more phosphorylated amino acid residues in the amino acid sequence of the RB1 protein.

[B-8.4]
The method according to any one of [B-1] to [B-7], wherein the hyperphosphorylated RB1 protein is RB1 protein having 15 or more phosphorylated amino acid residues in the amino acid sequence of the RB1 protein.

[B-8.5]
The method according to any one of [B-1] to [B-7], wherein the hyperphosphorylated RB1 protein is hyperphosphorylated human RB1 protein.

[B-8.6]
The method according to [B-8.5], wherein the hyperphosphorylated human RB1 protein is RB1 protein in which at least one amino acid residue selected from the group consisting of a threonine residue (T) at position 826, a threonine residue (T) at position 823, a threonine residue (T) at position 821, a serine residue (S) at position 816, a tyrosine residue (Y) at position 813, a serine residue (S) at position 811, a serine residue (S) at position 807, a tyrosine residue (Y) at position 805, a serine residue (S) at position 780, a threonine residue (T) at position 625, a threonine residue (T) at position 601, a threonine residue (T) at position 373, a serine residue (S) at position 360, a threonine residue (T) at position 356, a serine residue (S) at position 249, and a serine residue (S) at position 37 of SEQ ID No: 2 is phosphorylated.

[B-8.7]
The method according to [B-8.5], wherein the hyperphosphorylated human RB1 protein is RB1 protein in which at least one amino acid residue selected from the group consisting of a threonine residue (T) at position 826, a threonine residue (T) at position 821, a serine residue (S) at position 811, a serine residue (S) at position 807, a serine residue (S) at position 780, a threonine residue (T) at position 373, and a threonine residue (T) at position 356 of SEQ ID No: 2 is phosphorylated.

[B-8.8]
The method according to [B-8.5], wherein the hyperphosphorylated human RB1 protein is RB1 protein in which at least one amino acid residue selected from the group consisting of a threonine residue (T) at position 826, a threonine residue (T) at position 821, a serine residue (S) at position 811, and a serine residue (S) at position 807 of SEQ ID No: 2 is phosphorylated.

[B-8.9]
The method according to [B-8.5], wherein the hyperphosphorylated human RB1 protein is RB1 protein in which at least two amino acid residues selected from the group consisting of a threonine residue (T) at position 826, a threonine residue (T) at position 821, and a serine residue (S) at position 811 and/or a serine residue (S) at position 807 of SEQ ID No: 2 are phosphorylated.

[B-8.10]
The method according to [B-8.5], wherein the hyperphosphorylated human RB1 protein is RB1 protein in which a threonine residue (T) at position 826 and a threonine residue (T) at position 821 of SEQ ID No: 2 are phosphorylated, and a serine residue (S) at position 811 and/or a serine residue (S) at position 807 are/is phosphorylated.

[B-9]
The method according to any one of [B-1] to [B-8], wherein the MYT1 inhibitor is at least one selected from the group consisting of a low molecular compound, a polypeptide, and a polynucleotide.

[B-10]
The method according to [B-9], wherein the MYT1 inhibitor is a low molecular compound.

[B-11]
The method according to [B-10], wherein the low molecular compound is a compound having a molecular weight of 2000 g/mol or less.

[B-12]
The method according to [B-10], wherein the low molecular compound is a compound having a molecular weight of 1000 g/mol or less.

[B-13]
The method according to [B-9], wherein the MYT1 inhibitor is a polypeptide.

[B-14]
The method according to [B-13], wherein the polypeptide comprises an antibody.

[B-15]
The method according to [B-13], wherein the polypeptide is an anti-MYT1 antibody.

[B-16]
The method according to [B-9], wherein the MYT1 inhibitor is a polynucleotide.

[B-17]
The method according to [B-16], wherein the polynucleotide is at least one selected from the group consisting of a ribozyme, an antisense molecule, an inhibitor oligonucleotide, an aptamer, a micro RNA, and a small interfering RNA (siRNA).

[B-18]
The method according to [B-16], wherein the polynucleotide is at least one selected from the group consisting of an antisense nucleic acid, a micro RNA, and a small interfering RNA (siRNA).

[B-19]
The method according to any one of [B-1] to [B-12], wherein the MYT1 inhibitor is a compound represented by the formula (1): 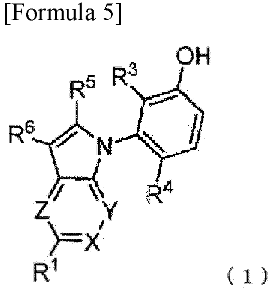
wherein X, Y, Z, R¹, R³, R⁴, R⁵, and R⁶ are as defined in the above [A-19],
or a salt thereof, or a solvate thereof.

[B-20]
The method according to [B-19], wherein the compound represented by the formula (1) comprises an atropisomer represented by the formula (1A) in a larger amount than each atropisomer: 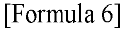 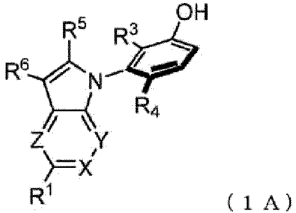 wherein X, Y, Z, R¹, R³, R⁴, R⁵, and R⁶ are as defined in the above [A-19].

[B-21]
The method according to any one of [B-1] to [B-20], wherein the MYT1 inhibitor is a compound represented by the formula (2): 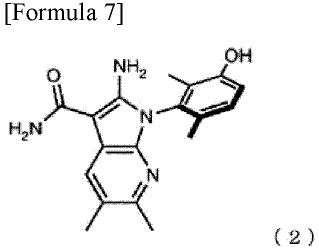 or a salt thereof, or a solvate thereof.

[B-21.5]
The method according to any one of [B-1] to [B-18], wherein the MYT1 inhibitor is a compound represented by the formula (3): 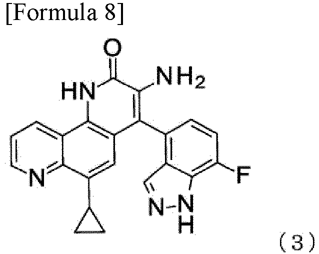 or a salt thereof, or a solvate thereof.

[B-22]
The method according to any one of [B-1] to [B-21.5], wherein the chemotherapeutic agent is at least one selected from the group consisting of an antimetabolite, an anticancer antibiotic, a mitosis inhibitor, a topoisomerase inhibitor, a platinating agent, an alkylating agent, and an antibody-drug conjugate.

[B-23]
The method according to [B-22], wherein the chemotherapeutic agent is an antimetabolite.

[B-24]
The method according to [B-22] or [B-23], wherein the antimetabolite is at least one selected from the group consisting of a purine antimetabolite, a pyrimidine antimetabolite, a folic acid antimetabolite, and a ribonucleotide reductase inhibitor.

[B-25]
The method according to [B-22], wherein the antimetabolite is a purine antimetabolite.

[B-26]
The method according to [B-24] or [B-25], wherein the purine antimetabolite is at least one selected from the group consisting of 6-thioguanine, 6-mercaptopurine, azathioprine, fludarabine, pentostatin, cladribine, clofarabine, and nelarabine.

[B-27]
The method according to [B-22], wherein the antimetabolite is a pyrimidine antimetabolite.

[B-28]
The method according to [B-24] or [B-27], wherein the pyrimidine antimetabolite is at least one selected from the group consisting of gemcitabine, cytarabine, fluorouracil, capecitabine, tegafur, azacitidine, trifluridine, and floxuridine.

[B-29]
The method according to [B-28], wherein the pyrimidine antimetabolite is gemcitabine.

[B-30]
The method according to [B-22], wherein the antimetabolite is a folic acid antimetabolite.

[B-31]
The method according to [B-24] or [B-30], wherein the folic acid antimetabolite is at least one selected from the group consisting of pemetrexed and methotrexate.

[B-32]
The method according to [B-31], wherein the folic acid antimetabolite is pemetrexed.

[B-33]
The method according to [B-22], wherein the antimetabolite is a ribonucleotide reductase inhibitor.

[B-34]
The method according to [B-24] or [B-33], wherein the ribonucleotide reductase inhibitor is hydroxyurea.

[B-35]
The method according to [B-22], wherein the chemotherapeutic agent is an anticancer antibiotic.

[B-36]
The method according to [B-22] or [B-35], wherein the anticancer antibiotic is at least one selected from the group consisting of bleomycin, actinomycin D, doxorubicin, daunorubicin, idarubicin, mitomycin, mitoxantrone, epirubicin, aclarubicin, and valrubicin.

[B-37]
The method according to [B-22], wherein the chemotherapeutic agent is a mitosis inhibitor.

[B-38]
The method according to [B-22] or [B-37], wherein the mitosis inhibitor is at least one selected from the group consisting of vinca alkaloid and a microtubule inhibitor.

[B-39]
The method according to [B-38], wherein the mitosis inhibitor is vinca alkaloid.

[B-40]
The method according to [B-22] or [B-39], wherein the vinca alkaloid is at least one selected from the group consisting of vincristine, vinblastine, and vinorelbine.

[B-41]
The method according to [B-38], wherein the mitosis inhibitor is a microtubule inhibitor.

[B-42]
The method according to [B-22] or [B-41], wherein the microtubule inhibitor is at least one selected from the group consisting of docetaxel, paclitaxel, eribulin, ixabepilone, and epothilone.

[B-43]
The method according to [B-22], wherein the chemotherapeutic agent is a topoisomerase inhibitor.

[B-44]
The method according to [B-22] or [B-43], wherein the topoisomerase inhibitor is at least one selected from the group consisting of topotecan, irinotecan, DXd, etoposide, and teniposide.

[B-45]
The method according to [B-44], wherein the topoisomerase inhibitor is at least one selected from the group consisting of irinotecan and DXd.

[B-46]
The method according to [B-22], wherein the chemotherapeutic agent is a platinating agent.

[B-47]
The method according to [B-22] or [B-46], wherein the platinating agent is at least one selected from the group consisting of cisplatin, carboplatin, and oxaliplatin.

[B-48]
The method according to [B-47], wherein the platinating agent is carboplatin.

[B-49]
The method according to [B-22], wherein the chemotherapeutic agent is an alkylating agent.

[B-50]
The method according to [B-22] or [B-49], wherein the alkylating agent is at least one selected from the group consisting of cyclophosphamide, ifosfamide, chlorambucil, melphalan, temozolomide, carmustine, lomustine, streptozocin, busulfan, procarbazine, dacarbazine, nimustine, ranimustine, bendamustine, altretamine, thiotepa, and mechlorethamine.

[B-51]
The method according to [B-22], wherein the chemotherapeutic agent is an antibody-drug conjugate.

[B-52]
The method according to [B-22] or [B-51], wherein the antibody-drug conjugate is at least one selected from the group consisting of trastuzumab deruxtecan, sacituzumab govitecan, tisotumab vedotin, enfortumab vedotin, trastuzumab emtansine, loncastuximab tesirine, moxetumomab pasudotox, belantamab mafodotin, polatuzumab vedotin, inotuzumab ozogamicin, brentuximab vedotin, and gemtuzumab ozogamicin.

[B-53]
The method according to [B-52], wherein the antibody-drug conjugate is at least one selected from the group consisting of trastuzumab deruxtecan and sacituzumab govitecan.

[B-54]
The method according to any one of [B-1] to [B-8], wherein the MYT1 inhibitor is a small interfering RNA (siRNA), and the chemotherapeutic agent is at least one selected from the group consisting of gemcitabine, pemetrexed, irinotecan, carboplatin, and trastuzumab deruxtecan.

[B-55]
The method according to any one of [B-1] to [B-8], wherein the MYT1 inhibitor is a small interfering RNA (siRNA), and the chemotherapeutic agent is gemcitabine.

[B-56]
The method according to any one of [B-1] to [B-8], wherein the MYT1 inhibitor is a small interfering RNA (siRNA), and the chemotherapeutic agent is fluorouracil.

[B-57]
The method according to any one of [B-1] to [B-8], wherein the MYT1 inhibitor is a small interfering RNA (siRNA), and the chemotherapeutic agent is pemetrexed.

[B-58]
The method according to any one of [B-1] to [B-8], wherein the MYT1 inhibitor is a small interfering RNA (siRNA), and the chemotherapeutic agent is etoposide.

[B-59]
The method according to any one of [B-1] to [B-8], wherein the MYT1 inhibitor is a small interfering RNA (siRNA), and the chemotherapeutic agent is irinotecan.

[B-60]
The method according to any one of [B-1] to [B-8], wherein the MYT1 inhibitor is a small interfering RNA (siRNA), and the chemotherapeutic agent is carboplatin.

[B-61]
The method according to any one of [B-1] to [B-8], wherein the MYT1 inhibitor is a small interfering RNA (siRNA), and the chemotherapeutic agent is trastuzumab deruxtecan.

[B-61.1]
The method according to any one of [B-1] to [B-8], wherein the MYT1 inhibitor is a compound represented by the formula (2) or a salt thereof, or a solvate thereof, and the chemotherapeutic agent is at least one selected from the group consisting of pemetrexed, gemcitabine, carboplatin, irinotecan, sacituzumab govitecan, DXd, and trastuzumab deruxtecan.

[B-61.11]
The method according to any one of [B-1] to [B-8], wherein the MYT1 inhibitor is a compound represented by the formula (2) or a salt thereof, or a solvate thereof, and the chemotherapeutic agent is pemetrexed.

[B-61.12]
The method according to any one of [B-1] to [B-8], wherein the MYT1 inhibitor is a compound represented by the formula (2) or a salt thereof, or a solvate thereof, and the chemotherapeutic agent is gemcitabine.

[B-61.13]
The method according to any one of [B-1] to [B-8], wherein the MYT1 inhibitor is a compound represented by the formula (2) or a salt thereof, or a solvate thereof, and the chemotherapeutic agent is carboplatin.

[B-61.14]
The method according to any one of [B-1] to [B-8], wherein the MYT1 inhibitor is a compound represented by the formula (2) or a salt thereof, or a solvate thereof, and the chemotherapeutic agent is irinotecan.

[B-61.15]
The method according to any one of [B-1] to [B-8], wherein the MYT1 inhibitor is a compound represented by the formula (2) or a salt thereof, or a solvate thereof, and the chemotherapeutic agent is sacituzumab govitecan.

[B-61.16]
The method according to any one of [B-1] to [B-8], wherein the MYT1 inhibitor is a compound represented by the formula (2) or a salt thereof, or a solvate thereof, and the chemotherapeutic agent is DXd.

[B-61.17]
The method according to any one of [B-1] to [B-8], wherein the MYT1 inhibitor is a compound represented by the formula (2) or a salt thereof, or a solvate thereof, and the chemotherapeutic agent is trastuzumab deruxtecan.

[B-61.2]
The method according to any one of [B-1] to [B-8], wherein the MYT1 inhibitor is a compound represented by the formula (3) or a salt thereof, or a solvate thereof, and the chemotherapeutic agent is at least one selected from the group consisting of pemetrexed, gemcitabine, carboplatin, irinotecan, sacituzumab govitecan, DXd, and trastuzumab deruxtecan.

[B-61.21]
The method according to any one of [B-1] to [B-8], wherein the MYT1 inhibitor is a compound represented by the formula (3) or a salt thereof, or a solvate thereof, and the chemotherapeutic agent is pemetrexed.

[B-61.22]
The method according to any one of [B-1] to [B-8], wherein the MYT1 inhibitor is a compound represented by the formula (3) or a salt thereof, or a solvate thereof, and the chemotherapeutic agent is gemcitabine.

[B-61.23]
The method according to any one of [B-1] to [B-8], wherein the MYT1 inhibitor is a compound represented by the formula (3) or a salt thereof, or a solvate thereof, and the chemotherapeutic agent is carboplatin.

[B-61.24]
The method according to any one of [B-1] to [B-8], wherein the MYT1 inhibitor is a compound represented by the formula (3) or a salt thereof, or a solvate thereof, and the chemotherapeutic agent is irinotecan.

[B-61.25]
The method according to any one of [B-1] to [B-8], wherein the MYT1 inhibitor is a compound represented by the formula (3) or a salt thereof, or a solvate thereof, and the chemotherapeutic agent is sacituzumab govitecan.

[B-61.26]
The method according to any one of [B-1] to [B-8], wherein the MYT1 inhibitor is a compound represented by the formula (3) or a salt thereof, or a solvate thereof, and the chemotherapeutic agent is DXd.

[B-61.27]
The method according to any one of [B-1] to [B-8], wherein the MYT1 inhibitor is a compound represented by the formula (3) or a salt thereof, or a solvate thereof, and the chemotherapeutic agent is trastuzumab deruxtecan.

[B-62]
The method according to any one of [B-1] to [B-61], wherein the MYT1 inhibitor and the chemotherapeutic agent are simultaneously or separately administered.

[B-63]
The method according to any one of [B-1] to [B-61], wherein the MYT1 inhibitor and the chemotherapeutic agent are administered as a combination drug.

[B-64]
The method according to any one of [B-1] to [B-63], wherein the cancer is solid cancer or blood cancer.

[B-65]
The method according to any one of [B-1] to [B-63], wherein the cancer is at least one selected from the group consisting of lung cancer, breast cancer, esophageal cancer, stomach cancer, bowel cancer, uterine cancer, ovarian cancer, pancreatic cancer, bladder cancer, thyroid cancer, skin cancer, head and neck cancer, kidney cancer, liver cancer, prostate cancer, adenoid cystic cancer, retinoblastoma, cerebral tumor, leukemia, malignant lymphoma, and multiple myeloma.

[B-66]
The method according to [B-65], wherein the cancer is at least one selected from the group consisting of lung cancer, breast cancer, bladder cancer, and ovarian cancer.

[B-67]
The method according to [B-66], wherein the cancer is lung cancer, breast cancer, or bladder cancer.

[B-68]
The method according to any one of [B-1] to [B-67], wherein the decrease in expression of an RB1 gene or protein is a decrease based on an expression level in a healthy individual-derived biological sample or a non-cancer tissue derived from the cancer patient.

[B-68.1]
The method according to any one of [B-1] to [B-67], wherein the positive expression of hyperphosphorylated RB1 protein is increased based on an expression level in a healthy individual-derived biological sample or a non-cancer tissue derived from the cancer patient.

[B-69]
The method according to any one of [B-1] to [B-68], wherein the patient is a patient in which amplification of a copy number of a CCNE1 gene is not detected.

[B-70]
The method according to any one of [B-1] to [B-69], wherein the above patient is not a mouse implanted with OVCAR3.

[B-71]
The method according to any one of [B-1] to [B-70], wherein the patient is human.

[C-1]
A method for suppressing growth of cancer cells in a cancer patient in which RB1 gene mutation positivity, a decrease in expression of an RB1 gene or protein, or positive expression of hyperphosphorylated RB1 protein is detected, the method comprising bringing an MYT1 inhibitor and a chemotherapeutic agent into contact with the cancer cells.

[C-1.1]
A method for suppressing growth of cancer cells in a cancer patient in which RB1 gene mutation positivity or a decrease in expression of an RB1 gene or protein is detected, the method comprising bringing an MYT1 inhibitor and a chemotherapeutic agent into contact with the cancer cells.

[C-1.2]
A method for suppressing growth of cancer cells in a cancer patient in which positive expression of hyperphosphorylated RB1 protein is detected, the method comprising bringing an MYT1 inhibitor and a chemotherapeutic agent into contact with the cancer cells.

[C-2]
The method according to [C-1], wherein the RB1 gene mutation is a mutation causing insertion, substitution, deletion, and/or addition of at least one amino acid residue to wild-type RB1 protein.

[C-3]

The method according to [C-1] or [C-2], wherein the RB1 gene mutation is a nonsense mutation, a frameshift mutation, a splice site mutation, or a homozygous or heterozygous deletion.

[C-4]
The method according to any one of [C-1] to [C-3], wherein the RB1 gene mutation is a mutation decreasing a function of RB1.

[C-5]
The method according to any one of [C-1] to [C-4], wherein the RB1 gene mutation is a human RB1 gene mutation.

[C-6]
The method according to [C-5], wherein the human RB1 gene mutation is at least one of the following (1) to (5):
(1) a codon corresponding to a serine residue (S) at position 82 of an amino acid sequence of SEQ ID No: 2 is replaced with a stop codon,
(2) a codon corresponding to an arginine residue (R) at position 467 of the amino acid sequence of SEQ ID No: 2 is replaced with a stop codon,
(3) in a codon corresponding to an amino acid residue at position 182, at least one base is inserted or deleted, a new reading frame starting from an isoleucine residue (I) is formed, and a third reading frame therefrom is a stop codon,
(4) a glutamic acid residue (E) at position 837 of the amino acid sequence of SEQ ID No: 2 is replaced with a lysine residue (K), and a part of the RB1 gene is homozygously deleted, and
(5) a glycine residue (G) at position 449 of the amino acid sequence of SEQ ID No: 2 is replaced with a glutamic acid residue (E), and a part of the RB1 gene is homozygously deleted.

[C-7]
The method according to any one of [C-1] to [C-6], wherein the decrease in expression of an RB1 gene or protein comprises a decrease in gene expression through methylation of an RB1 gene or a micro RNA.

[C-7.1]
The method according to any one of [C-1] to [C-6], wherein the hyperphosphorylated RB1 protein is RB1 protein having 3 or more phosphorylated amino acid residues in the amino acid sequence of the RB1 protein.

[C-7.2]
The method according to any one of [C-1] to [C-6], wherein the hyperphosphorylated RB1 protein is RB1 protein having 4 or more phosphorylated amino acid residues in the amino acid sequence of the RB1 protein.

[C-7.3]
The method according to any one of [C-1] to [C-6], wherein the hyperphosphorylated RB1 protein is RB1 protein having 8 or more phosphorylated amino acid residues in the amino acid sequence of the RB1 protein.

[C-7.4]
The method according to any one of [C-1] to [C-6], wherein the hyperphosphorylated RB1 protein is RB1 protein having 15 or more phosphorylated amino acid residues in the amino acid sequence of the RB1 protein.

[C-7.5]
The method according to any one of [C-1] to [C-6], wherein the hyperphosphorylated RB1 protein is hyperphosphorylated human RB1 protein.

[C-7.6]
The method according to [C-7.5], wherein the hyperphosphorylated human RB1 protein is RB1 protein in which at least one amino acid residue selected from the group consisting of a threonine residue (T) at position 826, a threonine residue (T) at position 823, a threonine residue (T) at position 821, a serine residue (S) at position 816, a tyrosine residue (Y) at position 813, a serine residue (S) at position 811, a serine residue (S) at position 807, a tyrosine residue (Y) at position 805, a serine residue (S) at position 780, a threonine residue (T) at position 625, a threonine residue (T) at position 601, a threonine residue (T) at position 373, a serine residue (S) at position 360, a threonine residue (T) at position 356, a serine residue (S) at position 249, and a serine residue (S) at position 37 of SEQ ID No: 2 is phosphorylated.

[C-7.7]
The method according to [C-7.5], wherein the hyperphosphorylated human RB1 protein is RB1 protein in which at least one amino acid residue selected from the group consisting of a threonine residue (T) at position 826, a threonine residue (T) at position 821, a serine residue (S) at position 811, a serine residue (S) at position 807, a serine residue (S) at position 780, a threonine residue (T) at position 373, and a threonine residue (T) at position 356 of SEQ ID No: 2 is phosphorylated.

[C-7.8]
The method according to [C-7.5], wherein the hyperphosphorylated human RB1 protein is RB1 protein in which at least one amino acid residue selected from the group consisting of a threonine residue (T) at position 826, a threonine residue (T) at position 821, a serine residue (S) at position 811, and a serine residue (S) at position 807 of SEQ ID No: 2 is phosphorylated.

[C-7.9]
The method according to [C-7.5], wherein the hyperphosphorylated human RB1 protein is RB1 protein in which at least two amino acid residues selected from the group consisting of a threonine residue (T) at position 826, a threonine residue (T) at position 821, and a serine residue (S) at position 811 and/or a serine residue (S) at position 807 of SEQ ID No: 2 are phosphorylated.

[C-7.10]
The method according to [C-7.5], wherein the hyperphosphorylated human RB1 protein is RB1 protein in which a threonine residue (T) at position 826 and a threonine residue (T) at position 821 of SEQ ID No: 2 are phosphorylated, and a serine residue (S) at position 811 and/or a serine residue (S) at position 807 are/is phosphorylated.

[C-8]
The method according to any one of [C-1] to [C-7], wherein the MYT1 inhibitor is at least one selected from the group consisting of a low molecular compound, a polypeptide, and a polynucleotide.

[C-9]
The method according to [C-8], wherein the MYT1 inhibitor is a low molecular compound.

[C-10]
The method according to [C-9], wherein the low molecular compound is a compound having a molecular weight of 2000 g/mol or less.

[C-11]
The method according to [C-9], wherein the low molecular compound is a compound having a molecular weight of 1000 g/mol or less.

[C-12]
The method according to [C-8], wherein the MYT1 inhibitor is a polypeptide.

[C-13]
The method according to [C-12], wherein the polypeptide comprises an antibody.

[C-14]
The method according to [C-12], wherein the polypeptide is an anti-MYT1 antibody.

[C-15]
The method according to [C-8], wherein the MYT1 inhibitor is a polynucleotide.

[C-16]
The method according to [C-15], wherein the polynucleotide is at least one selected from the group consisting of a ribozyme, an antisense molecule, an inhibitor oligonucleotide, an aptamer, a micro RNA, and a small interfering RNA (siRNA).

[C-17]
The method according to [C-15], wherein the polynucleotide is at least one selected from the group consisting of an antisense nucleic acid, a micro RNA, and a small interfering RNA (siRNA).

[C-18]
The method according to any one of [C-1] to [C-11], wherein the MYT1 inhibitor is a compound represented by the formula (1): 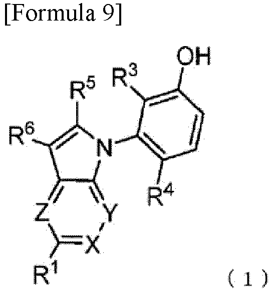
wherein X, Y, Z, R¹, R³, R⁴, R⁵, and R⁶ are as defined in the above [A-19],
or a salt thereof, or a solvate thereof.

[C-19]
The method according to [C-18], wherein the compound represented by the formula (1) comprises an atropisomer represented by the formula (1A) in a larger amount than each atropisomer: 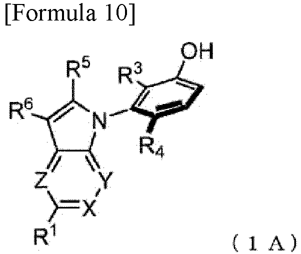 wherein X, Y, Z, R¹, R³, R⁴, R⁵, and R⁶ are as defined in the above [A-19].

[C-20]
The method according to any one of [C-1] to [C-19], wherein the MYT1 inhibitor is a compound represented by the formula (2): 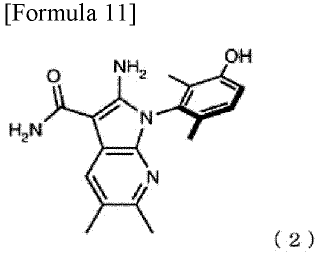 or a salt thereof, or a solvate thereof.

[C-20.5]
The method according to any one of [C-1] to [C-17], wherein the MYT1 inhibitor is a compound represented by the formula (3): 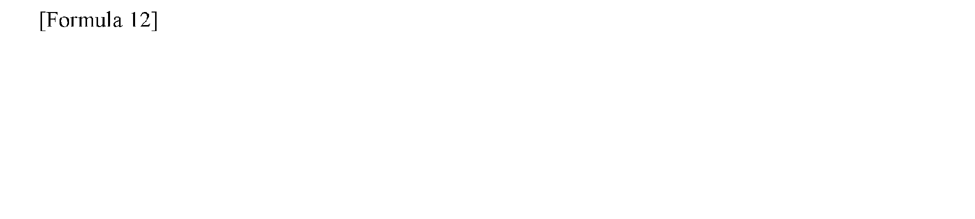 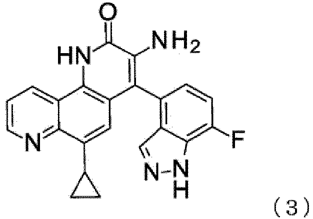 or a salt thereof, or a solvate thereof.

[C-21]
The method according to any one of [C-1] to [C-20.5], wherein the chemotherapeutic agent is at least one selected from the group consisting of an antimetabolite, an anticancer antibiotic, a mitosis inhibitor, a topoisomerase inhibitor, a platinating agent, an alkylating agent, and an antibody-drug conjugate.

[C-22]
The method according to [C-21], wherein the chemotherapeutic agent is an antimetabolite.

[C-23]
The method according to [C-21] or [C-22], wherein the antimetabolite is at least one selected from the group consisting of a purine antimetabolite, a pyrimidine antimetabolite, a folic acid antimetabolite, and a ribonucleotide reductase inhibitor.

[C-24]
The method according to [C-21], wherein the antimetabolite is a purine antimetabolite.

[C-25]
The method according to [C-23] or [C-24], wherein the purine antimetabolite is at least one selected from the group consisting of 6-thioguanine, 6-mercaptopurine, azathioprine, fludarabine, pentostatin, cladribine, clofarabine, and nelarabine.

[C-26]
The method according to [C-21], wherein the antimetabolite is a pyrimidine antimetabolite.

[C-27]
The method according to [C-23] or [C-26], wherein the pyrimidine antimetabolite is at least one selected from the group consisting of gemcitabine, cytarabine, fluorouracil, capecitabine, tegafur, azacitidine, trifluridine, and floxuridine.

[C-28]
The method according to [C-27], wherein the pyrimidine antimetabolite is gemcitabine.

[C-29]
The method according to [C-21], wherein the antimetabolite is a folic acid antimetabolite.

[C-30]
The method according to [C-23] or [C-29], wherein the folic acid antimetabolite is at least one selected from the group consisting of pemetrexed and methotrexate.

[C-31]
The method according to [C-30], wherein the folic acid antimetabolite is pemetrexed.

[C-32]
The method according to [C-21], wherein the antimetabolite is a ribonucleotide reductase inhibitor.

[C-33]
The method according to [C-23] or [C-32], wherein the ribonucleotide reductase inhibitor is hydroxyurea.

[C-34]
The method according to [C-21], wherein the chemotherapeutic agent is an anticancer antibiotic.

[C-35]
The method according to [C-21] or [C-34], wherein the anticancer antibiotic is at least one selected from the group consisting of bleomycin, actinomycin D, doxorubicin, daunorubicin, idarubicin, mitomycin, mitoxantrone, epirubicin, aclarubicin, and valrubicin.

[C-36]
The method according to [C-21], wherein the chemotherapeutic agent is a mitosis inhibitor.

[C-37]
The method according to [C-21] or [C-36], wherein the mitosis inhibitor is at least one selected from the group consisting of vinca alkaloid and a microtubule inhibitor.

[C-38]
The method according to [C-37], wherein the mitosis inhibitor is vinca alkaloid.

[C-39]
The method according to [C-37] or [C-38], wherein the vinca alkaloid is at least one selected from the group consisting of vincristine, vinblastine, and vinorelbine.

[C-40]
The method according to [C-37], wherein the mitosis inhibitor is a microtubule inhibitor.

[C-41]
The method according to [C-37] or [C-40], wherein the microtubule inhibitor is at least one selected from the group consisting of docetaxel, paclitaxel, eribulin, ixabepilone, and epothilone.

[C-42]
The method according to [C-21], wherein the chemotherapeutic agent is a topoisomerase inhibitor.

[C-43]
The method according to [C-21] or [C-42], wherein the topoisomerase inhibitor is at least one selected from the group consisting of topotecan, irinotecan, DXd, etoposide, and teniposide.

[C-44]
The method according to [C-43], wherein the topoisomerase inhibitor is at least one selected from the group consisting of irinotecan and DXd.

[C-45]
The method according to [C-21], wherein the chemotherapeutic agent is a platinating agent.

[C-46]
The method according to [C-21] or [C-45], wherein the platinating agent is at least one selected from the group consisting of cisplatin, carboplatin, and oxaliplatin.

[C-47]
The method according to [C-46], wherein the platinating agent is carboplatin.

[C-48]
The method according to [C-21], wherein the chemotherapeutic agent is an alkylating agent.

[C-49]
The method according to [C-21] or [C-48], wherein the alkylating agent is at least one selected from the group consisting of cyclophosphamide, ifosfamide, chlorambucil, melphalan, temozolomide, carmustine, lomustine, streptozocin, busulfan, procarbazine, dacarbazine, nimustine, ranimustine, bendamustine, altretamine, thiotepa, and mechlorethamine.

[C-50]
The method according to [C-21], wherein the chemotherapeutic agent is an antibody-drug conjugate.

[C-51]
The method according to [C-21] or [C-50], wherein the antibody-drug conjugate is at least one selected from the group consisting of trastuzumab deruxtecan, sacituzumab govitecan, tisotumab vedotin, enfortumab vedotin, trastuzumab emtansine, loncastuximab tesirine, moxetumomab pasudotox, belantamab mafodotin, polatuzumab vedotin, inotuzumab ozogamicin, brentuximab vedotin, and gemtuzumab ozogamicin.

[C-52]
The method according to [C-51], wherein the antibody-drug conjugate is at least one selected from the group consisting of trastuzumab deruxtecan and sacituzumab govitecan.

[C-53]
The method according to any one of [C-1] to [C-7], wherein the MYT1 inhibitor is a small interfering RNA (siRNA), and the chemotherapeutic agent is at least one selected from the group consisting of gemcitabine, pemetrexed, irinotecan, carboplatin, and trastuzumab deruxtecan.

[C-54]
The method according to any one of [C-1] to [C-7], wherein the MYT1 inhibitor is a small interfering RNA (siRNA), and the chemotherapeutic agent is gemcitabine.

[C-55]
The method according to any one of [C-1] to [C-7], wherein the MYT1 inhibitor is a small interfering RNA (siRNA), and the chemotherapeutic agent is fluorouracil.

[C-56]
The method according to any one of [C-1] to [C-7], wherein the MYT1 inhibitor is a small interfering RNA (siRNA), and the chemotherapeutic agent is pemetrexed.

[C-57]
The method according to any one of [C-1] to [C-7], wherein the MYT1 inhibitor is a small interfering RNA (siRNA), and the chemotherapeutic agent is etoposide.

[C-58]
The method according to any one of [C-1] to [C-7], wherein the MYT1 inhibitor is a small interfering RNA (siRNA), and the chemotherapeutic agent is irinotecan.

[C-59]
The method according to any one of [C-1] to [C-7], wherein the MYT1 inhibitor is a small interfering RNA (siRNA), and the chemotherapeutic agent is carboplatin.

[C-60]
The method according to any one of [C-1] to [C-7], wherein the MYT1 inhibitor is a small interfering RNA (siRNA), and the chemotherapeutic agent is trastuzumab deruxtecan.

[C-60.1]
The method according to any one of [C-1] to [C-8], wherein the MYT1 inhibitor is a compound represented by the formula (2) or a salt thereof, or a solvate thereof, and the chemotherapeutic agent is at least one selected from the group consisting of pemetrexed, gemcitabine, carboplatin, irinotecan, sacituzumab govitecan, DXd, and trastuzumab deruxtecan.

[C-60.11]
The method according to any one of [C-1] to [C-8], wherein the MYT1 inhibitor is a compound represented by the formula (2) or a salt thereof, or a solvate thereof, and the chemotherapeutic agent is pemetrexed.

[C-60.12]
The method according to any one of [C-1] to [C-8], wherein the MYT1 inhibitor is a compound represented by the formula (2) or a salt thereof, or a solvate thereof, and the chemotherapeutic agent is gemcitabine.

[C-60.13]
The method according to any one of [C-1] to [C-8], wherein the MYT1 inhibitor is a compound represented by the formula (2) or a salt thereof, or a solvate thereof, and the chemotherapeutic agent is carboplatin.

[C-60.14]
The method according to any one of [C-1] to [C-8], wherein the MYT1 inhibitor is a compound represented by the formula (2) or a salt thereof, or a solvate thereof, and the chemotherapeutic agent is irinotecan.

[C-60.15]
The method according to any one of [C-1] to [C-8], wherein the MYT1 inhibitor is a compound represented by the formula (2) or a salt thereof, or a solvate thereof, and the chemotherapeutic agent is sacituzumab govitecan.

[C-60.16]
The method according to any one of [C-1] to [C-8], wherein the MYT1 inhibitor is a compound represented by the formula (2) or a salt thereof, or a solvate thereof, and the chemotherapeutic agent is DXd.

[C-60.17]
The method according to any one of [C-1] to [C-8], wherein the MYT1 inhibitor is a compound represented by the formula (2) or a salt thereof, or a solvate thereof, and the chemotherapeutic agent is trastuzumab deruxtecan.

[C-60.2]
The method according to any one of [C-1] to [C-8], wherein the MYT1 inhibitor is a compound represented by the formula (3) or a salt thereof, or a solvate thereof, and the chemotherapeutic agent is at least one selected from the group consisting of pemetrexed, gemcitabine, carboplatin, irinotecan, sacituzumab govitecan, DXd, and trastuzumab deruxtecan.

[C-60.21]
The method according to any one of [C-1] to [C-8], wherein the MYT1 inhibitor is a compound represented by the formula (3) or a salt thereof, or a solvate thereof, and the chemotherapeutic agent is pemetrexed.

[C-60.22]
The method according to any one of [C-1] to [C-8], wherein the MYT1 inhibitor is a compound represented by the formula (3) or a salt thereof, or a solvate thereof, and the chemotherapeutic agent is gemcitabine.

[C-60.23]
The method according to any one of [C-1] to [C-8], wherein the MYT1 inhibitor is a compound represented by the formula (3) or a salt thereof, or a solvate thereof, and the chemotherapeutic agent is carboplatin.

[C-60.24]
The method according to any one of [C-1] to [C-8], wherein the MYT1 inhibitor is a compound represented by the formula (3) or a salt thereof, or a solvate thereof, and the chemotherapeutic agent is irinotecan.

[C-60.25]
The method according to any one of [C-1] to [C-8], wherein the MYT1 inhibitor is a compound represented by the formula (3) or a salt thereof, or a solvate thereof, and the chemotherapeutic agent is sacituzumab govitecan.

[C-60.26]
The method according to any one of [C-1] to [C-8], wherein the MYT1 inhibitor is a compound represented by the formula (3) or a salt thereof, or a solvate thereof, and the chemotherapeutic agent is DXd.

[C-60.27]
The method according to any one of [C-1] to [C-8], wherein the MYT1 inhibitor is a compound represented by the formula (3) or a salt thereof, or a solvate thereof, and the chemotherapeutic agent is trastuzumab deruxtecan.

[C-61]
The method according to any one of [C-1] to [C-60], wherein the MYT1 inhibitor and the chemotherapeutic agent are simultaneously or separately administered.

[C-62]
The method according to any one of [C-1] to [C-60], wherein the MYT1 inhibitor and the chemotherapeutic agent are administered as a combination drug.

[C-63]
The method according to any one of [C-1] to [C-62], wherein the cancer is solid cancer or blood cancer.

[C-64]
The method according to any one of [C-1] to [C-62], wherein the cancer is at least one selected from the group consisting of lung cancer, breast cancer, esophageal cancer, stomach cancer, bowel cancer, uterine cancer, ovarian cancer, pancreatic cancer, bladder cancer, thyroid cancer, skin cancer, head and neck cancer, kidney cancer, liver cancer, prostate cancer, adenoid cystic cancer, retinoblastoma, cerebral tumor, leukemia, malignant lymphoma, and multiple myeloma.

[C-65]
The method according to [C-64], wherein the cancer is at least one selected from the group consisting of lung cancer, breast cancer, bladder cancer, and ovarian cancer.

[C-66]
The method according to [C-64], wherein the cancer is lung cancer, breast cancer, or bladder cancer.

[C-67]
The method according to any one of [C-1] to [C-66], wherein the RB1 gene mutation positivity, or the decrease in expression of an RB1 gene or protein is detected in a cancer patient-derived biological sample.

[C-67.1]
The method according to any one of [C-1] to [C-66], wherein the positive expression of hyperphosphorylated RB1 protein is detected in a cancer patient-derived biological sample.

[C-68]
The method according to [C-67], wherein the cancer patient-derived biological sample is cancer cells.

[C-69]
The method according to any one of [C-1] to [C-68], wherein the decrease in expression of an RB1 gene or protein is a decrease based on an expression level in a healthy individual-derived biological sample or a non-cancer tissue derived from the cancer patient.

[C-69.1]
The method according to any one of [C-1] to [C-68], wherein the positive expression of hyperphosphorylated RB1 protein is determined from an increase in expression of the hyperphosphorylated RB1 protein based on an expression level in a healthy individual-derived biological sample or a non-cancer tissue derived from the cancer patient.

[C-70]
The method according to any one of [C-1] to [C-69], wherein the patient is a patient in which amplification of a copy number of a CCNE1 gene is not detected.

[C-71]
The method according to any one of [C-1] to [C-70], wherein the above patient is not a mouse implanted with OVCAR3.

[C-72]
The method according to any one of [C-1] to [C-71], wherein the patient is human.

[D-1]
A method for improving responsiveness to cancer treatment with a chemotherapeutic agent, wherein
the cancer is a cancer of a cancer patient in which RB1 gene mutation positivity, a decrease in expression of an RB1 gene or protein, or positive expression of hyperphosphorylated RB1 protein is detected, and
the method comprises administering an MYT1 inhibitor together with the chemotherapeutic agent to the cancer patient.

[D-1.1]
A method for improving responsiveness to cancer treatment with a chemotherapeutic agent, the method comprising:
administering an MYT1 inhibitor together with the chemotherapeutic agent to a cancer patient,
wherein the cancer is a cancer of the cancer patient in which RB1 gene mutation positivity or a decrease in expression of an RB1 gene or protein is detected.

[D-1.2]
A method for improving responsiveness to cancer treatment with a chemotherapeutic agent, the method comprising:
administering an MYT1 inhibitor together with the chemotherapeutic agent to a cancer patient,
wherein the cancer is a cancer of the cancer patient in which positive expression of hyperphosphorylated RB1 protein is detected.

[D-2]
The method according to [D-1], wherein the RB1 gene mutation comprises a mutation causing insertion, substitution, deletion, and/or addition of at least one amino acid residue to wild-type RB1 protein.

[D-3]
The method according to [D-1] or [D-2], wherein the RB1 gene mutation is a nonsense mutation, a frameshift mutation, a splice site mutation, or a homozygous or heterozygous deletion.

[D-4]
The method according to any one of [D-1] to [D-3], wherein the RB1 gene mutation is a mutation decreasing a function of RB1.

[D-5]
The method according to any one of [D-1] to [D-4], wherein the RB1 gene mutation is a human RB1 gene mutation.

[D-6]
The method according to [D-5], wherein the human RB1 gene mutation is at least one of the following (1) to (5):
(1) a codon corresponding to a serine residue (S) at position 82 of an amino acid sequence of SEQ ID No: 2 is replaced with a stop codon,
(2) a codon corresponding to an arginine residue (R) at position 467 of the amino acid sequence of SEQ ID No: 2 is replaced with a stop codon,
(3) in a codon corresponding to an amino acid residue at position 182, at least one base is inserted or deleted, a new reading frame starting from an isoleucine residue (I) is formed, and a third reading frame therefrom is a stop codon,
(4) a glutamic acid residue (E) at position 837 of the amino acid sequence of SEQ ID No: 2 is replaced with a lysine residue (K), and a part of the RB1 gene is homozygously deleted, and
(5) a glycine residue (G) at position 449 of the amino acid sequence of SEQ ID No: 2 is replaced with a glutamic acid residue (E), and a part of the RB1 gene is homozygously deleted.

[D-7]
The method according to any one of [D-1] to [D-6], wherein the decrease in expression of an RB1 gene or protein comprises a decrease in gene expression through methylation of an RB1 gene or a micro RNA.

[D-7.1]
The method according to any one of [D-1] to [D-6], wherein the hyperphosphorylated RB1 protein is RB1 protein having 3 or more phosphorylated amino acid residues in the amino acid sequence of the RB1 protein.

[D-7.2]
The method according to any one of [D-1] to [D-6], wherein the hyperphosphorylated RB1 protein is RB1 protein having 4 or more phosphorylated amino acid residues in the amino acid sequence of the RB1 protein.

[D-7.3]
The method according to any one of [D-1] to [D-6], wherein the hyperphosphorylated RB1 protein is RB1 protein having 8 or more phosphorylated amino acid residues in the amino acid sequence of the RB1 protein.

[D-7.4]
The method according to any one of [D-1] to [D-6], wherein the hyperphosphorylated RB1 protein is RB1 protein having 15 or more phosphorylated amino acid residues in the amino acid sequence of the RB1 protein.

[D-7.5]
The method according to any one of [D-1] to [D-6], wherein the hyperphosphorylated RB1 protein is hyperphosphorylated human RB1 protein.

[D-7.6]
The method according to [D-7.5], wherein the hyperphosphorylated human RB1 protein is RB1 protein in which at least one amino acid residue selected from the group consisting of a threonine residue (T) at position 826, a threonine residue (T) at position 823, a threonine residue (T) at position 821, a serine residue (S) at position 816, a tyrosine residue (Y) at position 813, a serine residue (S) at position 811, a serine residue (S) at position 807, a tyrosine residue (Y) at position 805, a serine residue (S) at position 780, a threonine residue (T) at position 625, a threonine residue (T) at position 601, a threonine residue (T) at position 373, a serine residue (S) at position 360, a threonine residue (T) at position 356, a serine residue (S) at position 249, and a serine residue (S) at position 37 of SEQ ID No: 2 is phosphorylated.

[D-7.7]
The method according to [D-7.5], wherein the hyperphosphorylated human RB1 protein is RB1 protein in which at least one amino acid residue selected from the group consisting of a threonine residue (T) at position 826, a threonine residue (T) at position 821, a serine residue (S) at position 811, a serine residue (S) at position 807, a serine residue (S) at position 780, a threonine residue (T) at position 373, and a threonine residue (T) at position 356 of SEQ ID No: 2 is phosphorylated.

[D-7.8]
The method according to [D-7.5], wherein the hyperphosphorylated human RB1 protein is RB1 protein in which at least one amino acid residue selected from the group consisting of a threonine residue (T) at position 826, a threonine residue (T) at position 821, a serine residue (S) at position 811, and a serine residue (S) at position 807 of SEQ ID No: 2 is phosphorylated.

[D-7.9]
The method according to [D-7.5], wherein the hyperphosphorylated human RB1 protein is RB1 protein in which at least two amino acid residues selected from the group consisting of a threonine residue (T) at position 826, a threonine residue (T) at position 821, and a serine residue (S) at position 811 and/or a serine residue (S) at position 807 of SEQ ID No: 2 are phosphorylated.

[D-7.10]
The method according to [D-7.5], wherein the hyperphosphorylated human RB1 protein is RB1 protein in which a threonine residue (T) at position 826 and a threonine residue (T) at position 821 of SEQ ID No: 2 are phosphorylated, and a serine residue (S) at position 811 and/or a serine residue (S) at position 807 are/is phosphorylated.

[D-8]
The method according to any one of [D-1] to [D-7], wherein the MYT1 inhibitor is at least one selected from the group consisting of a low molecular compound, a polypeptide, and a polynucleotide.

[D-9]
The method according to [D-8], wherein the MYT1 inhibitor is a low molecular compound.

[D-10]
The method according to [D-9], wherein the low molecular compound is a compound having a molecular weight of 2000 g/mol or less.

[D-11]
The method according to [D-9], wherein the low molecular compound is a compound having a molecular weight of 1000 g/mol or less.

[D-12]
The method according to [D-8], wherein the MYT1 inhibitor is a polypeptide.

[D-13]
The method according to [D-12], wherein the polypeptide comprises an antibody.

[D-14]
The method according to [D-12], wherein the polypeptide is an anti-MYT1 antibody.

[D-15]
The method according to [D-8], wherein the MYT1 inhibitor is a polynucleotide.

[D-16]
The method according to [D-15], wherein the polynucleotide is at least one selected from the group consisting of a ribozyme, an antisense molecule, an inhibitor oligonucleotide, an aptamer, a micro RNA, and a small interfering RNA (siRNA).

[D-17]
The method according to [D-15], wherein the polynucleotide is at least one selected from the group consisting of an antisense nucleic acid, a micro RNA, and a small interfering RNA (siRNA).

[D-18]
The method according to any one of [D-1] to [D-11], wherein the MYT1 inhibitor is a compound represented by the formula (1): 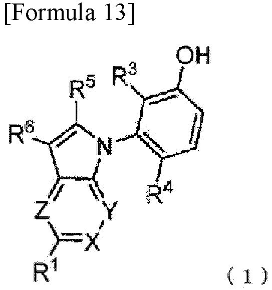
wherein X, Y, Z, R¹, R³, R⁴, R⁵*,* and R⁶ are as defined in the above [A-19],
or a salt thereof, or a solvate thereof.

[D-19]
The method according to [D-18], wherein the compound represented by the formula (1) comprises an atropisomer represented by the formula (1A) in a larger amount than each atropisomer: 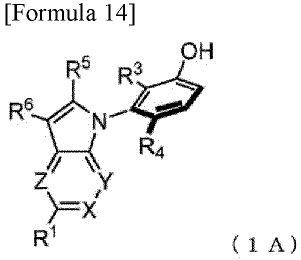 wherein X, Y, Z, R¹, R³, R⁴, R⁵*,* and R⁶ are as defined in the above [A-19].

[D-20]
The method according to any one of [D-1] to [D-18], wherein the MYT1 inhibitor is a compound represented by the formula (2): 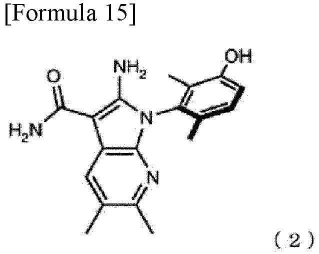 or a salt thereof, or a solvate thereof.

[D-20.5]
The method according to any one of [D-1] to [D-17], wherein the MYT1 inhibitor is a compound represented by the formula (3): 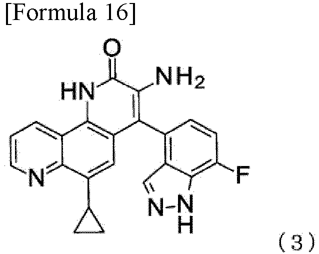 or a salt thereof, or a solvate thereof.

[D-21]
The method according to any one of [D-1] to [D-20.5], wherein the chemotherapeutic agent is at least one selected from the group consisting of an antimetabolite, an anticancer antibiotic, a mitosis inhibitor, a topoisomerase inhibitor, a platinating agent, an alkylating agent, and an antibody-drug conjugate.

[D-22]
The method according to [D-21], wherein the chemotherapeutic agent is an antimetabolite.

[D-23]
The method according to [D-21] or [D-22], wherein the antimetabolite is at least one selected from the group consisting of a purine antimetabolite, a pyrimidine antimetabolite, a folic acid antimetabolite, and a ribonucleotide reductase inhibitor.

[D-24]
The method according to [D-23], wherein the antimetabolite is a purine antimetabolite.

[D-25]
The method according to [D-23] or [D-24], wherein the purine antimetabolite is at least one selected from the group consisting of 6-thioguanine, 6-mercaptopurine, azathioprine, fludarabine, pentostatin, cladribine, clofarabine, and nelarabine.

[D-26]
The method according to [D-21], wherein the antimetabolite is a pyrimidine antimetabolite.

[D-27]
The method according to [D-21] or [D-26], wherein the pyrimidine antimetabolite is at least one selected from the group consisting of gemcitabine, cytarabine, fluorouracil, capecitabine, tegafur, azacitidine, trifluridine, and floxuridine.

[D-28]
The method according to [D-27], wherein the pyrimidine antimetabolite is gemcitabine.

[D-29]
The method according to [D-21], wherein the antimetabolite is a folic acid antimetabolite.

[D-30]
The method according to [D-21] or [D-29], wherein the folic acid antimetabolite is at least one selected from the group consisting of pemetrexed and methotrexate.

[D-31]
The method according to [D-30], wherein the folic acid antimetabolite is pemetrexed.

[D-32]
The method according to [D-21], wherein the antimetabolite is a ribonucleotide reductase inhibitor.

[D-33]
The method according to [D-23] or [D-32], wherein the ribonucleotide reductase inhibitor is hydroxyurea.

[D-34]
The method according to [D-21], wherein the chemotherapeutic agent is an anticancer antibiotic.

[D-35]
The method according to [D-21] or [D-34], wherein the anticancer antibiotic is at least one selected from the group consisting of bleomycin, actinomycin D, doxorubicin, daunorubicin, idarubicin, mitomycin, mitoxantrone, epirubicin, aclarubicin, and valrubicin.

[D-36]
The method according to [D-21], wherein the chemotherapeutic agent is a mitosis inhibitor.

[D-37]
The method according to [D-21] or [D-36], wherein the mitosis inhibitor is at least one selected from the group consisting of vinca alkaloid and a microtubule inhibitor.

[D-38]
The method according to [D-37], wherein the mitosis inhibitor is vinca alkaloid.

[D-39]
The method according to [D-37] or [D-38], wherein the vinca alkaloid is at least one selected from the group consisting of vincristine, vinblastine, and vinorelbine.

[D-40]
The method according to [D-37], wherein the mitosis inhibitor is a microtubule inhibitor.

[D-41]
The method according to [D-37] or [D-40], wherein the microtubule inhibitor is at least one selected from the group consisting of docetaxel, paclitaxel, eribulin, ixabepilone, and epothilone.

[D-42]
The method according to [D-21], wherein the chemotherapeutic agent is a topoisomerase inhibitor.

[D-43]
The method according to [D-21] or [D-42], wherein the topoisomerase inhibitor is at least one selected from the group consisting of topotecan, irinotecan, DXd, etoposide, and teniposide.

[D-44]
The method according to [D-43], wherein the topoisomerase inhibitor is at least one selected from the group consisting of irinotecan and DXd.

[D-45]
The method according to [D-21], wherein the chemotherapeutic agent is a platinating agent.

[D-46]
The method according to [D-21] or [D-45], wherein the platinating agent is at least one selected from the group consisting of cisplatin, carboplatin, and oxaliplatin.

[D-47]
The method according to [D-46], wherein the platinating agent is carboplatin.

[D-48]
The method according to [D-21], wherein the chemotherapeutic agent is an alkylating agent.

[D-49]
The method according to [D-21] or [D-48], wherein the alkylating agent is at least one selected from the group consisting of cyclophosphamide, ifosfamide, chlorambucil, melphalan, temozolomide, carmustine, lomustine, streptozocin, busulfan, procarbazine, dacarbazine, nimustine, ranimustine, bendamustine, altretamine, thiotepa, and mechlorethamine.

[D-50]
The method according to [D-21], wherein the chemotherapeutic agent is an antibody-drug conjugate.

[D-51]
The method according to [D-21] or [D-50], wherein the antibody-drug conjugate is at least one selected from the group consisting of trastuzumab deruxtecan, sacituzumab govitecan, tisotumab vedotin, enfortumab vedotin, trastuzumab emtansine, loncastuximab tesirine, moxetumomab pasudotox, belantamab mafodotin, polatuzumab vedotin, inotuzumab ozogamicin, brentuximab vedotin, and gemtuzumab ozogamicin.

[D-52]
The method according to [D-51], wherein the antibody-drug conjugate is at least one selected from the group consisting of trastuzumab deruxtecan and sacituzumab govitecan.

[D-53]
The method according to any one of [D-1] to [D-7], wherein the MYT1 inhibitor is a small interfering RNA (siRNA), and the chemotherapeutic agent is at least one selected from the group consisting of gemcitabine, pemetrexed, irinotecan, carboplatin, and trastuzumab deruxtecan.

[D-54]
The method according to any one of [D-1] to [D-7], wherein the MYT1 inhibitor is a small interfering RNA (siRNA), and the chemotherapeutic agent is gemcitabine.

[D-55]
The method according to any one of [D-1] to [D-7], wherein the MYT1 inhibitor is a small interfering RNA (siRNA), and the chemotherapeutic agent is fluorouracil.

[D-56]
The method according to any one of [D-1] to [D-7], wherein the MYT1 inhibitor is a small interfering RNA (siRNA), and the chemotherapeutic agent is pemetrexed.

[D-57]
The method according to any one of [D-1] to [D-7], wherein the MYT1 inhibitor is a small interfering RNA (siRNA), and the chemotherapeutic agent is etoposide.

[D-58]
The method according to any one of [D-1] to [D-7], wherein the MYT1 inhibitor is a small interfering RNA (siRNA), and the chemotherapeutic agent is irinotecan.

[D-59]
The method according to any one of [D-1] to [D-7], wherein the MYT1 inhibitor is a small interfering RNA (siRNA), and the chemotherapeutic agent is carboplatin.

[D-60]
The method according to any one of [D-1] to [D-7], wherein the MYT1 inhibitor is a small interfering RNA (siRNA), and the chemotherapeutic agent is trastuzumab deruxtecan.

[D-60.1]
The method according to any one of [D-1] to [D-7], wherein the MYT1 inhibitor is a compound represented by the formula (2) or a salt thereof, or a solvate thereof, and the chemotherapeutic agent is at least one selected from the group consisting of pemetrexed, gemcitabine, carboplatin, irinotecan, sacituzumab govitecan, DXd, and trastuzumab deruxtecan.

[D-60.11]
The method according to any one of [D-1] to [D-7], wherein the MYT1 inhibitor is a compound represented by the formula (2) or a salt thereof, or a solvate thereof, and the chemotherapeutic agent is pemetrexed.

[D-60.12]
The method according to any one of [D-1] to [D-7], wherein the MYT1 inhibitor is a compound represented by the formula (2) or a salt thereof, or a solvate thereof, and the chemotherapeutic agent is gemcitabine.

[D-60.13]
The method according to any one of [D-1] to [D-7], wherein the MYT1 inhibitor is a compound represented by the formula (2) or a salt thereof, or a solvate thereof, and the chemotherapeutic agent is carboplatin.

[D-60.14]
The method according to any one of [D-1] to [D-7], wherein the MYT1 inhibitor is a compound represented by the formula (2) or a salt thereof, or a solvate thereof, and the chemotherapeutic agent is irinotecan.

[D-60.15]
The method according to any one of [D-1] to [D-7], wherein the MYT1 inhibitor is a compound represented by the formula (2) or a salt thereof, or a solvate thereof, and the chemotherapeutic agent is sacituzumab govitecan.

[D-60.16]
The method according to any one of [D-1] to [D-7], wherein the MYT1 inhibitor is a compound represented by the formula (2) or a salt thereof, or a solvate thereof, and the chemotherapeutic agent is DXd.

[D-60.17]
The method according to any one of [D-1] to [D-7], wherein the MYT1 inhibitor is a compound represented by the formula (2) or a salt thereof, or a solvate thereof, and the chemotherapeutic agent is trastuzumab deruxtecan.

[D-60.2]
The method according to any one of [D-1] to [D-7], wherein the MYT1 inhibitor is a compound represented by the formula (3) or a salt thereof, or a solvate thereof, and the chemotherapeutic agent is at least one selected from the group consisting of pemetrexed, gemcitabine, carboplatin, irinotecan, sacituzumab govitecan, DXd, and trastuzumab deruxtecan.

[D-60.21]

The method according to any one of [D-1] to [D-7], wherein the MYT1 inhibitor is a compound represented by the formula (3) or a salt thereof, or a solvate thereof, and the chemotherapeutic agent is pemetrexed.

[D-60.22]
The method according to any one of [D-1] to [D-7], wherein the MYT1 inhibitor is a compound represented by the formula (3) or a salt thereof, or a solvate thereof, and the chemotherapeutic agent is gemcitabine.

[D-60.23]
The method according to any one of [D-1] to [D-7], wherein the MYT1 inhibitor is a compound represented by the formula (3) or a salt thereof, or a solvate thereof, and the chemotherapeutic agent is carboplatin.

[D-60.24]
The method according to any one of [D-1] to [D-7], wherein the MYT1 inhibitor is a compound represented by the formula (3) or a salt thereof, or a solvate thereof, and the chemotherapeutic agent is irinotecan.

[D-60.25]
The method according to any one of [D-1] to [D-7], wherein the MYT1 inhibitor is a compound represented by the formula (3) or a salt thereof, or a solvate thereof, and the chemotherapeutic agent is sacituzumab govitecan.

[D-60.26]

The method according to any one of [D-1] to [D-7], wherein the MYT1 inhibitor is a compound represented by the formula (3) or a salt thereof, or a solvate thereof, and the chemotherapeutic agent is DXd.

[D-60.27]
The method according to any one of [D-1] to [D-7], wherein the MYT1 inhibitor is a compound represented by the formula (3) or a salt thereof, or a solvate thereof, and the chemotherapeutic agent is trastuzumab deruxtecan.

[D-61]
The method according to any one of [D-1] to [D-60], wherein the MYT1 inhibitor and the chemotherapeutic agent are simultaneously or separately administered.

[D-62]
The method according to any one of [D-1] to [D-60], wherein the MYT1 inhibitor and the chemotherapeutic agent are administered as a combination drug.

[D-63]
The method according to any one of [D-1] to [D-62], wherein the cancer is solid cancer or blood cancer.

[D-64]
The method according to any one of [D-1] to [D-62], wherein the cancer is at least one selected from the group consisting of lung cancer, breast cancer, esophageal cancer, stomach cancer, bowel cancer, uterine cancer, ovarian cancer, pancreatic cancer, bladder cancer, thyroid cancer, skin cancer, head and neck cancer, kidney cancer, liver cancer, prostate cancer, adenoid cystic cancer, retinoblastoma, cerebral tumor, leukemia, malignant lymphoma, and multiple myeloma.

[D-65]
The method according to [D-64], wherein the cancer is at least one selected from the group consisting of lung cancer, breast cancer, bladder cancer, and ovarian cancer.

[D-66]
The method according to [D-64], wherein the cancer is lung cancer, breast cancer, or bladder cancer.

[D-67]
The method according to any one of [D-1] to [D-66], wherein the RB1 gene mutation positivity, or the decrease in expression of an RB1 gene or protein is detected in a cancer patient-derived biological sample.

[D-67.1]
The method according to any one of [D-1] to [D-66], wherein the positive expression of hyperphosphorylated RB1 protein is detected in a cancer patient-derived biological sample.

[D-68]
The method according to [D-67], wherein the cancer patient-derived biological sample is cancer cells.

[D-69]
The method according to any one of [D-1] to [D-68], wherein the decrease in expression of an RB1 gene or protein is a decrease based on an expression level in a healthy individual-derived biological sample or a non-cancer tissue derived from the cancer patient.

[D-69.1]
The method according to any one of [D-1] to [D-68], wherein the positive expression of hyperphosphorylated RB1 protein is determined from an increase in expression of the hyperphosphorylated RB1 protein based on an expression level in a healthy individual-derived biological sample or a non-cancer tissue derived from the cancer patient.

[D-70]
The method according to any one of [D-1] to [D-69], wherein the patient is a patient in which amplification of a copy number of a CCNE1 gene is not detected.

[D-71]
The method according to any one of [D-1] to [D-70], wherein the above patient is not a mouse implanted with OVCAR3.

[D-72]
The method according to any one of [D-1] to [D-71], wherein the patient is human.

[E-1]
A method for predicting responsiveness to treatment of cancer with a combination of an MYT1 inhibitor and a chemotherapeutic agent, the method comprising:
detecting or allowing a third person to detect the presence or absence of an RB1 gene mutation, the presence or absence of a decrease in expression of an RB1 gene or protein, or the presence or absence of the hyperphosphorylated RB1 protein, in a cancer patient-derived biological sample, and
determining the patient as having responsiveness to the treatment of cancer with the combination of the MYT1 inhibitor and the chemotherapeutic agent, when the RB1 gene mutation is positive, the expression of the RB1 gene or protein is decreased, or the expression of the hyperphosphorylated RB1 protein is positive.

[E-1.1]
A method for predicting responsiveness to treatment of cancer with a combination of an MYT1 inhibitor and a chemotherapeutic agent, the method comprising:
detecting or allowing a third person to detect the presence or absence of an RB1 gene mutation or the presence or absence of a decrease in expression of an RB1 gene or protein, in a cancer patient-derived biological sample, and
determining the patient as having responsiveness to the treatment of cancer with the combination of the MYT1 inhibitor and the chemotherapeutic agent, when the RB1 gene mutation is positive or the expression of the RB1 gene or protein is decreased.

[E-1.2]
A method for predicting responsiveness to treatment of cancer with a combination of an MYT1 inhibitor and a chemotherapeutic agent, the method comprising:
detecting or allowing a third person to detect the presence or absence of the hyperphosphorylated RB1 protein in a cancer patient-derived biological sample, and
determining the patient as having responsiveness to the treatment of cancer with the combination of the MYT1 inhibitor and the chemotherapeutic agent, when the expression of the hyperphosphorylated RB1 protein is positive.

[E-2]
The method according to [E-1], wherein the cancer patient-derived biological sample is cancer cells.

[E-3]
The method according to [E-1] or [E-2], wherein the RB1 gene mutation comprises a mutation causing insertion, substitution, deletion, and/or addition of at least one amino acid residue to wild-type RB1 protein.

[E-4]
The method according to any one of [E-1] to [E-3], wherein the RB1 gene mutation is a nonsense mutation, a frameshift mutation, a splice site mutation, or a homozygous or heterozygous deletion.

[E-5]
The method according to any one of [E-1] to [E-4], wherein the RB1 gene mutation is a mutation decreasing a function of RB1.

[E-6]
The method according to any one of [E-1] to [E-5], wherein the RB1 gene mutation is a human RB1 gene mutation.

[E-7]
The method according to [E-6], wherein the human RB1 gene mutation is at least one of the following (1) to (5):
(1) a codon corresponding to a serine residue (S) at position 82 of an amino acid sequence of SEQ ID No: 2 is replaced with a stop codon,
(2) a codon corresponding to an arginine residue (R) at position 467 of the amino acid sequence of SEQ ID No: 2 is replaced with a stop codon,
(3) in a codon corresponding to an amino acid residue at position 182, at least one base is inserted or deleted, a new reading frame starting from an isoleucine residue (I) is formed, and a third reading frame therefrom is a stop codon,
(4) a glutamic acid residue (E) at position 837 of the amino acid sequence of SEQ ID No: 2 is replaced with a lysine residue (K), and a part of the RB1 gene is homozygously deleted, and
(5) a glycine residue (G) at position 449 of the amino acid sequence of SEQ ID No: 2 is replaced with a glutamic acid residue (E), and a part of the RB1 gene is homozygously deleted.

[E-8]
The method according to any one of [E-1] to [E-7], wherein the decrease in expression of an RB1 gene or protein comprises a decrease in gene expression through methylation of an RB1 gene or a micro RNA.

[E-8.1]
The method according to any one of [E-1] to [E-7], wherein the hyperphosphorylated RB1 protein is RB1 protein having 3 or more phosphorylated amino acid residues in the amino acid sequence of the RB1 protein.

[E-8.2]
The method according to any one of [E-1] to [E-7], wherein the hyperphosphorylated RB1 protein is RB1 protein having 4 or more phosphorylated amino acid residues in the amino acid sequence of the RB1 protein.

[E-8.3]
The method according to any one of [E-1] to [E-7], wherein the hyperphosphorylated RB1 protein is RB1 protein having 8 or more phosphorylated amino acid residues in the amino acid sequence of the RB1 protein.

[E-8.4]
The method according to any one of [E-1] to [E-7], wherein the hyperphosphorylated RB1 protein is RB1 protein having 15 or more phosphorylated amino acid residues in the amino acid sequence of the RB1 protein.

[E-8.5]
The method according to any one of [E-1] to [E-7], wherein the hyperphosphorylated RB1 protein is hyperphosphorylated human RB1 protein.

[E-8.6]
The method according to [E-8.5], wherein the hyperphosphorylated human RB1 protein is RB1 protein in which at least one amino acid residue selected from the group consisting of a threonine residue (T) at position 826, a threonine residue (T) at position 823, a threonine residue (T) at position 821, a serine residue (S) at position 816, a tyrosine residue (Y) at position 813, a serine residue (S) at position 811, a serine residue (S) at position 807, a tyrosine residue (Y) at position 805, a serine residue (S) at position 780, a threonine residue (T) at position 625, a threonine residue (T) at position 601, a threonine residue (T) at position 373, a serine residue (S) at position 360, a threonine residue (T) at position 356, a serine residue (S) at position 249, and a serine residue (S) at position 37 of SEQ ID No: 2 is phosphorylated.

[E-8.7]
The method according to [E-8.5], wherein the hyperphosphorylated human RB1 protein is RB1 protein in which at least one amino acid residue selected from the group consisting of a threonine residue (T) at position 826, a threonine residue (T) at position 821, a serine residue (S) at position 811, a serine residue (S) at position 807, a serine residue (S) at position 780, a threonine residue (T) at position 373, and a threonine residue (T) at position 356 of SEQ ID No: 2 is phosphorylated.

[E-8.8]
The method according to [E-8.5], wherein the hyperphosphorylated human RB1 protein is RB1 protein in which at least one amino acid residue selected from the group consisting of a threonine residue (T) at position 826, a threonine residue (T) at position 821, a serine residue (S) at position 811, and a serine residue (S) at position 807 of SEQ ID No: 2 is phosphorylated.

[E-8.9]

The method according to [E-8.5], wherein the hyperphosphorylated human RB1 protein is RB1 protein in which at least two amino acid residues selected from the group consisting of a threonine residue (T) at position 826, a threonine residue (T) at position 821, and a serine residue (S) at position 811 and/or a serine residue (S) at position 807 of SEQ ID No: 2 are phosphorylated.

[E-8.10] The method according to [E-8.5], wherein the hyperphosphorylated human RB1 protein is RB1 protein in which a threonine residue (T) at position 826 and a threonine residue (T) at position 821 of SEQ ID No: 2 are phosphorylated, and a serine residue (S) at position 811 and/or a serine residue (S) at position 807 are/is phosphorylated.

[E-9]
The method according to any one of [E-1] to [E-8], wherein the MYT1 inhibitor is at least one selected from the group consisting of a low molecular compound, a polypeptide, and a polynucleotide.

[E-10]
The method according to [E-9], wherein the MYT1 inhibitor is a low molecular compound.

[E-11]
The method according to [E-9], wherein the low molecular compound is a compound having a molecular weight of 2000 g/mol or less.

[E-12]
The method according to [E-9], wherein the low molecular compound is a compound having a molecular weight of 1000 g/mol or less.

[E-13]
The method according to [E-9], wherein the MYT1 inhibitor is a polypeptide.

[E-14]
The method according to [E-13], wherein the polypeptide comprises an antibody.

[E-15]
The method according to [E-13], wherein the polypeptide is an anti-MYT1 antibody.

[E-16]
The method according to [E-9], wherein the MYT1 inhibitor is a polynucleotide.

[E-17]
The method according to [E-16], wherein the polynucleotide is at least one selected from the group consisting of a ribozyme, an antisense molecule, an inhibitor oligonucleotide, an aptamer, a micro RNA, and a small interfering RNA (siRNA).

[E-18]
The method according to [E-16], wherein the polynucleotide is at least one selected from the group consisting of an antisense nucleic acid, a micro RNA, and a small interfering RNA (siRNA).

[E-19]
The method according to any one of [E-1] to [E-12], wherein the MYT1 inhibitor is a compound represented by the formula (1): 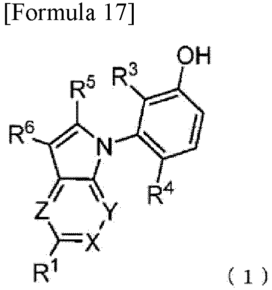
wherein X, Y, Z, R¹, R³, R⁴, R⁵*,* and R⁶ are as defined in the above [A-19],
or a salt thereof, or a solvate thereof.

[E-20]
The method according to [E-19], wherein the compound represented by the formula (1) comprises an atropisomer represented by the formula (1A) in a larger amount than each atropisomer: 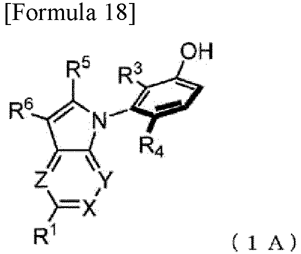 wherein X, Y, Z, R¹, R³, R⁴, R⁵*,* and R⁶ are as defined in the above [A-19].

[E-21]
The method according to any one of [E-1] to [E-20], wherein the MYT1 inhibitor is a compound represented by the formula (2): 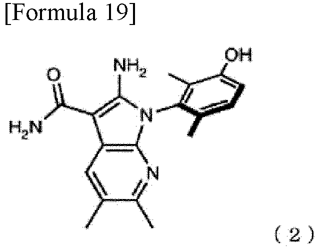 or a salt thereof, or a solvate thereof.

[E-21.5]
The method according to any one of [E-1] to [E-18], wherein the MYT1 inhibitor is a compound represented by the formula (3): 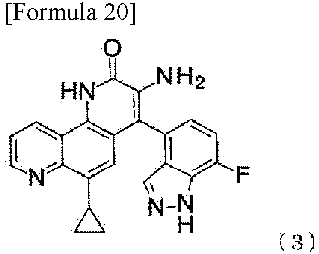 or a salt thereof, or a solvate thereof.

[E-22]
The method according to any one of [E-1] to [E-21.5], wherein the chemotherapeutic agent is at least one selected from the group consisting of an antimetabolite, an anticancer antibiotic, a mitosis inhibitor, a topoisomerase inhibitor, a platinating agent, an alkylating agent, and an antibody-drug conjugate.

[E-23]
The method according to [E-22], wherein the chemotherapeutic agent is an antimetabolite.

[E-24]
The method according to [E-22] or [E-23], wherein the antimetabolite is at least one selected from the group consisting of a purine antimetabolite, a pyrimidine antimetabolite, a folic acid antimetabolite, and a ribonucleotide reductase inhibitor.

[E-25]
The method according to [E-22], wherein the antimetabolite is a purine antimetabolite.

[E-26]
The method according to [E-24] or [E-25], wherein the purine antimetabolite is at least one selected from the group consisting of 6-thioguanine, 6-mercaptopurine, azathioprine, fludarabine, pentostatin, cladribine, clofarabine, and nelarabine.

[E-27]
The method according to [E-22], wherein the antimetabolite is a pyrimidine antimetabolite.

[E-28]
The method according to [E-24] or [E-27], wherein the pyrimidine antimetabolite is at least one selected from the group consisting of gemcitabine, cytarabine, fluorouracil, capecitabine, tegafur, azacitidine, trifluridine, and floxuridine.

[E-29]
The method according to [E-28], wherein the pyrimidine antimetabolite is gemcitabine.

[E-30]
The method according to [E-22], wherein the antimetabolite is a folic acid antimetabolite.

[E-31]
The method according to [E-24] or [E-30], wherein the folic acid antimetabolite is at least one selected from the group consisting of pemetrexed and methotrexate.

[E-32]
The method according to [E-31], wherein the folic acid antimetabolite is pemetrexed.

[E-33]
The method according to [E-22], wherein the antimetabolite is a ribonucleotide reductase inhibitor.

[E-34]
The method according to [E-24] or [E-33], wherein the ribonucleotide reductase inhibitor is hydroxyurea.

[E-35]
The method according to [E-22], wherein the chemotherapeutic agent is an anticancer antibiotic.

[E-36]
The method according to [E-22] or [E-35], wherein the anticancer antibiotic is at least one selected from the group consisting of bleomycin, actinomycin D, doxorubicin, daunorubicin, idarubicin, mitomycin, mitoxantrone, epirubicin, aclarubicin, and valrubicin.

[E-37]
The method according to [E-22], wherein the chemotherapeutic agent is a mitosis inhibitor.

[E-38]
The method according to [E-37], wherein the mitosis inhibitor is at least one selected from the group consisting of vinca alkaloid and a microtubule inhibitor.

[E-39]
The method according to [E-38], wherein the mitosis inhibitor is vinca alkaloid.

[E-40]
The method according to [E-38] or [E-39], wherein the vinca alkaloid is at least one selected from the group consisting of vincristine, vinblastine, and vinorelbine.

[E-41]
The method according to [E-37], wherein the mitosis inhibitor is a microtubule inhibitor.

[E-42]
The method according to [E-38] or [E-41], wherein the microtubule inhibitor is at least one selected from the group consisting of docetaxel, paclitaxel, eribulin, ixabepilone, and epothilone.

[E-43]
The method according to [E-22], wherein the chemotherapeutic agent is a topoisomerase inhibitor.

[E-44]
The method according to [E-22] or [E-43], wherein the topoisomerase inhibitor is at least one selected from the group consisting of topotecan, irinotecan, DXd, etoposide, and teniposide.

[E-45]
The method according to [E-44], wherein the topoisomerase inhibitor is at least one selected from the group consisting of irinotecan and DXd.

[E-46]
The method according to [E-22], wherein the chemotherapeutic agent is a platinating agent.

[E-47]
The method according to [E-22] or [E-46], wherein the platinating agent is at least one selected from the group consisting of cisplatin, carboplatin, and oxaliplatin.

[E-48]
The method according to [E-47], wherein the platinating agent is carboplatin.

[E-49]
The method according to [E-22], wherein the chemotherapeutic agent is an alkylating agent.

[E-50]
The method according to [E-22] or [E-49], wherein the alkylating agent is at least one selected from the group consisting of cyclophosphamide, ifosfamide, chlorambucil, melphalan, temozolomide, carmustine, lomustine, streptozocin, busulfan, procarbazine, dacarbazine, nimustine, ranimustine, bendamustine, altretamine, thiotepa, and mechlorethamine.

[E-51]
The method according to [E-22], wherein the chemotherapeutic agent is an antibody-drug conjugate.

[E-52]
The method according to [E-22] or [E-51], wherein the antibody-drug conjugate is at least one selected from the group consisting of trastuzumab deruxtecan, sacituzumab govitecan, tisotumab vedotin, enfortumab vedotin, trastuzumab emtansine, loncastuximab tesirine, moxetumomab pasudotox, belantamab mafodotin, polatuzumab vedotin, inotuzumab ozogamicin, brentuximab vedotin, and gemtuzumab ozogamicin.

[E-53]
The method according to [E-52], wherein the antibody-drug conjugate is at least one selected from the group consisting of trastuzumab deruxtecan and sacituzumab govitecan.

[E-54]
The method according to any one of [E-1] to [E-8], wherein the MYT1 inhibitor is a small interfering RNA (siRNA), and the chemotherapeutic agent is at least one selected from the group consisting of gemcitabine, pemetrexed, irinotecan, carboplatin, and trastuzumab deruxtecan.

[E-55]
The method according to any one of [E-1] to [E-8], wherein the MYT1 inhibitor is a small interfering RNA (siRNA), and the chemotherapeutic agent is gemcitabine.

[E-56]
The method according to any one of [E-1] to [E-8], wherein the MYT1 inhibitor is a small interfering RNA (siRNA), and the chemotherapeutic agent is fluorouracil.

[E-57]
The method according to any one of [E-1] to [E-8], wherein the MYT1 inhibitor is a small interfering RNA (siRNA), and the chemotherapeutic agent is pemetrexed.

[E-58]
The method according to any one of [E-1] to [E-8], wherein the MYT1 inhibitor is a small interfering RNA (siRNA), and the chemotherapeutic agent is etoposide.

[E-59]
The method according to any one of [E-1] to [E-8], wherein the MYT1 inhibitor is a small interfering RNA (siRNA), and the chemotherapeutic agent is irinotecan.

[E-60]
The method according to any one of [E-1] to [E-8], wherein the MYT1 inhibitor is a small interfering RNA (siRNA), and the chemotherapeutic agent is carboplatin.

[E-61]
The method according to any one of [E-1] to [E-8], wherein the MYT1 inhibitor is a small interfering RNA (siRNA), and the chemotherapeutic agent is trastuzumab deruxtecan.

[E-61.1]
The method according to any one of [E-1] to [E-8], wherein the MYT1 inhibitor is a compound represented by the formula (2) or a salt thereof, or a solvate thereof, and the chemotherapeutic agent is at least one selected from the group consisting of pemetrexed, gemcitabine, carboplatin, irinotecan, sacituzumab govitecan, DXd, and trastuzumab deruxtecan.

[E-61.11]
The method according to any one of [E-1] to [E-8], wherein the MYT1 inhibitor is a compound represented by the formula (2) or a salt thereof, or a solvate thereof, and the chemotherapeutic agent is pemetrexed.

[E-61.12]
The method according to any one of [E-1] to [E-8], wherein the MYT1 inhibitor is a compound represented by the formula (2) or a salt thereof, or a solvate thereof, and the chemotherapeutic agent is gemcitabine.

[E-61.13]
The method according to any one of [E-1] to [E-8], wherein the MYT1 inhibitor is a compound represented by the formula (2) or a salt thereof, or a solvate thereof, and the chemotherapeutic agent is carboplatin.

[E-61.14]
The method according to any one of [E-1] to [E-8], wherein the MYT1 inhibitor is a compound represented by the formula (2) or a salt thereof, or a solvate thereof, and the chemotherapeutic agent is irinotecan.

[E-61.15]
The method according to any one of [E-1] to [E-8], wherein the MYT1 inhibitor is a compound represented by the formula (2) or a salt thereof, or a solvate thereof, and the chemotherapeutic agent is sacituzumab govitecan.

[E-61.16]
The method according to any one of [E-1] to [E-8], wherein the MYT1 inhibitor is a compound represented by the formula (2) or a salt thereof, or a solvate thereof, and the chemotherapeutic agent is DXd.

[E-61.17]
The method according to any one of [E-1] to [E-8], wherein the MYT1 inhibitor is a compound represented by the formula (2) or a salt thereof, or a solvate thereof, and the chemotherapeutic agent is trastuzumab deruxtecan.

[E-61.2]
The method according to any one of [E-1] to [E-8], wherein the MYT1 inhibitor is a compound represented by the formula (3) or a salt thereof, or a solvate thereof, and the chemotherapeutic agent is at least one selected from the group consisting of pemetrexed, gemcitabine, carboplatin, irinotecan, sacituzumab govitecan, DXd, and trastuzumab deruxtecan.

[E-61.21]
The method according to any one of [E-1] to [E-8], wherein the MYT1 inhibitor is a compound represented by the formula (3) or a salt thereof, or a solvate thereof, and the chemotherapeutic agent is pemetrexed.

[E-61.22]
The method according to any one of [E-1] to [E-8], wherein the MYT1 inhibitor is a compound represented by the formula (3) or a salt thereof, or a solvate thereof, and the chemotherapeutic agent is gemcitabine.

[E-61.23]
The method according to any one of [E-1] to [E-8], wherein the MYT1 inhibitor is a compound represented by the formula (3) or a salt thereof, or a solvate thereof, and the chemotherapeutic agent is carboplatin.

[E-61.24]
The method according to any one of [E-1] to [E-8], wherein the MYT1 inhibitor is a compound represented by the formula (3) or a salt thereof, or a solvate thereof, and the chemotherapeutic agent is irinotecan.

[E-61.25]
The method according to any one of [E-1] to [E-8], wherein the MYT1 inhibitor is a compound represented by the formula (3) or a salt thereof, or a solvate thereof, and the chemotherapeutic agent is sacituzumab govitecan.

[E-61.26]
The method according to any one of [E-1] to [E-8], wherein the MYT1 inhibitor is a compound represented by the formula (3) or a salt thereof, or a solvate thereof, and the chemotherapeutic agent is DXd.

[E-61.27]
The method according to any one of [E-1] to [E-8], wherein the MYT1 inhibitor is a compound represented by the formula (3) or a salt thereof, or a solvate thereof, and the chemotherapeutic agent is trastuzumab deruxtecan.

[E-62]
The method according to any one of [E-1] to [E-61], wherein the MYT1 inhibitor and the chemotherapeutic agent are simultaneously or separately administered.

[E-63]
The method according to any one of [E-1] to [E-61], wherein the MYT1 inhibitor and the chemotherapeutic agent are administered as a combination drug.

[E-64]
The method according to any one of [E-1] to [E-63], wherein the cancer is solid cancer or blood cancer.

[E-65]
The method according to any one of [E-1] to [E-63], wherein the cancer is at least one selected from the group consisting of lung cancer, breast cancer, esophageal cancer, stomach cancer, bowel cancer, uterine cancer, ovarian cancer, pancreatic cancer, bladder cancer, thyroid cancer, skin cancer, head and neck cancer, kidney cancer, liver cancer, prostate cancer, adenoid cystic cancer, retinoblastoma, cerebral tumor, leukemia, malignant lymphoma, and multiple myeloma.

[E-66]
The method according to [E-65], wherein the cancer is at least one selected from the group consisting of lung cancer, breast cancer, bladder cancer, and ovarian cancer.

[E-67]
The method according to [E-65], wherein the cancer is lung cancer, breast cancer, or bladder cancer.

[E-68]
The method according to any one of [E-1] to [E-67], wherein the RB1 gene mutation positivity, or the decrease in expression of an RB1 gene or protein is detected in a cancer patient-derived biological sample.

[E-68.1]
The method according to any one of [E-1] to [E-67], wherein the positive expression of hyperphosphorylated RB1 protein is detected in a cancer patient-derived biological sample.

[E-69]
The method according to any one of [E-1] to [E-68], wherein the decrease in expression of an RB1 gene or protein is a decrease based on an expression level in a healthy individual-derived biological sample or a non-cancer tissue derived from the cancer patient.

[E-69.1]
The method according to any one of [E-1] to [E-68], wherein the positive expression of hyperphosphorylated RB1 protein is determined from an increase in expression of the hyperphosphorylated RB1 protein based on an expression level in a healthy individual-derived biological sample or a non-cancer tissue derived from the cancer patient.

[E-70]
The method according to any one of [E-1] to [E-69], wherein the patient is a patient in which amplification of a copy number of a CCNE1 gene is not detected.

[E-71]
The method according to any one of [E-1] to [E-70], wherein the above patient is not a mouse implanted with OVCAR3.

[E-72]
The method according to any one of [E-1] to [E-71], wherein the patient is human.

[F-1]
A method for selecting a cancer patient to which administration of a combination of an MYT1 inhibitor and a chemotherapeutic agent is more effective, the method comprising:
detecting or allowing a third person to detect the presence or absence of an RB1 gene mutation, the presence or absence of a decrease in expression of an RB1 gene or protein, or the presence or absence of the hyperphosphorylated RB1 protein, in a cancer patient-derived biological sample, and
determining the cancer patient as a cancer patient to which administration of the combination of the MYT1 inhibitor and the chemotherapeutic agent is more effective, based on the presence of the mutation, the decrease in expression, or the positivity of the expression of the hyperphosphorylated RB1 protein.

[F-1.1]
A method for selecting a cancer patient to which administration of a combination of an MYT1 inhibitor and a chemotherapeutic agent is more effective, the method comprising:
detecting or allowing a third person to detect the presence or absence of an RB1 gene mutation or the presence or absence of a decrease in expression of an RB1 gene or protein, in a cancer patient-derived biological sample, and
determining the cancer patient as a cancer patient to which administration of the combination of the MYT1 inhibitor and the chemotherapeutic agent is more effective, based on the presence of the mutation or the decrease in expression.

[F-1.2]
A method for selecting a cancer patient to which administration of a combination of an MYT1 inhibitor and a chemotherapeutic agent is more effective, the method comprising:
detecting or allowing a third person to detect the presence or absence of the hyperphosphorylated RB1 protein in a cancer patient-derived biological sample,
determining the cancer patient as a cancer patient to which administration of the combination of the MYT1 inhibitor and the chemotherapeutic agent is more effective, based on the positivity of the expression of the hyperphosphorylated RB1 protein.

[F-2]
The method according to [F-1], wherein the cancer patient-derived biological sample is cancer cells.

[F-3]
The method according to [F-1] or [F-2], wherein the RB1 gene mutation comprises a mutation causing insertion, substitution, deletion, and/or addition of at least one amino acid residue to wild-type RB1 protein.

[F-4]
The method according to any one of [F-1] to [F-3], wherein the RB1 gene mutation is a nonsense mutation, a frameshift mutation, a splice site mutation, or a homozygous or heterozygous deletion.

[F-5]
The method according to any one of [F-1] to [F-4], wherein the RB1 gene mutation is a mutation decreasing a function of RB1.

[F-6]
The method according to any one of [F-1] to [F-5], wherein the RB1 gene mutation is a human RB1 gene mutation.

[F-7]
The method according to [F-6], wherein the human RB1 gene mutation is at least one of the following (1) to (5):
(1) a codon corresponding to a serine residue (S) at position 82 of an amino acid sequence of SEQ ID No: 2 is replaced with a stop codon,
(2) a codon corresponding to an arginine residue (R) at position 467 of the amino acid sequence of SEQ ID No: 2 is replaced with a stop codon,
(3) in a codon corresponding to an amino acid residue at position 182, at least one base is inserted or deleted, a new reading frame starting from an isoleucine residue (I) is formed, and a third reading frame therefrom is a stop codon,
(4) a glutamic acid residue (E) at position 837 of the amino acid sequence of SEQ ID No: 2 is replaced with a lysine residue (K), and a part of the RB1 gene is homozygously deleted, and
(5) a glycine residue (G) at position 449 of the amino acid sequence of SEQ ID No: 2 is replaced with a glutamic acid residue (E), and a part of the RB1 gene is homozygously deleted.

[F-8]
The method according to any one of [F-1] to [F-7], wherein the decrease in expression of an RB1 gene or protein comprises a decrease in gene expression through methylation of an RB 1 gene or a micro RNA.

[F-8.1]
The method according to any one of [F-1] to [F-7], wherein the hyperphosphorylated RB 1 protein is RB1 protein having 3 or more phosphorylated amino acid residues in the amino acid sequence of the RB1 protein.

[F-8.2]
The method according to any one of [F-1] to [F-7], wherein the hyperphosphorylated RB 1 protein is RB1 protein having 4 or more phosphorylated amino acid residues in the amino acid sequence of the RB1 protein.

[F-8.3]
The method according to any one of [F-1] to [F-7], wherein the hyperphosphorylated RB1 protein is RB1 protein having 8 or more phosphorylated amino acid residues in the amino acid sequence of the RB1 protein.

[F-8.4]
The method according to any one of [F-1] to [F-7], wherein the hyperphosphorylated RB1 protein is RB1 protein having 15 or more phosphorylated amino acid residues in the amino acid sequence of the RB1 protein.

[F-8.5]
The method according to any one of [F-1] to [F-7], wherein the hyperphosphorylated RB1 protein is hyperphosphorylated human RB1 protein.

[F-8.6]
The method according to [F-8.5], wherein the hyperphosphorylated human RB1 protein is RB1 protein in which at least one amino acid residue selected from the group consisting of a threonine residue (T) at position 826, a threonine residue (T) at position 823, a threonine residue (T) at position 821, a serine residue (S) at position 816, a tyrosine residue (Y) at position 813, a serine residue (S) at position 811, a serine residue (S) at position 807, a tyrosine residue (Y) at position 805, a serine residue (S) at position 780, a threonine residue (T) at position 625, a threonine residue (T) at position 601, a threonine residue (T) at position 373, a serine residue (S) at position 360, a threonine residue (T) at position 356, a serine residue (S) at position 249, and a serine residue (S) at position 37 of SEQ ID No: 2 is phosphorylated.

[F-8.7]
The method according to [F-8.5], wherein the hyperphosphorylated human RB1 protein is RB1 protein in which at least one amino acid residue selected from the group consisting of a threonine residue (T) at position 826, a threonine residue (T) at position 821, a serine residue (S) at position 811, a serine residue (S) at position 807, a serine residue (S) at position 780, a threonine residue (T) at position 373, and a threonine residue (T) at position 356 of SEQ ID No: 2 is phosphorylated.

[F-8.8]
The method according to [F-8.5], wherein the hyperphosphorylated human RB1 protein is RB1 protein in which at least one amino acid residue selected from the group consisting of a threonine residue (T) at position 826, a threonine residue (T) at position 821, a serine residue (S) at position 811, and a serine residue (S) at position 807 of SEQ ID No: 2 is phosphorylated.

[F-8.9]
The method according to [F-8.5], wherein the hyperphosphorylated human RB1 protein is RB1 protein in which at least two amino acid residues selected from the group consisting of a threonine residue (T) at position 826, a threonine residue (T) at position 821, and a serine residue (S) at position 811 and/or a serine residue (S) at position 807 of SEQ ID No: 2 are phosphorylated.

[F-8.10]
The method according to [F-8.5], wherein the hyperphosphorylated human RB1 protein is RB1 protein in which a threonine residue (T) at position 826 and a threonine residue (T) at position 821 of SEQ ID No: 2 are phosphorylated, and a serine residue (S) at position 811 and/or a serine residue (S) at position 807 are/is phosphorylated.

[F-9]
The method according to any one of [F-1] to [F-8], wherein the MYT1 inhibitor is at least one selected from the group consisting of a low molecular compound, a polypeptide, and a polynucleotide.

[F-10]
The method according to [F-9], wherein the MYT1 inhibitor is a low molecular compound.

[F-11]
The method according to [F-9], wherein the low molecular compound is a compound having a molecular weight of 2000 g/mol or less.

[F-12]
The method according to [F-9], wherein the low molecular compound is a compound having a molecular weight of 1000 g/mol or less.

[F-13]
The method according to [F-9], wherein the MYT1 inhibitor is a polypeptide.

[F-14]
The method according to [F-13], wherein the polypeptide comprises an antibody.

[F-15]
The method according to [F-13], wherein the polypeptide is an anti-MYT1 antibody.

[F-16]
The method according to [F-9], wherein the MYT1 inhibitor is a polynucleotide.

[F-17]
The method according to [F-16], wherein the polynucleotide is at least one selected from the group consisting of a ribozyme, an antisense molecule, an inhibitor oligonucleotide, an aptamer, a micro RNA, and a small interfering RNA (siRNA).

[F-18]
The method according to [F-16], wherein the polynucleotide is at least one selected from the group consisting of an antisense nucleic acid, a micro RNA, and a small interfering RNA (siRNA).

[F-19]
The method according to any one of [F-1] to [F-12], wherein the MYT1 inhibitor is a compound represented by the formula (1): 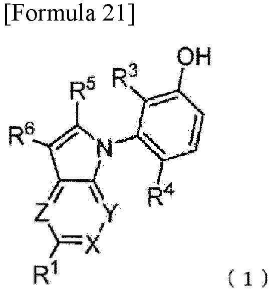
wherein X, Y, Z, R¹, R³, R⁴, R⁵*,* and R⁶ are as defined in the above [A-19],
or a salt thereof, or a solvate thereof.

[F-20]
The method according to [F-19], wherein the compound represented by the formula (1) comprises an atropisomer represented by the formula (1A) in a larger amount than each atropisomer: 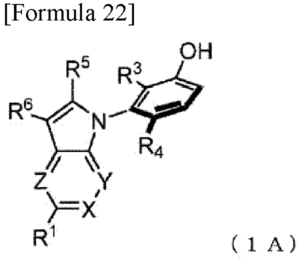 wherein X, Y, Z, R¹, R³, R⁴, R⁵*,* and R⁶ are as defined in the above [A-19].

[F-21]
The method according to any one of [F-1] to [F-20], wherein the MYT1 inhibitor is a compound represented by the formula (2): 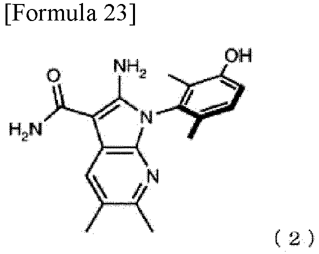 or a salt thereof, or a solvate thereof.

[F-21.5]
The method according to any one of [F-1] to [F-18], wherein the MYT1 inhibitor is a compound represented by the formula (3): 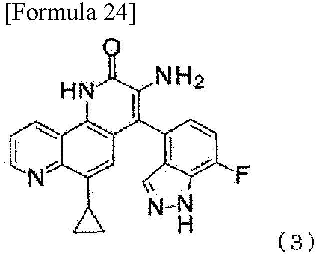 or a salt thereof, or a solvate thereof.

[F-22]
The method according to any one of [F-1] to [F-21.5], wherein the chemotherapeutic agent is at least one selected from the group consisting of an antimetabolite, an anticancer antibiotic, a mitosis inhibitor, a topoisomerase inhibitor, a platinating agent, an alkylating agent, and an antibody-drug conjugate.

[F-23]
The method according to [F-22], wherein the chemotherapeutic agent is an antimetabolite.

[F-24]
The method according to [F-22] or [F-23], wherein the antimetabolite is at least one selected from the group consisting of a purine antimetabolite, a pyrimidine antimetabolite, a folic acid antimetabolite, and a ribonucleotide reductase inhibitor.

[F-25]
The method according to [F-22], wherein the antimetabolite is a purine antimetabolite.

[F-26]
The method according to [F-24] or [F-25], wherein the purine antimetabolite is at least one selected from the group consisting of 6-thioguanine, 6-mercaptopurine, azathioprine, fludarabine, pentostatin, cladribine, clofarabine, and nelarabine.

[F-27]
The method according to [F-22], wherein the antimetabolite is a pyrimidine antimetabolite.

[F-28]
The method according to [F-24] or [F-27], wherein the pyrimidine antimetabolite is at least one selected from the group consisting of gemcitabine, cytarabine, fluorouracil, capecitabine, tegafur, azacitidine, trifluridine, and floxuridine.

[F-29]
The method according to [F-28], wherein the pyrimidine antimetabolite is gemcitabine.

[F-30]
The method according to [F-22], wherein the antimetabolite is a folic acid antimetabolite.

[F-31]
The method according to [F-24] or [F-30], wherein the folic acid antimetabolite is at least one selected from the group consisting of pemetrexed and methotrexate.

[F-32]
The method according to [F-31], wherein the folic acid antimetabolite is pemetrexed.

[F-33]
The method according to [F-22], wherein the antimetabolite is a ribonucleotide reductase inhibitor.

[F-34]
The method according to [F-24] or [F-33], wherein the ribonucleotide reductase inhibitor is hydroxyurea.

[F-35]
The method according to [F-22], wherein the chemotherapeutic agent is an anticancer antibiotic.

[F-36]
The method according to [F-22] or [F-35], wherein the anticancer antibiotic is at least one selected from the group consisting of bleomycin, actinomycin D, doxorubicin, daunorubicin, idarubicin, mitomycin, mitoxantrone, epirubicin, aclarubicin, and valrubicin.

[F-37]
The method according to [F-22], wherein the chemotherapeutic agent is a mitosis inhibitor.

[F-38]
The method according to [F-37], wherein the mitosis inhibitor is at least one selected from the group consisting of vinca alkaloid and a microtubule inhibitor.

[F-39]
The method according to [F-38], wherein the mitosis inhibitor is vinca alkaloid.

[F-40]
The method according to [F-38] or [F-39], wherein the vinca alkaloid is at least one selected from the group consisting of vincristine, vinblastine, and vinorelbine.

[F-41]
The method according to [F-37], wherein the mitosis inhibitor is a microtubule inhibitor.

[F-42]
The method according to [F-38] or [F-41], wherein the microtubule inhibitor is at least one selected from the group consisting of docetaxel, paclitaxel, eribulin, ixabepilone, and epothilone.

[F-43]
The method according to [F-22], wherein the chemotherapeutic agent is a topoisomerase inhibitor.

[F-44]
The method according to [F-22] or [F-43], wherein the topoisomerase inhibitor is at least one selected from the group consisting of topotecan, irinotecan, DXd, etoposide, and teniposide.

[F-45]
The method according to [F-44], wherein the topoisomerase inhibitor is at least one selected from the group consisting of irinotecan and DXd.

[F-46]
The method according to [F-22], wherein the chemotherapeutic agent is a platinating agent.

[F-47]
The method according to [F-22] or [F-46], wherein the platinating agent is at least one selected from the group consisting of cisplatin, carboplatin, and oxaliplatin.

[F-48]
The method according to [F-47], wherein the platinating agent is carboplatin.

[F-49]
The method according to [F-22], wherein the chemotherapeutic agent is an alkylating agent.

[F-50]
The method according to [F-22] or [F-49], wherein the alkylating agent is at least one selected from the group consisting of cyclophosphamide, ifosfamide, chlorambucil, melphalan, temozolomide, carmustine, lomustine, streptozocin, busulfan, procarbazine, dacarbazine, nimustine, ranimustine, bendamustine, altretamine, thiotepa, and mechlorethamine.

[F-51]
The method according to [F-22], wherein the chemotherapeutic agent is an antibody-drug conjugate.

[F-52]
The method according to [F-22] or [F-51], wherein the antibody-drug conjugate is at least one selected from the group consisting of trastuzumab deruxtecan, sacituzumab govitecan, tisotumab vedotin, enfortumab vedotin, trastuzumab emtansine, loncastuximab tesirine, moxetumomab pasudotox, belantamab mafodotin, polatuzumab vedotin, inotuzumab ozogamicin, brentuximab vedotin, and gemtuzumab ozogamicin.

[F-53]
The method according to [F-52], wherein the antibody-drug conjugate is at least one selected from the group consisting of trastuzumab deruxtecan and sacituzumab govitecan.

[F-54]
The method according to any one of [F-1] to [F-8], wherein the MYT1 inhibitor is a small interfering RNA (siRNA), and the chemotherapeutic agent is at least one selected from the group consisting of gemcitabine, pemetrexed, irinotecan, carboplatin, and trastuzumab deruxtecan.

[F-55]
The method according to any one of [F-1] to [F-8], wherein the MYT1 inhibitor is a small interfering RNA (siRNA), and the chemotherapeutic agent is gemcitabine.

[F-56]
The method according to any one of [F-1] to [F-8], wherein the MYT1 inhibitor is a small interfering RNA (siRNA), and the chemotherapeutic agent is fluorouracil.

[F-57]
The method according to any one of [F-1] to [F-8], wherein the MYT1 inhibitor is a small interfering RNA (siRNA), and the chemotherapeutic agent is pemetrexed.

[F-58]
The method according to any one of [F-1] to [F-8], wherein the MYT1 inhibitor is a small interfering RNA (siRNA), and the chemotherapeutic agent is etoposide.

[F-59]
The method according to any one of [F-1] to [F-8], wherein the MYT1 inhibitor is a small interfering RNA (siRNA), and the chemotherapeutic agent is irinotecan.

[F-60]
The method according to any one of [F-1] to [F-8], wherein the MYT1 inhibitor is a small interfering RNA (siRNA), and the chemotherapeutic agent is carboplatin.

[F-61]
The method according to any one of [F-1] to [F-8], wherein the MYT1 inhibitor is a small interfering RNA (siRNA), and the chemotherapeutic agent is trastuzumab deruxtecan.

[F-61.1]
The method according to any one of [F-1] to [F-8], wherein the MYT1 inhibitor is a compound represented by the formula (2) or a salt thereof, or a solvate thereof, and the chemotherapeutic agent is at least one selected from the group consisting of pemetrexed, gemcitabine, carboplatin, irinotecan, sacituzumab govitecan, DXd, and trastuzumab deruxtecan.

[F-61.11]
The method according to any one of [F-1] to [F-8], wherein the MYT1 inhibitor is a compound represented by the formula (2) or a salt thereof, or a solvate thereof, and the chemotherapeutic agent is pemetrexed.

[F-61.12]
The method according to any one of [F-1] to [F-8], wherein the MYT1 inhibitor is a compound represented by the formula (2) or a salt thereof, or a solvate thereof, and the chemotherapeutic agent is gemcitabine.

[F-61.13]
The method according to any one of [F-1] to [F-8], wherein the MYT1 inhibitor is a compound represented by the formula (2) or a salt thereof, or a solvate thereof, and the chemotherapeutic agent is carboplatin.

[F-61.14]
The method according to any one of [F-1] to [F-8], wherein the MYT1 inhibitor is a compound represented by the formula (2) or a salt thereof, or a solvate thereof, and the chemotherapeutic agent is irinotecan.

[F-61.15]
The method according to any one of [F-1] to [F-8], wherein the MYT1 inhibitor is a compound represented by the formula (2) or a salt thereof, or a solvate thereof, and the chemotherapeutic agent is sacituzumab govitecan.

[F-61.16]
The method according to any one of [F-1] to [F-8], wherein the MYT1 inhibitor is a compound represented by the formula (2) or a salt thereof, or a solvate thereof, and the chemotherapeutic agent is DXd.

[F-61.17]
The method according to any one of [F-1] to [F-8], wherein the MYT1 inhibitor is a compound represented by the formula (2) or a salt thereof, or a solvate thereof, and the chemotherapeutic agent is trastuzumab deruxtecan.

[F-61.2]
The method according to any one of [F-1] to [F-8], wherein the MYT1 inhibitor is a compound represented by the formula (3) or a salt thereof, or a solvate thereof, and the chemotherapeutic agent is at least one selected from the group consisting of pemetrexed, gemcitabine, carboplatin, irinotecan, sacituzumab govitecan, DXd, and trastuzumab deruxtecan.

[F-61.21]
The method according to any one of [F-1] to [F-8], wherein the MYT1 inhibitor is a compound represented by the formula (3) or a salt thereof, or a solvate thereof, and the chemotherapeutic agent is pemetrexed.

[F-61.22]
The method according to any one of [F-1] to [F-8], wherein the MYT1 inhibitor is a compound represented by the formula (3) or a salt thereof, or a solvate thereof, and the chemotherapeutic agent is gemcitabine.

[F-61.23]
The method according to any one of [F-1] to [F-8], wherein the MYT1 inhibitor is a compound represented by the formula (3) or a salt thereof, or a solvate thereof, and the chemotherapeutic agent is carboplatin.

[F-61.24]
The method according to any one of [F-1] to [F-8], wherein the MYT1 inhibitor is a compound represented by the formula (3) or a salt thereof, or a solvate thereof, and the chemotherapeutic agent is irinotecan.

[F-61.25]
The method according to any one of [F-1] to [F-8], wherein the MYT1 inhibitor is a compound represented by the formula (3) or a salt thereof, or a solvate thereof, and the chemotherapeutic agent is sacituzumab govitecan.

[F-61.26]
The method according to any one of [F-1] to [F-8], wherein the MYT1 inhibitor is a compound represented by the formula (3) or a salt thereof, or a solvate thereof, and the chemotherapeutic agent is DXd.

[F-61.27]
The method according to any one of [F-1] to [F-8], wherein the MYT1 inhibitor is a compound represented by the formula (3) or a salt thereof, or a solvate thereof, and the chemotherapeutic agent is trastuzumab deruxtecan.

[F-62]
The method according to any one of [F-1] to [F-61], wherein the MYT1 inhibitor and the chemotherapeutic agent are simultaneously or separately administered.

[F-63]
The method according to any one of [F-1] to [F-61], wherein the MYT1 inhibitor and the chemotherapeutic agent are administered as a combination drug.

[F-64]
The method according to any one of [F-1] to [F-63], wherein the cancer is solid cancer or blood cancer.

[F-65]
The method according to any one of [F-1] to [F-63], wherein the cancer is at least one selected from the group consisting of lung cancer, breast cancer, esophageal cancer, stomach cancer, bowel cancer, uterine cancer, ovarian cancer, pancreatic cancer, bladder cancer, thyroid cancer, skin cancer, head and neck cancer, kidney cancer, liver cancer, prostate cancer, adenoid cystic cancer, retinoblastoma, cerebral tumor, leukemia, malignant lymphoma, and multiple myeloma.

[F-66]
The method according to [F-65], wherein the cancer is at least one selected from the group consisting of lung cancer, breast cancer, bladder cancer, and ovarian cancer.

[F-67]
The method according to [F-65], wherein the cancer is lung cancer, breast cancer, or bladder cancer.

[F-68]
The method according to any one of [F-1] to [F-67], wherein the RB1 gene mutation positivity, or the decrease in expression of an RB1 gene or protein is detected in a cancer patient-derived biological sample.

[F-68.1]
The method according to any one of [F-1] to [F-67], wherein the positive expression of hyperphosphorylated RB1 protein is detected in a cancer patient-derived biological sample.

[F-69]
The method according to any one of [F-1] to [F-68], wherein the decrease in expression of an RB1 gene or protein is a decrease based on an expression level in a healthy individual-derived biological sample or a non-cancer tissue derived from the cancer patient.

[F-69.1]
The method according to any one of [F-1] to [F-68], wherein the positive expression of hyperphosphorylated RB1 protein is determined from an increase in expression of the hyperphosphorylated RB1 protein based on an expression level in a healthy individual-derived biological sample or a non-cancer tissue derived from the cancer patient.

[F-70]
The method according to any one of [F-1] to [F-69], wherein the patient is a patient in which amplification of a copy number of a CCNE1 gene is not detected.

[F-71]
The method according to any one of [F-1] to [F-70], wherein the above patient is not a mouse implanted with OVCAR3.

[F-72]
The method according to any one of [F-1] to [F-71], wherein the patient is human.

[G-1]
A method for screening a compound effective for treatment or prevention of cancer in a cancer patient in which RB1 gene mutation positivity, a decrease in expression of an RB1 gene or protein, or positive expression of hyperphosphorylated RB1 protein is detected, the method comprising:
measuring MYT1 inhibitory activity of a candidate compound; and
selecting a candidate compound having the MYT1 inhibitory activity as a compound effective for treatment of cancer.

[G-1.1]
A method for screening a compound effective for treatment or prevention of cancer in a cancer patient in which RB1 gene mutation positivity or a decrease in expression of an RB1 gene or protein is detected, the method comprising:
measuring MYT1 inhibitory activity of a candidate compound; and
selecting a candidate compound having the MYT1 inhibitory activity as a compound effective for treatment of cancer.

[G-1.2]
A method for screening a compound effective for treatment or prevention of cancer in a cancer patient in which positive expression of hyperphosphorylated RB1 protein is detected, the method comprising:
measuring MYT1 inhibitory activity of a candidate compound; and
selecting a candidate compound having the MYT1 inhibitory activity as a compound effective for treatment of cancer.

[G-2]
The method according to [G-1], wherein the RB1 gene mutation comprises a mutation causing insertion, substitution, deletion, and/or addition of at least one amino acid residue to wild-type RB1 protein.

[G-3]
The method according to [G-1] or [G-2], wherein the RB1 gene mutation is a nonsense mutation, a frameshift mutation, a splice site mutation, or a homozygous or heterozygous deletion.

[G-4]
The method according to any one of [G-1] to [G-3], wherein the RB1 gene mutation is a mutation decreasing a function of RB1.

[G-5]
The method according to any one of [G-1] to [G-4], wherein the RB1 gene mutation is a human RB1 gene mutation.

[G-6]
The method according to [G-5], wherein the human RB1 gene mutation is at least one of the following (1) to (5):
(1) a codon corresponding to a serine residue (S) at position 82 of an amino acid sequence of SEQ ID No: 2 is replaced with a stop codon,
(2) a codon corresponding to an arginine residue (R) at position 467 of the amino acid sequence of SEQ ID No: 2 is replaced with a stop codon,
(3) in a codon corresponding to an amino acid residue at position 182, at least one base is inserted or deleted, a new reading frame starting from an isoleucine residue (I) is formed, and a third reading frame therefrom is a stop codon,
(4) a glutamic acid residue (E) at position 837 of the amino acid sequence of SEQ ID No: 2 is replaced with a lysine residue (K), and a part of the RB1 gene is homozygously deleted, and
(5) a glycine residue (G) at position 449 of the amino acid sequence of SEQ ID No: 2 is replaced with a glutamic acid residue (E), and a part of the RB1 gene is homozygously deleted.

[G-7]
The method according to any one of [G-1] to [G-6], wherein the decrease in expression of an RB1 gene or protein comprises a decrease in gene expression through methylation of an RB 1 gene or a micro RNA.

[G-7.1]
The method according to any one of [G-1] to [G-6], wherein the hyperphosphorylated RB1 protein is RB1 protein having 3 or more phosphorylated amino acid residues in the amino acid sequence of the RB 1 protein.

[G-7.2]
The method according to any one of [G-1] to [G-6], wherein the hyperphosphorylated RB1 protein is RB1 protein having 4 or more phosphorylated amino acid residues in the amino acid sequence of the RB1 protein.

[G-7.3]
The method according to any one of [G-1] to [G-6], wherein the hyperphosphorylated RB1 protein is RB1 protein having 8 or more phosphorylated amino acid residues in the amino acid sequence of the RB1 protein.

[G-7.4]
The method according to any one of [G-1] to [G-6], wherein the hyperphosphorylated RB1 protein is RB1 protein having 15 or more phosphorylated amino acid residues in the amino acid sequence of the RB1 protein.

[G-7.5]
The method according to any one of [G-1] to [G-6], wherein the hyperphosphorylated RB1 protein is hyperphosphorylated human RB1 protein.

[G-7.6]
The method according to [G-7.5], wherein the hyperphosphorylated human RB1 protein is RB1 protein in which at least one amino acid residue selected from the group consisting of a threonine residue (T) at position 826, a threonine residue (T) at position 823, a threonine residue (T) at position 821, a serine residue (S) at position 816, a tyrosine residue (Y) at position 813, a serine residue (S) at position 811, a serine residue (S) at position 807, a tyrosine residue (Y) at position 805, a serine residue (S) at position 780, a threonine residue (T) at position 625, a threonine residue (T) at position 601, a threonine residue (T) at position 373, a serine residue (S) at position 360, a threonine residue (T) at position 356, a serine residue (S) at position 249, and a serine residue (S) at position 37 of SEQ ID No: 2 is phosphorylated.

[G-7.7]
The method according to [G-7.5], wherein the hyperphosphorylated human RB1 protein is RB1 protein in which at least one amino acid residue selected from the group consisting of a threonine residue (T) at position 826, a threonine residue (T) at position 821, a serine residue (S) at position 811, a serine residue (S) at position 807, a serine residue (S) at position 780, a threonine residue (T) at position 373, and a threonine residue (T) at position 356 of SEQ ID No: 2 is phosphorylated.

[G-7.8]
The method according to [G-7.5], wherein the hyperphosphorylated human RB1 protein is RB1 protein in which at least one amino acid residue selected from the group consisting of a threonine residue (T) at position 826, a threonine residue (T) at position 821, a serine residue (S) at position 811, and a serine residue (S) at position 807 of SEQ ID No: 2 is phosphorylated.

[G-7.9]
The method according to [G-7.5], wherein the hyperphosphorylated human RB1 protein is RB1 protein in which at least two amino acid residues selected from the group consisting of a threonine residue (T) at position 826, a threonine residue (T) at position 821, and a serine residue (S) at position 811 and/or a serine residue (S) at position 807 of SEQ ID No: 2 are phosphorylated.

[G-7.10]
The method according to [G-7.5], wherein the hyperphosphorylated human RB1 protein is RB1 protein in which a threonine residue (T) at position 826 and a threonine residue (T) at position 821 of SEQ ID No: 2 are phosphorylated and a serine residue (S) at position 811 and/or a serine residue (S) at position 807 are/is phosphorylated.

[G-8]
The method according to any one of [G-1] to [G-7], wherein the MYT1 inhibitor is at least one selected from the group consisting of a low molecular compound, a polypeptide, and a polynucleotide.

[G-9]
The method according to [G-8], wherein the MYT1 inhibitor is a low molecular compound.

[G-10]
The method according to [G-9], wherein the low molecular compound is a compound having a molecular weight of 2000 g/mol or less.

[G-11]
The method according to [G-9], wherein the low molecular compound is a compound having a molecular weight of 1000 g/mol or less.

[G-12]
The method according to [G-8], wherein the MYT1 inhibitor is a polypeptide.

[G-13]
The method according to [G-12], wherein the polypeptide comprises an antibody.

[G-14]
The method according to [G-12], wherein the polypeptide is an anti-MYT1 antibody.

[G-15]
The method according to [G-8], wherein the MYT1 inhibitor is a polynucleotide.

[G-16]
The method according to [G-15], wherein the polynucleotide is at least one selected from the group consisting of a ribozyme, an antisense molecule, an inhibitor oligonucleotide, an aptamer, a micro RNA, and a small interfering RNA (siRNA).

[G-17]
The method according to [G-15], wherein the polynucleotide is at least one selected from the group consisting of an antisense nucleic acid, a micro RNA, and a small interfering RNA (siRNA).

[G-18]
The method according to any one of [G-1] to [G-11], wherein the MYT1 inhibitor is a compound represented by the formula (1): 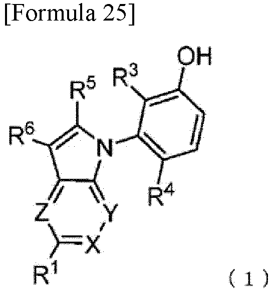
wherein X, Y, Z, R¹, R³, R⁴, R⁵*,* and R⁶ are as defined in the above [A-19],
or a salt thereof, or a solvate thereof.

[G-19]
The method according to [G-18], wherein the compound represented by the formula (1) comprises an atropisomer represented by the formula (1A) in a larger amount than each atropisomer: 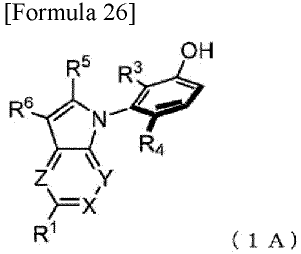 wherein X, Y, Z, R¹, R³, R⁴, R⁵*,* and R⁶ are as defined in the above [A-19].

[G-20]
The method according to any one of [G-1] to [G-19], wherein the MYT1 inhibitor is a compound represented by the formula (2): 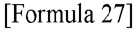 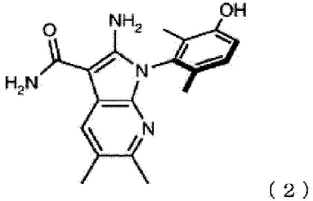 or a salt thereof, or a solvate thereof.

[G-20.5]
The method according to any one of [G-1] to [G-17], wherein the MYT1 inhibitor is a compound represented by the formula (3): 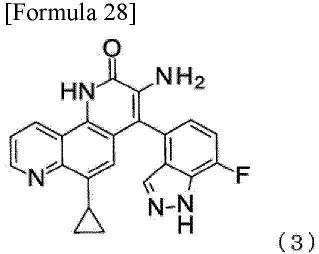 or a salt thereof, or a solvate thereof.

[G-21]
The method according to any one of [G-1] to [G-20.5], wherein the MYT1 inhibitor and the candidate compound are simultaneously or separately administered.

[G-22]
The method according to any one of [G-1] to [G-20], wherein the MYT1 inhibitor and the candidate compound are administered as a combination drug.

[G-23]
The method according to any one of [G-1] to [G-22], wherein the cancer is solid cancer or blood cancer.

[G-24]
The method according to any one of [G-1] to [G-22], wherein the cancer is at least one selected from the group consisting of lung cancer, breast cancer, esophageal cancer, stomach cancer, bowel cancer, uterine cancer, ovarian cancer, pancreatic cancer, bladder cancer, thyroid cancer, skin cancer, head and neck cancer, kidney cancer, liver cancer, prostate cancer, adenoid cystic cancer, retinoblastoma, cerebral tumor, leukemia, malignant lymphoma, and multiple myeloma.

[G-25]
The method according to [G-24], wherein the cancer is at least one selected from the group consisting of lung cancer, breast cancer, bladder cancer, and ovarian cancer.

[G-26]
The method according to [G-24], wherein the cancer is lung cancer, breast cancer, or bladder cancer.

[G-27]
The method according to any one of [G-1] to [G-26], wherein the RB1 gene mutation positivity, or the decrease in expression of an RB1 gene or protein is detected in a cancer patient-derived biological sample.

[G-27.1]
The method according to any one of [G-1] to [G-26], wherein the positive expression of hyperphosphorylated RB1 protein is detected in a cancer patient-derived biological sample.

[G-28]
The method according to any one of [G-1] to [G-27], wherein the decrease in expression of an RB1 gene or protein is a decrease based on an expression level in a healthy individual-derived biological sample or a non-cancer tissue derived from the cancer patient.

[G-28.1]
The method according to any one of [G-1] to [G-27], wherein the positive expression of hyperphosphorylated RB1 protein is determined from an increase in expression of the hyperphosphorylated RB1 protein based on an expression level in a healthy individual-derived biological sample or a non-cancer tissue derived from the cancer patient.

[G-29]
The method according to [G-27], wherein the cancer patient-derived biological sample is cancer cells.

[G-30]
The method according to any one of [G-1] to [G-29], wherein the patient is a patient in which amplification of a copy number of a CCNE1 gene is not detected.

[G-31]
The method according to any one of [G-1] to [G-30], wherein the above patient is not a mouse implanted with OVCAR3.

[G-32]
The method according to any one of [G-1] to [G-31], wherein the patient is human.

[H-1]
An MYT1 inhibitor for use in combination with a chemotherapeutic agent in treatment or prevention of cancer in a cancer patient in which RB1 gene mutation positivity, a decrease in expression of an RB1 gene or protein, or positive expression of hyperphosphorylated RB1 protein is detected.

[H-1.1]
An MYT1 inhibitor for use in combination with a chemotherapeutic agent in treatment or prevention of cancer in a cancer patient in which RB1 gene mutation positivity or a decrease in expression of an RB1 gene or protein is detected.

[H-1.2]
An MYT1 inhibitor for use in combination with a chemotherapeutic agent in treatment or prevention of cancer in a cancer patient in which positive expression of hyperphosphorylated RB1 protein is detected.

[H-2]
The MYT1 inhibitor according to [H-1], wherein the RB1 gene mutation comprises a mutation causing insertion, substitution, deletion, and/or addition of at least one amino acid residue to wild-type RB1 protein.

[H-3]
The MYT1 inhibitor according to [H-1] or [H-2], wherein the RB1 gene mutation is a nonsense mutation, a frameshift mutation, a splice site mutation, or a homozygous or heterozygous deletion.

[H-4]
The MYT1 inhibitor according to any one of [H-1] to [H-3], wherein the RB1 gene mutation is a mutation decreasing a function of RB1.

[H-5]
The MYT1 inhibitor according to any one of [H-1] to [H-4], wherein the RB1 gene mutation is a human RB1 gene mutation.

[H-6]
The MYT1 inhibitor according to [H-5], wherein the human RB1 gene mutation is at least one of the following (1) to (5):
(1) a codon corresponding to a serine residue (S) at position 82 of an amino acid sequence of SEQ ID No: 2 is replaced with a stop codon,
(2) a codon corresponding to an arginine residue (R) at position 467 of the amino acid sequence of SEQ ID No: 2 is replaced with a stop codon,
(3) in a codon corresponding to an amino acid residue at position 182, at least one base is inserted or deleted, a new reading frame starting from an isoleucine residue (I) is formed, and a third reading frame therefrom is a stop codon,
(4) a glutamic acid residue (E) at position 837 of the amino acid sequence of SEQ ID No: 2 is replaced with a lysine residue (K), and a part of the RB1 gene is homozygously deleted, and
(5) a glycine residue (G) at position 449 of the amino acid sequence of SEQ ID No: 2 is replaced with a glutamic acid residue (E), and a part of the RB1 gene is homozygously deleted.

[H-7]
The MYT1 inhibitor according to any one of [H-1] to [H-6], wherein the decrease in expression of an RB1 gene or protein comprises a decrease in gene expression through methylation of an RB1 gene or a micro RNA.

[H-7.1]
The MYT1 inhibitor according to any one of [H-1] to [H-6], wherein the hyperphosphorylated RB1 protein is RB1 protein having 3 or more phosphorylated amino acid residues in the amino acid sequence of the RB1 protein.

[H-7.2]
The MYT1 inhibitor according to any one of [H-1] to [H-6], wherein the hyperphosphorylated RB1 protein is RB1 protein having 4 or more phosphorylated amino acid residues in the amino acid sequence of the RB1 protein.

[H-7.3]
The MYT1 inhibitor according to any one of [H-1] to [H-6], wherein the hyperphosphorylated RB1 protein is RB1 protein having 8 or more phosphorylated amino acid residues in the amino acid sequence of the RB1 protein.

[H-7.4]
The MYT1 inhibitor according to any one of [H-1] to [H-6], wherein the hyperphosphorylated RB1 protein is RB1 protein having 15 or more phosphorylated amino acid residues in the amino acid sequence of the RB1 protein.

[H-7.5]
The MYT1 inhibitor according to any one of [H-1] to [H-6], wherein the hyperphosphorylated RB1 protein is hyperphosphorylated human RB1 protein.

[H-7.6]
The MYT1 inhibitor according to [H-7.5], wherein the hyperphosphorylated human RB1 protein is RB1 protein in which at least one amino acid residue selected from the group consisting of a threonine residue (T) at position 826, a threonine residue (T) at position 823, a threonine residue (T) at position 821, a serine residue (S) at position 816, a tyrosine residue (Y) at position 813, a serine residue (S) at position 811, a serine residue (S) at position 807, a tyrosine residue (Y) at position 805, a serine residue (S) at position 780, a threonine residue (T) at position 625, a threonine residue (T) at position 601, a threonine residue (T) at position 373, a serine residue (S) at position 360, a threonine residue (T) at position 356, a serine residue (S) at position 249, and a serine residue (S) at position 37 of SEQ ID No: 2 is phosphorylated.

[H-7.7]
The MYT1 inhibitor according to [H-7.5], wherein the hyperphosphorylated human RB1 protein is RB1 protein in which at least one amino acid residue selected from the group consisting of a threonine residue (T) at position 826, a threonine residue (T) at position 821, a serine residue (S) at position 811, a serine residue (S) at position 807, a serine residue (S) at position 780, a threonine residue (T) at position 373, and a threonine residue (T) at position 356 of SEQ ID No: 2 is phosphorylated.

[H-7.8]
The MYT1 inhibitor according to [H-7.5], wherein the hyperphosphorylated human RB1 protein is RB1 protein in which at least one amino acid residue selected from the group consisting of a threonine residue (T) at position 826, a threonine residue (T) at position 821, a serine residue (S) at position 811, and a serine residue (S) at position 807 of SEQ ID No: 2 is phosphorylated.

[H-7.9]
The MYT1 inhibitor according to [H-7.5], wherein the hyperphosphorylated human RB1 protein is RB1 protein in which at least two amino acid residues selected from the group consisting of a threonine residue (T) at position 826, a threonine residue (T) at position 821, and a serine residue (S) at position 811 and/or a serine residue (S) at position 807 of SEQ ID No: 2 are phosphorylated.

[H-7.10]
The MYT1 inhibitor according to [H-7.5], wherein the hyperphosphorylated human RB1 protein is RB1 protein in which a threonine residue (T) at position 826 and a threonine residue (T) at position 821 of SEQ ID No: 2 are phosphorylated, and a serine residue (S) at position 811 and/or a serine residue (S) at position 807 are/is phosphorylated.

[H-8]
The MYT1 inhibitor according to any one of [H-1] to [H-7], wherein the MYT1 inhibitor is at least one selected from the group consisting of a low molecular compound, a polypeptide, and a polynucleotide.

[H-9]
The MYT1 inhibitor according to [H-8], wherein the MYT1 inhibitor is a low molecular compound.

[H-10]
The MYT1 inhibitor according to [H-9], wherein the low molecular compound is a compound having a molecular weight of 2000 g/mol or less.

[H-11]
The MYT1 inhibitor according to [H-9], wherein the low molecular compound is a compound having a molecular weight of 1000 g/mol or less.

[H-12]
The MYT1 inhibitor according to [H-8], wherein the MYT1 inhibitor is a polypeptide.

[H-13]
The MYT1 inhibitor according to [H-12], wherein the polypeptide comprises an antibody.

[H-14]
The MYT1 inhibitor according to [H-12], wherein the MYT1 inhibitor is an anti-MYT1 antibody.

[H-15]
The MYT1 inhibitor according to [H-8], wherein the MYT1 inhibitor is a polynucleotide.

[H-16]
The MYT1 inhibitor according to [H-15], wherein the polynucleotide is at least one polynucleotide selected from the group consisting of a ribozyme, an antisense molecule, an inhibitor oligonucleotide, an aptamer, a micro RNA, and a small interfering RNA (siRNA).

[H-17]
The MYT1 inhibitor according to [H-15], wherein the polynucleotide is at least one polynucleotide selected from the group consisting of an antisense nucleic acid, a micro RNA, and a small interfering RNA (siRNA).

[H-18]
The MYT1 inhibitor according to any one of [H-1] to [H-11], wherein the MYT1 inhibitor is a compound represented by the formula (1): 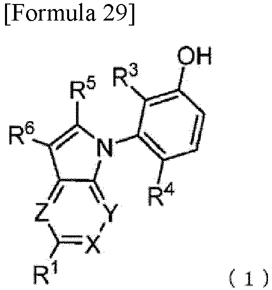
wherein X, Y, Z, R¹, R³, R⁴, R⁵, and R⁶ are as defined in the above [A-19],
or a salt thereof, or a solvate thereof.

[H-19]
The MYT1 inhibitor according to [H-18], wherein the compound represented by the formula (1) comprises an atropisomer represented by the formula (1A) in a larger amount than each atropisomer: 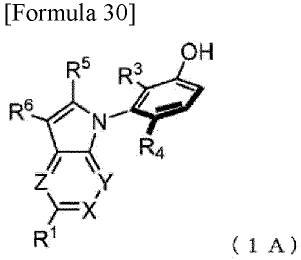 wherein X, Y, Z, R¹, R³, R⁴, R⁵, and R⁶ are as defined in the above [A-19].

[H-20]
The MYT1 inhibitor according to any one of [H-1] to [H-19], wherein the MYT1 inhibitor is a compound represented by the formula (2): 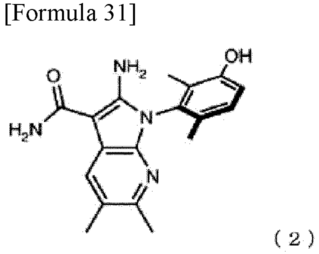 or a salt thereof, or a solvate thereof.

[H-20.5]
The MYT1 inhibitor according to any one of [H-1] to [H-17], wherein the MYT1 inhibitor is a compound represented by the formula (3): 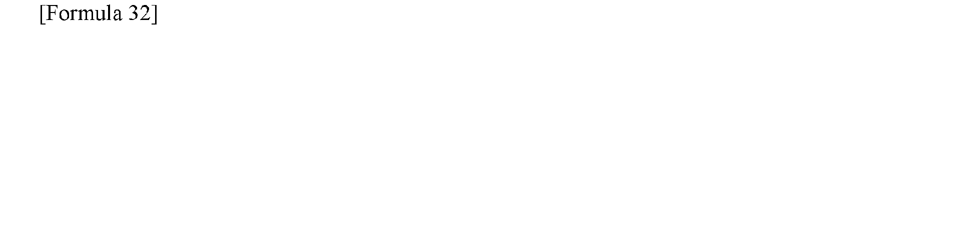 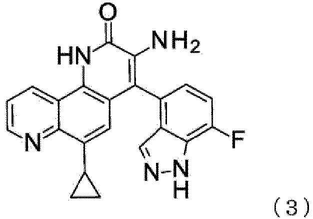 or a salt thereof, or a solvate thereof.

[H-21]
The MYT1 inhibitor according to any one of [H-1] to [H-20.5], wherein the chemotherapeutic agent is at least one selected from the group consisting of an antimetabolite, an anticancer antibiotic, a mitosis inhibitor, a topoisomerase inhibitor, a platinating agent, an alkylating agent, and an antibody-drug conjugate.

[H-22]
The MYT1 inhibitor according to [H-21], wherein the chemotherapeutic agent is an antimetabolite.

[H-23]
The MYT1 inhibitor according to [H-21] or [H-22], wherein the antimetabolite is at least one selected from the group consisting of a purine antimetabolite, a pyrimidine antimetabolite, a folic acid antimetabolite, and a ribonucleotide reductase inhibitor.

[H-24]
The MYT1 inhibitor according to [H-21], wherein the antimetabolite is a purine antimetabolite.

[H-25]
The MYT1 inhibitor according to [H-23] or [H-24], wherein the purine antimetabolite is at least one selected from the group consisting of 6-thioguanine, 6-mercaptopurine, azathioprine, fludarabine, pentostatin, cladribine, clofarabine, and nelarabine.

[H-26]
The MYT1 inhibitor according to [H-21], wherein the antimetabolite is a pyrimidine antimetabolite.

[H-27]
The MYT1 inhibitor according to [H-23] or [H-26], wherein the pyrimidine antimetabolite is at least one selected from the group consisting of gemcitabine, cytarabine, fluorouracil, capecitabine, tegafur, azacitidine, trifluridine, and floxuridine.

[H-28]
The MYT1 inhibitor according to [H-27], wherein the pyrimidine antimetabolite is gemcitabine.

[H-29]
The MYT1 inhibitor according to [H-21], wherein the antimetabolite is a folic acid antimetabolite.

[H-30]
The MYT1 inhibitor according to [H-23] or [H-29], wherein the folic acid antimetabolite is at least one selected from the group consisting of pemetrexed and methotrexate.

[H-31]
The MYT1 inhibitor according to [H-30], wherein the folic acid antimetabolite is pemetrexed.

[H-32]
The MYT1 inhibitor according to [H-21], wherein the antimetabolite is a ribonucleotide reductase inhibitor.

[H-33]
The MYT1 inhibitor according to [H-23] or [H-32], wherein the ribonucleotide reductase inhibitor is hydroxyurea.

[H-34]
The MYT1 inhibitor according to [H-21], wherein the chemotherapeutic agent is an anticancer antibiotic.

[H-35]
The MYT1 inhibitor according to [H-21] or [H-34], wherein the anticancer antibiotic is at least one selected from the group consisting of bleomycin, actinomycin D, doxorubicin, daunorubicin, idarubicin, mitomycin, mitoxantrone, epirubicin, aclarubicin, and valrubicin.

[H-36]
The MYT1 inhibitor according to [H-21], wherein the chemotherapeutic agent is a mitosis inhibitor.

[H-37]
The MYT1 inhibitor according to [H-21] or [H-36], wherein the mitosis inhibitor is at least one selected from the group consisting of vinca alkaloid and a microtubule inhibitor.

[H-38]
The MYT1 inhibitor according to [H-37], wherein the mitosis inhibitor is vinca alkaloid.

[H-39]
The MYT1 inhibitor according to [H-37] or [H-38], wherein the vinca alkaloid is at least one selected from the group consisting of vincristine, vinblastine, and vinorelbine.

[H-40]
The MYT1 inhibitor according to [H-37], wherein the mitosis inhibitor is a microtubule inhibitor.

[H-41]
The MYT1 inhibitor according to [H-37] or [H-40], wherein the microtubule inhibitor is at least one selected from the group consisting of docetaxel, paclitaxel, eribulin, ixabepilone, and epothilone.

[H-42]
The MYT1 inhibitor according to [H-21], wherein the chemotherapeutic agent is a topoisomerase inhibitor.

[H-43]
The MYT1 inhibitor according to [H-21] or [H-42], wherein the topoisomerase inhibitor is at least one selected from the group consisting of topotecan, irinotecan, DXd, etoposide, and teniposide.

[H-44]
The MYT1 inhibitor according to [H-43], wherein the topoisomerase inhibitor is at least one selected from the group consisting of irinotecan and DXd.

[H-45]
The MYT1 inhibitor according to [H-21], wherein the chemotherapeutic agent is a platinating agent.

[H-46]
The MYT1 inhibitor according to [H-21] or [H-45], wherein the platinating agent is at least one selected from the group consisting of cisplatin, carboplatin, and oxaliplatin.

[H-47]
The MYT1 inhibitor according to [H-46], wherein the platinating agent is carboplatin.

[H-48]
The MYT1 inhibitor according to [H-21], wherein the chemotherapeutic agent is an alkylating agent.

[H-49]
The MYT1 inhibitor according to [H-21] or [H-48], wherein the alkylating agent is at least one selected from the group consisting of cyclophosphamide, ifosfamide, chlorambucil, melphalan, temozolomide, carmustine, lomustine, streptozocin, busulfan, procarbazine, dacarbazine, nimustine, ranimustine, bendamustine, altretamine, thiotepa, and mechlorethamine.

[H-50]
The MYT1 inhibitor according to [H-21], wherein the chemotherapeutic agent is an antibody-drug conjugate.

[H-51]
The MYT1 inhibitor according to [H-21] or [H-50], wherein the antibody-drug conjugate is at least one selected from the group consisting of trastuzumab deruxtecan, sacituzumab govitecan, tisotumab vedotin, enfortumab vedotin, trastuzumab emtansine, loncastuximab tesirine, moxetumomab pasudotox, belantamab mafodotin, polatuzumab vedotin, inotuzumab ozogamicin, brentuximab vedotin, and gemtuzumab ozogamicin.

[H-52]
The MYT1 inhibitor according to [H-51], wherein the antibody-drug conjugate is at least one selected from the group consisting of trastuzumab deruxtecan and sacituzumab govitecan.

[H-53]
The MYT1 inhibitor according to any one of [H-1] to [H-7], wherein the chemotherapeutic agent is at least one selected from the group consisting of gemcitabine, pemetrexed, irinotecan, carboplatin, and trastuzumab deruxtecan.

[H-54]
The MYT1 inhibitor according to any one of [H-1] to [H-7], wherein the MYT1 inhibitor is a small interfering RNA (siRNA), and the chemotherapeutic agent is gemcitabine.

[H-55]
The MYT1 inhibitor according to any one of [H-1] to [H-7], wherein the MYT1 inhibitor is a small interfering RNA (siRNA), and the chemotherapeutic agent is fluorouracil.

[H-56]
The MYT1 inhibitor according to any one of [H-1] to [H-7], wherein the MYT1 inhibitor is a small interfering RNA (siRNA), and the chemotherapeutic agent is pemetrexed.

[H-57]
The MYT1 inhibitor according to any one of [H-1] to [H-7], wherein the MYT1 inhibitor is a small interfering RNA (siRNA), and the chemotherapeutic agent is etoposide.

[H-58]
The MYT1 inhibitor according to any one of [H-1] to [H-7], wherein the MYT1 inhibitor is a small interfering RNA (siRNA), and the chemotherapeutic agent is irinotecan.

[H-59]
The MYT1 inhibitor according to any one of [H-1] to [H-7], wherein the MYT1 inhibitor is a small interfering RNA (siRNA), and the chemotherapeutic agent is carboplatin.

[H-60]
The MYT1 inhibitor according to any one of [H-1] to [H-7], wherein the MYT1 inhibitor is a small interfering RNA (siRNA), and the chemotherapeutic agent is trastuzumab deruxtecan.

[H-60.1]
The MYT1 inhibitor according to any one of [H-1] to [H-7], wherein the MYT1 inhibitor is a compound represented by the formula (2) or a salt thereof, or a solvate thereof, and the chemotherapeutic agent is at least one selected from the group consisting of pemetrexed, gemcitabine, carboplatin, irinotecan, sacituzumab govitecan, DXd, and trastuzumab deruxtecan.

[H-60.11]
The MYT1 inhibitor according to any one of [H-1] to [H-7], wherein the MYT1 inhibitor is a compound represented by the formula (2) or a salt thereof, or a solvate thereof, and the chemotherapeutic agent is pemetrexed.

[H-60.12]
The MYT1 inhibitor according to any one of [H-1] to [H-7], wherein the MYT1 inhibitor is a compound represented by the formula (2) or a salt thereof, or a solvate thereof, and the chemotherapeutic agent is gemcitabine.

[H-60.13]
The MYT1 inhibitor according to any one of [H-1] to [H-7], wherein the MYT1 inhibitor is a compound represented by the formula (2) or a salt thereof, or a solvate thereof, and the chemotherapeutic agent is carboplatin.

[H-60.14]
The MYT1 inhibitor according to any one of [H-1] to [H-7], wherein the MYT1 inhibitor is a compound represented by the formula (2) or a salt thereof, or a solvate thereof, and the chemotherapeutic agent is irinotecan.

[H-60.15]
The MYT1 inhibitor according to any one of [H-1] to [H-7], wherein the MYT1 inhibitor is a compound represented by the formula (2) or a salt thereof, or a solvate thereof, and the chemotherapeutic agent is sacituzumab govitecan.

[H-60.16]
The MYT1 inhibitor according to any one of [H-1] to [H-7], wherein the MYT1 inhibitor is a compound represented by the formula (2) or a salt thereof, or a solvate thereof, and the chemotherapeutic agent is DXd.

[H-60.17]
The MYT1 inhibitor according to any one of [H-1] to [H-7], wherein the MYT1 inhibitor is a compound represented by the formula (2) or a salt thereof, or a solvate thereof, and the chemotherapeutic agent is trastuzumab deruxtecan.

[H-60.2]
The MYT1 inhibitor according to any one of [H-1] to [H-7], wherein the MYT1 inhibitor is a compound represented by the formula (3) or a salt thereof, or a solvate thereof, and the chemotherapeutic agent is at least one selected from the group consisting of pemetrexed, gemcitabine, carboplatin, irinotecan, sacituzumab govitecan, DXd, and trastuzumab deruxtecan.

[H-60.21]
The MYT1 inhibitor according to any one of [H-1] to [H-7], wherein the MYT1 inhibitor is a compound represented by the formula (3) or a salt thereof, or a solvate thereof, and the chemotherapeutic agent is pemetrexed.

[H-60.22]
The MYT1 inhibitor according to any one of [H-1] to [H-7], wherein the MYT1 inhibitor is a compound represented by the formula (3) or a salt thereof, or a solvate thereof, and the chemotherapeutic agent is gemcitabine.

[H-60.23]
The MYT1 inhibitor according to any one of [H-1] to [H-7], wherein the MYT1 inhibitor is a compound represented by the formula (3) or a salt thereof, or a solvate thereof, and the chemotherapeutic agent is carboplatin.

[H-60.24]
The MYT1 inhibitor according to any one of [H-1] to [H-7], wherein the MYT1 inhibitor is a compound represented by the formula (3) or a salt thereof, or a solvate thereof, and the chemotherapeutic agent is irinotecan.

[H-60.25]
The MYT1 inhibitor according to any one of [H-1] to [H-7], wherein the MYT1 inhibitor is a compound represented by the formula (3) or a salt thereof, or a solvate thereof, and the chemotherapeutic agent is sacituzumab govitecan.

[H-60.26]
The MYT1 inhibitor according to any one of [H-1] to [H-7], wherein the MYT1 inhibitor is a compound represented by the formula (3) or a salt thereof, or a solvate thereof, and the chemotherapeutic agent is DXd.

[H-60.27]
The MYT1 inhibitor according to any one of [H-1] to [H-7], wherein the MYT1 inhibitor is a compound represented by the formula (3) or a salt thereof, or a solvate thereof, and the chemotherapeutic agent is trastuzumab deruxtecan.

[H-61]
The MYT1 inhibitor according to any one of [H-1] to [H-60], wherein the MYT1 inhibitor and the chemotherapeutic agent are simultaneously or separately administered.

[H-62]
The MYT1 inhibitor according to any one of [H-1] to [H-59], wherein the MYT1 inhibitor and the chemotherapeutic agent are administered as a combination drug.

[H-63]
The MYT1 inhibitor according to any one of [H-1] to [H-62], wherein the cancer is solid cancer or blood cancer.

[H-64]
The MYT1 inhibitor according to any one of [H-1] to [H-62], wherein the cancer is at least one selected from the group consisting of lung cancer, breast cancer, esophageal cancer, stomach cancer, bowel cancer, uterine cancer, ovarian cancer, pancreatic cancer, bladder cancer, thyroid cancer, skin cancer, head and neck cancer, kidney cancer, liver cancer, prostate cancer, adenoid cystic cancer, retinoblastoma, cerebral tumor, leukemia, malignant lymphoma, and multiple myeloma.

[H-65]
The MYT1 inhibitor according to [H-64], wherein the cancer is at least one selected from the group consisting of lung cancer, breast cancer, bladder cancer, and ovarian cancer.

[H-66]
The MYT1 inhibitor according to [H-65], wherein the cancer is lung cancer, breast cancer, or bladder cancer.

[H-67]
The MYT1 inhibitor according to any one of [H-1] to [H-66], wherein the RB1 gene mutation positivity, or the decrease in expression of an RB1 gene or protein is detected in a cancer patient-derived biological sample.

[H-67.1]
The MYT1 inhibitor according to any one of [H-1] to [H-66], wherein the positive expression of hyperphosphorylated RB1 protein is detected in a cancer patient-derived biological sample.

[H-68]
The MYT1 inhibitor according to [H-67], wherein the cancer patient-derived biological sample is cancer cells.

[H-69]
The MYT1 inhibitor according to any one of [H-1] to [H-68], wherein the decrease in expression of an RB1 gene or protein is a decrease based on an expression level in a healthy individual-derived biological sample or a non-cancer tissue derived from the cancer patient.

[H-69.1]
The MYT1 inhibitor according to any one of [H-1] to [H-68], wherein the positive expression of hyperphosphorylated RB1 protein is determined from an increase in expression of the hyperphosphorylated RB1 protein based on an expression level in a healthy individual-derived biological sample or a non-cancer tissue derived from the cancer patient.

[H-70]
The MYT1 inhibitor according to any one of [H-1] to [H-69], wherein the patient is a patient in which amplification of a copy number of a CCNE1 gene is not detected.

[H-71]
The MYT1 inhibitor according to any one of [H-1] to [H-70], wherein the above patient is not a mouse implanted with OVCAR3.

[H-72]
The MYT1 inhibitor according to any one of [H-1] to [H-71], wherein the patient is human.

[I-1]
A chemotherapeutic agent for use in combination with an MYT1 inhibitor in treatment or prevention of cancer in a cancer patient in which RB1 gene mutation positivity, a decrease in expression of an RB1 gene or protein, or positive expression of hyperphosphorylated RB1 protein is detected.

[I-1.1]
A chemotherapeutic agent for use in combination with an MYT1 inhibitor in cancer treatment or prevention in a cancer patient in which RB1 gene mutation positivity or a decrease in expression of an RB1 gene or protein is detected.

[I-1.2]
A chemotherapeutic agent for use in combination with an MYT1 inhibitor in treatment or prevention of cancer in a cancer patient in which positive expression of hyperphosphorylated RB1 protein is detected.

[I-2]
The chemotherapeutic agent according to [1-1], wherein the RB1 gene mutation comprises a mutation causing insertion, substitution, deletion, and/or addition of at least one amino acid residue to wild-type RB1 protein.

[I-3]
The chemotherapeutic agent according to [I-1] or [I-2], wherein the RB1 gene mutation is a nonsense mutation, a frameshift mutation, a splice site mutation, or a homozygous or heterozygous deletion.

[I-4]
The chemotherapeutic agent according to any one of [I-1] to [I-3], wherein the RB1 gene mutation is a mutation decreasing a function of RB1.

[I-5]
The chemotherapeutic agent according to any one of [I-1] to [I-4], wherein the RB1 gene mutation is a human RB1 gene mutation.

[I-6]
The chemotherapeutic agent according to [I-5], wherein the human RB1 gene mutation is at least one of the following (1) to (5):
(1) a codon corresponding to a serine residue (S) at position 82 of an amino acid sequence of SEQ ID No: 2 is replaced with a stop codon,
(2) a codon corresponding to an arginine residue (R) at position 467 of the amino acid sequence of SEQ ID No: 2 is replaced with a stop codon,
(3) in a codon corresponding to an amino acid residue at position 182, at least one base is inserted or deleted, a new reading frame starting from an isoleucine residue (I) is formed, and a third reading frame therefrom is a stop codon,
(4) a glutamic acid residue (E) at position 837 of the amino acid sequence of SEQ ID No: 2 is replaced with a lysine residue (K), and a part of the RB1 gene is homozygously deleted, and
(5) a glycine residue (G) at position 449 of the amino acid sequence of SEQ ID No: 2 is replaced with a glutamic acid residue (E), and a part of the RB1 gene is homozygously deleted.

[I-7]
The chemotherapeutic agent according to any one of [I-1] to [I-6], wherein the decrease in expression of an RB1 gene or protein comprises a decrease in gene expression through methylation of an RB1 gene or a micro RNA.

[I-7.1]
The chemotherapeutic agent according to any one of [I-1] to [I-6], wherein the hyperphosphorylated RB1 protein is RB1 protein having 3 or more phosphorylated amino acid residues in the amino acid sequence of the RB1 protein.

[I-7.2]
The chemotherapeutic agent according to any one of [I-1] to [I-6], wherein the hyperphosphorylated RB1 protein is RB1 protein having 4 or more phosphorylated amino acid residues in the amino acid sequence of the RB1 protein.

[I-7.3]
The chemotherapeutic agent according to any one of [I-1] to [I-6], wherein the hyperphosphorylated RB1 protein is RB1 protein having 8 or more phosphorylated amino acid residues in the amino acid sequence of the RB1 protein.

[I-7.4]
The chemotherapeutic agent according to any one of [I-1] to [I-6], wherein the hyperphosphorylated RB1 protein is RB1 protein having 15 or more phosphorylated amino acid residues in the amino acid sequence of the RB1 protein.

[I-7.5]
The chemotherapeutic agent according to any one of [I-1] to [I-6], wherein the hyperphosphorylated RB1 protein is hyperphosphorylated human RB1 protein.

[I-7.6]
The chemotherapeutic agent according to [I-7.5], wherein the hyperphosphorylated human RB1 protein is RB1 protein in which at least one amino acid residue selected from the group consisting of a threonine residue (T) at position 826, a threonine residue (T) at position 823, a threonine residue (T) at position 821, a serine residue (S) at position 816, a tyrosine residue (Y) at position 813, a serine residue (S) at position 811, a serine residue (S) at position 807, a tyrosine residue (Y) at position 805, a serine residue (S) at position 780, a threonine residue (T) at position 625, a threonine residue (T) at position 601, a threonine residue (T) at position 373, a serine residue (S) at position 360, a threonine residue (T) at position 356, a serine residue (S) at position 249, and a serine residue (S) at position 37 of SEQ ID No: 2 is phosphorylated.

[I-7.7]
The chemotherapeutic agent according to [I-7.5], wherein the hyperphosphorylated human RB1 protein is RB1 protein in which at least one amino acid residue selected from the group consisting of a threonine residue (T) at position 826, a threonine residue (T) at position 821, a serine residue (S) at position 811, a serine residue (S) at position 807, a serine residue (S) at position 780, a threonine residue (T) at position 373, and a threonine residue (T) at position 356 of SEQ ID No: 2 is phosphorylated.

[I-7.8]
The chemotherapeutic agent according to [I-7.5], wherein the hyperphosphorylated human RB1 protein is RB1 protein in which at least one amino acid residue selected from the group consisting of a threonine residue (T) at position 826, a threonine residue (T) at position 821, a serine residue (S) at position 811, and a serine residue (S) at position 807 of SEQ ID No: 2 is phosphorylated.

[I-7.9]
The chemotherapeutic agent according to [I-7.5], wherein the hyperphosphorylated human RB1 protein is RB1 protein in which at least two amino acid residues selected from the group consisting of a threonine residue (T) at position 826, a threonine residue (T) at position 821, and a serine residue (S) at position 811 and/or a serine residue (S) at position 807 of SEQ ID No: 2 are phosphorylated.

[I-7.10]
The chemotherapeutic agent according to [I-7.5], wherein the hyperphosphorylated human RB1 protein is RB1 protein in which a threonine residue (T) at position 826 and a threonine residue (T) at position 821 of SEQ ID No: 2 are phosphorylated, and a serine residue (S) at position 811 and/or a serine residue (S) at position 807 are/is phosphorylated.

[I-8]
The chemotherapeutic agent according to any one of [I-1] to [I-7], wherein the MYT1 inhibitor is at least one selected from the group consisting of a low molecular compound, a polypeptide, and a polynucleotide.

[I-9]
The chemotherapeutic agent according to [I-8], wherein the MYT1 inhibitor is a low molecular compound.

[I-10]
The chemotherapeutic agent according to [I-9], wherein the low molecular compound is a compound having a molecular weight of 2000 g/mol or less.

[I-11]
The chemotherapeutic agent according to [I-9], wherein the low molecular compound is a compound having a molecular weight of 1000 g/mol or less.

[I-12]
The chemotherapeutic agent according to [I-8], wherein the MYT1 inhibitor is a polypeptide.

[I-13]
The chemotherapeutic agent according to [I-12], wherein the polypeptide comprises an antibody.

[I-14]
The chemotherapeutic agent according to [1-12], wherein the polypeptide is an anti-MYT1 antibody.

[I-15]
The chemotherapeutic agent according to [I-8], wherein the MYT1 inhibitor is a polynucleotide.

[I-16]
The chemotherapeutic agent according to [I-15], wherein the polynucleotide is at least one selected from the group consisting of a ribozyme, an antisense molecule, an inhibitor oligonucleotide, an aptamer, a micro RNA, and a small interfering RNA (siRNA).

[I-17]
The chemotherapeutic agent according to [I-15], wherein the polynucleotide is at least one selected from the group consisting of an antisense nucleic acid, a micro RNA, and a small interfering RNA (siRNA).

[I-18]
The chemotherapeutic agent according to any one of [I-1] to [I-11], wherein the MYT1 inhibitor is a compound represented by the formula (1): 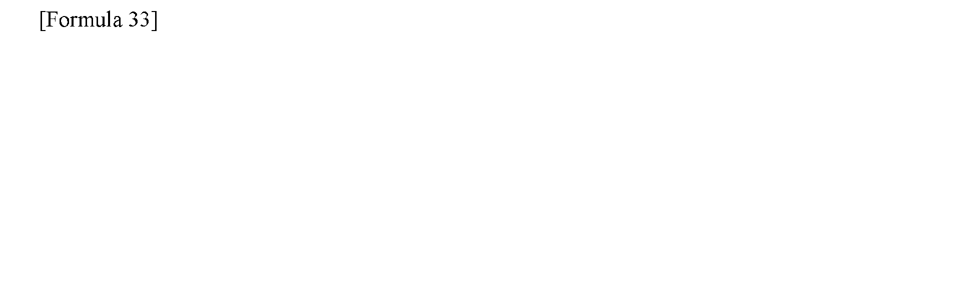 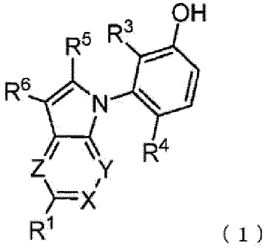
wherein X, Y, Z, R¹, R³, R⁴, R⁵, and R⁶ are as defined in the above [A-19],
or a salt thereof, or a solvate thereof.

[I-19]
The chemotherapeutic agent according to [I-18], wherein the compound represented by the formula (1) comprises an atropisomer represented by the formula (1A) in a larger amount than each atropisomer: 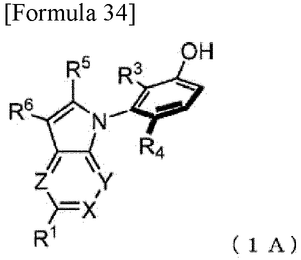 wherein X, Y, Z, R¹, R³, R⁴, R⁵, and R⁶ are as defined in the above [A-19].

[I-20]
The chemotherapeutic agent according to any one of [I-1] to [I-19], wherein the MYT1 inhibitor is a compound represented by the formula (2): 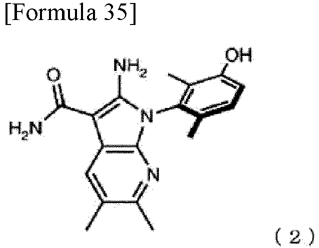 or a salt thereof, or a solvate thereof.

[I-20.5]
The chemotherapeutic agent according to any one of [I-1] to [I-17], wherein the MYT1 inhibitor is a compound represented by the formula (3): 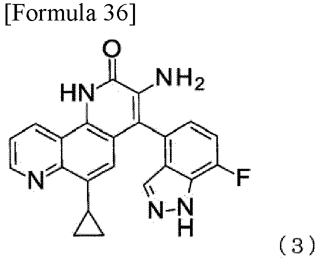 or a salt thereof, or a solvate thereof.

[I-21]
The chemotherapeutic agent according to any one of [I-1] to [I-20.5], wherein the chemotherapeutic agent is at least one selected from the group consisting of an antimetabolite, an anticancer antibiotic, a mitosis inhibitor, a topoisomerase inhibitor, a platinating agent, an alkylating agent, and an antibody-drug conjugate.

[I-22]
The chemotherapeutic agent according to [1-21], wherein the chemotherapeutic agent is an antimetabolite.

[I-23]
The chemotherapeutic agent according to [I-21] or [I-22], wherein the antimetabolite is at least one selected from the group consisting of a purine antimetabolite, a pyrimidine antimetabolite, a folic acid antimetabolite, and a ribonucleotide reductase inhibitor.

[I-24]
The chemotherapeutic agent according to [1-21], wherein the antimetabolite is a purine antimetabolite.

[I-25]
The chemotherapeutic agent according to [I-23] or [I-24], wherein the purine antimetabolite is at least one selected from the group consisting of 6-thioguanine, 6-mercaptopurine, azathioprine, fludarabine, pentostatin, cladribine, clofarabine, and nelarabine.

[I-26]
The chemotherapeutic agent according to [1-21], wherein the antimetabolite is a pyrimidine antimetabolite.

[I-27]
The chemotherapeutic agent according to [I-23] or [I-24], wherein the pyrimidine antimetabolite is at least one selected from the group consisting of gemcitabine, cytarabine, fluorouracil, capecitabine, tegafur, azacitidine, trifluridine, and floxuridine.

[I-28]
The chemotherapeutic agent according to [I-27], wherein the pyrimidine antimetabolite is gemcitabine.

[I-29]
The chemotherapeutic agent according to [1-21], wherein the antimetabolite is a folic acid antimetabolite.

[I-30]
The chemotherapeutic agent according to [I-23] or [I-29], wherein the folic acid antimetabolite is at least one selected from the group consisting of pemetrexed and methotrexate.

[I-31]
The chemotherapeutic agent according to [I-30], wherein the folic acid antimetabolite is pemetrexed.

[I-32]
The chemotherapeutic agent according to [1-21], wherein the antimetabolite is a ribonucleotide reductase inhibitor.

[I-33]
The chemotherapeutic agent according to [I-23] or [I-32], wherein the ribonucleotide reductase inhibitor is hydroxyurea.

[I-34]
The chemotherapeutic agent according to [1-21], wherein the chemotherapeutic agent is an anticancer antibiotic.

[I-35]
The chemotherapeutic agent according to [I-21] or [I-34], wherein the anticancer antibiotic is at least one selected from the group consisting of bleomycin, actinomycin D, doxorubicin, daunorubicin, idarubicin, mitomycin, mitoxantrone, epirubicin, aclarubicin, and valrubicin.

[I-36]
The chemotherapeutic agent according to [1-21], wherein the chemotherapeutic agent is a mitosis inhibitor.

[I-37]
The chemotherapeutic agent according to [I-21] or [I-36], wherein the mitosis inhibitor is at least one selected from the group consisting of vinca alkaloid and a microtubule inhibitor.

[I-38]
The chemotherapeutic agent according to [I-37], wherein the mitosis inhibitor is vinca alkaloid.

[I-39]
The chemotherapeutic agent according to [I-37] or [I-38], wherein the vinca alkaloid is at least one selected from the group consisting of vincristine, vinblastine, and vinorelbine.

[I-40]
The chemotherapeutic agent according to [I-37], wherein the mitosis inhibitor is a microtubule inhibitor.

[I-41]
The chemotherapeutic agent according to [I-37] or [I-40], wherein the microtubule inhibitor is at least one selected from the group consisting of docetaxel, paclitaxel, eribulin, ixabepilone, and epothilone.

[I-42]
The chemotherapeutic agent according to [1-21], wherein the chemotherapeutic agent is a topoisomerase inhibitor.

[I-43]
The chemotherapeutic agent according to [I-21] or [I-42], wherein the topoisomerase inhibitor is at least one selected from the group consisting of topotecan, irinotecan, DXd, etoposide, and teniposide.

[I-44]
The chemotherapeutic agent according to [I-43], wherein the topoisomerase inhibitor is at least one selected from the group consisting of irinotecan and DXd.

[I-45]
The chemotherapeutic agent according to [1-21], wherein the chemotherapeutic agent is a platinating agent.

[I-46]
The chemotherapeutic agent according to [I-21] or [I-45], wherein the platinating agent is at least one selected from the group consisting of cisplatin, carboplatin, and oxaliplatin.

[I-47]
The chemotherapeutic agent according to [I-46], wherein the platinating agent is carboplatin.

[I-48]
The chemotherapeutic agent according to [1-21], wherein the chemotherapeutic agent is an alkylating agent.

[I-49]
The chemotherapeutic agent according to [I-21] or [I-48], wherein the alkylating agent is at least one selected from the group consisting of cyclophosphamide, ifosfamide, chlorambucil, melphalan, temozolomide, carmustine, lomustine, streptozocin, busulfan, procarbazine, dacarbazine, nimustine, ranimustine, bendamustine, altretamine, thiotepa, and mechlorethamine.

[I-50]
The chemotherapeutic agent according to [1-21], wherein the chemotherapeutic agent is an antibody-drug conjugate.

[I-51]
The chemotherapeutic agent according to [I-21] or [I-50], wherein the antibody-drug conjugate is at least one selected from the group consisting of trastuzumab deruxtecan, sacituzumab govitecan, tisotumab vedotin, enfortumab vedotin, trastuzumab emtansine, loncastuximab tesirine, moxetumomab pasudotox, belantamab mafodotin, polatuzumab vedotin, inotuzumab ozogamicin, brentuximab vedotin, and gemtuzumab ozogamicin.

[I-52]
The chemotherapeutic agent according to [I-51], wherein the antibody-drug conjugate is at least one selected from the group consisting of trastuzumab deruxtecan and sacituzumab govitecan.

[I-53]
The chemotherapeutic agent according to any one of [I-1] to [I-7], wherein the MYT1 inhibitor is a small interfering RNA (siRNA).

[I-54]
The chemotherapeutic agent according to any one of [I-1] to [I-7], wherein the MYT1 inhibitor is a small interfering RNA (siRNA), and the chemotherapeutic agent is gemcitabine.

[I-55]
The chemotherapeutic agent according to any one of [I-1] to [I-7], wherein the MYT1 inhibitor is a small interfering RNA (siRNA), and the chemotherapeutic agent is fluorouracil.

[I-56]
The chemotherapeutic agent according to any one of [I-1] to [I-7], wherein the MYT1 inhibitor is a small interfering RNA (siRNA), and the chemotherapeutic agent is pemetrexed.

[I-57]
The chemotherapeutic agent according to any one of [I-1] to [I-7], wherein the MYT1 inhibitor is a small interfering RNA (siRNA), and the chemotherapeutic agent is etoposide.

[I-58]
The chemotherapeutic agent according to any one of [I-1] to [I-7], wherein the MYT1 inhibitor is a small interfering RNA (siRNA), and the chemotherapeutic agent is irinotecan.

[I-59]
The chemotherapeutic agent according to any one of [I-1] to [I-7], wherein the MYT1 inhibitor is a small interfering RNA (siRNA), and the chemotherapeutic agent is carboplatin.

[I-60]
The chemotherapeutic agent according to any one of [I-1] to [I-7], wherein the MYT1 inhibitor is a small interfering RNA (siRNA), and the chemotherapeutic agent is trastuzumab deruxtecan.

[I-60.1]
The chemotherapeutic agent according to any one of [I-1] to [I-7], wherein the MYT1 inhibitor is a compound represented by the formula (2) or a salt thereof, or a solvate thereof, and the chemotherapeutic agent is at least one selected from the group consisting of pemetrexed, gemcitabine, carboplatin, irinotecan, sacituzumab govitecan, DXd, and trastuzumab deruxtecan.

[I-60.11]
The chemotherapeutic agent according to any one of [I-1] to [I-7], wherein the MYT1 inhibitor is a compound represented by the formula (2) or a salt thereof, or a solvate thereof, and the chemotherapeutic agent is pemetrexed.

[I-60.12]
The chemotherapeutic agent according to any one of [I-1] to [I-7], wherein the MYT1 inhibitor is a compound represented by the formula (2) or a salt thereof, or a solvate thereof, and the chemotherapeutic agent is gemcitabine.

[I-60.13]
The chemotherapeutic agent according to any one of [I-1] to [I-7], wherein the MYT1 inhibitor is a compound represented by the formula (2) or a salt thereof, or a solvate thereof, and the chemotherapeutic agent is carboplatin.

[I-60.14]
The chemotherapeutic agent according to any one of [I-1] to [I-7], wherein the MYT1 inhibitor is a compound represented by the formula (2) or a salt thereof, or a solvate thereof, and the chemotherapeutic agent is irinotecan.

[I-60.15]
The chemotherapeutic agent according to any one of [I-1] to [I-7], wherein the MYT1 inhibitor is a compound represented by the formula (2) or a salt thereof, or a solvate thereof, and the chemotherapeutic agent is sacituzumab govitecan.

[I-60.16]
The chemotherapeutic agent according to any one of [I-1] to [I-7], wherein the MYT1 inhibitor is a compound represented by the formula (2) or a salt thereof, or a solvate thereof, and the chemotherapeutic agent is DXd.

[I-60.17]
The chemotherapeutic agent according to any one of [I-1] to [I-7], wherein the MYT1 inhibitor is a compound represented by the formula (2) or a salt thereof, or a solvate thereof, and the chemotherapeutic agent is trastuzumab deruxtecan.

[I-60.2]
The chemotherapeutic agent according to any one of [I-1] to [I-7], wherein the MYT1 inhibitor is a compound represented by the formula (3) or a salt thereof, or a solvate thereof, and the chemotherapeutic agent is at least one selected from the group consisting of pemetrexed, gemcitabine, carboplatin, irinotecan, sacituzumab govitecan, DXd, and trastuzumab deruxtecan.

[1-60.21]
The chemotherapeutic agent according to any one of [I-1] to [I-7], wherein the MYT1 inhibitor is a compound represented by the formula (3) or a salt thereof, or a solvate thereof, and the chemotherapeutic agent is pemetrexed.

[I-60.22]
The chemotherapeutic agent according to any one of [I-1] to [I-7], wherein the MYT1 inhibitor is a compound represented by the formula (3) or a salt thereof, or a solvate thereof, and the chemotherapeutic agent is gemcitabine.

[I-60.23]
The chemotherapeutic agent according to any one of [I-1] to [I-7], wherein the MYT1 inhibitor is a compound represented by the formula (3) or a salt thereof, or a solvate thereof, and the chemotherapeutic agent is carboplatin.

[I-60.24]
The chemotherapeutic agent according to any one of [I-1] to [I-7], wherein the MYT1 inhibitor is a compound represented by the formula (3) or a salt thereof, or a solvate thereof, and the chemotherapeutic agent is irinotecan.

[I-60.25]
The chemotherapeutic agent according to any one of [I-1] to [I-7], wherein the MYT1 inhibitor is a compound represented by the formula (3) or a salt thereof, or a solvate thereof, and the chemotherapeutic agent is sacituzumab govitecan.

[I-60.26]
The chemotherapeutic agent according to any one of [I-1] to [I-7], wherein the MYT1 inhibitor is a compound represented by the formula (3) or a salt thereof, or a solvate thereof, and the chemotherapeutic agent is DXd.

[I-60.27]
The chemotherapeutic agent according to any one of [I-1] to [I-7], wherein the MYT1 inhibitor is a compound represented by the formula (3) or a salt thereof, or a solvate thereof, and the chemotherapeutic agent is trastuzumab deruxtecan.

[I-61]
The chemotherapeutic agent according to any one of [I-1] to [I-60], wherein the MYT1 inhibitor and the chemotherapeutic agent are simultaneously or separately administered.

[I-62]
The chemotherapeutic agent according to any one of [I-1] to [I-60], wherein the MYT1 inhibitor and the chemotherapeutic agent are administered as a combination drug.

[I-63]
The chemotherapeutic agent according to any one of [I-1] to [I-62], wherein the cancer is solid cancer or blood cancer.

[I-64]
The chemotherapeutic agent according to any one of [I-1] to [I-62], wherein the cancer is at least one selected from the group consisting of lung cancer, breast cancer, esophageal cancer, stomach cancer, bowel cancer, uterine cancer, ovarian cancer, pancreatic cancer, bladder cancer, thyroid cancer, skin cancer, head and neck cancer, kidney cancer, liver cancer, prostate cancer, adenoid cystic cancer, retinoblastoma, cerebral tumor, leukemia, malignant lymphoma, and multiple myeloma.

[I-65]
The chemotherapeutic agent according to [I-64], wherein the cancer is at least one selected from the group consisting of lung cancer, breast cancer, bladder cancer, and ovarian cancer.

[I-66]
The chemotherapeutic agent according to [I-64], wherein the cancer is lung cancer, breast cancer, or bladder cancer.

[I-67]
The chemotherapeutic agent according to any one of [I-1] to [I-66], wherein the RB1 gene mutation positivity, or the decrease in expression of an RB1 gene or protein is detected in a cancer patient-derived biological sample.

[1-67.1]
The chemotherapeutic agent according to any one of [I-1] to [I-66], wherein the positive expression of hyperphosphorylated RB1 protein is detected in a cancer patient-derived biological sample.

[I-68]
The chemotherapeutic agent according to [I-67], wherein the cancer patient-derived biological sample is cancer cells.

[I-69]
The chemotherapeutic agent according to any one of [I-1] to [I-68], wherein the decrease in expression of an RB1 gene or protein is a decrease based on an expression level in a healthy individual-derived biological sample or a non-cancer tissue derived from the cancer patient.

[I-69.1]
The chemotherapeutic agent according to any one of [I-1] to [I-68], wherein the positive expression of hyperphosphorylated RB1 protein is determined from an increase in expression of the hyperphosphorylated RB1 protein based on an expression level in a non-cancer tissue derived from the cancer patient.

[I-70]
The chemotherapeutic agent according to any one of [I-1] to [I-69], wherein the patient is a patient in which amplification of a copy number of a CCNE1 gene is not detected.

[I-71]
The chemotherapeutic agent according to any one of [I-1] to [I-70], wherein the above patient is not a mouse implanted with OVCAR3.

[I-72]
The chemotherapeutic agent according to any one of [I-1] to [I-71], wherein the patient is human.

[J-1]
Use of an MYT1 inhibitor for production of a pharmaceutical composition for treatment or prevention of cancer in a cancer patient in which RB1 gene mutation positivity, a decrease in expression of an RB1 gene or protein, or positive expression of hyperphosphorylated RB1 protein is detected, the MYT1 inhibitor being administered in combination with a chemotherapeutic agent.

[J-1.1]
Use of an MYT1 inhibitor for production of a pharmaceutical composition for treatment or prevention of cancer in a cancer patient in which RB1 gene mutation positivity or a decrease in expression of an RB1 gene or protein is detected, the MYT1 inhibitor being administered in combination with a chemotherapeutic agent.

[J-1.2]
Use of an MYT1 inhibitor for production of a pharmaceutical composition for treatment or prevention of cancer in a cancer patient in which positive expression of hyperphosphorylated RB1 protein is detected, the MYT1 inhibitor being administered in combination with a chemotherapeutic agent.

[J-2]
The use according to [J-1], wherein the RB1 gene mutation comprises a mutation causing insertion, substitution, deletion, and/or addition of at least one amino acid residue to wild-type RB1 protein.

[J-3]
The use according to [J-1] or [J-2], wherein the RB1 gene mutation is a nonsense mutation, a frameshift mutation, a splice site mutation, or a homozygous or heterozygous deletion.

[J-4]
The use according to any one of [J-1] to [J-3], wherein the RB1 gene mutation is a mutation decreasing a function of RB1.

[J-5]
The use according to any one of [J-1] to [J-4], wherein the RB1 gene mutation is a human RB1 gene mutation.

[J-6]
The use according to [J-5], wherein the human RB1 gene mutation is at least one of the following (1) to (5):
(1) a codon corresponding to a serine residue (S) at position 82 of an amino acid sequence of SEQ ID No: 2 is replaced with a stop codon,
(2) a codon corresponding to an arginine residue (R) at position 467 of the amino acid sequence of SEQ ID No: 2 is replaced with a stop codon,
(3) in a codon corresponding to an amino acid residue at position 182, at least one base is inserted or deleted, a new reading frame starting from an isoleucine residue (I) is formed, and a third reading frame therefrom is a stop codon,
(4) a glutamic acid residue (E) at position 837 of the amino acid sequence of SEQ ID No: 2 is replaced with a lysine residue (K), and a part of the RB1 gene is homozygously deleted, and
(5) a glycine residue (G) at position 449 of the amino acid sequence of SEQ ID No: 2 is replaced with a glutamic acid residue (E), and a part of the RB1 gene is homozygously deleted.

[J-7]
The use according to any one of [J-1] to [J-6], wherein the decrease in expression of an RB1 gene or protein comprises a decrease in gene expression through methylation of an RB1 gene or a micro RNA.

[J-7.1]
The use according to any one of [J-1] to [J-6], wherein the hyperphosphorylated RB1 protein is RB1 protein having 3 or more phosphorylated amino acid residues in the amino acid sequence of the RB1 protein.

[J-7.2]
The use according to any one of [J-1] to [J-6], wherein the hyperphosphorylated RB1 protein is RB1 protein having 4 or more phosphorylated amino acid residues in the amino acid sequence of the RB1 protein.

[J-7.3]
The use according to any one of [J-1] to [J-6], wherein the hyperphosphorylated RB1 protein is RB1 protein having 8 or more phosphorylated amino acid residues in the amino acid sequence of the RB1 protein.

[J-7.4]
The use according to any one of [J-1] to [J-6], wherein the hyperphosphorylated RB1 protein is RB1 protein having 15 or more phosphorylated amino acid residues in the amino acid sequence of the RB1 protein.

[J-7.5]
The use according to any one of [J-1] to [J-6], wherein the hyperphosphorylated RB1 protein is hyperphosphorylated human RB1 protein.

[J-7.6]
The use according to [J-7.5], wherein the hyperphosphorylated human RB1 protein is RB1 protein in which at least one amino acid residue selected from the group consisting of a threonine residue (T) at position 826, a threonine residue (T) at position 823, a threonine residue (T) at position 821, a serine residue (S) at position 816, a tyrosine residue (Y) at position 813, a serine residue (S) at position 811, a serine residue (S) at position 807, a tyrosine residue (Y) at position 805, a serine residue (S) at position 780, a threonine residue (T) at position 625, a threonine residue (T) at position 601, a threonine residue (T) at position 373, a serine residue (S) at position 360, a threonine residue (T) at position 356, a serine residue (S) at position 249, and a serine residue (S) at position 37 of SEQ ID No: 2 is phosphorylated.

[J-7.7]
The use according to [J-7.5], wherein the hyperphosphorylated human RB1 protein is RB 1 protein in which at least one amino acid residue selected from the group consisting of a threonine residue (T) at position 826, a threonine residue (T) at position 821, a serine residue (S) at position 811, a serine residue (S) at position 807, a serine residue (S) at position 780, a threonine residue (T) at position 373, and a threonine residue (T) at position 356 of SEQ ID No: 2 is phosphorylated.

[J-7.8]
The use according to [J-7.5], wherein the hyperphosphorylated human RB1 protein is RB 1 protein in which at least one amino acid residue selected from the group consisting of a threonine residue (T) at position 826, a threonine residue (T) at position 821, a serine residue (S) at position 811, and a serine residue (S) at position 807 of SEQ ID No: 2 is phosphorylated.

[J-7.9]
The use according to [J-7.5], wherein the hyperphosphorylated human RB1 protein is RB 1 protein in which at least two amino acid residues selected from the group consisting of a threonine residue (T) at position 826, a threonine residue (T) at position 821, and a serine residue (S) at position 811 and/or a serine residue (S) at position 807 of SEQ ID No: 2 are phosphorylated.

[J-7.10]
The use according to [J-7.5], wherein the hyperphosphorylated human RB1 protein is RB1 protein in which a threonine residue (T) at position 826 and a threonine residue (T) at position 821 of SEQ ID No: 2 are phosphorylated, and a serine residue (S) at position 811 and/or a serine residue (S) at position 807 are/is phosphorylated.

[J-8]
The use according to any one of [J-1] to [J-7], wherein the MYT1 inhibitor is at least one selected from the group consisting of a low molecular compound, a polypeptide, and a polynucleotide.

[J-9]
The use according to [J-8], wherein the MYT1 inhibitor is a low molecular compound.

[J-10]
The use according to [J-9], wherein the low molecular compound is a compound having a molecular weight of 2000 g/mol or less.

[J-11]
The use according to [J-9], wherein the low molecular compound is a compound having a molecular weight of 1000 g/mol or less.

[J-12]
The use according to [J-8], wherein the MYT1 inhibitor is a polypeptide.

[J-13]
The use according to [J-12], wherein the polypeptide comprises an antibody.

[J-14]
The use according to [J-12], wherein the polypeptide is an anti-MYT1 antibody.

[J-15]
The use according to [J-8], wherein the MYT1 inhibitor is a polynucleotide.

[J-16]
The use according to [J-15], wherein the polynucleotide is at least one selected from the group consisting of a ribozyme, an antisense molecule, an inhibitor oligonucleotide, an aptamer, a micro RNA, and a small interfering RNA (siRNA).

[J-17]
The use according to [J-15], wherein the polynucleotide is at least one selected from the group consisting of an antisense nucleic acid, a micro RNA, and a small interfering RNA (siRNA).

[J-18]
The use according to any one of [J-1] to [J-11], wherein the MYT1 inhibitor is a compound represented by the formula (1): 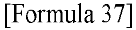 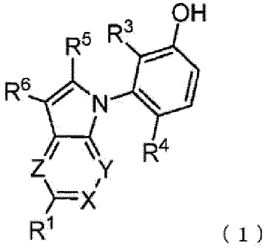
wherein X, Y, Z, R¹, R³, R⁴, R⁵, and R⁶ are as defined in the above [A-19],
or a salt thereof, or a solvate thereof.

[J-19]
The use according to [J-18], wherein the compound represented by the formula (1) comprises an atropisomer represented by the formula (1A) in a larger amount than each atropisomer: 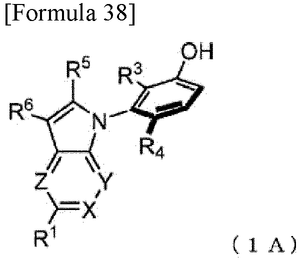 wherein X, Y, Z, R¹, R³, R⁴, R⁵, and R⁶ are as defined in the above [A-19].

[J-20]
The use according to any one of [J-1] to [J-19], wherein the MYT1 inhibitor is a compound represented by the formula (2): 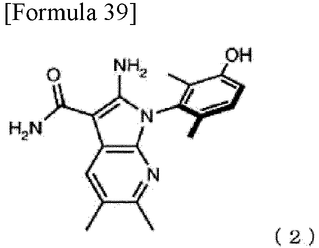 or a salt thereof, or a solvate thereof.

[J-20.5]
The use according to any one of [J-1] to [J-17], wherein the MYT1 inhibitor is a compound represented by the formula (3): 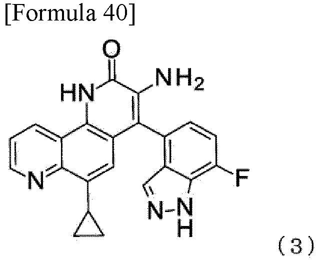 or a salt thereof, or a solvate thereof.

[J-21]
The use according to any one of [J-1] to [J-20.5], wherein the chemotherapeutic agent is at least one selected from the group consisting of an antimetabolite, an anticancer antibiotic, a mitosis inhibitor, a topoisomerase inhibitor, a platinating agent, an alkylating agent, and an antibody-drug conjugate.

[J-22]
The use according to [J-21], wherein the chemotherapeutic agent is an antimetabolite.

[J-23]
The use according to [J-21] or [J-22], wherein the antimetabolite is at least one selected from the group consisting of a purine antimetabolite, a pyrimidine antimetabolite, a folic acid antimetabolite, and a ribonucleotide reductase inhibitor.

[J-24]
The use according to [J-21], wherein the antimetabolite is a purine antimetabolite.

[J-25]
The use according to [J-23] or [J-24], wherein the purine antimetabolite is at least one selected from the group consisting of 6-thioguanine, 6-mercaptopurine, azathioprine, fludarabine, pentostatin, cladribine, clofarabine, and nelarabine.

[J-26]
The use according to [J-21], wherein the antimetabolite is a pyrimidine antimetabolite.

[J-27]
The use according to [J-23] or [J-26], wherein the pyrimidine antimetabolite is at least one selected from the group consisting of gemcitabine, cytarabine, fluorouracil, capecitabine, tegafur, azacitidine, trifluridine, and floxuridine.

[J-28]
The use according to [J-27], wherein the pyrimidine antimetabolite is gemcitabine.

[J-29]
The use according to [J-21], wherein the antimetabolite is a folic acid antimetabolite.

[J-30]
The use according to [J-23] or [J-29], wherein the folic acid antimetabolite is at least one selected from the group consisting of pemetrexed and methotrexate.

[J-31]
The use according to [J-30], wherein the folic acid antimetabolite is pemetrexed.

[J-32]
The use according to [J-21], wherein the antimetabolite is a ribonucleotide reductase inhibitor.

[J-33]
The use according to [J-23] or [J-32], wherein the ribonucleotide reductase inhibitor is hydroxyurea.

[J-34]
The use according to [J-21], wherein the chemotherapeutic agent is an anticancer antibiotic.

[J-35]
The use according to [J-21] or [J-34], wherein the anticancer antibiotic is at least one selected from the group consisting of bleomycin, actinomycin D, doxorubicin, daunorubicin, idarubicin, mitomycin, mitoxantrone, epirubicin, aclarubicin, and valrubicin.

[J-36]
The use according to [J-21], wherein the chemotherapeutic agent is a mitosis inhibitor.

[J-37]
The use according to [J-21] or [J-36], wherein the mitosis inhibitor is at least one selected from the group consisting of vinca alkaloid and a microtubule inhibitor.

[J-38]
The use according to [J-37], wherein the mitosis inhibitor is vinca alkaloid.

[J-39]
The use according to [J-37] or [J-38], wherein the vinca alkaloid is at least one selected from the group consisting of vincristine, vinblastine, and vinorelbine.

[J-40]
The use according to [J-37], wherein the mitosis inhibitor is a microtubule inhibitor.

[J-41]
The use according to [J-37] or [J-40], wherein the microtubule inhibitor is at least one selected from the group consisting of docetaxel, paclitaxel, eribulin, ixabepilone, and epothilone.

[J-42]
The use according to [J-21], wherein the chemotherapeutic agent is a topoisomerase inhibitor.

[J-43]
The use according to [J-21] or [J-42], wherein the topoisomerase inhibitor is at least one selected from the group consisting of topotecan, irinotecan, DXd, etoposide, and teniposide.

[J-44]
The use according to [J-43], wherein the topoisomerase inhibitor is at least one selected from the group consisting of irinotecan and DXd.

[J-45]
The use according to [J-21], wherein the chemotherapeutic agent is a platinating agent.

[J-46]
The use according to [J-21] or [J-45], wherein the platinating agent is at least one selected from the group consisting of cisplatin, carboplatin, and oxaliplatin.

[J-47]
The use according to [J-46], wherein the platinating agent is carboplatin.

[J-48]
The use according to [J-21], wherein the chemotherapeutic agent is an alkylating agent.

[J-49]
The use according to [J-21] or [J-48], wherein the alkylating agent is at least one selected from the group consisting of cyclophosphamide, ifosfamide, chlorambucil, melphalan, temozolomide, carmustine, lomustine, streptozocin, busulfan, procarbazine, dacarbazine, nimustine, ranimustine, bendamustine, altretamine, thiotepa, and mechlorethamine.

[J-50]
The use according to [J-21], wherein the chemotherapeutic agent is an antibody-drug conjugate.

[J-51]
The use according to [J-21] or [J-50], wherein the antibody-drug conjugate is at least one selected from the group consisting of trastuzumab deruxtecan, sacituzumab govitecan, tisotumab vedotin, enfortumab vedotin, trastuzumab emtansine, loncastuximab tesirine, moxetumomab pasudotox, belantamab mafodotin, polatuzumab vedotin, inotuzumab ozogamicin, brentuximab vedotin, and gemtuzumab ozogamicin.

[J-52]
The use according to [J-51], wherein the antibody-drug conjugate is at least one selected from the group consisting of trastuzumab deruxtecan and sacituzumab govitecan.

[J-53]
The use according to any one of [J-1] to [J-7], wherein the chemotherapeutic agent is at least one selected from the group consisting of gemcitabine, pemetrexed, irinotecan, carboplatin, and trastuzumab deruxtecan.

[J-54]
The use according to any one of [J-1] to [J-7], wherein the MYT1 inhibitor is a small interfering RNA (siRNA), and the chemotherapeutic agent is gemcitabine.

[J-55]
The use according to any one of [J-1] to [J-7], wherein the MYT1 inhibitor is a small interfering RNA (siRNA), and the chemotherapeutic agent is fluorouracil.

[J-56]
The use according to any one of [J-1] to [J-7], wherein the MYT1 inhibitor is a small interfering RNA (siRNA), and the chemotherapeutic agent is pemetrexed.

[J-57]
The use according to any one of [J-1] to [J-7], wherein the MYT1 inhibitor is a small interfering RNA (siRNA), and the chemotherapeutic agent is etoposide.

[J-58]
The use according to any one of [J-1] to [J-7], wherein the MYT1 inhibitor is a small interfering RNA (siRNA), and the chemotherapeutic agent is irinotecan.

[J-59]
The use according to any one of [J-1] to [J-7], wherein the MYT1 inhibitor is a small interfering RNA (siRNA), and the chemotherapeutic agent is carboplatin.

[J-60]
The use according to any one of [J-1] to [J-7], wherein the MYT1 inhibitor is a small interfering RNA (siRNA), and the chemotherapeutic agent is trastuzumab deruxtecan.

[J-60.1]
The use according to any one of [J-1] to [J-7], wherein the MYT1 inhibitor is a compound represented by the formula (2) or a salt thereof, or a solvate thereof, and the chemotherapeutic agent is at least one selected from the group consisting of pemetrexed, gemcitabine, carboplatin, irinotecan, sacituzumab govitecan, DXd, and trastuzumab deruxtecan.

[J-60.11]
The use according to any one of [J-1] to [J-7], wherein the MYT1 inhibitor is a compound represented by the formula (2) or a salt thereof, or a solvate thereof, and the chemotherapeutic agent is pemetrexed.

[J-60.12]
The use according to any one of [J-1] to [J-7], wherein the MYT1 inhibitor is a compound represented by the formula (2) or a salt thereof, or a solvate thereof, and the chemotherapeutic agent is gemcitabine.

[J-60.13]
The use according to any one of [J-1] to [J-7], wherein the MYT1 inhibitor is a compound represented by the formula (2) or a salt thereof, or a solvate thereof, and the chemotherapeutic agent is carboplatin.

[J-60.14]
The use according to any one of [J-1] to [J-7], wherein the MYT1 inhibitor is a compound represented by the formula (2) or a salt thereof, or a solvate thereof, and the chemotherapeutic agent is irinotecan.

[J-60.15]
The use according to any one of [J-1] to [J-7], wherein the MYT1 inhibitor is a compound represented by the formula (2) or a salt thereof, or a solvate thereof, and the chemotherapeutic agent is sacituzumab govitecan.

[J-60.16]
The use according to any one of [J-1] to [J-7], wherein the MYT1 inhibitor is a compound represented by the formula (2) or a salt thereof, or a solvate thereof, and the chemotherapeutic agent is DXd.

[J-60.17]
The use according to any one of [J-1] to [J-7], wherein the MYT1 inhibitor is a compound represented by the formula (2) or a salt thereof, or a solvate thereof, and the chemotherapeutic agent is trastuzumab deruxtecan.

[J-60.2]
The use according to any one of [J-1] to [J-7], wherein the MYT1 inhibitor is a compound represented by the formula (3) or a salt thereof, or a solvate thereof, and the chemotherapeutic agent is at least one selected from the group consisting of pemetrexed, gemcitabine, carboplatin, irinotecan, sacituzumab govitecan, DXd, and trastuzumab deruxtecan.

[J-60.21]
The use according to any one of [J-1] to [J-7], wherein the MYT1 inhibitor is a compound represented by the formula (3) or a salt thereof, or a solvate thereof, and the chemotherapeutic agent is pemetrexed.

[J-60.22]
The use according to any one of [J-1] to [J-7], wherein the MYT1 inhibitor is a compound represented by the formula (3) or a salt thereof, or a solvate thereof, and the chemotherapeutic agent is gemcitabine.

[J-60.23]
The use according to any one of [J-1] to [J-7], wherein the MYT1 inhibitor is a compound represented by the formula (3) or a salt thereof, or a solvate thereof, and the chemotherapeutic agent is carboplatin.

[J-60.24]
The use according to any one of [J-1] to [J-7], wherein the MYT1 inhibitor is a compound represented by the formula (3) or a salt thereof, or a solvate thereof, and the chemotherapeutic agent is irinotecan.

[J-60.25]
The use according to any one of [J-1] to [J-7], wherein the MYT1 inhibitor is a compound represented by the formula (3) or a salt thereof, or a solvate thereof, and the chemotherapeutic agent is sacituzumab govitecan.

[J-60.26]
The use according to any one of [J-1] to [J-7], wherein the MYT1 inhibitor is a compound represented by the formula (3) or a salt thereof, or a solvate thereof, and the chemotherapeutic agent is DXd.

[J-60.27]
The use according to any one of [J-1] to [J-7], wherein the MYT1 inhibitor is a compound represented by the formula (3) or a salt thereof, or a solvate thereof, and the chemotherapeutic agent is trastuzumab deruxtecan.

[J-61]
The use according to any one of [J-1] to [J-60], wherein the MYT1 inhibitor and the chemotherapeutic agent are simultaneously or separately administered.

[J-62]
The use according to any one of [J-1] to [J-60], wherein the MYT1 inhibitor and the chemotherapeutic agent are administered as a combination drug.

[J-63]
The use according to any one of [J-1] to [J-62], wherein the cancer is solid cancer or blood cancer.

[J-64]
The use according to any one of [J-1] to [J-61], wherein the cancer is at least one selected from the group consisting of lung cancer, breast cancer, esophageal cancer, stomach cancer, bowel cancer, uterine cancer, ovarian cancer, pancreatic cancer, bladder cancer, thyroid cancer, skin cancer, head and neck cancer, kidney cancer, liver cancer, prostate cancer, adenoid cystic cancer, retinoblastoma, cerebral tumor, leukemia, malignant lymphoma, and multiple myeloma.

[J-65]
The use according to [J-64], wherein the cancer is at least one selected from the group consisting of lung cancer, breast cancer, bladder cancer, and ovarian cancer.

[J-66]
The use according to [J-64], wherein the cancer is lung cancer, breast cancer, or bladder cancer.

[J-67]
The use according to any one of [J-1] to [J-66], wherein the RB1 gene mutation positivity, or the decrease in expression of an RB1 gene or protein is detected in a cancer patient-derived biological sample.

[J-67.1]
The use according to any one of [J-1] to [J-66], wherein the positive expression of hyperphosphorylated RB1 protein is detected in a cancer patient-derived biological sample.

[J-68]
The use according to [J-67], wherein the cancer patient-derived biological sample is cancer cells.

[J-69]
The use according to any one of [J-1] to [J-68], wherein the decrease in expression of an RB1 gene or protein is a decrease based on an expression level in a healthy individual-derived biological sample or a non-cancer tissue derived from the cancer patient.

[J-69.1]
The use according to any one of [J-1] to [J-68], wherein the positive expression of hyperphosphorylated RB1 protein is determined from an increase in expression of the hyperphosphorylated RB1 protein based on an expression level in a healthy individual-derived biological sample or a non-cancer tissue derived from the cancer patient.

[J-70]
The use according to any one of [J-1] to [J-69], wherein the patient is a patient in which amplification of a copy number of a CCNE1 gene is not detected.

[J-71]
The use according to any one of [J-1] to [J-70], wherein the above patient is not a mouse implanted with OVCAR3.

[J-72]
The use according to any one of [J-1] to [J-71], wherein the patient is human.

[K-1]
Use of a chemotherapeutic agent for production of a pharmaceutical composition for treatment or prevention of cancer in a cancer patient in which RB1 gene mutation positivity, a decrease in expression of an RB1 gene or protein, or positive expression of hyperphosphorylated RB1 protein is detected, the chemotherapeutic agent being administered in combination with an MYT1 inhibitor.

[K-1.1]
Use of a chemotherapeutic agent for production of a pharmaceutical composition for treatment or prevention of cancer in a cancer patient in which RB1 gene mutation positivity or a decrease in expression of an RB1 gene or protein is detected, the chemotherapeutic agent being administered in combination with an MYT1 inhibitor.

[K-1.2]
Use of a chemotherapeutic agent for production of a pharmaceutical composition for treatment or prevention of cancer in a cancer patient in which positive expression of hyperphosphorylated RB1 protein is detected, the chemotherapeutic agent being administered in combination with an MYT1 inhibitor.

[K-2]
The use according to [K-1], wherein the RB1 gene mutation comprises a mutation causing insertion, substitution, deletion, and/or addition of at least one amino acid residue to wild-type RB1 protein.

[K-3]
The use according to [K-1] or [K-2], wherein the RB1 gene mutation is a nonsense mutation, a frameshift mutation, a splice site mutation, or a homozygous or heterozygous deletion.

[K-4]
The use according to any one of [K-1] to [K-3], wherein the RB1 gene mutation is a mutation decreasing a function of RB1.

[K-5]
The use according to any one of [K-1] to [K-4], wherein the RB1 gene mutation is a human RB1 gene mutation.

[K-6]
The use according to [K-5], wherein the human RB1 gene mutation is at least one of the following (1) to (5):
(1) a codon corresponding to a serine residue (S) at position 82 of an amino acid sequence of SEQ ID No: 2 is replaced with a stop codon,
(2) a codon corresponding to an arginine residue (R) at position 467 of the amino acid sequence of SEQ ID No: 2 is replaced with a stop codon,
(3) in a codon corresponding to an amino acid residue at position 182, at least one base is inserted or deleted, a new reading frame starting from an isoleucine residue (I) is formed, and a third reading frame therefrom is a stop codon,
(4) a glutamic acid residue (E) at position 837 of the amino acid sequence of SEQ ID No: 2 is replaced with a lysine residue (K), and a part of the RB1 gene is homozygously deleted, and
(5) a glycine residue (G) at position 449 of the amino acid sequence of SEQ ID No: 2 is replaced with a glutamic acid residue (E), and a part of the RB1 gene is homozygously deleted.

[K-7]
The use according to any one of [K-1] to [K-6], wherein the decrease in expression of an RB1 gene or protein comprises a decrease in gene expression through methylation of an RB 1 gene or a micro RNA.

[K-7.1]
The use according to any one of [K-1] to [K-6], wherein the hyperphosphorylated RB1 protein is RB1 protein having 3 or more phosphorylated amino acid residues in the amino acid sequence of the RB1 protein.

[K-7.2]
The use according to any one of [K-1] to [K-6], wherein the hyperphosphorylated RB1 protein is RB1 protein having 4 or more phosphorylated amino acid residues in the amino acid sequence of the RB1 protein.

[K-7.3]
The use according to any one of [K-1] to [K-6], wherein the hyperphosphorylated RB1 protein is RB1 protein having 8 or more phosphorylated amino acid residues in the amino acid sequence of the RB1 protein.

[K-7.4]
The use according to any one of [K-1] to [K-6], wherein the hyperphosphorylated RB1 protein is RB1 protein having 15 or more phosphorylated amino acid residues in the amino acid sequence of the RB1 protein.

[K-7.5]
The use according to any one of [K-1] to [K-6], wherein the hyperphosphorylated RB1 protein is hyperphosphorylated human RB1 protein.

[K-7.6]
The use according to [K-7.5], wherein the hyperphosphorylated human RB1 protein is RB 1 protein in which at least one amino acid residue selected from the group consisting of a threonine residue (T) at position 826, a threonine residue (T) at position 823, a threonine residue (T) at position 821, a serine residue (S) at position 816, a tyrosine residue (Y) at position 813, a serine residue (S) at position 811, a serine residue (S) at position 807, a tyrosine residue (Y) at position 805, a serine residue (S) at position 780, a threonine residue (T) at position 625, a threonine residue (T) at position 601, a threonine residue (T) at position 373, a serine residue (S) at position 360, a threonine residue (T) at position 356, a serine residue (S) at position 249, and a serine residue (S) at position 37 of SEQ ID No: 2 is phosphorylated.

[K-7.7]
The use according to [K-7.5], wherein the hyperphosphorylated human RB1 protein is RB 1 protein in which at least one amino acid residue selected from the group consisting of a threonine residue (T) at position 826, a threonine residue (T) at position 821, a serine residue (S) at position 811, a serine residue (S) at position 807, a serine residue (S) at position 780, a threonine residue (T) at position 373, and a threonine residue (T) at position 356 of SEQ ID No: 2 is phosphorylated.

[K-7.8]
The use according to [K-7.5], wherein the hyperphosphorylated human RB1 protein is RB 1 protein in which at least one amino acid residue selected from the group consisting of a threonine residue (T) at position 826, a threonine residue (T) at position 821, a serine residue (S) at position 811, and a serine residue (S) at position 807 of SEQ ID No: 2 is phosphorylated.

[K-7.9]
The use according to [K-7.5], wherein the hyperphosphorylated human RB1 protein is RB 1 protein in which at least two amino acid residues selected from the group consisting of a threonine residue (T) at position 826, a threonine residue (T) at position 821, and a serine residue (S) at position 811 and/or a serine residue (S) at position 807 of SEQ ID No: 2 are phosphorylated.

[K-7.10]
The use according to [K-7.5], wherein the hyperphosphorylated human RB1 protein is RB1 protein in which a threonine residue (T) at position 826 and a threonine residue (T) at position 821 of SEQ ID No: 2 are phosphorylated, and a serine residue (S) at position 811 and/or a serine residue (S) at position 807 are/is phosphorylated.

[K-8]
The use according to any one of [K-1] to [K-7], wherein the MYT1 inhibitor is at least one selected from the group consisting of a low molecular compound, a polypeptide, and a polynucleotide.

[K-9]
The use according to [K-8], wherein the MYT1 inhibitor is a low molecular compound.

[K-10]
The use according to [K-9], wherein the low molecular compound is a compound having a molecular weight of 2000 g/mol or less.

[K-11]
The use according to [K-9], wherein the low molecular compound is a compound having a molecular weight of 1000 g/mol or less.

[K-12]
The use according to [K-9], wherein the MYT1 inhibitor is a polypeptide.

[K-13]
The use according to [K-12], wherein the polypeptide comprises an antibody.

[K-14]
The use according to [K-12], wherein the polypeptide is an anti-MYT1 antibody.

[K-15]
The use according to [K-8], wherein the MYT1 inhibitor is a polynucleotide.

[K-16]
The use according to [K-15], wherein the polynucleotide is at least one selected from the group consisting of a ribozyme, an antisense molecule, an inhibitor oligonucleotide, an aptamer, a micro RNA, and a small interfering RNA (siRNA).

[K-17]
The use according to [K-15], wherein the polynucleotide is at least one selected from the group consisting of an antisense nucleic acid, a micro RNA, and a small interfering RNA (siRNA).

[K-18]
The use according to any one of [K-1] to [K-11], wherein the MYT1 inhibitor is a compound represented by the formula (1): 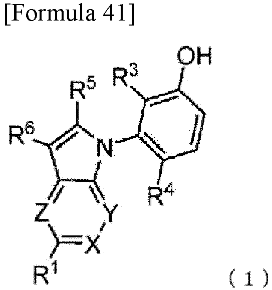
wherein X, Y, Z, R¹, R³, R⁴, R⁵, and R⁶ are as defined in the above [A-19],
or a salt thereof, or a solvate thereof.

[K-19]
The use according to [K-18], wherein the compound represented by the formula (1) comprises an atropisomer represented by the formula (1A) in a larger amount than each atropisomer: 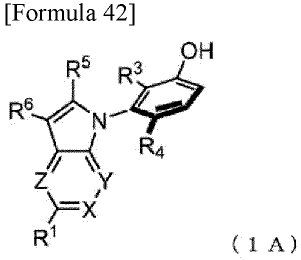 wherein X, Y, Z, R¹, R³, R⁴, R⁵, and R⁶ are as defined in the above [A-19].

[K-20]
The use according to any one of [K-1] to [K-19], wherein the MYT1 inhibitor is a compound represented by the formula (2): 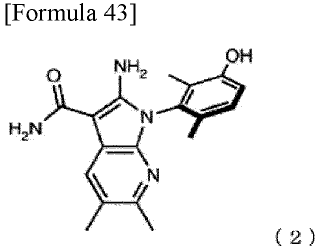 or a salt thereof, or a solvate thereof.

[K-20.5]
The use according to any one of [K-1] to [K-17], wherein the MYT1 inhibitor is a compound represented by the formula (3): 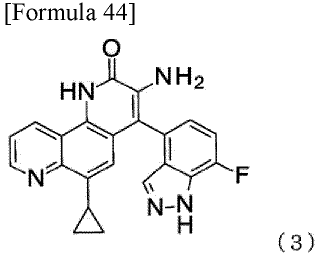 or a salt thereof, or a solvate thereof.

[K-21]
The use according to any one of [K-1] to [K-20.5], wherein the chemotherapeutic agent is at least one selected from the group consisting of an antimetabolite, an anticancer antibiotic, a mitosis inhibitor, a topoisomerase inhibitor, a platinating agent, an alkylating agent, and an antibody-drug conjugate.

[K-22]
The use according to [K-21], wherein the chemotherapeutic agent is an antimetabolite.

[K-23]
The use according to [K-21] or [K-22], wherein the antimetabolite is at least one selected from the group consisting of a purine antimetabolite, a pyrimidine antimetabolite, a folic acid antimetabolite, and a ribonucleotide reductase inhibitor.

[K-24]
The use according to [K-21], wherein the antimetabolite is a purine antimetabolite.

[K-25]
The use according to [K-23] or [K-24], wherein the purine antimetabolite is at least one selected from the group consisting of 6-thioguanine, 6-mercaptopurine, azathioprine, fludarabine, pentostatin, cladribine, clofarabine, and nelarabine.

[K-26]
The use according to [K-21], wherein the antimetabolite is a pyrimidine antimetabolite.

[K-27]
The use according to [K-23] or [K-26], wherein the pyrimidine antimetabolite is at least one selected from the group consisting of gemcitabine, cytarabine, fluorouracil, capecitabine, tegafur, azacitidine, trifluridine, and floxuridine.

[K-28]
The use according to [K-27], wherein the pyrimidine antimetabolite is gemcitabine.

[K-29]
The use according to [K-21], wherein the antimetabolite is a folic acid antimetabolite.

[K-30]
The use according to [K-23] or [K-29], wherein the folic acid antimetabolite is at least one selected from the group consisting of pemetrexed and methotrexate.

[K-31]
The use according to [K-30], wherein the folic acid antimetabolite is pemetrexed.

[K-32]
The use according to [K-21], wherein the antimetabolite is a ribonucleotide reductase inhibitor.

[K-33]
The use according to [K-23] or [K-32], wherein the ribonucleotide reductase inhibitor is hydroxyurea.

[K-34]
The use according to [K-21], wherein the chemotherapeutic agent is an anticancer antibiotic.

[K-35]
The use according to [K-21] or [K-34], wherein the anticancer antibiotic is at least one selected from the group consisting of bleomycin, actinomycin D, doxorubicin, daunorubicin, idarubicin, mitomycin, mitoxantrone, epirubicin, aclarubicin, and valrubicin.

[K-36]
The use according to [K-21], wherein the chemotherapeutic agent is a mitosis inhibitor.

[K-37]
The use according to [K-21] or [K-36], wherein the mitosis inhibitor is at least one selected from the group consisting of vinca alkaloid and a microtubule inhibitor.

[K-38]
The use according to [K-37], wherein the mitosis inhibitor is vinca alkaloid.

[K-39]
The use according to [K-37] or [K-38], wherein the vinca alkaloid is at least one selected from the group consisting of vincristine, vinblastine, and vinorelbine.

[K-40]
The use according to [K-37], wherein the mitosis inhibitor is a microtubule inhibitor.

[K-41]
The use according to [K-37] or [K-40], wherein the microtubule inhibitor is at least one selected from the group consisting of docetaxel, paclitaxel, eribulin, ixabepilone, and epothilone.

[K-42]
The use according to [K-21], wherein the chemotherapeutic agent is a topoisomerase inhibitor.

[K-43]
The use according to [K-21] or [K-42], wherein the topoisomerase inhibitor is at least one selected from the group consisting of topotecan, irinotecan, DXd, etoposide, and teniposide.

[K-44]
The use according to [K-43], wherein the topoisomerase inhibitor is at least one selected from the group consisting of irinotecan and DXd.

[K-45]
The use according to [K-21], wherein the chemotherapeutic agent is a platinating agent.

[K-46]
The use according to [K-21] or [K-45], wherein the platinating agent is at least one selected from the group consisting of cisplatin, carboplatin, and oxaliplatin.

[K-47]
The use according to [K-46], wherein the platinating agent is carboplatin.

[K-48]
The use according to [K-21], wherein the chemotherapeutic agent is an alkylating agent.

[K-49]
The use according to [K-21] or [K-48], wherein the alkylating agent is at least one selected from the group consisting of cyclophosphamide, ifosfamide, chlorambucil, melphalan, temozolomide, carmustine, lomustine, streptozocin, busulfan, procarbazine, dacarbazine, nimustine, ranimustine, bendamustine, altretamine, thiotepa, and mechlorethamine.

[K-50]
The use according to [K-21], wherein the chemotherapeutic agent is an antibody-drug conjugate.

[K-51]
The use according to [K-21] or [K-50], wherein the antibody-drug conjugate is at least one selected from the group consisting of trastuzumab deruxtecan, sacituzumab govitecan, tisotumab vedotin, enfortumab vedotin, trastuzumab emtansine, loncastuximab tesirine, moxetumomab pasudotox, belantamab mafodotin, polatuzumab vedotin, inotuzumab ozogamicin, brentuximab vedotin, and gemtuzumab ozogamicin.

[K-52]
The use according to [K-51], wherein the antibody-drug conjugate is at least one selected from the group consisting of trastuzumab deruxtecan and sacituzumab govitecan.

[K-53]
The use according to any one of [K-1] to [K-7], wherein the MYT1 inhibitor is a small interfering RNA (siRNA).

[K-54]
The use according to any one of [K-1] to [K-7], wherein the MYT1 inhibitor is a small interfering RNA (siRNA), and the chemotherapeutic agent is gemcitabine.

[K-55]
The use according to any one of [K-1] to [K-7], wherein the MYT1 inhibitor is a small interfering RNA (siRNA), and the chemotherapeutic agent is fluorouracil.

[K-56]
The use according to any one of [K-1] to [K-7], wherein the MYT1 inhibitor is a small interfering RNA (siRNA), and the chemotherapeutic agent is pemetrexed.

[K-57]
The use according to any one of [K-1] to [K-7], wherein the MYT1 inhibitor is a small interfering RNA (siRNA), and the chemotherapeutic agent is etoposide.

[K-58]
The use according to any one of [K-1] to [K-7], wherein the MYT1 inhibitor is a small interfering RNA (siRNA), and the chemotherapeutic agent is irinotecan.

[K-59]
The use according to any one of [K-1] to [K-7], wherein the MYT1 inhibitor is a small interfering RNA (siRNA), and the chemotherapeutic agent is carboplatin.

[K-60]
The use according to any one of [K-1] to [K-7], wherein the MYT1 inhibitor is a small interfering RNA (siRNA), and the chemotherapeutic agent is trastuzumab deruxtecan.

[K-60.1]
The use according to any one of [K-1] to [K-7], wherein the MYT1 inhibitor is a compound represented by the formula (2) or a salt thereof, or a solvate thereof, and the chemotherapeutic agent is at least one selected from the group consisting of pemetrexed, gemcitabine, carboplatin, irinotecan, sacituzumab govitecan, DXd, and trastuzumab deruxtecan.

[K-60.11]
The use according to any one of [K-1] to [K-7], wherein the MYT1 inhibitor is a compound represented by the formula (2) or a salt thereof, or a solvate thereof, and the chemotherapeutic agent is pemetrexed.

[K-60.12]
The use according to any one of [K-1] to [K-7], wherein the MYT1 inhibitor is a compound represented by the formula (2) or a salt thereof, or a solvate thereof, and the chemotherapeutic agent is gemcitabine.

[K-60.13]
The use according to any one of [K-1] to [K-7], wherein the MYT1 inhibitor is a compound represented by the formula (2) or a salt thereof, or a solvate thereof, and the chemotherapeutic agent is carboplatin.

[K-60.14]
The use according to any one of [K-1] to [K-7], wherein the MYT1 inhibitor is a compound represented by the formula (2) or a salt thereof, or a solvate thereof, and the chemotherapeutic agent is irinotecan.

[K-60.15]
The use according to any one of [K-1] to [K-7], wherein the MYT1 inhibitor is a compound represented by the formula (2) or a salt thereof, or a solvate thereof, and the chemotherapeutic agent is sacituzumab govitecan.

[K-60.16]
The use according to any one of [K-1] to [K-7], wherein the MYT1 inhibitor is a compound represented by the formula (2) or a salt thereof, or a solvate thereof, and the chemotherapeutic agent is DXd.

[K-60.17]
The use according to any one of [K-1] to [K-7], wherein the MYT1 inhibitor is a compound represented by the formula (2) or a salt thereof, or a solvate thereof, and the chemotherapeutic agent is trastuzumab deruxtecan.

[K-60.2]
The use according to any one of [K-1] to [K-7], wherein the MYT1 inhibitor is a compound represented by the formula (3) or a salt thereof, or a solvate thereof, and the chemotherapeutic agent is at least one selected from the group consisting of pemetrexed, gemcitabine, carboplatin, irinotecan, sacituzumab govitecan, DXd, and trastuzumab deruxtecan.

[K-60.21]
The use according to any one of [K-1] to [K-7], wherein the MYT1 inhibitor is a compound represented by the formula (3) or a salt thereof, or a solvate thereof, and the chemotherapeutic agent is pemetrexed.

[K-60.22]
The use according to any one of [K-1] to [K-7], wherein the MYT1 inhibitor is a compound represented by the formula (3) or a salt thereof, or a solvate thereof, and the chemotherapeutic agent is gemcitabine.

[K-60.23]
The use according to any one of [K-1] to [K-7], wherein the MYT1 inhibitor is a compound represented by the formula (3) or a salt thereof, or a solvate thereof, and the chemotherapeutic agent is carboplatin.

[K-60.24]
The use according to any one of [K-1] to [K-7], wherein the MYT1 inhibitor is a compound represented by the formula (3) or a salt thereof, or a solvate thereof, and the chemotherapeutic agent is irinotecan.

[K-60.25]
The use according to any one of [K-1] to [K-7], wherein the MYT1 inhibitor is a compound represented by the formula (3) or a salt thereof, or a solvate thereof, and the chemotherapeutic agent is sacituzumab govitecan.

[K-60.26]
The use according to any one of [K-1] to [K-7], wherein the MYT1 inhibitor is a compound represented by the formula (3) or a salt thereof, or a solvate thereof, and the chemotherapeutic agent is DXd.

[K-60.27]
The use according to any one of [K-1] to [K-7], wherein the MYT1 inhibitor is a compound represented by the formula (3) or a salt thereof, or a solvate thereof, and the chemotherapeutic agent is trastuzumab deruxtecan.

[K-61]
The use according to any one of [K-1] to [K-60], wherein the MYT1 inhibitor and the chemotherapeutic agent are simultaneously or separately administered.

[K-62]
The use according to any one of [K-1] to [K-60], wherein the MYT1 inhibitor and the chemotherapeutic agent are administered as a combination drug.

[K-63]
The use according to any one of [K-1] to [K-62], wherein the cancer is solid cancer or blood cancer.

[K-64]
The use according to any one of [K-1] to [K-62], wherein the cancer is at least one selected from the group consisting of lung cancer, breast cancer, esophageal cancer, stomach cancer, bowel cancer, uterine cancer, ovarian cancer, pancreatic cancer, bladder cancer, thyroid cancer, skin cancer, head and neck cancer, kidney cancer, liver cancer, prostate cancer, adenoid cystic cancer, retinoblastoma, cerebral tumor, leukemia, malignant lymphoma, and multiple myeloma.

[K-65]
The use according to [K-64], wherein the cancer is at least one selected from the group consisting of lung cancer, breast cancer, bladder cancer, and ovarian cancer.

[K-66]
The use according to [K-64], wherein the cancer is lung cancer, breast cancer, or bladder cancer.

[K-67]
The use according to any one of [K-1] to [K-66], wherein the RB1 gene mutation positivity, or the decrease in expression of an RB1 gene or protein is detected in a cancer patient-derived biological sample.

[K-67.1]
The use according to any one of [K-1] to [K-66], wherein the positive expression of hyperphosphorylated RB1 protein is detected in a cancer patient-derived biological sample.

[K-68]
The use according to [K-67], wherein the cancer patient-derived biological sample is cancer cells.

[K-69]
The use according to any one of [K-1] to [K-68], wherein the decrease in expression of an RB1 gene or protein is a decrease based on an expression level in a healthy individual-derived biological sample or a non-cancer tissue derived from the cancer patient.

[K-69.1]
The method according to any one of [K-1] to [K-68], wherein the positive expression of hyperphosphorylated RB1 protein is determined from an increase in expression of the hyperphosphorylated RB1 protein based on an expression level in a healthy individual-derived biological sample or a non-cancer tissue derived from the cancer patient.

[K-70]
The use according to any one of [K-1] to [K-69], wherein the patient is a patient in which amplification of a copy number of a CCNE1 gene is not detected.

[K-71]
The use according to any one of [K-1] to [K-69], wherein the above patient is not a mouse implanted with OVCAR3.

[K-72]
The use according to any one of [K-1] to [K-71], wherein the patient is human.

In the above numbering, the number cited in a dependent item also includes the same number but with a different number after a decimal point, unless otherwise specified. For example, [A-21] cited in a dependent item shows that not only [A-21] but also [A-21.5] or the like is included. The same applies to other numbers.

### [Advantageous Effect of Invention]

The present invention can provide a method for treating or preventing cancer in a patient in which RB1 gene mutation positivity, a decrease in expression of an RB1 gene or protein, or positive expression of hyperphosphorylated RB1 protein is detected.

### [Brief Description of Drawings]

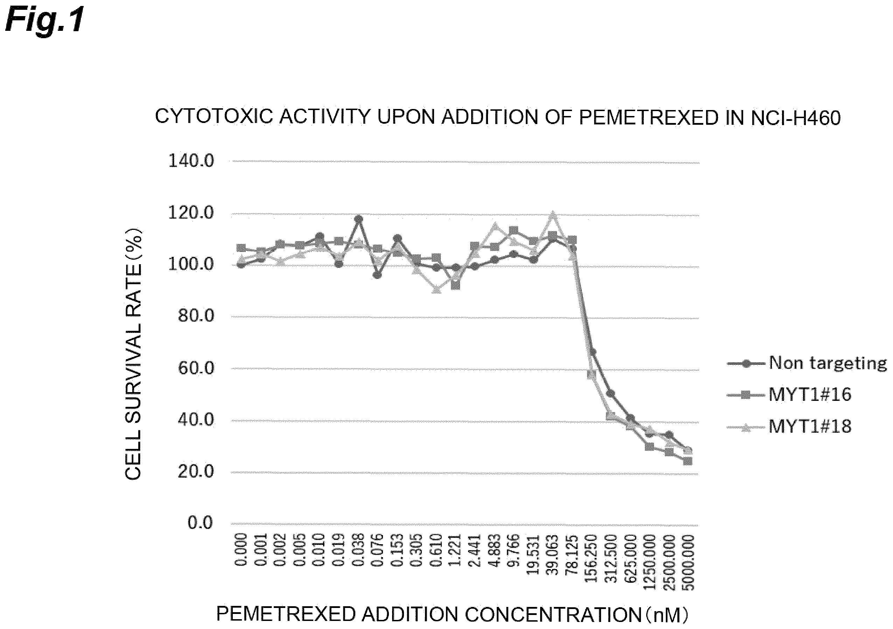

[Figure 1] Figure 1 is a graph showing the cytotoxic activity caused by pemetrexed treatment upon suppression of MYT1 expression of cancer cell line NCI-H460.

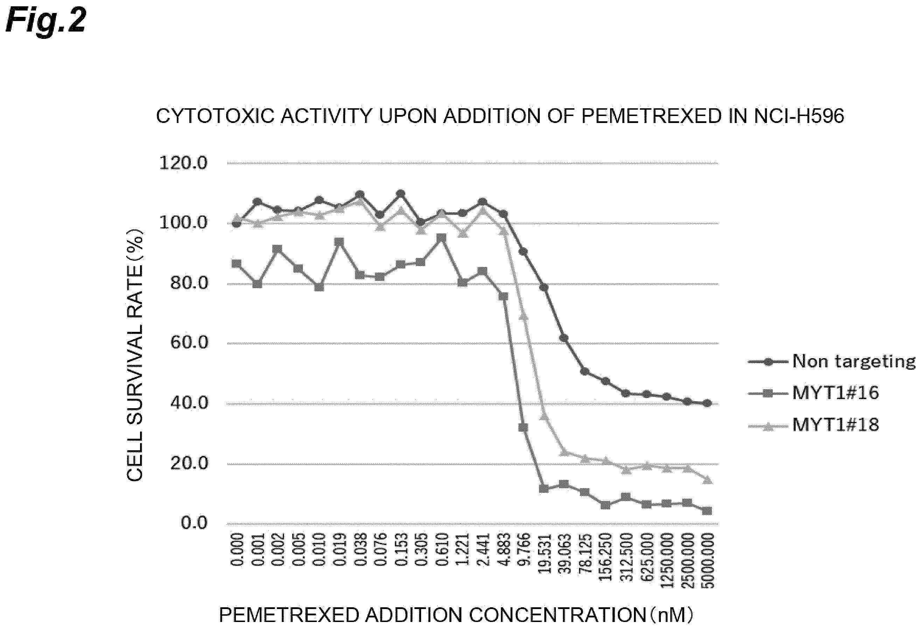

[Figure 2] Figure 2 is a graph showing the cytotoxic activity caused by pemetrexed treatment upon suppression of MYT1 expression of cancer cell line NCI-H596.

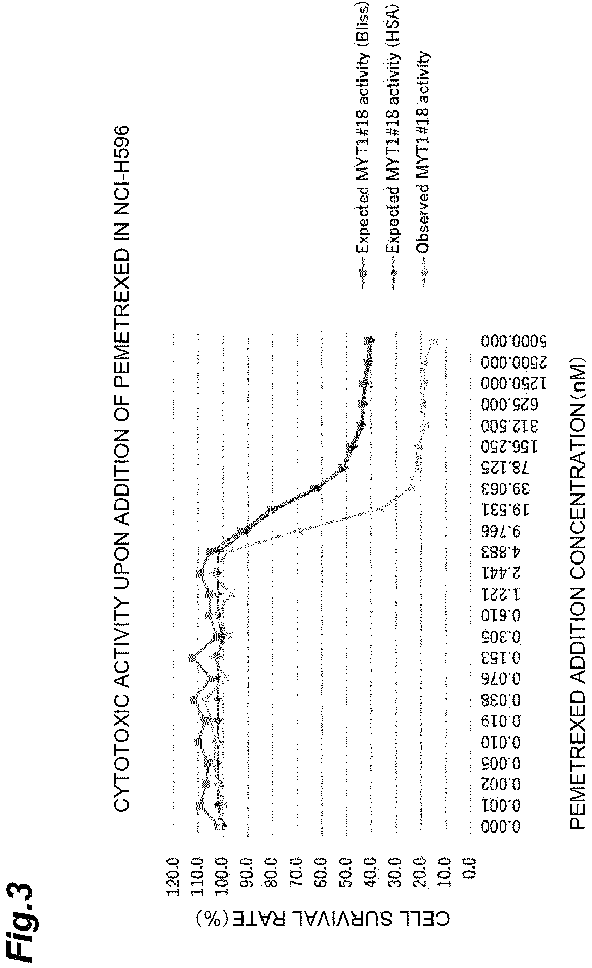

[Figure 3] Figure 3 is a graph showing the cytotoxic activity caused by pemetrexed treatment in cancer cell line NCI-H596.

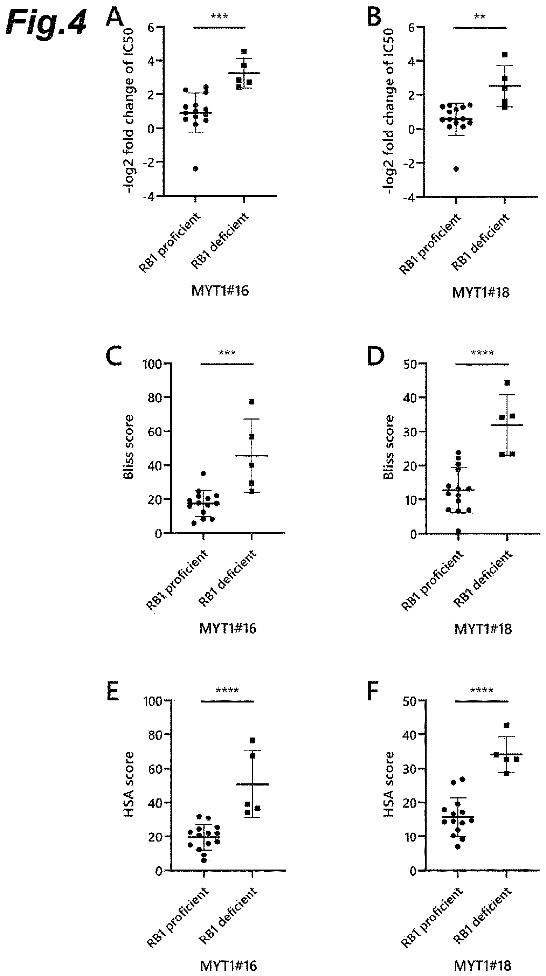

[Figure 4] Figures 4A and 4B are graphs showing the -Log2 values of the fold change of IC₅₀ upon treatment with MYT1 siRNA #16 or #18 in RB1 wild-type cell lines and RB1 mutant cell lines. Figures 4C and 4D are graphs showing the maximum values of the Bliss score upon treatment with MYT1 siRNA #16 or #18 in the RB1 wild-type cell lines and the RB1 mutant cell lines. Figures 4E and 4F are graphs showing the maximum values of the HSA score upon treatment with MYT1 siRNA #16 or #18 in the RB1 wild-type cell lines and the RB1 mutant cell lines.

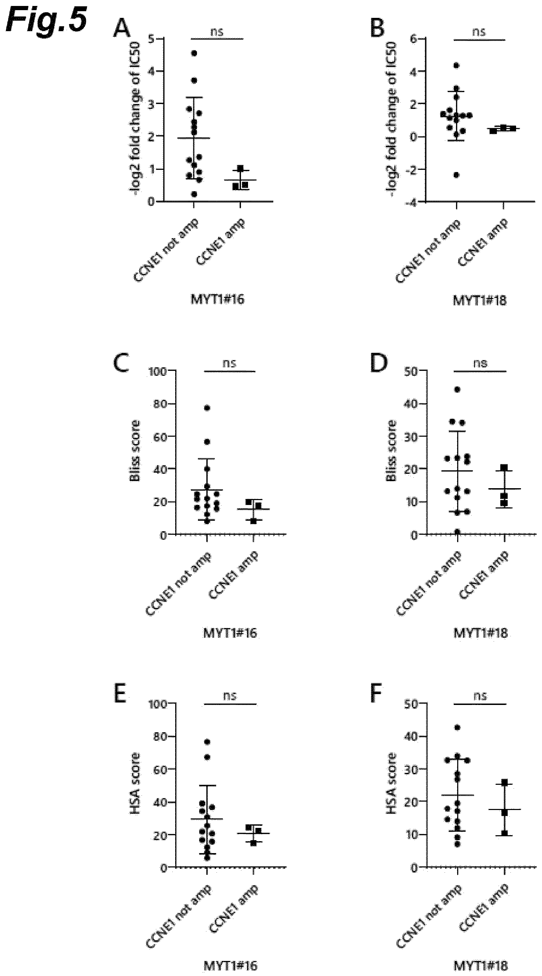

[Figure 5] Figures 5A and 5B are graphs showing the -Log2 values of the fold change of IC₅₀ upon treatment with MYT1 siRNA #16 or #18 in the CCNE1 gene-not-amplified cell lines and the CCNE1 gene-amplified cell lines. Figures 5C and 5D are graphs showing the maximum values of the Bliss score upon treatment with MYT1 siRNA #16 or #18 in the CCNE1 gene-not-amplified cell lines and the CCNE1 gene-amplified cell lines. Figures 5E and 5F are graphs showing the maximum values of the HSA score upon treatment with MYT1 siRNA #16 or #18 in the CCNE1 gene-not-amplified cell lines and the CCNE1 gene-amplified cell lines.

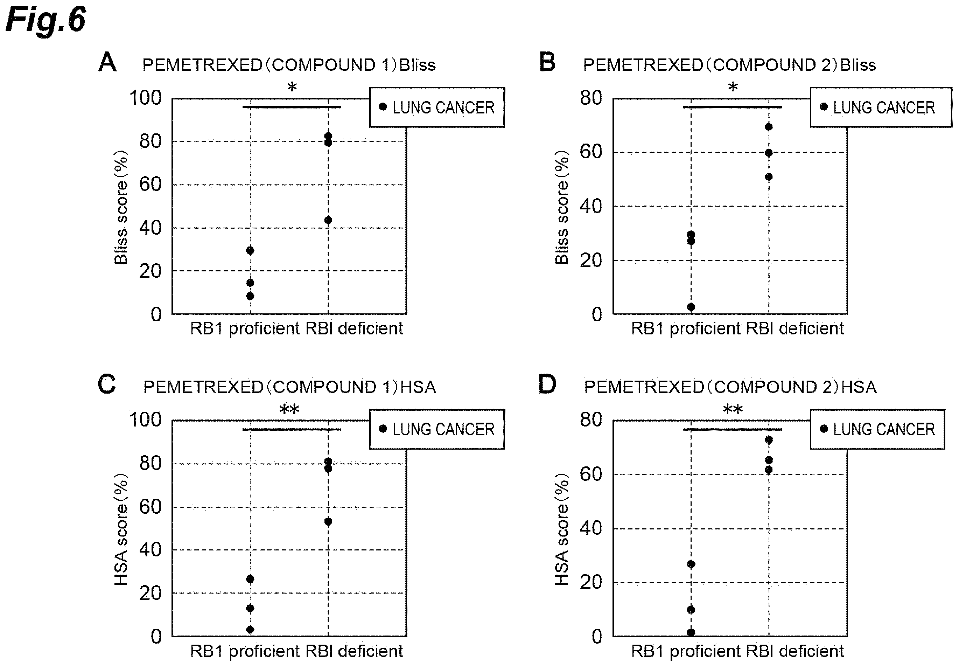

[Figure 6] Figures 6A and 6B are graphs showing the maximum values of the Bliss score when pemetrexed and the compound 1 or the compound 2 are added in RB1 wild-type cell lines (RB 1 proficient) and RB1 mutant cell lines (RB 1 deficient). Figures 6C and 6D are graphs showing the maximum values of HSA score when pemetrexed and the compound 1 or the compound 2 are added in the RB1 wild-type cell lines (RB1 proficient) and the RB1 mutant cell lines (RB1 deficient).

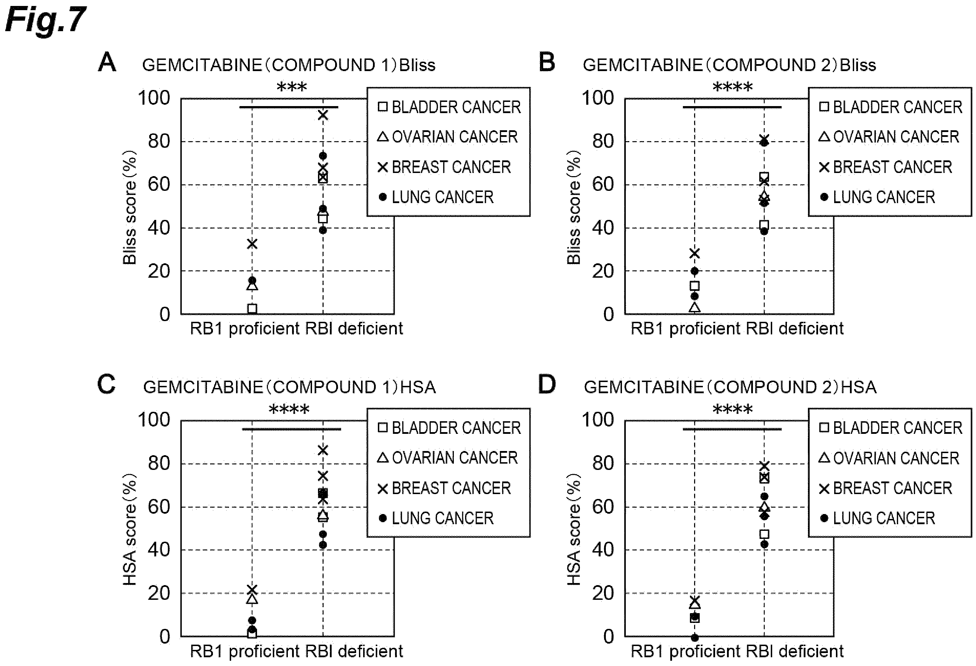

[Figure 7] Figures 7A and 7B are graphs showing the maximum values of the Bliss score when gemcitabine and the compound 1 or the compound 2 are added in RB1 wild-type cell lines (RB 1 proficient) and RB1 mutant cell lines (RB 1 deficient). Figures 7C and 7D are graphs showing the maximum values of the HSA score when gemcitabine and the compound 1 or the compound 2 are added in the RB1 wild-type cell lines (RB 1 proficient) and the RB1 mutant cell lines (RB 1 deficient).

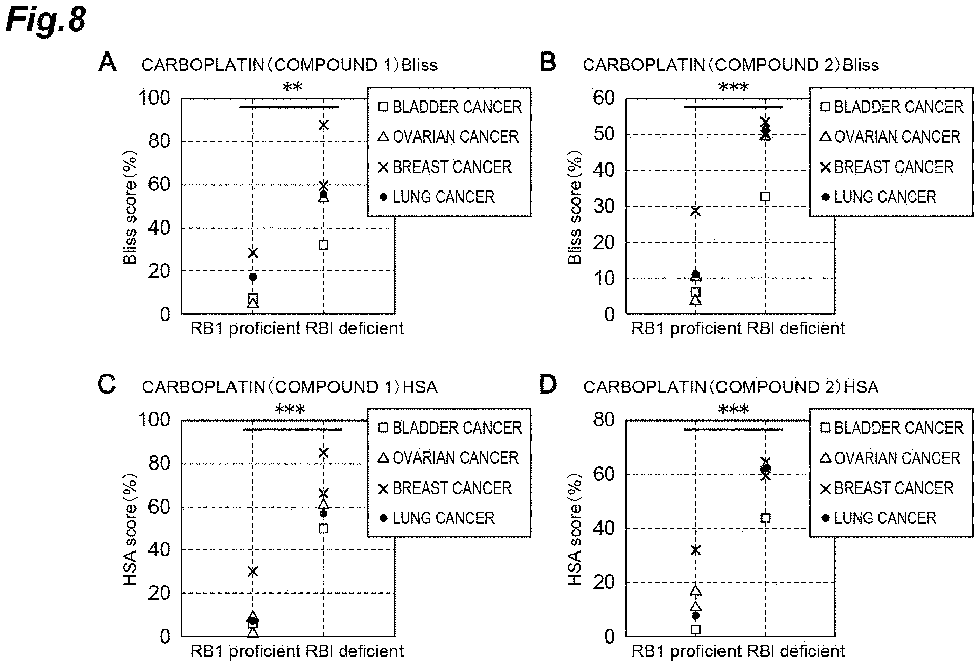

[Figure 8] Figures 8A and 8B are graphs showing the maximum values of the Bliss score when carboplatin and the compound 1 or the compound 2 are added in RB1 wild-type cell lines (RB 1 proficient) and RB1 mutant cell lines (RB1 deficient). Figures 8C and 8D are graphs showing the maximum values of the HSA score when carboplatin and the compound 1 or the compound 2 are added in the RB1 wild-type cell lines (RB1 proficient) and the RB1 mutant cell lines (RB1 deficient).

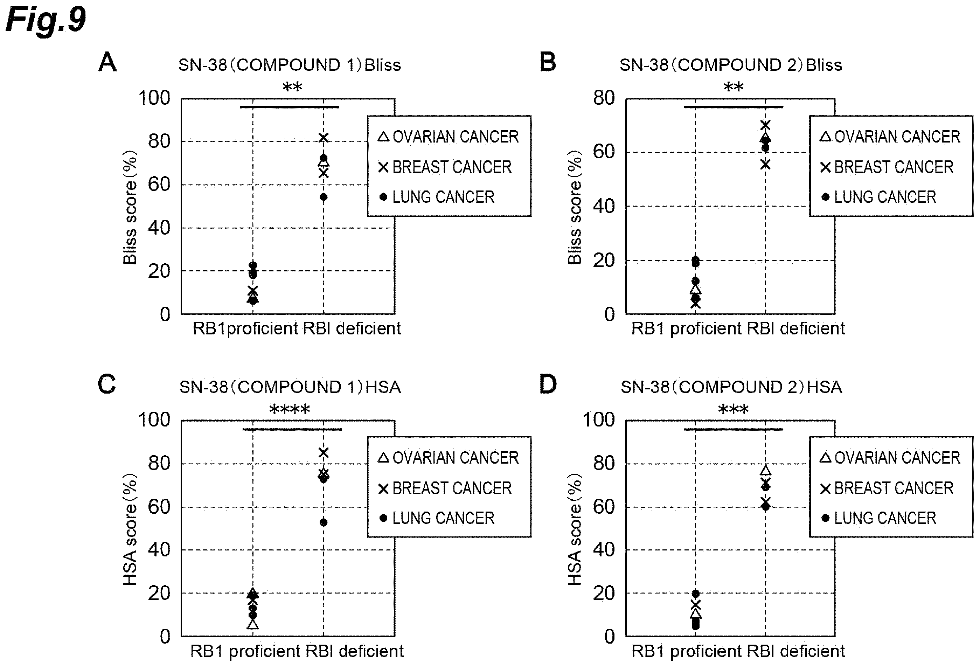

[Figure 9] Figures 9A and 9B are graphs showing the maximum values of the Bliss score when SN-38 and the compound 1 or the compound 2 are added in RB1 wild-type cell lines (RB1 proficient) and RB1 mutant cell lines (RB1 deficient). Figures 9C and 9D are graphs showing the maximum values of the HSA score when SN-38 and the compound 1 or the compound 2 are added in the RB1 wild-type cell lines (RB 1 proficient) and the RB1 mutant cell lines (RB 1 deficient).

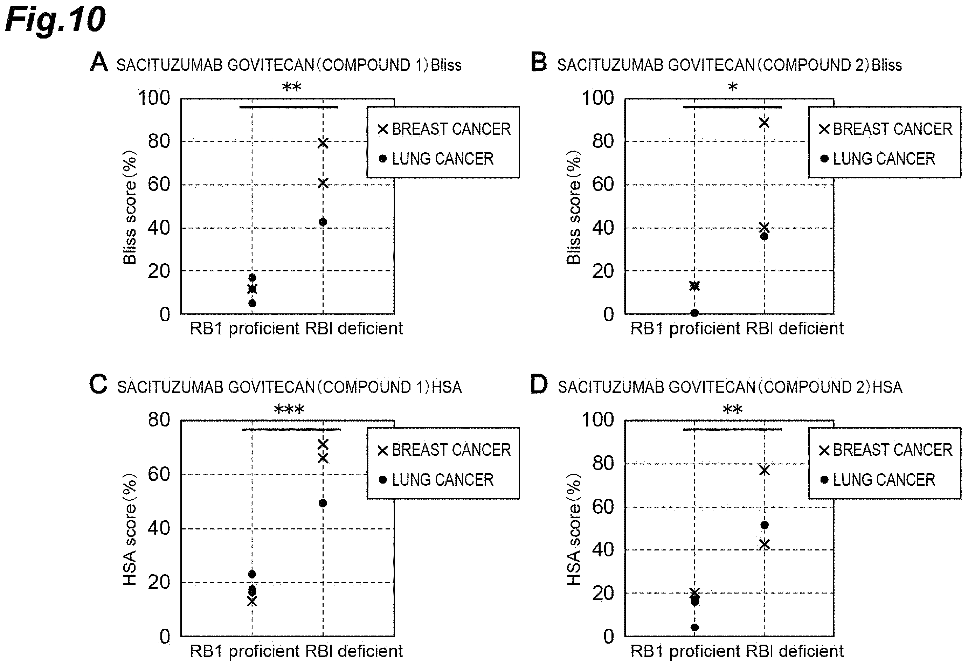

[Figure 10] Figures 10A and 10B are graphs showing the maximum values of the Bliss score when SG and the compound 1 or the compound 2 are added in RB1 wild-type cell lines (RB 1 proficient) and RB1 mutant cell lines (RB 1 deficient). Figures 10C and 10D are graphs showing the maximum values of the HSA score when SG and the compound 1 or the compound 2 are added in the RB1 wild-type cell lines (RB 1 proficient) and the RB1 mutant cell lines (RB 1 deficient).

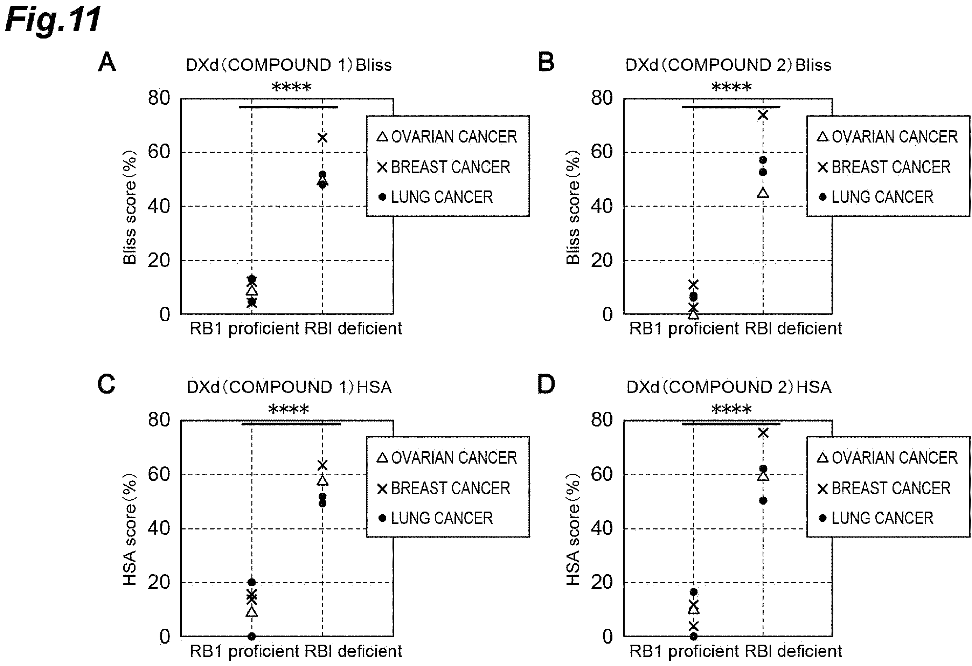

[Figure 11] Figures 11A and 11B are graphs showing the maximum values of Bliss score when DXd and the compound 1 or the compound 2 are added in RB1 wild-type cell lines (RB 1 proficient) and RB1 mutant cell lines (RB 1 deficient). Figures 11C and 11D are graphs showing the maximum values of the HSA score when DXd and the compound 1 or the compound 2 are added in the RB1 wild-type cell lines (RB 1 proficient) and the RB1 mutant cell lines (RB 1 deficient).

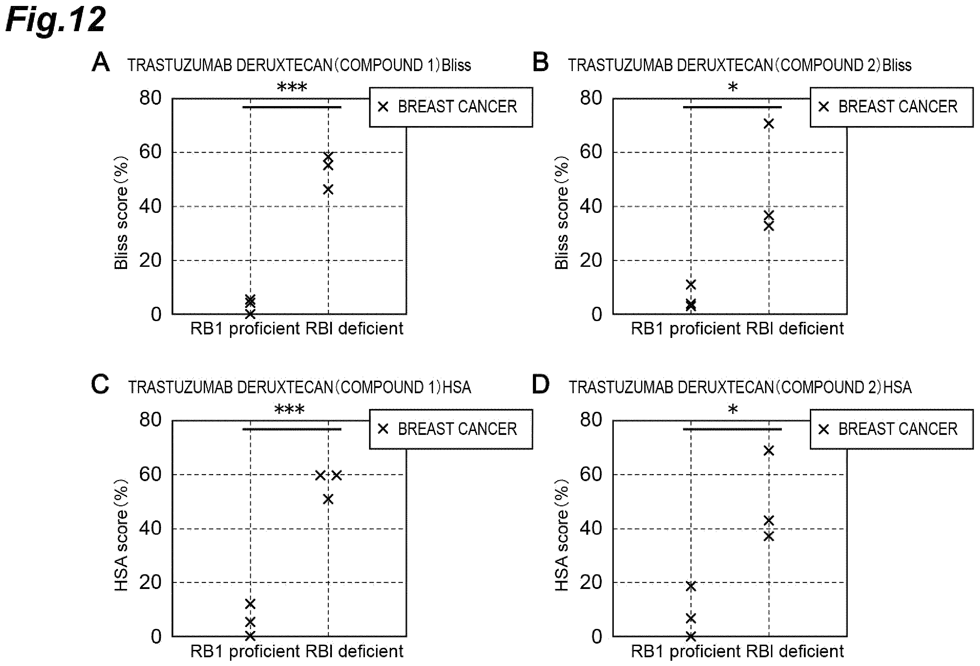

[Figure 12] Figures 12A and 12B are graphs showing the maximum values of Bliss score when T-DXd and the compound 1 or the compound 2 are added in RB1 wild-type cell lines (RB1 proficient) and RB1 mutant cell lines (RB 1 deficient). Figures 12C and 12D are graphs showing the maximum values of the HSA score when T-DXd and the compound 1 or the compound 2 are added in the RB1 wild-type cell lines (RB 1 proficient) and the RB1 mutant cell lines (RB1 deficient).

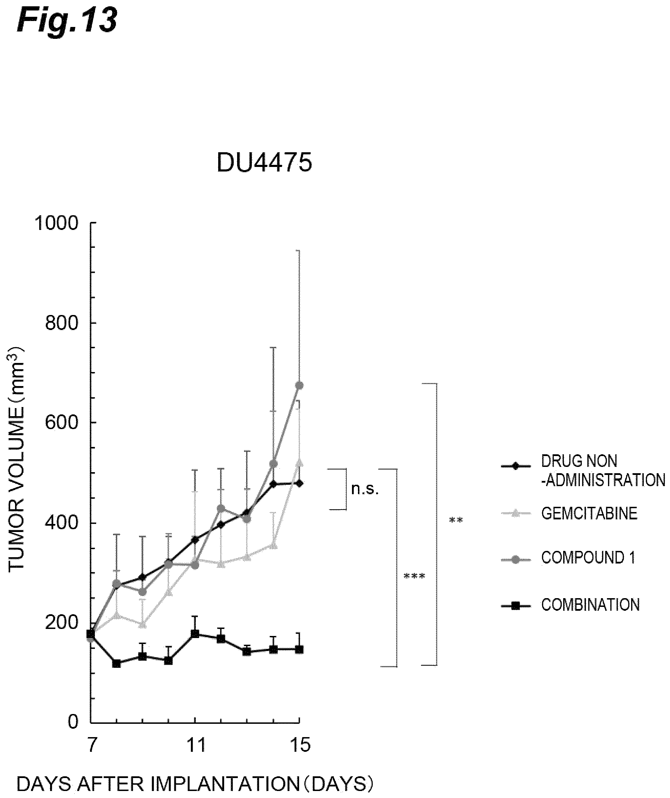

[Figure 13] Figure 13 is a diagram showing the change of tumor volume with time when the compound 1 and gemcitabine are administered in combination in DU4475 tumor-bearing mice.

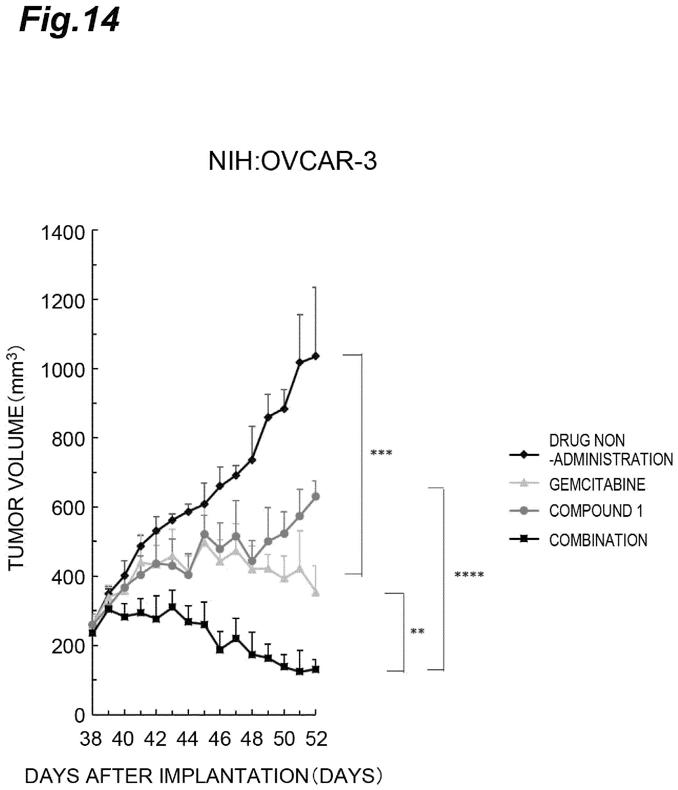

[Figure 14] Figure 14 is a diagram showing the change of tumor volume with time when the compound 1 and gemcitabine are administered in combination in OVCAR-3 tumor-bearing mice.

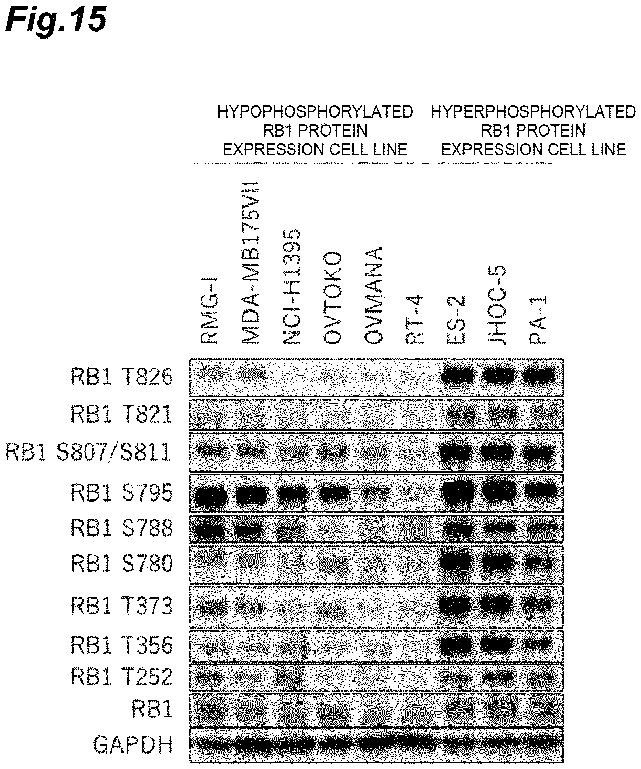

[Figure 15] Figure 15 shows the results of blotting obtained by using ChemiDoc Touch MP (manufactured by Bio-Rad Laboratories, Inc.).

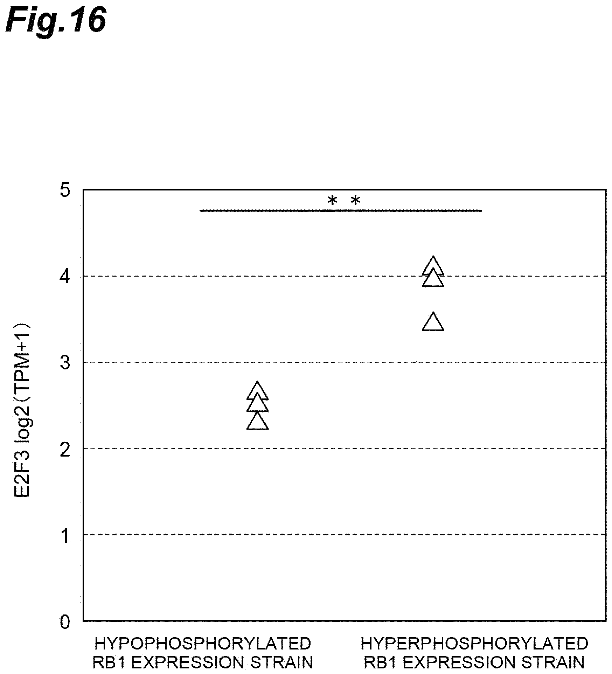

[Figure 16] Figure 16 is a plot showing a comparison in the expression level of the E2F3 gene in cell lines with positive expression of hypophosphorylated RB1 protein and cell lines with positive expression of hyperphosphorylated RB1 protein.

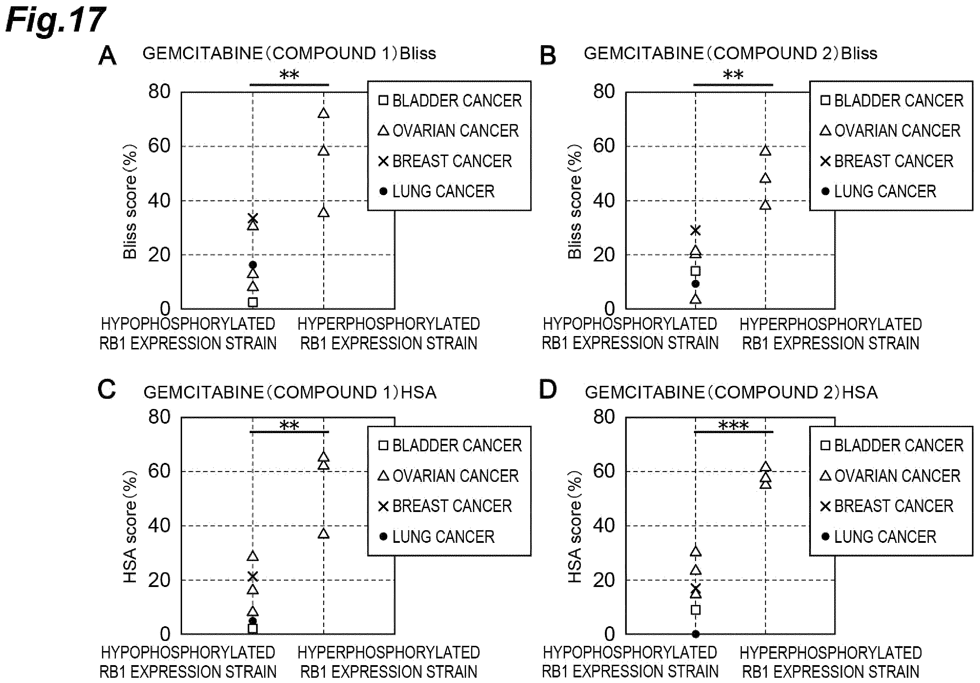

[Figure 17] Figures 17A to 17D are graphs plotting the maximum values of the Bliss score or the maximum values of the HSA score for cells with positive expression of hypophosphorylated RB1 protein and cells with positive expression of hyperphosphorylated RB1 protein. Figures 17A and 17B are graphs comparing the maximum values of the Bliss score in combined administration of gemcitabine and the compound 1 and combined administration of gemcitabine and the compound 2, respectively. Figures 17C and 17D are graphs comparing the maximum values of the HSA score in combined administration of gemcitabine and the compound 1 and combined administration of gemcitabine and the compound 2, respectively.

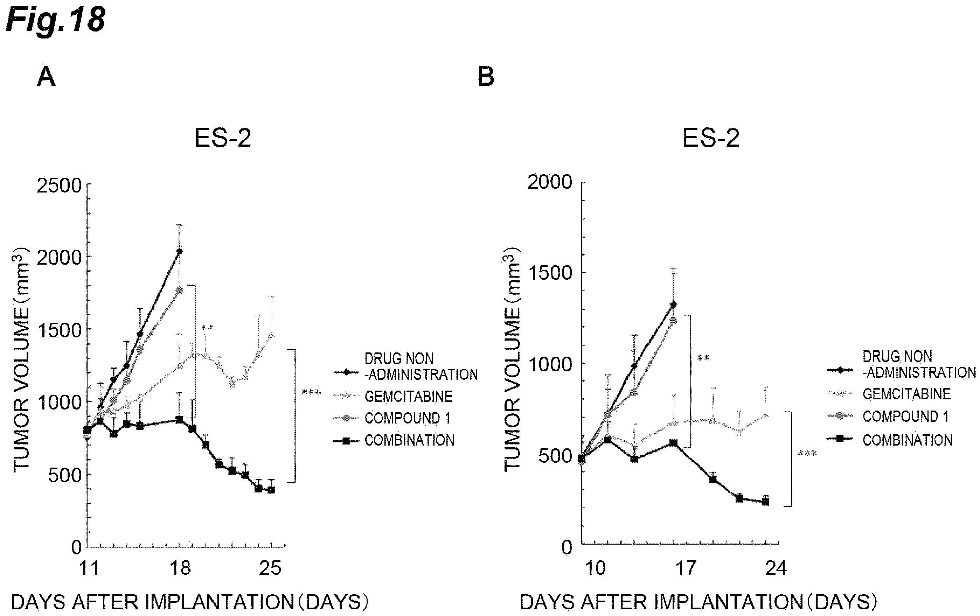

[Figure 18] Figure 18A is a graph showing the change of tumor volume with time after implantation of ES-2 in the drug non-administration group, the compound 1 single administration group, the gemcitabine single administration group, and the combination administration group. Figure 18B is a graph showing the change of tumor volume with time after implantation of ES-2 in the drug non-administration group, the compound 2 single administration group, the gemcitabine single administration group, and the combination administration group.

### [Description of Embodiments]

Embodiments of the present invention will be described. However, the present invention is not limited to the following embodiments. The medicament for treatment or prevention and the treatment or prevention method of the present invention may be administered or applied to humans. As used herein, the term "to" that indicates a range includes values of both ends thereof. For example, "A to B" means the range from A to B. The term "about" as used herein means, when used in combination with a numerical value, the range of +10% and - 10% of the numerical value. In the present invention, the meaning of the term "and/or" includes any combinations in which "and" and "or" are appropriately combined. Specifically, for example, "A, B, and/or C" includes the following seven variations; (i) A, (ii) B, (iii) C, (iv) A and B, (v) A and C, (vi) B and C, and (vii) A, B, and C.

The term "combined administration" means two or more ingredients are used in combination. For example, use of the MYT1 inhibitor (hereinafter, also referred to as the "first ingredient") and the chemotherapeutic agent (hereinafter, also referred to as the "second ingredient") in combination includes an "aspect in which a single formulation containing the first ingredient and second ingredient is administered" (that is, an aspect in which the first ingredient and the second ingredient are used in combination as a combination drug), and "an aspect in which each of the first ingredient and the second ingredient is simultaneously or separately administered as separate formulations". In the latter aspect, a formulation containing the first ingredient may be administered first, or a formulation containing the second ingredient may be administered first. The latter aspect may be any of an "aspect in which the first ingredient and the second ingredient are separately formulated and simultaneously administered by the same route of administration", an "aspect in which the first ingredient and second ingredient are separately formulated and separately administered by the same route of administration with a time difference", an "aspect in which the first ingredient and the second ingredient are separately formulated and simultaneously administered by different routes of administration (administered at different sites of the same patient)", and an "aspect in which the first ingredient and the second ingredient are separately formulated and separately administered by different routes of administration with a time difference". In the case of the "aspect in which the first ingredient and the second ingredient are separately formulated and simultaneously administered by the same route of administration", both formulations may be mixed immediately before administration. The term "separately" means that a certain formulation is administered before or after administration of another formulation.

In other words, the term "in combination" is said to be a use method for allowing another ingredient to be present in the body of a patient, in a state where one ingredient is present in the body of the patient. That is, an aspect in which the first ingredient and the second ingredient are administered so as to be simultaneously present in the body of a patient, for example, in the blood is preferable, and an aspect in which certain formulations are simultaneously administered to a patient, or, after a certain formulation is administered, another formulation is administered within 48 hours is preferable.

The "treatment of cancer" in the present invention means a decrease in the number of cancer cells in an individual, suppression of the growth of cancer cells, a decrease in tumor volume, a decrease in tumor weight, suppression of the metastasis of cancer cells, or amelioration of various symptoms caused by cancer, and combinations thereof. The "prevention of cancer" in the present invention means prevention of new cancer cells from occurring, prevention of an increase in the number of cancer cells due to regrowth of the decreased cancer cells, prevention of the regrowth of the cancer cells whose growth is suppressed, prevention of a re-increase of the decreased tumor volume or weight, and combinations thereof.

### [First embodiment]

One embodiment of the present invention is a pharmaceutical composition comprising, in combination with a chemotherapeutic agent, an MYT1 inhibitor as an active ingredient for treatment or prevention of cancer of a cancer patient in which RB1 gene mutation positivity, a decrease in expression of an RB1 gene or protein, or positive expression of hyperphosphorylated RB1 protein is detected.

RB1 (retinoblastoma gene, also called Rb or RB) is a gene encoding RB1 protein which is a typical cell cycle-regulating factor, and is involved in the G1/S checkpoint. RB1 protein (also called RB1 or pRb) suppresses the action of E2F by forming a complex with a transcription factor E2F which induces expression of a gene involved in the transition of cell cycle from the G1 phase to the S phase. When the action of E2F is suppressed, the transition from the G1 phase to the S phase is inhibited.

In cell cycle, typically, cyclin D is synthesized by the cell growth stimulation and bonds to CDK4 to form a complex. The complex is phosphorylated (activated) by CAK to phosphorylate RB protein. When RB protein is phosphorylated, the transcription factor E2F attached to RB protein is released, which induces the expression of the gene group necessary for the progress of the S phase or DNA replication. When the RB1 gene has a mutation (in particular, a mutation that decreases the function of the RB1 protein), the expression level of the RB1 gene or protein is decreased, or the RB1 gene is hyperphosphorylated, the cell cycle progresses.

As used herein, "RB1 gene mutation positivity" means that, when a nucleotide sequence corresponding to the RB1 gene of a subject is analyzed, any of the mutations (e.g., a mutation causing insertion, substitution, deletion, and/or addition of at least one amino acid residue to wild-type RB1 protein) is found in the nucleotide sequence to the nucleotide sequence of a wild-type RB1 gene, or when the mutation to the nucleotide sequence of the RB1 gene is reflected in the change of a base in a transcription product or the change of an amino acid in a translation product (e.g., insertion, substitution, deletion, and/or addition of at least one amino acid residue as compared with the wild-type RB1 protein), the change is detected in the transcription product or the translation product. In a specific embodiment, the RB1 gene mutation positivity is detected in a cancer patient-derived biological sample (e.g., cancer cell). The term "detecting a mutation" as used herein means that a mutation on genome DNA is detected in principle. When the mutation on genome DNA is reflected in a change of a base in a transcription product or a change of an amino acid in a translation product, the meaning also includes detection of the change of the transcription product or the translation product (i.e. indirect detection). The preferred aspect of the method of the present specification is a method for detecting a mutation by directly determining a nucleotide sequence of the region of a gene for RB1 in a cancer cell. The method for detecting the RB1 gene mutation positivity is not particularly limited, but for example, the RB1 gene mutation positivity can be confirmed and determined by next generation sequencer (NGS).

In the present invention, the term "region of a gene for RB1" means a certain region on genome DNA including a gene for RB1. The regions also each independently include, in addition to translated regions, untranslated regions such as an expression control region for the relevant gene (e.g. promotor region or enhancer region), a 3'-end untranslated region for the relevant gene, and the like. In this method, first, a DNA sample is prepared from a biological sample. Examples of the DNA sample include genome DNA samples, and cDNA samples prepared by reverse transcription from RNA.

The method for extracting genome DNA or RNA from a biological sample is not particularly limited, and a known method can be appropriately selected and used. Examples of the method for extracting genome DNA include a SDS phenol method (a method in which protein of a tissue stored in a urea-containing solution or ethanol is denatured with a proteinase (proteinase K), a surfactant (SDS) and phenol, and DNA is precipitated and extracted from the tissue with ethanol), and DNA extraction methods using Clean Columns (registered trademark, manufactured by NexTec Co., Ltd.), AquaPure (registered trademark, manufactured by Bio-Rad Laboratories, Inc.), ZR Plant/Seed DNA Kit (manufactured by Zymo Research), AquaGenomicSolution (registered trademark, manufactured by Mo Bi Tec GmbH), prepGEM (registered trademark, manufactured by ZyGEM LLC) and BuccalQuick (registered trademark, manufactured by TrimGen Corporation).

The method for extracting RNA from a biological sample and the method for preparing cDNA from extracted RNA is not particularly limited, and a known method can be appropriately selected and used. Examples thereof include extraction methods using phenol and a chaotropic salt (more specifically, extraction methods using a commercially available kit such as TRIzol (manufactured by Invitrogen Corporation) or ISOGEN (manufactured by Wako Pure Chemical Industries, Ltd.)), and methods using another commercially available kit (e.g. RNAPrep Total RNA Extraction Kit (manufactured by Beckman Coulter Inc.), RNeasy Mini (manufactured by QIAGEN N.V.) or RNA Extraction Kit (manufactured by Pharmacia Biotech, Inc.)). Further, the reverse transcriptase used for preparation of cDNA from extracted RNA is not particularly limited, and examples thereof include reverse transcriptases derived from retroviruses such as RAV (Rous associated virus) and AMV (Avian myeloblastosis virus), and reverse transcriptases derived from mouse retroviruses such as MMLV (Moloney murine leukemia virus).

In this aspect, subsequently, DNA containing the region of a gene for RB1 is isolated, and the nucleotide sequence of the isolated DNA is determined. The isolation of DNA can be performed by PCR with genome DNA or RNA as a template, or the like using a pair of oligonucleotide primers designed to sandwich all or part of the region of a gene for RB1. The determination of the nucleotide sequence of the isolated DNA can be performed by a method known to those skilled in the art, such as a Maxam-Gilbert method or a Sanger method. It is also possible to use a next-generation sequencer which enables analysis such that nucleotide sequences of genes can be read rapidly and comprehensively, etc.

By comparing the determined nucleotide sequence of DNA or cDNA (for example, when the biological sample is a cancer patient-derived sample, the nucleotide sequence of DNA or cDNA derived from a non-cancer tissue of the same patient, or a known database), the presence or absence of a mutation in the region of a gene for RB1 in a cancer cell of the biological sample can be determined.

As the method for detecting a mutation in the region of a gene for RB1, various methods capable of detecting a mutation can be used in addition to methods for directly determining the nucleotide sequence of DNA or cDNA.

For example, the detection of a mutation in the present invention can also be performed by the following method. First, a DNA or cDNA sample is prepared from a biological sample. Subsequently, an oligonucleotide probe is prepared which has a nucleotide sequence complementary to a nucleotide sequence containing a mutation site of the region of a gene for RB1 and is labeled with a reporter fluorescent dye and a quencher fluorescent dye. The oligonucleotide probe is hybridized to the DNA or cDNA sample, and the nucleotide sequence containing the mutation site of the region of a gene for RB1 is amplified using, as a template, the DNA or cDNA sample to which the oligonucleotide probe is hybridized. Fluorescence generated by the reporter fluorescent dye due to degradation of the oligonucleotide probe which is caused by the amplification is detected, and the detected fluorescence is then compared to a control. Examples of such a method include a double-dye probe method, so called a TaqMan (registered trademark) probe method.

In still another method, a DNA or cDNA sample is prepared from a biological sample. Subsequently, in a reaction system containing an intercalator which generated fluorescence when inserted between DNA double strands, a nucleotide sequence containing a mutation site of the region of a gene for RB1 is amplified using the DNA or cDNA sample as a template. The temperature of the reaction system is changed, a variation in intensity of fluorescence generated by the intercalator is detected, and the variation in intensity of the fluorescence with the detected change in temperature is compared to a control. Examples of such a method include a HRM (high resolution melting) analysis method.

In still another method, first, a DNA or cDNA sample is prepared from a biological sample. Subsequently, DNA containing all or part of the region of a gene for RB1 is amplified. Further, the amplified DNA is cleaved by a restriction enzyme. Subsequently, DNA fragments are separated according to the sizes thereof. Subsequently, the size of the detected DNA fragment is compared to a control. Examples of such a method include methods utilizing restriction fragment length polymorphism (RFLP), and a PCR-RFLP method.

In still another method, first, a DNA or cDNA sample is prepared from a biological sample. Subsequently, DNA containing all or part of the region of a gene for RB1 is amplified. Further, the amplified DNA is dissociated into single-stranded DNA. Subsequently, the dissociated single-stranded DNA is separated on a non-denaturing gel. The mobility of the separated single-stranded DNA on the gel is compared to a control. Examples of such a method include a PCR-SSCP (single-strand conformation polymorphism) method.

In still another method, first, a DNA or cDNA sample is prepared from a biological sample. Subsequently, DNA containing all or part of the region of a gene for RB1 is amplified. Further, the amplified DNA is separated on a gel in which the concentration of a DNA denaturant increases in steps. Subsequently, the mobility of the separated DNA on the gel is compared to a control. Examples of such a method include a denaturant gradient gel electrophoresis (DGGE) method.

As still another method, there is a method using DNA prepared from a biological sample and containing a mutation site of the region of a gene for RB1, and a substrate on which an oligonucleotide probe hybridized to the DNA is fixed. Examples of such a method include a DNA array method.

In still another method, first, a DNA or cDNA sample is prepared from a biological sample. An "oligonucleotide primer having a nucleotide sequence complementary to bases on the 3' side of the bases of all or part of the region of a gene for RB1 by one base and a nucleotide sequence on the 3' side thereof" is prepared. Subsequently, with the DNA as a template, a ddNTP primer elongation reaction is carried out using the primer. Subsequently, the primer elongation reaction product is applied to a mass analyzer to perform mass measurement. Subsequently, the gene type is determined from the result of the mass measurement. Subsequently, the determined gene type is compared to a control. Examples of such a method include a MALDI-TOF/MS method.

In still another method, first, a DNA or cDNA sample is prepared from a biological sample. Subsequently, an oligonucleotide probe consisting of 5'-"nucleotide sequence complementary to the bases of all or part of the region of a gene for RB1 and a nucleotide sequence on the 5' side thereof"-"nucleotide sequence which is not hybridized to bases on the 3' side of all or part of the region of a gene for RB1 by one base and a nucleotide sequence on the 3' side thereof"-3' (flap) is prepared. An "oligonucleotide probe having a nucleotide sequence complementary to the bases of all or part of the region of a gene for RB1 and a nucleotide sequence on the 3' side thereof" is prepared. Subsequently, the two oligonucleotide probes are hybridized to the prepared DNA or cDNA sample. Subsequently, the hybridized DNA is cleaved by a single-stranded DNA cleavage enzyme to liberate the flap. The single-stranded DNA cleavage enzyme is not particularly limited, and examples thereof include cleavases. In this method, subsequently, an oligonucleotide probe which has a sequence complementary to the flap and is labeled with reporter fluorescence and quencher fluorescence is hybridized to the flap. Subsequently, the intensity of generated fluorescence is measured. Subsequently, the measured fluorescence intensity is compared to a control. Examples of such a method include an Invader method.

In still another method, first, a DNA or cDNA sample is prepared from a biological sample. Subsequently, DNA containing all or part of the region of a gene for RB1 is amplified. The amplified DNA is dissociated into single-stranded DNA, and only one strand is separated from the dissociated single-stranded DNA. Subsequently, an elongation reaction is carried out base by base from near the bases of all or part of the region of a gene for RB1, pyrophosphoric acid generated at this time is enzymatically caused to emit light, and the intensity of emission is measured. The measured fluorescence intensity is compared to a control. Examples of such a method include a Pyrosequencing method.

In still another method, first, a DNA or cDNA sample is prepared from a biological sample. Subsequently, DNA containing all or part of the region of a gene for RB1 is amplified. Subsequently, an "oligonucleotide primer having a nucleotide sequence complementary to bases on the 3' side of the bases of all or part of the region of a gene for RB1 by one base and a nucleotide sequence on the 3' side thereof" is prepared. Subsequently, with the amplified DNA as a template, a single-base elongation reaction is carried out using the prepared primer in the presence of a fluorescently labeled nucleotide. The polarization degree of fluorescence is measured. Subsequently, the measured polarization degree of fluorescence is compared to a control. Examples of such a method include an AcycloPrime method.

In still another method, first, a DNA or cDNA sample is prepared from a biological sample. Subsequently, DNA containing all or part of the region of a gene for RB1 is amplified. Subsequently, an "oligonucleotide primer having a nucleotide sequence complementary to bases on the 3' side of the bases of all or part of the region of a gene for RB1 by one base and a nucleotide sequence on the 3' side thereof" is prepared. Subsequently, with the amplified DNA as a template, a single-base elongation reaction is carried out using the prepared primer in the presence of a fluorescently labeled nucleotide. Subsequently, the type of base used for the single-base elongation reaction is determined. Subsequently, the determined type of base is compared to a control. Examples of such a method include a SNuPE method.

When the mutation is associated with a change of an amino acid in RB1 protein, the sample prepared from the biological sample may be protein. Here, for detecting a mutation, a method using a molecule binding specifically to a site at which a change of amino acids occurs due to the mutation, peptide mass fingerprinting method (PMF), a protein sequencer (Edman degradation method), or the like can be used.

As used herein, "a decrease in the expression of an RB1 gene or protein" means that, when an RB1 gene or protein of a subject is analyzed, the expression level of the RB1 gene or protein thereof is lower compared to that of the control (e.g., the expression level in a healthy individual or in a non-cancer tissue of the same patient). In a specific embodiment, the decrease in the expression level of the RB1 gene or protein is detected in a cancer patient-derived biological sample (e.g., cancer cell).

Examples of the method for detecting a decrease in the expression of an RB1 gene include, but are not particularly limited to, a method in which the expression level of RB1 is detected at a transcriptional level or a translational level, and compared to the control. In the method for detecting the expression level of an RB1 gene at a transcriptional level, first, RNA or cDNA is prepared from a biological sample. The method for extracting RNA from a biological sample and the method for preparing cDNA from extracted RNA is not particularly limited, and a known method can be appropriately selected and used. Examples thereof include extraction methods using phenol and a chaotropic salt (more specifically, extraction methods using a commercially available kit such as TRIzol (manufactured by Invitrogen Corporation) or ISOGEN (manufactured by Wako Pure Chemical Industries, Ltd.)), and methods using another commercially available kit (e.g. RNAPrep Total RNA Extraction Kit (manufactured by Beckman Coulter Inc.), RNeasy Mini (manufactured by QIAGEN N.V.) or RNA Extraction Kit (manufactured by Pharmacia Biotech, Inc.)). Further, the reverse transcriptase used for preparation of cDNA from extracted RNA is not particularly limited, and examples thereof include reverse transcriptases derived from retroviruses such as RAV (Rous associated virus) and AMV (Avian myeloblastosis virus), and reverse transcriptases derived from mouse retroviruses such as MMLV (Moloney murine leukemia virus).

Subsequently, an oligonucleotide primer or an oligonucleotide probe is used for an amplification reaction or a hybridization reaction, and an amplified product or a hybrid product thereof is detected. As such a method, for example, a RT-PCR method, a Northern blot method, a dot blot method, a DNA array method, an in situ hybridization method, a RNase protection assay method, mRNA-seq, or the like can be used. Those skilled in the art can design an oligonucleotide primer or an oligonucleotide probe suitable for each method in a conventional manner on the basis of a nucleotide sequence of cDNA for RB1.

It is known in the art that one of causes of a decrease in expression of a gene is excessive methylation of a promotor. Therefore, the presence or absence of a suppressed function of RB1 may be detected using methylation of a gene promotor for RB1 as an indicator. For detection of methylation of a promotor, it is possible to use, for example, a known method such as a method in which a change of a nucleotide sequence after treatment with bisulfite having an activity that converts methylated cytosine into uracil is directly detected by determination of the nucleotide sequence, or indirectly detected using a restriction endonuclease which can recognize (cleave) a nucleotide sequence before the bisulfite treatment and cannot recognize (cleave) a nucleotide sequence after the bisulfite treatment.

The method for detecting the decrease in expression of RB1 protein is not particularly limited, but for example, the decrease can be confirmed and determined by IHC (immunohistochemistry) using an antibody specific to RB1 protein. In a method for detecting protein using an antibody, first, a protein sample is prepared from a biological sample. Subsequently, using an antibody specific to RB1 protein, an antigen-antibody reaction is used, and RB1 protein is detected. When the sample is labeled with antibody specific to RB1 protein, RB1 protein can be directly detected, and when the sample is not labeled, a labeled molecule which recognizes the antibody (e.g. secondary antibody or protein A) can be further applied to indirectly detect RB1 protein using the label of the molecule. As such a method, for example, an immunohistochemistry (immunostaining) method, a Western blotting method, an ELISA method, flow cytometry, imaging cytometry, radioimmunoassay, an immunoprecipitation method, or an analysis method using an antibody array can be used. This method also has an advantage that additional information such as a form or a distribution state of cancer cells in a tissue can also be obtained immunohistochemically.

The type, the origin, and the like of an antibody used are not particularly limited, and a monoclonal antibody is preferable. An oligoclonal antibody (mixture of several antibodies or dozens of antibodies) or a polyclonal antibody can also be used as long as it is possible to detect RB1 protein with sufficient specificity. Functional fractions of antibodies such as Fab, Fab', F(ab')₂, Fv, scFv, sc(Fv)₂, dsFv and diabodies, and multimers (e.g. dimers, trimers, tetramers and polymers) thereof can also be used. Such an anti-RB1 protein antibody may be a marketed product.

The RB1 protein can also be detected by mass spectrometry (MS). In particular, analysis by a mass spectrometer coupled with liquid chromatography (LC/MS) is sensitive, and therefore advantageous. Detection by mass spectrometry can be performed by, for example, labeling the protein sample with the protein, fractionating the labeled protein, subjecting the fractionated protein to mass analysis, and identifying RB1 protein from the mass analysis value. As the label, an isotopic labeling reagent known in the art can be used, and an appropriate labeling reagent can be obtained as a marketed product. The fractionation can be performed by a method known in the art, and for example, a commercially available ion-exchange column or the like can be used.

As used herein, the amplification of the copy number of the CCNE1 gene can be determined in the diagnosis or prognostic assay by an evaluation of the copy number of the CCNE1 gene using a cancer patient-derived biological sample (e.g., next generation sequencer, digital PCR, array CGH method, or FISH method).

As used herein, the "patient" may be mice, rats, guinea pigs, monkeys, dogs, sheep, horses, or humans. In the present invention, the "cancer patient" may be not only those affected with a cancer, but those possibly affected with a cancer. In a specific embodiment, a subject to be treated or prevented is a cancer patient in which an increase in the CCNE1 gene is not detected as compared to a control. In addition, in a specific embodiment, the subject to be treated or prevented is not a mouse implanted with OVCAR3. The pharmaceutical composition is suitably applicable to humans.

The "cancer patient-derived biological sample" as used herein is not particularly limited as long as it is a biological sample allowing the presence or absence of the RB1 gene mutation, the presence or absence of the decrease in expression of an RB1 gene or protein, or the presence or absence of the hyperphosphorylated RB1 protein to be detected, and a specimen material such as a cancer biopsy specimen material, blood, urine, body cavity fluid or tumor cell-derived circulating DNA (circulating tumor DNA: ctDNA). The cancer patient-derived biological sample may be a protein extract or a nucleic acid extract obtained from the specimen material (e.g. an mRNA extract, or a cDNA preparation or a cRNA preparation prepared from an mRNA extract). The "biological sample" as used herein includes cancer patient-derived samples and cancer cell culture-derived samples.

The RB1 gene mutation may include mutations that cause insertion, deletion, or addition of at least one amino acid residue, or substitution of an existing amino acid residue to the wild-type RB1 protein. The RB1 gene mutation may be a nonsense mutation, a frameshift mutation, a splice site mutation, or a homozygous or heterozygous deletion. The RB1 gene mutation is preferably a mutation that decreases the function of RB1. The "mutation that decreases the function of RB1" can be confirmed, for example, by Internet <URL: https://www.oncokb.org/gene/RB1> [searched on February 20, 2023].

A nucleotide sequence of typical DNA (cDNA) of a wild-type human RB1 gene is set forth as SEQ ID NO: 1 (NCBI reference number: NM_000321.3), and a typical amino acid sequence of a wild-type human RB1 gene is set forth as SEQ ID NO: 2 (NCBI reference number: NP_000312.2). In the case of the human RB1 gene, the RB1 gene mutation may include a mutation that causes a base sequence different from the human RB1 genomic sequences set forth as 48, 303, 751 to 48, 481, 890 of NCBI reference number: NC_13.11, a base sequence different from the human RB1 base sequences set forth as 4921 to 5161 of NCBI reference number: NG_9009.1, or an amino acid sequence different from the amino acid sequence of human RB1 protein set forth as SEQ ID No: 2, and may be a mutation that causes at least one of the following (1) to (5). Note that, sequences may have individual differences depending on the polymorphism and the like, even in RB1 having no mutation.
(1) The codon corresponding to the serine residue (S) at position 82 of the amino acid sequence of SEQ ID No: 2 is replaced with a stop codon,
(2) the codon corresponding to the arginine residue (R) at position 467 of the amino acid sequence of SEQ ID No: 2 is replaced with a stop codon,
(3) in the codon corresponding to the amino acid residue at position 182 of the amino acid sequence of SEQ ID No: 2, at least one base is inserted or deleted, a new reading frame starting from an isoleucine residue (I) is formed, and the third reading frame therefrom is a stop codon,
(4) the glutamic acid residue (E) at position 837 of the amino acid sequence of SEQ ID No: 2 is replaced with a lysine residue (K), and a part of the RB1 gene is homozygously deleted, and
(5) the glycine residue (G) at position 449 of the amino acid sequence of SEQ ID No: 2 is replaced with a glutamic acid residue (E), and a part of the RB1 gene is homozygously deleted.

As used herein, the decrease in expression of an RB1 gene or protein includes a decrease in gene expression through methylation of the RB1 gene or micro RNA.

As used herein, "hyperphosphorylated RB1 protein" refers to RB1 protein having 2 or more phosphorylated amino acid residues (also referred to as phosphorylated sites) in the amino acid sequence of the RB1 protein, and examples thereof include RB1 proteins having preferably 4 or more phosphorylated sites, more preferably 8 or more phosphorylated sites, and most preferably 15 or more phosphorylated sites.

In the case of human RB1 protein, for example, the "positive expression of hyperphosphorylated RB1 protein" may be determined from phosphorylation of at least one amino acid residue selected from the group consisting of a threonine residue (T) at position 826, a threonine residue (T) at position 823, a threonine residue (T) at position 821, a serine residue (S) at position 816, a tyrosine residue (Y) at position 813, a serine residue (S) at position 811, a serine residue (S) at position 807, a tyrosine residue (Y) at position 805, a serine residue (S) at position 780, a threonine residue (T) at position 625, a threonine residue (T) at position 601, a threonine residue (T) at position 373, a serine residue (S) at position 360, a threonine residue (T) at position 356, a serine residue (S) at position 249, and a serine residue (S) at position 37 of SEQ ID No: 2 (NCBI reference number: NP_000312.2) which is a typical amino acid sequence of human wild-type RB1 protein.

In the case of human RB1 protein, the "positive expression of hyperphosphorylated RB1 protein" is preferably determined from phosphorylation of at least one amino acid residue selected from the group consisting of a threonine residue (T) at position 826, a threonine residue (T) at position 821, a serine residue (S) at position 811, a serine residue (S) at position 807, a serine residue (S) at position 780, a threonine residue (T) at position 373, and a threonine residue (T) at position 356 of SEQ ID No: 2.

In the case of human RB1 protein, the "positive expression of hyperphosphorylated RB1 protein" is more preferably determined from phosphorylation of at least one amino acid residue selected from the group consisting of a threonine residue (T) at position 826, a threonine residue (T) at position 821, a serine residue (S) at position 811, and a serine residue (S) at position 807 of SEQ ID No: 2.

In the case of human RB1 protein, the "positive expression of hyperphosphorylated RB1 protein" is further preferably determined from phosphorylation of at least two amino acid residues selected from the group consisting of a threonine residue (T) at position 826, a threonine residue (T) at position 821, and a serine residue (S) at position 811 and/or a serine residue (S) at position 807.

In the case of human RB1 protein, the "positive expression of hyperphosphorylated RB1 protein" is most preferably determined from phosphorylation of a threonine residue (T) at position 826 and a threonine residue (T) at position 821 of SEQ ID No: 2, and phosphorylation of a serine residue (S) at position 811 and/or a serine residue (S) at position 807.

In a specific embodiment, the hyperphosphorylated RB1 protein is detected in a cancer patient-derived biological sample (e.g., cancer cell).

The positive expression of hyperphosphorylated RB1 protein can be, without particular limitation, but for example, detected and determined by IHC (immunohistochemistry) using an antibody specific to phosphorylated RB1 protein. It is also possible to use an antibody specific to phosphorylated RB1 protein in which a plurality of amino acid residues is phosphorylated, in combination. In a method for detecting protein using an antibody, first, a protein sample is prepared from a biological sample. Subsequently, using an antibody specific to phosphorylated RB1 protein, an antigen-antibody reaction is used, and phosphorylated RB1 protein is detected. When the sample is labeled with the antibody specific to phosphorylated RB1 protein, phosphorylated RB1 protein can be directly detected, but when the sample is not labeled, a labeled molecule which recognizes the antibody (e.g., secondary antibody or protein A) can be further applied to indirectly detect phosphorylated RB1 protein using the label of the molecule. As such a method, for example, an immunohistochemistry (immunostaining) method, a Western blotting method, an ELISA method, flow cytometry, imaging cytometry, radioimmunoassay, an immunoprecipitation method, or an analysis method using an antibody array can be used. This method also has an advantage that additional information such as a form or a distribution state of cancer cells in a tissue can also be obtained immunohistochemically.

In a specific embodiment, the pharmaceutical composition contains an MYT1 inhibitor (the first ingredient) as an active ingredient. MYT1 protein is a protein that is also referred to as membrane-associated tyrosine- and threonine-specific CDC2-inhibitory kinase and is encoded by a PKMYT1 gene. MYT1 protein is mainly localized in endoplasmic reticulum and Golgi bodies, is a type of Wee kinases including Wee1 protein and Wee1b protein, and negatively regulates CDK1-cyclin-B complexes that promote the progress from the G2 phase to the M phase. Usually, the MYT1 protein is inactivated at the start of the M phase of the cell cycle, and negatively acts in the DNA replication checkpoint phase (G2/M phase). Overexpression of MYT1 is observed in various carcinomas including hepatocellular carcinoma and clear cell renal cell carcinoma.

The MYT1 inhibitor according to the present embodiment may be at least one selected from the group consisting of a low molecular compound, a polypeptide, and a polynucleotide. When the MYT1 inhibitor is a low molecular compound, a compound having a molecular weight of 2000 g/mol or less is preferable, and a compound having a molecular weight of 1000 g/mol or less is more preferable as the MYT1 inhibitor. When the MYT1 inhibitor is a polypeptide, an antibody (e.g., anti-MYT1 antibody) may be used as the MYT1 inhibitor. When the MYT1 inhibitor is a polynucleotide, the polynucleotide may be selected from the group consisting of a ribozyme, an antisense molecule, an inhibitor oligonucleotide, an aptamer, a micro RNA, and a small interfering RNA (siRNA), and is preferably an antisense nucleic acid, a micro RNA, or a small interfering RNA (siRNA).

As used herein, the term "MYT1 inhibitor" means a substance that reduces MYT1 activity in vitro, in a cell culture system, or in animals, and for example, is a substance whose inhibitory activity IC₅₀ of MYT1 measured is 10 µM or less, 5 µM or less, or 1 µM or less. In a specific MYT1 inhibitor, IC₅₀ of MYT1 may be 100 nM or less, 10 nM or less, 3 nM or less, 100 pM or less, or 10 pM or less. More preferably, IC₅₀ of MYT1 is, for example, 1 nM to 1 µM, 1 nM to 750 nM, 1 nM to 500 nM, or 1 nM to 250 nM. Further preferably, IC₅₀ of MYT1 is, for example, less than 20 nM, or 1 nM to 20 nM. IC₅₀ of MYT1 is not particularly limited, and for example, can be measured by the method described in Non Patent Literature 8.

As the MYT1 inhibitor, a substance well known to those skilled in the art may be used. For example, as the MYT1 inhibitor according to the present embodiment, a compound represented by the formula (1): 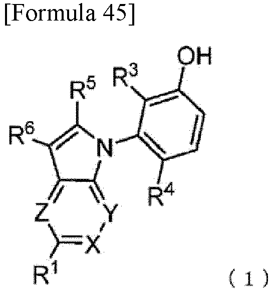
wherein each of X, Y, and Z is independently N or CR²;
each of R¹ and R² is independently a hydrogen atom, optionally substituted C₁₋₆ alkyl, optionally substituted C₂₋₆ alkenyl, optionally substituted C₂₋₆ alkynyl, optionally substituted C₃₋₈ cycloalkyl, optionally substituted C₃₋₈ cycloalkenyl, optionally substituted C₂₋₉ heterocyclyl, optionally substituted C₂₋₉ heterocyclyl C₁₋₆ alkyl, optionally substituted C₆₋₁₀ aryl, optionally substituted C₁₋₉ heteroaryl, optionally substituted C₁₋₉ heteroaryl C₁₋₆ alkyl, a halogen atom, cyano, -N(R⁷)₂, -OR⁷, -C(O)N(R⁸)₂, -SO₂N(R⁸)₂, -SO₂R^{7A}, or -Q-R^{7B}, or R¹ form optionally substituted C₃-₆ alkylene together with one R² adjacent to R¹;
each of R³ and R⁴ is independently optionally substituted C₁₋₆ alkyl or a halogen atom;
R⁵ is a hydrogen atom or -N(R⁷)₂;
R⁶ is -C(O)NH(R⁸), -C(O)R^{7A}, or -SO₂R^{7A};
R⁷ is each independently a hydrogen atom, optionally substituted C₁₋₆ alkyl, optionally substituted C₆₋₁₀ aryl C₁₋₆ alkyl, optionally substituted C₃₋₈ cycloalkyl, optionally substituted C₆₋₁₀ aryl, optionally substituted C₂₋₉ heterocyclyl, optionally substituted C₁₋₉ heteroaryl, optionally substituted C₁₋₉ heteroaryl C₁₋₆ alkyl, or -SO₂R^{7A}, or two R⁷ form optionally substituted C₂₋₉ heterocyclyl in combination with an atom in contact with both of them;
R^{7A} is each independently optionally substituted C₁₋₆ alkyl, optionally substituted C₃₋₈ cycloalkyl, or optionally substituted C₆₋₁₀ aryl;
R^{7B} is each independently hydroxyl, optionally substituted C₁₋₆ alkyl, optionally substituted C₆₋₁₀ aryl, optionally substituted C₂₋₉ heterocyclyl, optionally substituted C₁₋₉ heteroaryl, -N(R⁷)₂, -C(O)N(R⁸)₂, -SO₂N(R⁸)₂, -SO₂R^{7A}, or optionally substituted alkoxy;
R⁸ is each independently a hydrogen atom, optionally substituted C₁₋₆ alkyl, optionally substituted C₂₋₆ alkoxyalkyl, optionally substituted C₆₋₁₀ aryl C₁₋₆ alkyl, optionally substituted C₆₋₁₀ aryl, optionally substituted C₃₋₈ cycloalkyl, or optionally substituted C₁₋₉ heteroaryl in combination with an atom in contact with both of them; and
Q is optionally substituted C₁₋₆ alkylene, optionally substituted C₂₋₆ alkenylene, optionally substituted C₂₋₆ alkynylene, optionally substituted C₃₋₈ cycloalkylene, optionally substituted C₃₋₈ cycloalkenylene, optionally substituted C₆₋₁₀ arylene, optionally substituted C₂₋₉ heterocyclylene, or optionally substituted C₁₋₉ heteroarylene,
or a salt thereof, or a solvate thereof described in WO 2021/195781 can be used.

As used herein, the term "alkyl" means an unsubstituted or substituted linear or branched saturated hydrocarbon group. When unsubstituted, the alkyl has 1 to 12 carbon atoms, unless otherwise stated. In a specific preferred embodiment, the unsubstituted alkyl has 1 to 6 carbon atoms, and examples thereof include methyl, ethyl, n-propyl, isopropyl, n-butyl, sec-butyl, isobutyl, tert-butyl; and neopentyl. In the case of the substituted alkyl, the alkyl may be substituted with four or more substituents independently selected from the group consisting of amino, alkoxy, aryl, aryloxy, azide, cycloalkyl, cycloalkoxy, cycloalkenyl, cycloalkynyl, halo, heterocyclyl, (heterocyclyl)oxy, heteroaryl, hydroxy, nitro, thiol, silyl, cyano, alkylsulfonyl, alkylsulphenyl, =O, =S, -C(O)R or -SO₂R (wherein R is amino), and =NR' (wherein R' is a hydrogen atom, alkyl, aryl, or heterocyclyl). Each of these substituents themselves may be unsubstituted, or each group may be substituted with an unsubstituted substituent defined herein.

As used herein, the term "alkylene" means divalent alkyl. Optionally substituted alkylene means alkylene which may be unsubstituted or substituted, as described herein for alkyl.

As used herein, the term "alkenyl" represents a noncyclic monovalent linear or branched hydrocarbon group that contains 1, 2, or 3 carbon-carbon double bonds. Non-limited examples of alkenyl include ethenyl, prop-1-enyl, prop-2-enyl, 1-methylethenyl, -2-enyl, -3-enyl, 1-methylprop-1-enyl, 2-methylprop-1-enyl, and 1-methylprop-2-enyl. Optionally substituted alkenyl means alkenyl which may be unsubstituted or substituted, as described herein for alkyl.

As used herein, the term "alkenylene" means divalent alkenyl. Optionally substituted alkenylene may be means alkenylene which unsubstituted or substituted, as described herein for alkenyl.

As used herein, the term "alkanoyl" represents a hydrogen atom or an alkyl group bonding to a parent molecule group through a carbonyl group, and examples thereof include formyl (that is, a carboxy aldehyde group), acetyl, propionyl, butyryl, and isobutyryl. An unsubstituted alkanoyl group contains 1 to 7 carbon atoms. Alkanoyl may be unsubstituted or substituted (e.g., C₁₋₇ alkanoyl), as described for alkyl. Moreover, groups (e.g., aryloyl, cycloalkanoyl, and (heterocyclyl)oyl) obtained by adding "oyl" to the end of other groups defined herein (e.g., aryl, cycloalkyl, and heterocyclyl) represent carbonyl groups substituted with aryl, cycloalkyl, and heterocyclyl, respectively. These substituents may further optionally be substituted in substituent parts (e.g., aryl, cycloalkyl, and heterocyclyl).

As used herein, the term "alkoxy" means a substituent represented by the formula -OR (wherein R is C₁₋₆ alkyl), unless otherwise stated. In some embodiments, alkyl may be further substituted as defined herein. The term "alkoxy" can be combined with any of the other terms defined herein, for example, aryl, cycloalkyl, or heterocyclyl, and can define substituted aryl, alkoxy, cycloalkyl, alkoxy, and heterocyclyl(heterocyclyl)alkoxy groups. Each of these groups represents alkoxy substituted with aryl, cycloalkyl, or heterocyclyl. These groups may have a substituent in the individual part as defined herein.

As used herein, the term "alkoxyalkyl" means a substituent represented by the formula -L-O-R (wherein L is C₁₋₆ alkylene, and R is C₁₋₆ alkyl). Optionally substituted alkoxyalkyl means alkoxyalkyl which may be unsubstituted or substituted, as described herein for alkyl.

As used herein, the term "alkylamino" means a group represented by the formula - N(R^{N1})₂ or -NHR^{N1} (wherein R^{N1} is alkyl). The alkyl moiety R^{N1} of alkylamino may be unsubstituted or substituted, as described herein for alkyl. Any substituent itself on the substituted alkylamino may be unsubstituted or substituted.

As used herein, the term "alkylsulfenyl" means a group represented by the formula -S-(alkyl). Alkylsulfenyl may be unsubstituted or substituted, as described herein for alkyl.

As used herein, the term "alkynylsulfinyl" means a group represented by the formula - S(O)-(alkyl). Alkylsulfinyl may be substituted alkylsulfinyl, as described for the term "alkyl".

As used herein, the term "alkylsulfonyl" means a group represented by the formula - S(O)₂-(alkyl). Alkylsulfonyl may be optionally substituted, as defined for alkyl.

As used herein, the term "alkynyl" means a monovalent linear or branched hydrocarbon group having 2 to 6 carbon atoms that includes at least (one) carbon-carbon triple bond. Examples of alkynyl include ethynyl and 1-propynyl. Alkynyl may be unsubstituted or substituted.

As used herein, the term "alkynylene" means a divalent alkynyl group. Optionally substituted alkynylene means alkynylene which may be unsubstituted or substituted, as described herein for alkynyl.

As used herein, the term "amino" means a group represented by -N(R^{N1})₂ (wherein, when amino is unsubstituted, both R^{N1} are hydrogen atoms, and when amino is substituted, each R^{N1} is independently a hydrogen atom, -OH, -NO₂, -N(R^{N2})₂, -SO₂OR^{N2}, -SO₂R^{N2}, -SOR^{N2}, - C(O)OR^{N2}, an N-protective group, alkyl, alkenyl, alkynyl, alkoxy, aryl, arylalkyl, aryloxy, cycloalkyl, cycloalkenyl, heteroalkyl, or heterocyclyl, and at least one RN1 is not a hydrogen atom; and each R^{N2} is independently H, alkyl, or aryl). Each of these substituents may be unsubstituted, or may be substituted with an unsubstituted substituent defined herein. In a specific embodiment, amino is unsubstituted amino (that is, -NH₂) or substituted amino (e.g., - NHR^{N1} (wherein R^{N1} is independently -OH, SO₂OR^{N2}, -SO₂R^{N2}, -SOR^{N2}, -COOR^{N2}, optionally substituted alkyl, or optionally substituted aryl, and each R^{N2} is optionally substituted alkyl or optionally substituted aryl)). In a specific embodiment, substituted amino may be substituted alkylamino, as described herein for alkyl. In a specific embodiment, an amino group is -NHR^{N1} (wherein R^{N1} is optionally substituted alkyl).

As used herein, the term "aryl" means a mono-, bi- or polycyclic carbocycle having one or two aromatic rings. Aryl may contain 6 to 10 carbon atoms. All the atoms in an unsubstituted carbocyclic aryl are carbon atoms. Non-limited examples of the carbocyclic aryl include phenyl, naphthyl, 1,2-dihydronaphthyl, 1,2,3,4-tetrahydronaphthyl, fluorenyl, indanyl, and indenyl. Aryl may be unsubstituted, and may be substituted with 1, 2, 3, 4, or 5 substituents selected from alkenyl, alkynyl, alkoxy, alkylsulfinyl, alkylsulfenyl, alkylsulfonyl, amino, aryl, aryloxy, azide, cycloalkyl, cycloalkoxy, cycloalkenyl, cycloalkynyl, halo, heteroalkyl, heterocyclyl, (heterocyclyl)oxy, hydroxy, nitro, thiol, silyl, -(CH₂)ₙ-C(O)R^{A}, - C(O)R, and -SO₂R (wherein R is amino or alkyl, R^{A} is a hydrogen atom or alkyl, and n is 0 or 1). Each of these substituents may be unsubstituted, or may be substituted with an unsubstituted substituent, as defined herein.

As used herein, the term "arylalkyl" means an alkyl group substituted with an aryl group. Aryl and alkyl may optionally be substituted as an individual group, as defined herein.

As used herein, the term "arylene" means a divalent aryl group. Optionally substituted arylene means arylene which may be unsubstituted or substituted, as described herein for aryl.

As used herein, the term "aryloxy" means a group represented by the formula -OR (wherein R is an aryl group), unless otherwise stated. The aryl group may be optionally substituted in optionally substituted aryloxy, as described herein for aryl.

As used herein, the term "azide" means a group represented by the formula -N₃.

As used herein, the term "cycloalkenyl" means a nonaromatic carbocyclic group (e.g., C₃₋₁₀ cycloalkenyl) having at least one double bond in the ring and 3 to 10 carbon atoms, unless otherwise stated. Non-limited examples of cycloalkenyl include cycloprop-1-enyl, cycloprop-2-enyl, cyclobut-1-enyl, cyclobut-1-enyl, cyclobut-2-enyl, cyclopent-1-enyl, cyclopent-2-enyl, cyclopent-3-enyl, norborn-1-yl, norbornen-2-yl, norbornen-5-yl, and norbornen-7-yl. The cycloalkenyl group may be unsubstituted or substituted, as described for cycloalkyl.

As used herein, the term "cycloalkenylalkyl" means alkyl substituted with cycloalkenyl, as defined herein. The cycloalkenyl and alkyl moieties may be substituted as an individual group defined herein.

As used herein, the term "cycloalkenylene" means divalent cycloalkenyl. Optionally substituted cycloalkenylene means cycloalkenylene which may be unsubstituted or substituted, as described herein for cycloalkyl.

As used herein, the term "cycloalkoxy" means a group represented by the formula -OR (wherein R is a cycloalkyl group), unless otherwise stated. In some embodiments, the cycloalkyl moiety may be unsubstituted or substituted, as described herein for cycloalkyl.

As used herein, the term "cycloalkyl" means a cyclic alkyl group having 3 to 10 carbon atoms (e.g., C₃₋₁₀ cycloalkyl), unless otherwise stated. Cycloalkyl may be monocyclic or bicyclic. Bicyclic cycloalkyl may be of bicyclo[p.q.0]alkyl type (wherein p and q are independently 1, 2, 3, 4, 5, 6, or 7, and the sum of p and q is 2, 3, 4, 5, 6, 7, or 8). Alternatively, bicyclic cycloalkyl may have a crosslinked cycloalkyl structure (e.g., bicyclo[p.q.r]alkyl (wherein r is 1, 2, or 3, each of p and q is 1, 2, 3, 4, 5, or 6, and the sum of p, q, and r is 3, 4, 5, 6, 7, or 8)). Cycloalkyl may have a spirocyclic structure (e.g., spiro[p.q]alkyl, wherein each of p and q is independently 2, 3, 4, 5, 6, or 7, and the sum of p and q is 4, 5, 6, 7, 8, or 9). Non-limited examples of cycloalkyl include cyclopropyl, cyclobutyl, cyclopentyl, cyclohexyl, cycloheptyl, 1-bicyclo[2.2.1]heptyl, 2-bicyclo[2.2.1]heptyl, 5-bicyclo[2.2.1]heptyl, 7-bicyclo[2.2.1]heptyl, and decanyl. Cycloalkyl may be unsubstituted or substituted with 1, 2, 3, 4, or 5 substituents independently selected from the group consisting of alkyl, alkenyl, alkynyl, alkoxy, alkylsulfenyl, alkylsulfonyl, amino, aryl, aryloxy, azide, cycloalkyl, cycloalkoxy, cycloalkenyl, cycloalkynyl, halo, heteroalkyl, heterocyclyl, (heterocyclyl)oxy, heteroaryl, hydroxy, nitro, thiol, silyl, cyano, =O, =S, -SO₂R (wherein R is optionally substituted amino or =NR', and R' may be substituted with a hydrogen atom, alkyl, aryl, or heterocyclyl), and - CON(R^{A})₂ (wherein each R^{A} is independently a hydrogen atom or alkyl, or both R^{A} are bonded together with atoms forming heterocyclyl). Each of these substituents themselves may be unsubstituted, or each substituent may be substituted with an unsubstituted substituent defined herein.

As used herein, the term "cycloalkyl" means an alkyl group substituted with a cycloalkyl group, as defined herein. The cycloalkyl moiety and the alkyl moiety may optionally be substituted as an individual group described herein.

As used herein, the term "cycloalkylene" means a divalent cycloalkyl group. Optionally substituted cycloalkylene means cycloalkylene which may be unsubstituted or substituted, as described herein for cycloalkyl.

As used herein, the term "cycloalkynyl" means a monovalent carbocyclic group having one or two carbon-carbon triple bonds and 8 to 12 carbon atoms, unless otherwise stated. Cycloalkynyl may contain a trans-ring bond or bridge structure. Non-limited examples of cycloalkynyl include cyclooctanyl, cyclononyl, cyclodecanyl, and cyclodecadienyl. Optionally substituted cycloalkynyl means cycloalkynyl which may be unsubstituted or substituted, as described herein for cycloalkyl.

As used herein, the term "halo" means a halogen atom selected from bromine, chlorine, iodine, and fluorine.

As used herein, the term "heteroalkyl" means an alkyl, alkenyl, or alkynyl group interrupted once by one or two heteroatoms, twice each independently by one or two heteroatoms, 3 times each independently by one or two heteroatoms, or 4 times each independently by one or two heteroatoms. Each heteroatom is independently an oxygen atom, a nitrogen atom, or a sulfur atom. In a specific embodiment, the heteroatom is an oxygen atom or a nitrogen atom, and all the heteroalkyl groups do not contain two continuous oxygen atoms or sulfur atoms. The heteroalkyl group may be unsubstituted or substituted. When heteroalkyl is substituted and the substituent is bonded to a heteroatom, the substituent is selected depending on the property and valence of the heteroatom. Therefore, the substituent bonded to the heteroatom is selected from the group consisting of =O, -N(R^{N2})₂, -SO₂OR^{N3}, - SO₂R^{N2}, -SOR^{N3}, -COOR^{N3}, an N-protective group, alkyl, alkenyl, alkynyl, aryl, cycloalkyl, cycloalkenyl, cycloalkynyl, heterocyclyl, or cyano, each R^{N2} is independently a hydrogen atom, alkyl, cycloalkyl, cycloalkenyl, cycloalkynyl, aryl, or heterocyclyl, and each R^{N3} is independently alkyl, cycloalkyl, cycloalkenyl, cycloalkynyl, aryl, or heterocyclyl. Each of these substituents themselves may be unsubstituted or substituted.

As used herein, the term "heteroaryl alkyl" means alkyl substituted with heteroaryl, as defined herein. The heteroaryl moiety and the alkyl moiety may optionally be substituted as an individual group described herein.

As used herein, the term "heteroarylene" means divalent heteroaryl. Optionally substituted heteroarylene means heteroarylene which may be unsubstituted or substituted, as described herein for heteroaryl.

As used herein, the term "heteroaryloxy" means a group represented by the formula -OR (wherein R is heteroaryl). Heteroaryloxy may be unsubstituted or substituted, as described herein for heterocyclyl.

As used herein, the term "heterocyclyl" means a monocyclic, bicyclic, tricyclic, or spiro 3-, 4-, 5-, 6-, 7- or 8-membered ring, unless otherwise stated, and contains 1, 2, 3, or 4 heteroatoms independently selected from the group consisting of nitrogen atoms, oxygen atoms, and sulfur atoms, unless otherwise stated. In a specific embodiment, heterocyclyl has a monocyclic, bicyclic, tricyclic, or tetracyclic ring structure containing 1, 2, 3, or 4 heteroatoms, unless otherwise stated, and contains 1, 2, 3, or 4 heteroatoms independently selected from the group consisting of nitrogen atoms, oxygen atoms, and sulfur atoms, unless otherwise stated. Heterocyclyl may be aromatic or nonaromatic. Nonaromatic 5-membered heterocyclyl has zero or one double bond, nonaromatic 6-membered and 7-membered heterocyclyl groups has 0, 1, or 2 double bonds, a nonaromatic 8-membered heterocyclyl group has 0, 1, or 2 double bonds and/or zero or one carbon-carbon triple bond. The heterocyclyl group contains 1 to 16 carbon atoms, unless otherwise stated. Specific heterocyclyl can contain at most 9 carbon atoms. Examples of nonaromatic heterocyclyl include pyrrolinyl, pyrrolidinyl, pyrazolinonyl, pyrazolidinyl, imidazolinyl, imidazolidinyl, piperidinyl, homopiperidinyl, piperazinyl, pyridazinyl, oxazolidinyl, iso oxazolidinyl, morpholinyl, thiomorpholinyl, thiazolidinyl, isothiazolidinyl, tetrahydrofuranyl, dihydrofuranyl, tetrahydrothienyl, dihydrothienyl, dihydroindolyl, tetrahydroquinolyl, tetrahydroisoquinolyl, pyranyl, dihydropyranyl, and dithiazolyl. When a heterocyclic ring structure has at least one aromatic resonance structure or at least one aromatic tautomer, such a structure is aromatic heterocyclyl (that is, heteroaryl). Non-limited examples of heteroaryl include benzimidazolyl, benzofuryl, benzothiazolyl, benzothienyl, benzoxazolyl, furyl, imidazolyl, indolyl, isoindazolyl, isoquinolyl, isothiazolyl, isooxazolyl, oxadiazolyl, oxazolyl, prinyl, pyrrolyl, pyridinyl, pyrazinyl, pyrimidinyl, quinazolinyl, quinolinyl, thiadiazolyl (e.g., 1,3,4-thiadiazole), thiazolyl, thienyl, triazolyl, and tetrazolyl. The term "heterocyclyl" means a heterocyclic compound having a cross-linking polycyclic structure in which one or more carbon atoms and/or heteroatoms cross-link two nonadjacent atoms in a monocyclic ring (e.g., quinuclidine, tropeine, or diazabicyclo[2.2.2]octane). The term "heterocyclyl" includes bicyclic, tricyclic, and tetracyclic groups in which any of the above heterocyclic rings is condensed with 1, 2, or 3 carbocyclic rings (e.g., an aryl ring, a cyclohexane ring, a cyclohexene ring, a cyclopentane ring, a cyclopentene ring, or other monocyclic heterocycles). Examples of the condensed heterocycle include 1,2,3,5,8,8a-hexahydroindolysine, 2,3-dihydrobenzofuran, 2,3-dihydroindole, and 2,3-dihydrobenzothiophene. Heterocyclyl may be unsubstituted, or substituted with 1, 2, 3, 4, or 5 substituents independently selected from the group consisting of alkyl, alkenyl, alkynyl, alkoxy, alkylsulfinyl, alkylsulfenyl, alkylsulfonyl, amino, aryl, aryloxy, azide, cycloalkyl, cycloalkoxy, cycloalkenyl, cycloalkynyl, halo, heteroalkyl, heterocyclyl, heterocyclyloxy, hydroxy, nitro, thiol, silyl, cyano, -C(O)R, or -SO₂R (wherein R is amino or alkyl, =O, =S, =NR' (wherein R' is a hydrogen atom, alkyl, aryl, or heterocyclyl)). Each of these substituents may be unsubstituted, or each substituent may be substituted with an unsubstituted substituent defined herein.

As used herein, the term "heterocyclylalkyl" means alkyl substituted with heterocyclyl, as defined herein. The heterocyclyl moiety and the alkyl moiety may optionally be substituted as an individual group described herein.

As used herein, the term "heterocyclylene" means divalent heterocyclyl. Optionally substituted heterocyclylene means heterocyclylene which may be unsubstituted or substituted, as described herein for heterocyclyl.

As used herein, the term "heterocyclyloxy" means a group represented by the formula - OR (wherein R is heterocyclyl), unless otherwise stated. Heterocyclyloxy may be unsubstituted or substituted, as described herein for heterocyclyl.

The compound of the present disclosure may be a salt thereof, and preferably a chemically or pharmaceutically acceptable salt thereof. In addition, the compound of the present disclosure or a salt thereof may be a solvate thereof, and preferably a chemically or pharmaceutically acceptable solvate thereof. Examples of the salt of the compound of the present disclosure include hydrochlorides; hydrobromides; hydroiodides; phosphates; phosphonates; hydrosulfates; methanesulfonates, sulfonates such as p-toluenesulfonates; carboxylates such as acetates, citrates, maleates, tartarates, succinates, and salicylates; alkaline metal salts such as lithium salts, sodium salts, and potassium salts; alkaline earth metal salts such as magnesium salts and calcium salts; and ammonium salts such as ammonium salts, alkylammonium salts, dialkylammonium salts, trialkylammonium salts, and tetraalkylammonium salts. These salts are produced, for example, by bringing the compound into contact with an acid or a base available for the production of the medicament.

In the present disclosure, a solvate of a compound refers to a molecule group formed from the compound and the solvent. When the solvent is water, the solvate is referred to as a hydrate. As the solvate of the compound of the present disclosure, a hydrate is preferable, and such a hydrate is specifically a monohydrate to decahydrate, preferably a monohydrate to pentahydrate, and further preferably a monohydrate to trihydrate. Examples of the solvate of the compound of the present disclosure include not only solvates with a single solvent such as water, alcohol (e.g., methanol, ethanol, 1-propanol, and 2-propanol), or dimethylformamide, but also solvates with a plurality of solvents.

The compound represented by the formula (1) can form an atropisomer by the bond between a nitrogen atom constituting a pyrrole ring and a phenyl group. The MYT1 inhibitor according to the present embodiment preferably contains a larger amount of the atropisomer represented by the formula (1A): 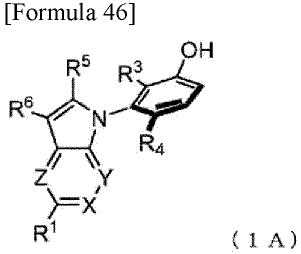
wherein each of X, Y, and Z is independently N or CR²;
each of R¹ and R² is independently a hydrogen atom, optionally substituted C₁₋₆ alkyl, optionally substituted C₂₋₆ alkenyl, optionally substituted C₂₋₆ alkynyl, optionally substituted C₃₋₈ cycloalkyl, optionally substituted C₃₋₈ cycloalkenyl, optionally substituted C₂₋₉ heterocyclyl, optionally substituted C₂₋₉ heterocyclyl C₁₋₆ alkyl, optionally substituted C₆₋₁₀ aryl, optionally substituted C₁₋₉ heteroaryl, optionally substituted C₁₋₉ heteroaryl C₁₋₆ alkyl, a halogen atom, cyano, -N(R⁷)₂, -OR⁷, -C(O)N(R⁸)₂, -SO₂N(R⁸)₂, -SO₂R^{7A}, or -Q-R^{7B}, or R¹ form optionally substituted C₃₋₆ alkylene together with one R² adjacent to R¹;
each of R³ and R⁴ is independently optionally substituted C₁₋₆ alkyl or a halogen atom;
R⁵ is a hydrogen atom or -N(R⁷)₂;
R⁶ is -C(O)NH(R⁸), -C(O)R^{7A}, or -SO₂R^{7A};
R⁷ is each independently a hydrogen atom, optionally substituted C₁₋₆ alkyl, optionally substituted C₆₋₁₀ aryl, C₁₋₆ alkyl, optionally substituted C₃₋₈ cycloalkyl, optionally substituted C₆₋₁₀ aryl, optionally substituted C₂₋₉ heterocyclyl, optionally substituted C₁₋₉ heterocyclyl, optionally substituted C₁₋₉ heteroaryl C₁₋₆ alkyl, or -SO₂R^{7A}, or two R⁷ form optionally substituted C₂₋₉ heterocyclyl in combination with an atom in contact with both of them;
R^{7A} is each independently optionally substituted C₁₋₆ alkyl, optionally substituted C₃₋₈ cycloalkyl, or optionally substituted C₆₋₁₀ aryl;
R^{7B} is each independently hydroxyl, optionally substituted C₁₋₆ alkyl, optionally substituted C₆₋₁₀ aryl, optionally substituted C₂₋₉ heterocyclyl, optionally substituted C₁₋₉ heteroaryl, -N(R⁷)₂, -C(O)N(R⁸)₂, -SO₂N(R⁸)₂, -SO₂R^{7A}, or optionally substituted alkoxy;
R⁸ is each independently a hydrogen atom, optionally substituted C₁₋₆ alkyl, optionally substituted C₂₋₆ alkoxyalkyl, optionally substituted C₆₋₁₀ aryl C₁₋₆ alkyl, optionally substituted C₆₋₁₀ aryl, optionally substituted C₃₋₈ cycloalkyl, or optionally substituted C₁₋₉ heteroaryl, or two R⁸ form optionally substituted C₂₋₉ heterocyclyl in combination with an atom in contact with both of them; and
Q is optionally substituted C₁₋₆ alkylene, optionally substituted C₂₋₆ alkenylene, optionally substituted C₂₋₆ alkynylene, optionally substituted C₃₋₈ cycloalkylene, optionally substituted C₃₋₈ cycloalkenylene, optionally substituted C₆₋₁₀ arylene, optionally substituted C₂₋₉ heterocyclylene, or optionally substituted C₁₋₉ heteroarylene.

The MYT1 inhibitor according to the present embodiment is further preferably a compound represented by the formula (2): 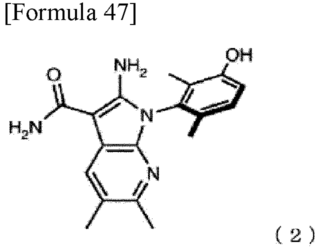 or a salt thereof, or a solvate thereof.

As the MYT1 inhibitor according to the present embodiment, a compound represented by the formula (3), a salt thereof, or a solvate thereof can be used.

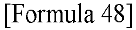

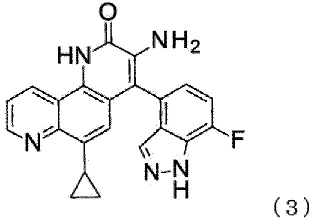

The pharmaceutical composition containing the MYT1 inhibitor (the first ingredient) as an active ingredient is used in combination with the chemotherapeutic agent (the second ingredient). In an embodiment, the pharmaceutical composition containing the MYT1 inhibitor is simultaneously or separately administered with the chemotherapeutic agent. In another embodiment, the pharmaceutical composition containing the MYT1 inhibitor is administered as a combination drug with the chemotherapeutic agent.

The dose of the MYT1 inhibitor is preferably 0.001 to 50 mg (0.001 to 50 mg/kg/day), more preferably 0.001 to 20 mg/kg/day, and further preferably 0.002 to 10 mg/kg/day per day per kg body weight of the subject to be administered. When the dose of the MYT1 inhibitor falls within these ranges, the effect of treating or preventing cancer is further enhanced. The dosing frequency of the MYT1 inhibitor may be, for example, once a week or more, twice a week, once daily, or twice daily.

The chemotherapeutic agent is a chemically synthesized substance having anticancer activity (hereinafter, also referred to as an anticancer agent). The chemotherapeutic agent may be, for example, selected from the group consisting of an antimetabolite, an anticancer antibiotic, a mitosis inhibitor, a topoisomerase inhibitor, a platinating agent, an alkylating agent, and an antibody-drug conjugate.

The antimetabolite is a substance having a chemical structure similar to a metabolite (e.g., purine, pyrimidine, folic acid, and ribonucleotide) and competing with or inhibiting the metabolic mechanism of an organism. Examples of the antimetabolite include purine antimetabolites, pyrimidine antimetabolites, folic acid antimetabolites, and ribonucleotide reductase inhibitors. The antimetabolite is more preferably a purine antimetabolite.

The purine antimetabolite has a chemical structure similar to a purine nucleic base and inhibits the metabolism of purine. Examples of the purine antimetabolite include 6-thioguanine, 6-mercaptopurine, azathioprine, fludarabine, pentostatin, cladribine, clofarabine, and nelarabine.

The pyrimidine antimetabolite has a chemical structure similar to a pyrimidine nucleic base and inhibits the metabolism of pyrimidine. Examples of the pyrimidine antimetabolite include gemcitabine, cytarabine, fluorouracil, capecitabine, tegafur, azacitidine, trifluridine, and floxuridine. The pyrimidine antimetabolite is more preferably gemcitabine.

An antifolate is a substance that inhibits DNA synthesis by suppressing or inhibiting the action of the enzyme that reduces folic acid to active folic acid essential for nucleic acid biosynthesis. Examples of the antifolate include methotrexate and pemetrexed. The antifolate is more preferably pemetrexed.

The ribonucleotide reductase inhibitor is a substance for inhibiting DNA biosynthesis by suppressing or inhibiting the enzyme that reduces ribonucleotide to deoxyribonucleotide. Examples of the ribonucleotide reductase inhibitor include hydroxyurea.

The anticancer antibiotic is an agent synthesized with reference to a chemical substance produced by microorganisms (also referred to as the antibiotic), and is a substance exhibiting an antitumor effect by breaking the cell membrane of cancer cells or inhibiting the synthesis of DNA or RNA. Examples of the anticancer antibiotic include bleomycin, actinomycin D, doxorubicin, daunorubicin, idarubicin, mitomycin, mitoxantrone, epirubicin, aclarubicin, and valrubicin.

The mitosis inhibitor is a substance for inhibiting the division of cancer cells by inhibiting the process of the division of the cell cycle, and is classified into vinca alkaloid and microtubule inhibitors (including taxane-based compounds). Vinca alkaloid is a compound made with reference to the chemical structure of the compound obtained from Catharanthus roseus, and stops the cell cycle in the middle of the division by acting on the tubulin protein of microtubules that form spindles in the process of the cell division. Examples of vinca alkaloid include vincristine, vinblastine, and vinorelbine. The microtubule inhibitor is a compound containing a taxane-based compound made with reference to the chemical structure of the compound obtained from Taxus cuspidata and inhibiting the cell division by promoting the polymerization of microtubules or inhibiting the depolymerization of microtubules in the process of the cell division. Examples of the microtubule inhibitor include taxane-based compounds (e.g., docetaxel and paclitaxel), eribulin, ixabepilone, and epothilone.

The topoisomerase inhibitor is a substance for inhibiting topoisomerase I or II that is necessary for DNA replication in cells in which the growth of cancer cells and the like is active. Topoisomerase I is an enzyme that cuts one of the double helix of DNA and involves in recombination, and topoisomerase II is an enzyme that cuts both strands of the double helix and involves in recombination. Examples of the topoisomerase inhibitor include topotecan, irinotecan, DXd, etoposide, and teniposide. The topoisomerase inhibitor is more preferably irinotecan or DXd.

The platinating agent is a compound that inhibits DNA replication by binding to the DNA of cancer cells and induces self-destruction (apoptosis) of cancer cells. Examples of the platinating agent include cisplatin, carboplatin, and oxaliplatin. The platinating agent is more preferably carboplatin.

The alkylating agent is a compound that binds to DNA of cancer cells and the like and inhibits DNA replication. Examples of the alkylating agent include cyclophosphamide, ifosfamide, chlorambucil, melphalan, temozolomide, carmustine, lomustine, streptozocin, busulfan, procarbazine, dacarbazine, nimustine, ranimustine, bendamustine, altretamine, thiotepa, and mechlorethamine.

The antibody-drug conjugate (ADC) is a substance obtained by binding a compound having an anticancer effect to an antibody that recognizes a specific molecule of cancer cells, and the compound having an anticancer effect is typically bonded to the antibody through a linker. When the antibody specifically recognizes cancer cells, the compound bonded to the antibody can be selectively delivered to the cancer cells. Examples of the antibody-drug conjugate include trastuzumab deruxtecan (T-DXd), sacituzumab govitecan (SG), tisotumab vedotin, enfortumab vedotin, trastuzumab emtansine, loncastuximab tesirine, moxetumomab pasudotox, belantamab mafodotin, polatuzumab vedotin, inotuzumab ozogamicin, brentuximab vedotin, and gemtuzumab ozogamicin. The antibody-drug conjugate is more preferably trastuzumab deruxtecan or sacituzumab govitecan.

In the present embodiment, a preferred combination of the MYT1 inhibitor and the chemotherapeutic agent is such that the MYT1 inhibitor is a small interfering RNA (siRNA) and the chemotherapeutic agent is at least one selected from the group consisting of gemcitabine, pemetrexed, irinotecan, carboplatin, and an antibody-drug conjugate that contains DXd as a payload (e.g., trastuzumab deruxtecan (used as Enhertu)), or the MYT1 inhibitor is the compound represented by the formula (2), or a salt thereof, or a solvate thereof, or the compound represented by (3) or a salt thereof, or a solvate thereof and the chemotherapeutic agent is at least one selected from the group consisting of pemetrexed, gemcitabine, carboplatin, irinotecan, an antibody-drug conjugate that contains SN-38 as a payload (e.g., sacituzumab govitecan), an antibody-drug conjugate that contains DXd as a payload (e.g., trastuzumab deruxtecan (used as Enhertu)).

The pharmaceutical composition is particularly suitable for treatment or prevention of cancer of a patient in which RB 1 gene mutation positivity, a decrease in expression of an RB 1 gene or protein, or positive expression of hyperphosphorylated RB1 protein is detected.

The cancer may be solid cancer or blood cancer. In a specific embodiment, the cancer is lung cancer, breast cancer, esophageal cancer, stomach cancer, bowel cancer, uterine cancer, ovarian cancer, pancreatic cancer, bladder cancer, thyroid cancer, skin cancer, head and neck cancer, kidney cancer, liver cancer, prostate cancer, adenoid cystic cancer, retinoblastoma, cerebral tumor, leukemia, malignant lymphoma, or multiple myeloma. The pharmaceutical composition is more suitable for treatment or prevention of lung cancer, breast cancer, bladder cancer, or ovarian cancer, and especially treatment or prevention of lung cancer, breast cancer, or bladder cancer.

The dose of the chemotherapeutic agent is preferably 0.005 to 300 mg per day per kg body weight of the subject to be administered (0.005 to 300 mg/kg/day), more preferably 0.01 to 250 mg/kg/day, and further preferably 0.02 to 200 mg/kg/day. When the dose of the chemotherapeutic agent falls within the range, the effect of treating or preventing cancer is further enhanced. The dosing frequency of the chemotherapeutic agent may be, for example, once a week or more, twice a week, once daily, or twice daily.

The doses of the MYT1 inhibitor (the first ingredient) and the chemotherapeutic agent (the second ingredient) are preferably such that the first ingredient is 0.0001 to 50 mg/kg/day and the second ingredient is 0.005 to 300 mg/kg/day, more preferably such that the first ingredient is 0.001 to 20 mg/kg/day and the second ingredient is 0.01 to 250 mg/kg/day, and further preferably such that the first ingredient is 0.002 to 10 mg/kg/day and the second ingredient is 0.02 to 200 mg/kg/day per kg body weight of the subject to be administered. When the doses of the first ingredient and second ingredient fall within the range, the effect of treating or preventing cancer is further enhanced.

In the present invention, examples of administration methods include oral, rectal, parenteral (intravenous, intramuscular, subcutaneous, percutaneous absorption), intracisternal, intravaginal, intraperitoneal, intravesical, or local (injections, intravenous drips, powders, ointments, gels, or cream) administration, and by inhalation (a buccal or nasal spray). Examples of dosage forms include tablets, capsules, granules, powders, pills, aqueous and nonaqueous oral solutions and suspensions, and parenteral solutions packaged in containers adapted for subdivision into individual doses. The dosage forms can be adapted for various administration methods including controlled release formulations, such as subcutaneous implants.

The first ingredient may be administered by, for example, any of the above administration methods, and the second ingredient may be administered by, for example, the same administration method as the first ingredient or an administration method different from the first ingredient. The dosing interval between the first ingredient and the second ingredient may be, for example, an interval of 0 days to 14 days, an interval of 0 days to 10 days, or an interval of 0 days to 7 days. The dosing interval can be determined using, for example, AUC, Cmax, Tmax, the elimination half-life, and the state of health of the subject, as the criteria. For example, the first ingredient may be orally administered (tablets, capsules, or the like) and the second ingredient may be administered by intravenous drips. For example, both the first ingredient and the second ingredient may be orally administered (tablets, capsules, or the like). For example, the first ingredient may be administered by intravenous drips and the second ingredient may be orally administered (tablets, capsules, or the like). For example, both the first ingredient and the second ingredient may be administered by intravenous drips. In such a case, the first ingredient and second ingredient may be administered at any interval, such as three times daily, twice daily, once daily, once a week, and once every two weeks. More specifically, both the first ingredient and the second ingredient may be administered once daily, and in this case, they may be administered before, between, or after meals. Before, between, or after meals may be before, between, or after any of breakfast, lunch, dinner, supper, or snack. When the dosing interval between the first ingredient and the second ingredient falls within 24 hours, it means that both ingredients are administered once daily. If necessary, the first ingredient and the second ingredient are respectively administered twice daily and once daily, once daily and once daily, once daily and twice daily, once daily and once every two days, once daily and once every three days, once daily and once every seven days, three times daily and once every seven days, or twice daily and once every seven days, in some cases. If necessary, only the dosing interval of the second ingredient may be increased or reduced, and only the dosing interval of the first ingredient may be increased or reduced. Administration of the second ingredient may be started on the start day of administration of the first ingredient, and the start day of administration of the first ingredient and the start day of administration of the second ingredient may be different from each other. For the duration of administration, the total number of courses may be one course or more, or two courses or more, with 7 days as one course. Each course may be continuously carried out, or a rest period may be provided. A rest period may be provided in a course. If necessary, it is also possible to continuously administer only the first ingredient and stop administration of the second ingredient in a course, or stop administration of only the first ingredient and continuously administer the second ingredient. The first ingredient and the second ingredient may be included in the same tablet, capsule, or the like.

The pharmaceutical composition according to the present embodiment may contain a medicinally acceptable additive in addition to the MYT1 inhibitor (the first ingredient). As the medicinally acceptable additive, those skilled in the art can appropriately select a well-known additive, and if necessary, can appropriately use a plurality of additives. The additive is appropriately selected depending on the oral agent or injection, and for example, may be one mixed with water, ethanol, physiological saline, liquid paraffin, surfactant, sucrose, or the like, or may be one obtained by encapsulating the obtained mixture in a capsule.

The pharmaceutical composition according to the present embodiment can be administered, for example, in combination with a pharmaceutical composition containing an MYT1 inhibitor described as the second embodiment (combined administration).

One aspect of the present embodiment is an MYT1 inhibitor for use in combination with a chemotherapeutic agent in treatment or prevention of cancer in a patient in which RB1 gene mutation positivity, a decrease in expression of an RB1 gene or protein, or positive expression of hyperphosphorylated RB1 protein is detected.

Another aspect of the present embodiment is use of an MYT1 inhibitor for production of a medicament for treatment or prevention of cancer in a patient in which RB1 gene mutation positivity, a decrease in expression of an RB1 gene or protein, or positive expression of hyperphosphorylated RB1 protein is detected, the MYT1 inhibitor being administered in combination with a chemotherapeutic agent.

### [Second embodiment]

The second embodiment of the present invention is a pharmaceutical composition comprising, in combination with an MYT1 inhibitor, a chemotherapeutic agent as an active ingredient for treatment or prevention of cancer of a patient in which RB1 gene mutation positivity, a decrease in expression of an RB1 gene or protein, or positive expression of hyperphosphorylated RB1 protein is detected.

Each term in the pharmaceutical composition according to the present embodiment (e.g., MYT1 inhibitor, chemotherapeutic agent, RB1 gene mutation, administration method, type of cancer) can be referred to the definitions in the first embodiment. The pharmaceutical composition according to the present embodiment can be administered, for example, in combination with the pharmaceutical composition containing the MYT1 inhibitor described as the first embodiment (combined administration).

In a specific embodiment, the pharmaceutical composition contains the chemotherapeutic agent (the second ingredient) as an active ingredient. The pharmaceutical composition containing the chemotherapeutic agent as an active ingredient is used in combination with the MYT1 inhibitor (the first ingredient). In an embodiment, the pharmaceutical composition containing the chemotherapeutic agent is simultaneously or separately administered with the MYT1 inhibitor. In another embodiment, the pharmaceutical composition containing the chemotherapeutic agent is administered as a combination drug with the MYT1 inhibitor.

In the present embodiment, a preferred combination of the MYT1 inhibitor and the chemotherapeutic agent is such that the MYT1 inhibitor is a small interfering RNA (siRNA) and the chemotherapeutic agent is at least one selected from the group consisting of gemcitabine, pemetrexed, irinotecan (a prodrug of SN-38), carboplatin, and an antibody-drug conjugate that contains DXd as a payload (e.g., trastuzumab deruxtecan (used as Enhertu)), or the MYT1 inhibitor is at least one selected from the compound represented by the formula (2), or a salt thereof, or a solvate thereof, and the compound represented by the formula (3) or a salt thereof, or a solvate thereof, and the chemotherapeutic agent is at least one selected from the group consisting of pemetrexed, gemcitabine, carboplatin, irinotecan, an antibody-drug conjugate that contains SN-38 as a payload (e.g., sacituzumab govitecan), an antibody-drug conjugate that contains DXd as a payload (e.g., trastuzumab deruxtecan (used as Enhertu)).

The doses of the MYT1 inhibitor (the first ingredient) and the chemotherapeutic agent (the second ingredient) are preferably such that the first ingredient is 0.0001 to 50 mg/kg/day and the second ingredient is 0.005 to 300 mg/kg/day, more preferably such that the first ingredient is 0.001 to 20 mg/kg/day and the second ingredient is 0.01 to 250 mg/kg/day, and further preferably such that the first ingredient is 0.002 to 10 mg/kg/day and the second ingredient is 0.02 to 200 mg/kg/day per kg body weight of the subject to be administered. When the doses of the first ingredient and second ingredient fall within the range, the effect of treating or preventing cancer is further enhanced.

The pharmaceutical composition according to the present embodiment may contain a medicinally acceptable additive in addition to the chemotherapeutic agent (the second ingredient). As the medicinally acceptable additive, those skilled in the art can appropriately select a well-known additive, and if necessary, can appropriately use a plurality of additives. The additive is appropriately selected depending on the oral agent or injection, and for example, may be one mixed with water, ethanol, physiological saline, liquid paraffin, surfactant, sucrose, or the like, or may be one obtained by encapsulating the obtained mixture in a capsule.

One aspect of the present embodiment is also a chemotherapeutic agent for use in combination with an MYT1 inhibitor in treatment or prevention of cancer in a patient in which RB1 gene mutation positivity, a decrease in expression of an RB1 gene or protein, or positive expression of hyperphosphorylated RB1 protein is detected.

Another aspect of the present embodiment is use of a chemotherapeutic agent for production of a medicament for treatment or prevention of cancer in a patient in which RB1 gene mutation positivity, a decrease in expression of an RB1 gene or protein, or positive expression of hyperphosphorylated RB1 protein is detected, the chemotherapeutic agent being administered in combination with an MYT1 inhibitor.

### [Third embodiment]

The third embodiment of the present invention is a method for treating or preventing cancer of a patient in which RB1 gene mutation positivity, a decrease in expression of an RB1 gene or protein, or positive expression of hyperphosphorylated RB1 protein is detected, the method comprising administering a combination of a chemotherapeutic agent and an MYT1 inhibitor to the cancer patient.

The doses of the MYT1 inhibitor (the first ingredient) and the chemotherapeutic agent (the second ingredient) are preferably such that the first ingredient is 0.0001 to 50 mg/kg/day and the second ingredient is 0.005 to 300 mg/kg/day, more preferably such that the first ingredient is 0.001 to 20 mg/kg/day and the second ingredient is 0.01 to 250 mg/kg/day, and further preferably such that the first ingredient is 0.002 to 10 mg/kg/day and the second ingredient is 0.02 to 200 mg/kg/day per kg body weight of the subject to be administered. When the doses of the first ingredient and second ingredient fall within the range, the effect of treating or preventing cancer is further enhanced.

When the combination of the chemotherapeutic agent and the MYT1 inhibitor is administered, both the MYT1 inhibitor and the chemotherapeutic agent may be simultaneously administered, or may be separately administered with a certain dosing interval. Routes of administration of each of the MYT1 inhibitor and the chemotherapeutic agent may be the same or different from each other. The MYT1 inhibitor and the chemotherapeutic agent may be administered in a form of a combination drug containing the MYT1 inhibitor and the chemotherapeutic agent. That is, the pharmaceutical composition may contain both the MYT1 inhibitor and the chemotherapeutic agent.

### [Fourth embodiment]

The fourth embodiment of the present invention is a method for suppressing the growth of cancer cells in a subject in which RB1 gene mutation positivity, a decrease in expression of an RB 1 gene or protein, or positive expression of hyperphosphorylated RB1 protein is detected, the method comprising bringing an MYT1 inhibitor and a chemotherapeutic agent into contact with the cancer cells.

The method according to the present embodiment comprises any form of in vivo, in vitro, and ex vivo. For example, the subject in which RB1 gene mutation positivity, a decrease in expression of an RB1 gene or protein, or positive expression of hyperphosphorylated RB 1 protein is detected may be an animal in which RB1 gene mutation positivity, a decrease in expression of an RB1 gene or protein, or positive expression of hyperphosphorylated RB 1 protein is detected. Examples of animal species include mice, rats, guinea pigs, monkeys, dogs, sheep, horses, or humans. In the case of in vivo, the growth of cancer cells can be suppressed by administering the MYT1 inhibitor and the chemotherapeutic agent to the above subject. In the case of in vitro, the growth of cancer cells can be suppressed by adding the MYT1 inhibitor and the chemotherapeutic agent to the system containing cancer cells collected from the above subject. In the case of ex vivo, the growth of cancer cells can be suppressed by collecting an organ containing cancer cells (e.g., lung in the case of lung cancer) from the subject, and administering the MYT1 inhibitor and the chemotherapeutic agent to the organ. The doses of the first ingredient (MYT1 inhibitor) and the second ingredient (chemotherapeutic agent) can be appropriately set depending on the amount of cancer cells and the types of the MYT1 inhibitor and the chemotherapeutic agent to be used.

### [Fifth embodiment]

The fifth embodiment of the present invention is a method for improving responsiveness to cancer treatment with a chemotherapeutic agent, the method comprising administering an MYT1 inhibitor together with the chemotherapeutic agent to a cancer patient, wherein the cancer is cancer of the patient in which RB1 gene mutation positivity, a decrease in expression of an RB1 gene or protein, or positive expression of hyperphosphorylated RB1 protein is detected.

In the treatment of cancer of a patient in which RB1 gene mutation positivity, a decrease in expression of an RB1 gene or protein, or positive expression of hyperphosphorylated RB 1 protein is detected, the method according to the present embodiment comprises administering the chemotherapeutic agent in combination with the MYT1 inhibitor to the patient. The efficacy of cancer treatment by the chemotherapeutic agent can be improved by administering the chemotherapeutic agent in combination with the MYT1 inhibitor.

When the MYT1 inhibitor is administered to the cancer patient together with the chemotherapeutic agent, both the MYT1 inhibitor and the chemotherapeutic agent may be simultaneously administered, or may be separately administered with a certain dosing interval. Routes of administration of each of the MYT1 inhibitor and the chemotherapeutic agent may be the same or different from each other. The MYT1 inhibitor and the chemotherapeutic agent may be administered in a form of a combination drug containing the MYT1 inhibitor and the chemotherapeutic agent. Even in a patient to which the therapeutic effect of an existing chemotherapeutic agent is insufficient, the chemotherapeutic agent may exhibit sufficient therapeutic efficacy by being combined with the MYT1 inhibitor.

The doses of the MYT1 inhibitor (the first ingredient) and the chemotherapeutic agent (the second ingredient) are preferably such that the first ingredient is 0.0001 to 50 mg/kg/day and the second ingredient is 0.005 to 300 mg/kg/day, more preferably such that the first ingredient is 0.001 to 20 mg/kg/day and the second ingredient is 0.01 to 250 mg/kg/day, and further preferably such that the first ingredient is 0.002 to 10 mg/kg/day and the second ingredient is 0.02 to 200 mg/kg/day per kg body weight of the subject to be administered. When the doses of the first ingredient and second ingredient fall within the range, the effect of treating or preventing cancer is further enhanced.

### [Sixth embodiment]

The sixth embodiment of the present invention is a method for predicting responsiveness to treatment of cancer with a combination of an MYT1 inhibitor and a chemotherapeutic agent, the method comprising detecting or allowing a third person to detect the presence or absence of an RB1 gene mutation, the presence or absence of a decrease in expression of an RB1 gene or protein, or the presence or absence of the hyperphosphorylated RB1 protein, in a cancer patient-derived cancer cell, and determining the cancer patient as having responsiveness to the treatment of cancer with the combination of the MYT1 inhibitor and the chemotherapeutic agent, when the RB1 gene mutation is positive, the expression of the RB1 gene or protein is decreased, or the expression of the hyperphosphorylated RB1 protein is positive.

In the method according to the present embodiment, when the presence or absence of an RB1 gene mutation, the presence or absence of a decrease in expression of an RB1 gene or protein, or the presence or absence of the hyperphosphorylated RB1 protein is detected or detected by a third person, and the RB1 gene mutation is positive, or the expression of the RB1 gene or protein is decreased, or the expression of the hyperphosphorylated RB1 protein is positive in cancer cells collected from a cancer patient which is a subject to be treated, the cancer patient is determined to have responsiveness (efficacy) to the treatment in which the combination of the MYT1 inhibitor and the chemotherapeutic agent is administered.

According to the method according to the present embodiment, even in a patient to which the therapeutic efficacy of existing chemotherapeutic agents is insufficient, as long as the cancer patient is a patient in which RB1 gene mutation is positive, the expression of the RB1 gene or protein is decreased, or the expression of hyperphosphorylated RB1 protein is positive, the chemotherapeutic agent can be determined as having therapeutic efficacy by administering the chemotherapeutic agent in combination with the MYT1 inhibitor. As a result, it is possible to present effective chemotherapy in advance to a cancer patient that could not obtain a sufficient therapeutic effect only from existing chemotherapeutic agents.

The detection of the presence or absence of the RB1 gene mutation, the presence or absence of the decrease in expression of an RB1 gene or protein, or the presence or absence of the hyperphosphorylated RB1 protein in cancer cells can be conducted by a method well known to the skilled in the art. Examples thereof include a direct sequencing method, a PCR method, a TaqMan Genotyping method, and a next generation sequencing method.

One aspect of the present embodiment is also a method for selecting a cancer patient for which the administration of a combination of an MYT1 inhibitor and a chemotherapeutic agent is more effective. The method comprises detecting or allowing a third person to detect the presence or absence of an RB1 gene mutation, the presence or absence of a decrease in expression of an RB1 gene or protein, or the presence or absence of the hyperphosphorylated RB1 protein, in cancer patient-derived cancer cells, and determining the cancer patient as a cancer patient for which the administration of the combination of an MYT1 inhibitor and a chemotherapeutic agent is more effective, based on the presence of the mutation, the decrease in expression, or the positivity of the expression of the hyperphosphorylated RB1 protein.

Another aspect of the present embodiment is also a method for diagnosing a specific cancer patient that the administration of a combination of an MYT1 inhibitor and a chemotherapeutic agent is more effective for cancer treatment. The method comprises detecting or allowing a third person to detect the presence or absence of an RB1 gene mutation, the presence or absence of a decrease in expression of an RB1 gene or protein, or the presence or absence of the hyperphosphorylated RB1 protein, in cancer patient-derived cancer cells, and determining the cancer patient as a cancer patient for which the administration of the combination of an MYT1 inhibitor and a chemotherapeutic agent is more effective, based on the presence of the mutation.

### [Seventh embodiment]

The seventh embodiment of the present invention is a method for screening a compound effective for treatment or prevention of cancer in a patient in which RB1 gene mutation positivity, a decrease in expression of an RB1 gene or protein, or positive expression of hyperphosphorylated RB1 protein is detected, the method comprising measuring MYT1 inhibitory activity of a candidate compound, and selecting a candidate compound having MYT1 inhibitory activity as a compound effective for treatment of cancer.

The method according to the present embodiment comprises measuring the MYT1 inhibitory activity of a candidate compound, and selecting the candidate compound as the compound effective for treatment of cancer when the candidate compound has the MYT1 inhibitory activity.

In the present invention, the MYT1 inhibitor means a substance capable of directly or indirectly neutralizing, blocking, inhibiting, reducing, or preventing the MYT1 activity. Examples of the activity of MYT1 protein include threonine kinase activity or tyrosine kinase activity.

Inhibition of the threonine kinase activity or tyrosine kinase activity of MYT1 by the compound can be confirmed by, for example, treating a purified MYT1 protein standard sample with the test compound, adding ATP, and using the degree of hydrolysis of ATP as the criterion. The degree of ATP hydrolysis can be evaluated by using Kinase-Glo (manufactured by Promega) or ADP-Glo (manufactured by Promega) (Non Patent Literature 8). When the threonine kinase activity or tyrosine kinase activity of MYT1 is inhibited by the test compound, a decrease in the ATP hydrolysis is confirmed, in comparison to the control (e.g., the degree of ATP hydrolysis by MYT1 which is not treated with the test compound).

Inhibition of the threonine kinase activity of MYT1 protein by the compound can be confirmed by, for example, treating a purified MYT1 protein standard sample with the test compound, adding CDK1 which is a substrate of MYT1, and using the degree of phosphorylation of Thr at position 14 of CDK1 as the criterion. The degree of phosphorylation at the position can be evaluated by a Western blotting method using an antibody that specifically recognizes phosphorylation of Thr at position 14 of CDK1 (manufactured by Abcam plc), an ELISA method using an antibody that specifically recognizes CDK1 and the antibody that specifically recognizes phosphorylation of Thr at position 14 of CDK1, or an AlphaLISA method using the antibody that specifically recognizes CDK1 and the antibody that specifically recognizes phosphorylation of Thr at position 14 of CDK1 (manufactured by PerkinElmer, Inc.). When the threonine kinase activity of MYT1 is inhibited by the test compound, a decrease in phosphorylation is confirmed, in comparison to the control (e.g., the degree of phosphorylation of Thr at position 14 of CDK1 by MYT1 which is not treated with the test compound).

Inhibition of the threonine kinase activity of MYT1 by the compound can be confirmed by, for example, using the degree of phosphorylation of Thr at position 14 of CDK1 which is the substrate protein of the threonine kinase activity of MYT1 as the criterion, to a lysate of a cell treated with the test compound. The degree of phosphorylation at the position can be evaluated by a Western blotting method using an antibody that specifically recognizes phosphorylation of Thr at position 14 of CDK1 (manufactured by Abcam plc), an ELISA method using an antibody that specifically recognizes CDK1 and the antibody that specifically recognizes phosphorylation of Thr at position 14 of CDK1, or an AlphaLISA method using the antibody that specifically recognizes CDK1 and the antibody that specifically recognizes phosphorylation of Thr at position 14 of CDK1 (manufactured by PerkinElmer, Inc.) (Non Patent Literature 8). When the threonine kinase activity of MYT1 is inhibited by the test compound, a decrease in phosphorylation is confirmed, in comparison to the control (e.g., the degree of phosphorylation of Thr at position 14 of CDK1 in a lysate of a cell which is not treated with the test compound).

Inhibition of the expression of MYT1 by the compound can be confirmed by, for example, detecting a decrease in expression of MYT1 in a cell treated with the test compound. Examples of the method for detecting a decrease in expression of MYT1 include a method in which normally, the expression level of MYT1 is detected at a transcriptional level or a translational level, and compared to a control (e.g. expression level in a cell which is not treated with the test compound) to confirm that the expression level is lower than the control.

In the method for detecting the expression level of MYT1 at a transcriptional level, first, RNA or cDNA is prepared from a cell treated with the test compound. The method for extracting RNA from the cell is not particularly limited, and a known method can be appropriately selected and used. Examples thereof include extraction methods using phenol and a chaotropic salt (more specifically, extraction methods using a commercially available kit such as TRIzol (manufactured by Invitrogen Corporation) or ISOGEN (manufactured by Wako Pure Chemical Industries, Ltd.)), and methods using another commercially available kit (e.g. RNAPrep Total RNA Extraction Kit (manufactured by Beckman Coulter Inc.), RNeasy Mini (manufactured by QIAGEN N.V.) or RNA Extraction Kit (manufactured by Pharmacia Biotech, Inc.)). Further, the reverse transcriptase used for preparation of cDNA from extracted RNA is not particularly limited, and examples thereof include reverse transcriptases derived from retroviruses such as RAV (Rous associated virus) and AMV (Avian myeloblastosis virus), and reverse transcriptases derived from mouse retroviruses such as MMLV (Moloney murine leukemia virus).

Subsequently, an oligonucleotide primer or an oligonucleotide probe is used for an amplification reaction or a hybridization reaction, and an amplified product or a hybrid product thereof is detected. As such a method, for example, an RT-PCR method, a Northern blot method, a dot blot method, a DNA array method, an in situ hybridization method, an RNase protection assay method, mRNA-seq, or the like can be used. Those skilled in the art can design an oligonucleotide primer or an oligonucleotide probe suitable for each method in a conventional manner on the basis of a nucleotide sequence of cDNA for MYT1.

In the method for detecting the expression level of MYT1 at a translational level, first, a protein sample is prepared from a cell treated with the test compound. Subsequently, using an antibody specific to MYT1 protein, an antigen-antibody reaction is carried out, and MYT1 protein is detected. In such a method for detecting protein using an antibody, for example, an antibody specific to MYT1 protein is added to the protein sample to carry out an antigen-antibody reaction, and binding of the antibody to MYT1 protein is detected. When the sample is labeled with antibody specific to MYT1 protein, MYT1 protein can be directly detected, and when the sample is not labeled, a labeled molecule that recognizes the antibody (e.g. secondary antibody or protein A) can be further applied to indirectly detect MYT1 protein using the label of the molecule. As such a method, for example, an immunohistochemistry (immunostaining) method, a Western blotting method, an ELISA method, flow cytometry, imaging cytometry, radioimmunoassay, an immunoprecipitation method, or an analysis method using an antibody array can be used.

The type, the origin, and the like of an antibody used are not particularly limited, and a monoclonal antibody is preferable. An oligoclonal antibody (mixture of several antibodies or dozens of antibodies) or a polyclonal antibody can also be used as long as it is possible to detect MYT1 protein with sufficient specificity. Functional fractions of antibodies such as Fab, Fab', F(ab')2, Fv, scFv, sc(Fv)2, dsFv and diabodies, and multimers (e.g. dimers, trimers, tetramers, and polymers) thereof can also be used. Such an anti-MYT1 protein antibody may be a marketed product.

The MYT1 protein can also be detected by mass spectrometry (MS). In particular, analysis by a mass spectrometer coupled with liquid chromatography (LC/MS) is sensitive, and therefore advantageous. Detection by mass spectrometry can be performed by, for example, labeling the protein sample with the protein, fractionating the labeled protein, subjecting the fractionated protein to mass analysis, and identifying MYT1 protein from the mass analysis value. As the label, an isotopic labeling reagent known in the art can be used, and an appropriate labeling reagent can be obtained as a marketed product. The fractionation can be performed by a method known in the art, and for example, a commercially available ionexchange column or the like can be used.

As used herein, the term "having MYT1 inhibitory activity" means that the MYT1 activity is reduced in vitro, in a cell culture system, or in animals, and for example, the inhibitory activity IC₅₀ of MYT1 measured is preferably 10 µM or less, 5 µM or less, or 1 µM or less. A compound having an MYT1 inhibitory activity (IC₅₀) of 100 nM or less, 10 nM or less, 3 nM or less, 100 pM or less, or 10 pM or less is more preferable. A compound having an MYT1 inhibitory activity (IC₅₀) of 1 nM to 1 µM, 1 nM to 750 nM, 1 nM to 500 nM, or 1 nM to 250 nM is further preferable. A compound having an MYT1 inhibitory activity (IC₅₀) of less than 20 nM, or 1 nM to 20 nM is particularly preferable. A compound having higher MYT1 inhibitory activity (having lower IC₅₀) can be selected as the compound more effective for treatment or prevention of cancer in a patient in which RB1 gene mutation positivity, a decrease in expression of an RB1 gene or protein, or positive expression of hyperphosphorylated RB1 protein is detected.

The therapeutic efficacy of the compound selected by the method according to the present embodiment is further enhanced by using the compound in combination with a chemotherapeutic agent in treatment of cancer in a patient in which RB1 gene mutation positivity, a decrease in expression of an RB1 gene or protein, or positive expression of hyperphosphorylated RB1 protein is detected.

### [Examples]

The content of the present invention will be further described in Examples below, but the present invention is not limited to the content. All the compounds and reagents were obtained from commercial suppliers or synthesized by known methods.

### (Example 1: Cytotoxic activity evaluation using small interfering RNA (siRNA))

### 1. Experimental material and method

### (1) Cell line

NCI-H441 (received from ATCC), NCI-H460 (received from ATCC), A549 (received from ATCC), EKVX (received from NCI), NCI-H1993 (received from ATCC), NCI-H2122 (received from ATCC), PC-9 (received from RIKEN), NCI-H292 (received from ATCC), NCI-H1666 (received from ATCC), NCI-H661 (received from ATCC), NCI-H322 (received from ECACC), HOP-62 (received from NCI), ABC-1 (received from human tissue bank), NCI-H358 (received from ATCC), ChaGo-K-1 (received from ATCC), NCI-H2110 (received from ATCC), Lu65 (received from human tissue bank), NCI-H596 (received from ATCC), and NCI-H1155 (received from ATCC) (all of which are non-small cell lung cancers) were used in the evaluation of the present invention.

NCI-H441, NCI-H460, A549, EKVX, NCI-H2122, PC-9, NCI-H292, NCI-H1666, NCI-H661, NCI-H322, HOP-62, ABC-1, and NCI-H358 are the RB1 wild-type cancer cell lines having no RB1 gene mutation, and RB1 functions in these cell lines. NCI-H1993 and PC-9 are the RB1 wild-type cancer cell lines having no mutations causing insertion, substitution, and/or addition of at least one amino acid residue to wild-type RB1 protein and having the expression of RB1, and RB1 functions in these cell lines. ChaGo-K-1 and NCI-H2110 are cancer cell lines in which a specific region of the RB1 gene is homozygously deleted and which further have a missense mutation of E837K and a missense mutation of G449E, respectively. Lu65, NCI-H596, and NCI-H1155 are cancer cell lines having a nonsense mutation or a frameshift mutation in the RB1 gene. A decrease in RB1 function is caused in these cell lines. The information on these cancer cell lines and gene mutations are summarized in Table 1.

**[Table 1]**

| Cell line | Tissue | Subtype | Status of RB1 gene |
|---|---|---|---|
| NCI-H441 | Lung | NSCLC Adenocarcinoma | No alteration¹ |
| NCI-H460 | Lung | NSCLC Large Cell | No alteration¹ |
| A549 | Lung | NSCLC Adenocarcinoma | No alteration¹ |
| EKVX | Lung | NSCLC Adenocarcinoma | No alteration¹ |
| NCI-H1993 | Lung | NSCLC Adenocarcinoma | No alteration^{1, 2} |
| NCI-H2122 | Lung | NSCLC Adenocarcinoma | No alteration¹ |
| PC-9 | Lung | NSCLC Adenocarcinoma | No alteration^{1, 3} |
| NCI-H292 | Lung | NSCLC Mucoepidermoid | No alteration¹ |
| NCI-H1666 | Lung | NSCLC Adenocarcinoma | No alteration¹ |
| NCI-H661 | Lung | NSCLC Large Cell | No alteration¹ |
| NCI-H322 | Lung | NSCLC Adenocarcinoma | No alteration¹ |
| HOP-62 | Lung | NSCLC Adenocarcinoma | No alteration¹ |
| ABC-1 | Lung | NSCLC Adenocarcinoma | No alteration¹ |
| NCI-H358 | Lung | NSCLC Adenocarcinoma | No alteration¹ |
| ChaGo-K-1 | Lung | NSCLC | E837K, HOMDEL¹ |
| NCI-H2110 | Lung | NSCLC | G449E, HOMDEL¹ |
| LU65 | Lung | NSCLC Large Cell | S82*¹ |
| NCI-H596 | Lung | NSCLC Adenosquamous | S182Ifs*3¹ |
| NCI-H1155 | Lung | NSCLC Large Cell | R467*¹ |

| | | | |
|---|---|---|---|
| ¹ Nature 2019 May; 569(7757): 503-508. ² Nat Commun. 2021 Dec 3; 12(1): 7064. ³ Cancer Discov. 2019 Feb; 9(2): 230-247. | | | |

NCI-H441, NCI-H460, EKVX, NCI-H2122, NCI-H292, NCI-H1666, NCI-H322, ABC-1, NCI-H358, ChaGo-K-1, NCI-H2110, NCI-H596, and NCI-H1155 are CCNE1 gene-not-amplified cell lines having no amplification of CCNE1 gene. Lu65 is a cancer cell line in which CCNE1 gene is homozygously deleted, and is a CCNE1 gene-not-amplified cell line. A549, HOP-62, and NCI-H661 are CCNE1 gene amplified cell lines having amplification of CCNE1 gene. There is no known information on the presence or absence of amplification of CCNE1 gene in PC-9 and NCI-H1993. The information on these cancer cell lines and gene mutations are summarized in Table 2.

**[Table 2]**

| Cell line | Tissue | Subtype | Status of CCNE1 gene |
|---|---|---|---|
| NCI-H441 | Lung | NSCLC Adenocarcinoma | No alteration¹ |
| NCI-H460 | Lung | NSCLC Large Cell | No alteration¹ |
| A549 | Lung | NSCLC Adenocarcinoma | AMP¹ |
| EKVX | Lung | NSCLC Adenocarcinoma | No alteration¹ |
| NCI-H1993 | Lung | NSCLC Adenocarcinoma | Not profiled¹ |
| NCI-H2122 | Lung | NSCLC Adenocarcinoma | No alteration¹ |
| PC-9 | Lung | NSCLC Adenocarcinoma | Not profiled¹ |
| NCI-H292 | Lung | NSCLC Mucoepidermoid | No alteration¹ |
| NCI-H1666 | Lung | NSCLC Adenocarcinoma | No alteration¹ |
| NCI-H661 | Lung | NSCLC Large Cell | AMP¹ |
| NCI-H322 | Lung | NSCLC Adenocarcinoma | No alteration¹ |
| HOP-62 | Lung | NSCLC Adenocarcinoma | AMP¹ |
| ABC-1 | Lung | NSCLC Adenocarcinoma | No alteration¹ |
| NCI-H358 | Lung | NSCLC Adenocarcinoma | No alteration¹ |
| ChaGo-K-1 | Lung | NSCLC | No alteration¹ |
| NCI-H2110 | Lung | NSCLC | No alteration¹ |
| LU65 | Lung | NSCLC Large Cell | HOMDEL¹ |
| NCI-H596 | Lung | NSCLC Adenosquamous | No alteration¹ |
| NCI-H1155 | Lung | NSCLC Large Cell | No alteration¹ |

| | | | |
|---|---|---|---|
| 1 Nature 2019 May; 569(7757): 503-508. | | | |

### (2) Small interfering RNA (siRNA)

ON-TARGETplus individual siRNA (manufactured by Dharmacon) was used for the suppression of expression of each gene. Lipofectamine RNAiMAX (manufactured by Thermo Fisher Scientific, Inc., product code: 13778150) was used for transfection. In addition, non-targeted siRNA (ON-TARGET plus Non-targeting Control, Dharmacon product code: D-001810-01) was used as the negative control. The nucleotide sequence of non-targeted siRNA is shown as SEQ ID No: 3. For dilution of each siRNA, 1 × siRNA buffer obtained by diluting 5 × siRNA buffer (manufactured by Dharmacon: B-002000-UB-100) to 5 times with nuclease free water (manufactured by Ambion, Inc.: AM9932) was used.

### (3) Pemetrexed cytotoxicity test upon suppression of expression of MYT1

In each cancer cell line shown in Table 1 and Table 2 of the above (1), the cytotoxic activity caused by pemetrexed treatment upon suppression of expression of MYT1 was evaluated. Each cell line was cultured using culture solutions described in the following Table 3 in a cell culture flask (manufactured by Corning Inc.: 430641U), the cell surface was washed with PBS (manufactured by Nacalai tesque, Inc.: 14249-24), then trypsin (manufactured by Nacalai tesque, Inc.: 35554-64) was added thereto, and the mixture was incubated at 37°C for 5 min and then suspended using each culture solution. The number of cells was measured using an automatic cell counter (manufactured by Chemometec: NC-200) and Via1-Casette (manufactured by Chemometec: 941-0011), then cells were adjusted to achieve the number of cells seeded described in the following Table 4 using each culture solution, and 32 µL/mL of each cell line was seeded in a 384 well-plate (Greiner).

**[Table 3]**

| Cell line | Medium composition |
|---|---|
| NCI-H441 | RPMI-1640 (SIGMA, R8758-500 mL), 10% FBS (SIGMA, 172012) |
| NCI-H460 | RPMI-1640 (SIGMA, R8758-500 mL), 10% FBS (SIGMA, 172012) |
| A549 | D-MEM (SIGMA, D5796), 10% FBS (SIGMA, 172012) |
| EKVX | RPMI-1640 (SIGMA, R8758-500 mL), 10% FBS (SIGMA, 172012) |
| NCI-H1993 | RPMI-1640 (SIGMA, R8758-500 mL), 10% FBS (SIGMA, 172012), 10 mM HEPES (SIGMA, H0887), 4.5g/L glucose (SIGMA G8769). 1 mM sodium pyruvate (GIBCO. 11360-070) |
| NCI-H2122 | RPMI-1640 (SIGMA, R8758-500 mL), 10% FBS (SIGMA, 172012), 10 mM HEPES (SIGMA, H0887), 4.5g/L glucose (SIGMA G8769), 1 mM sodium pyruvate (GIBCO, 11360-070) |
| PC-9 | RPMI-1640 (SIGMA, R8758-500 mL), 10% FBS (SIGMA, 172012) |
| NCI-H292 | RPMI-1640 (SIGMA, R8758-500 mL), 10% FBS (SIGMA, 172012) |
| NCI-H1666 | D-MEM:Ham's F-12=1:1 (GIBCO, 11330-032), 2 mML-glutamine (SIGMA, G7513), 0.02 mg/mL insulin (GIBCO, 12585-014), 0.01 mg/mL transferrin (SIGMA, T8158), 25 nM Sodium selenite (SIGMA, S9133), 50 nM hydrocortisone (SIGMA, M6909), 1 ng/mL hEGF (R&D systems, 236-EG), 0.01 mM Ethanolamine (SIGMA, E0135), 0.01 mM O-phosphorylethanolamine (SIGMA, P0503), 100 pM T3 (SIGMA. T5516). 0.5% (w/v) BSA (SIGMA, A9576) |
| NCI-H661 | RPMI-1640 (SIGMA, R8758-500 mL), 10% FBS (SIGMA, 172012), 10 mM HEPES (SIGMA, H0887), 4.5g/L glucose (SIGMA G8769), 1 mM sodium pyruvate (GIBCO, 11360-070) |
| NCI-H322 | RPMI-1640 (SIGMA, R8758-500 mL), 10% FBS (SIGMA, 172012) |
| HOP-62 | RPMI-1640 (SIGMA, R8758-500 mL), 10% FBS (SIGMA, 172012) |
| ABC-1 | E-MEM (SIGMA, M4655)m 10% FBS (SIGMA, 172012) |
| NCI-H358 | RPMI-1640 (SIGMA, R8758-500 mL), 10% FBS (SIGMA, 172012) |
| ChaGo-K-1 | RPMI-1640 (SIGMA, R8758-500 mL), 10% FBS (SIGMA, 172012), 0.1 mM NEAA (GIBCO, 11140-050), 1 mM sodium pyruvate (GIBCO. 11360-070) |
| NCI-H2110 | RPMI-1640 (SIGMA, R8758-500 mL), 10% FBS (SIGMA, 172012), 10 mM HEPES (SIGMA, H0887), 4.5g/L glucose (SIGMA G8769). 1 mM sodium pyruvate (GIBCO. 11360-070) |
| LU65 | RPMI-1640 (SIGMA, R8758-500 mL), 10% FBS (SIGMA, 172012) |
| NCI-H596 | RPMI-1640 (SIGMA, R8758-500 mL), 10% FBS (SIGMA, 172012) |
| NCI-H1155 | D-MEM:Ham's F-12=1:1 (GIBCO, 11330-032), 0.02 mg/mL insulin (GIBCO, 12585-014), 0.01 mg/mL transferrin (SIGMA, T8158), 25 nM Sodium selenite (SIGMA, S9133), 50 nM hydrocortisone (SIGMA, M6909), 1 ng/mL hEGF (R&D systems, 236-EG), 0.01 mM Ethanolamine (SIGMA, E0135), 0.01 mM O-phosphorylethanolamine (SIGMA, P0503), 100 pM T3 (SIGMA, T5516), 0.5% (w/v) BSA (SIGMA, A9576), 0.5 mM Sodium pyruvate (GIBCO, 11360-070), 10 mM HEPES (SIGMA, H0087), 2 mM Glutamine (SIGMA, G6013) |

**[Table 4]**

| Cell line | Number of cells seeded (cells/well) | siRNA concentration (nM) |
|---|---|---|
| NCI-H441 | 1000 | 10 |
| NCI-H460 | 1000 | 10 |
| A549 | 1000 | 10 |
| EKVX | 1000 | 10 |
| NCI-H1993 | 1000 | 10 |
| NCI-H2122 | 1000 | 10 |
| PC-9 | 1000 | 10 |
| NCI-H292 | 1000 | 10 |
| NCI-H1666 | 1000 | 10 |
| NCI-H661 | 1000 | 10 |
| NCI-H322 | 1000 | 10 |
| HOP-62 | 1000 | 10 |
| ABC-1 | 1000 | 10 |
| NCI-H358 | 1000 | 10 |
| ChaGo-K-1 | 1000 | 10 |
| NCI-H2110 | 1000 | 10 |
| LU65 | 1000 | 10 |
| NCI-H596 | 1000 | 10 |
| NCI-H1155 | 500 | 10 |

Then, using Opti-MEM (manufactured by Thermo Fischer Scientific, Inc.: 31985-062), Lipofectamine RNAiMAX (manufactured by Thermo Fischer Scientific, Inc.: 13778150) was diluted to 100 times, and each of 20 µM of test substance (MYT1 siRNA #16 and MYT1 siRNA #18) or 20 µM of negative control siRNA (ON-TARGET plus Non-targeting Control) was diluted to 400 times, and then 8 µL/well of the mixed solution was added to the plates on which the above cells were seeded. Thereafter, the plates were shaken to transfect each siRNA at a final concentration of 10 nM. Dharmacon product code: J-005026-16 was used as MYT1 siRNA #16, and the nucleotide sequence of siRNA is shown as SEQ ID No: 4. Dharmacon product code: J-005026-18 was used as MYT1 siRNA #18, and the nucleotide sequence of siRNA is shown as SEQ ID No: 5.

After 1 day of culture, 10 µL/mL of pemetrexed (manufactured by Tokyo Kasei Kogyo Co., Ltd.) diluted with DMSO and PBS was added. The pemetrexed concentration was set to have a final concentration of 0 to 20 µM, and when the cell survival rate treated with 5 µM of pemetrexed in the treatment of negative control siRNA was 50% or more, the maximum concentration was increased until the treatment with 20 µM of pemetrexed, and the evaluation was carried out. After addition of pemetrexed, cells were cultured for 7 days, and intracellular ATP which is a cell survival marker was measured using CellTiter-Glo2.0 Cell Viability Assay (manufactured by Promega Corporation) and used in the monitoring of the number of cells.

The cell survival rate is calculated by setting the cell survival rate in negative control siRNA-treated cells which were not treated with pemetrexed to 100%, and the cytotoxic activity is represented by subtracting the survival rate from the value of 100%. As representative examples, the cytotoxic activities of NCI-H460 and NCI-H596 caused by pemetrexed treatment upon suppression of MYT1 expression are shown in Figures 1 and 2, respectively.

IC₅₀ (50% Inhibition Concentration (µM)) of pemetrexed in each cell was calculated from each pemetrexed treatment concentration and cell survival curve. IC₅₀ of pemetrexed upon treatment of negative control siRNA and MYT1 siRNA (#16, #18) and the cytotoxic activity upon no addition of pemetrexed and treatment with MYT1 siRNA (#16, #18) alone were described in Table 5. Based on the cytotoxic activity upon treatment of negative control siRNA at each pemetrexed concentration and the cytotoxic activity when MYT1 siRNA (#16) or MYT1 siRNA (#18) was treated without pemetrexed treatment, IC₅₀ was calculated from the dose-response curve estimated based on the Bliss independence upon treatment of MYT1 siRNA (#16) and MYT1 siRNA (#18) at each pemetrexed concentration. The -Log2 value of fold change of IC₅₀ and the maximum value of Bliss score, which are indexes of the combination effect, were calculated by comparing with the actually obtained cell survival curve, and described in Table 6 (see Non Patent Literature 9). In addition, HSA score, which is an index of the combination effect different from the Bliss independence, was calculated, and the maximum value thereof was described in Table 5 (see Non Patent Literature 9).

As a represented example, the cell survival curve predicted based on the observed cell survival curve and the Bliss independence of NCI-H596, and the cell survival curve predicted based on HSA were shown in Figure 3. In Figure 4, the -Log2 value of fold change of IC₅₀, the maximum value of Bliss score, and the maximum value of HSA score were plotted separately for the cell line whose RB1 gene is wild type and where RB1 functions, and the cell line whose RB1 gene is mutant and where a decrease in RB1 function is caused, and Mean ± SD of each of them was shown. Student's t-test was carried out to calculate p values, and asterisks were described in Figure 4 based on the values (ns: p ≥ 0.05, *: p < 0.05, **: p < 0.01, ***: p < 0.001, ****: p < 0.0001). In addition, the -Log2 value of fold change of IC₅₀, the maximum value of Bliss score, and the maximum value of HSA score are plotted separately for the CCNE1 gene-not-amplified cell line and the CCNE1 gene amplified cell line, and Mean ± SD of each of them is shown. Student's t-test was carried out to calculate p values, and asterisks were described in Figure 5 based on the values (ns: p ≥ 0.05, *: p < 0.05, **: p < 0.01, ***: p < 0.001, ****: p < 0.0001).

**[Table 5]**

| Cell line | IC50 (nM) | | | Cytotoxic activity of siRNA alone (%) | | Predicted IC50 based on Bliss (nM) | |
|---|---|---|---|---|---|---|---|
| | NT | MYT1 #16 | MYT1 #18 | MYT1 #16 | MYT1 #18 | MYT1 #16 | MYT1 #18 |
| NCI-H441 | 883.916 | 95.561 | 865.108 | 12.986 | 22.238 | 461.690 | 170.459 |
| NCI-H460 | 667.217 | 465.143 | 562.809 | -6.609 | -2.387 | 805.595 | 716.635 |
| A549 | 2852.397 | 1533.915 | 1952.736 | -3.683 | 0.844 | 3066.904 | 2820.321 |
| EKVX | 404.198 | 165.981 | 263.082 | -1.125 | 1.053 | 426.188 | 384.361 |
| NCI-H1993 | 180.559 | 24.572 | 105.991 | 35.910 | 10.347 | 4.704 | 117.041 |
| NCI-H2122 | 159.314 | 13.471 | 47.196 | 11.825 | 5.899 | 58.336 | 103.643 |
| PC-9 | 145.251 | 32.868 | 77.700 | -5.180 | -9.517 | 176.605 | 206.344 |
| NCI-H292 | 390.653 | 71.145 | 186.885 | 19.878 | 11.155 | 82.588 | 204.002 |
| NCI-H1666 | 618.531 | 472.503 | 291.293 | -4.467 | 1.456 | 744.711 | 581.438 |
| NCI-H661 | 315.183 | 45.688 | 128.729 | 20.313 | 9.766 | 64.827 | 192.765 |
| NCI-H322 | 1598.537 | 1028.909 | 804.599 | -10.363 | -6.271 | 2222.711 | 1955.521 |
| HOP-62 | 175.108 | 98.115 | 91.893 | 8.347 | 10.654 | 134.182 | 117.126 |
| ABC-1 | 972.973 | 362.671 | 313.342 | 3.870 | 7.811 | 869.660 | 774.096 |
| NCI-H358 | 986.491 | 530.737 | 333.356 | 0.021 | 2.693 | 985.528 | 871.636 |
| ChaGo-K-1 | 913.691 | 133.466 | 192.109 | -1.049 | -2.748 | 953.883 | 1021.447 |
| NCI-H2110 | 226.935 | 10.513 | 41.415 | 15.886 | 9.660 | 68.562 | 127.226 |
| LU65 | 1068.495 | 134.378 | 140.025 | 8.109 | -0.297 | 726.736 | 1082.726 |
| NCI-H596 | 384.837 | 7.015 | 20.987 | 13.549 | -2.058 | 164.652 | 430.362 |
| NCI-H1155 | 9844.578 | 672.589 | 1650.028 | 5.782 | 30.960 | 8835.252 | 4069.395 |

**[Table 6]**

| Cell line | IC50 fold change (-log2) | | Bliss score maximum value | | HSA score maximum value | |
|---|---|---|---|---|---|---|
| | MYT1 #16 | MYT1 #18 | MYT1 #16 | MYT1 #18 | MYT1 #16 | MYT1 #18 |
| NCI-H441 | 2.272 | -2.343 | 21.600 | 0.784 | 30.794 | 17.896 |
| NCI-H460 | 0.792 | 0.349 | 17.443 | 11.230 | 9.093 | 9.099 |
| A549 | 1.000 | 0.530 | 17.634 | 9.581 | 15.047 | 10.174 |
| EKVX | 1.360 | 0.547 | 16.454 | 6.653 | 15.771 | 7.060 |
| NCI-H1993 | -2.385 | 0.143 | 5.736 | 7.046 | 21.843 | 14.207 |
| NCI-H2122 | 2.115 | 1.135 | 15.749 | 13.938 | 20.659 | 17.140 |
| PC-9 | 2.426 | 1.409 | 35.148 | 18.833 | 31.604 | 14.476 |
| NCI-H292 | 0.215 | 0.126 | 19.031 | 6.895 | 21.907 | 11.985 |
| NCI-H1666 | 0.656 | 0.997 | 8.127 | 13.160 | 5.753 | 13.970 |
| NCI-H661 | 0.505 | 0.583 | 8.081 | 11.699 | 22.502 | 16.633 |
| NCI-H322 | 1.111 | 1.281 | 24.679 | 23.827 | 16.828 | 19.509 |
| HOP-62 | 0.452 | 0.350 | 20.165 | 20.375 | 24.453 | 25.849 |
| ABC-1 | 1.262 | 1.305 | 21.970 | 22.139 | 25.475 | 26.822 |
| NCI-H358 | 0.893 | 1.387 | 12.322 | 13.168 | 12.336 | 14.592 |
| ChaGo-K-1 | 2.837 | 2.411 | 40.066 | 34.464 | 39.075 | 32.605 |
| NCI-H2110 | 2.705 | 1.619 | 24.584 | 23.200 | 36.712 | 28.531 |
| LU65 | 2.435 | 2.951 | 29.374 | 34.154 | 34.405 | 33.984 |
| NCI-H596 | 4.553 | 4.358 | 56.598 | 44.329 | 67.277 | 42.707 |
| NCI-H1155 | 3.715 | 1.302 | 77.287 | 23.341 | 76.586 | 32.708 |

### 2. Results

As shown in Table 5, Table 6, and Figure 4, a significant enhancement in the IC₅₀, Bliss score, and HSA score of the cytotoxic activity caused by pemetrexed upon suppression of expression of MYT1 was confirmed in the cancer cell line in which an RB1 gene mutation is observed and a decrease in RB 1 function is caused, as compared with the cancer cell line in which no RB 1 gene mutation is observed and RB 1 functions. These results demonstrated that the cytotoxic activity of the chemotherapeutic agent such as pemetrexed can be effectively enhanced by suppressing the expression of MYT1 in the cancer cell line in which an RB1 gene mutation is observed and a decrease in RB1 function is caused. As shown in Table 5, Table 6, and Figure 5, a significant enhancement in the IC₅₀, Bliss score, and HSA score of the cytotoxic activity caused by pemetrexed upon suppression of expression of MYT1 was not observed in the cell line in which amplification of the CCNE1 gene is caused, as compared with the cell line in which amplification of the CCNE1 gene is not caused.

### (Example 2: Test for binding to ATP binding site of MYT1 and MYT1 kinase activity inhibition test)

Examples and meanings of the abbreviations as used herein are listed below.
DCM: dichloromethane
DMA: N,N-dimethylacetamide
DMSO: dimethyl sulfoxide
EtOH: ethanol
TFA: trifluoroacetylacetic acid
THF: tetrahydrofuran
TBME: tert-butyl methyl ether
HATU: O-(7-azabenzotriazol-1-yl)-N,N,N',N'-tetramethyluronium hexafluorophosphate
XPhos Pd G4: (2-dicyclohexylphosphino-2',4',6'-triisopropyl-1,1'-biphenyl)(2-(2'-(N-methyl)amino-1,1'-biphenyl))palladium(II) methanesulfonate (CAS Number: 1599466-81-5)

Mass spectrum data were obtained using a single quadrupole mass detector (LCMS-2020) equipped with an ultra-high performance liquid chromatography (Nexera UC) manufactured by SHIMADZU CORPORATION, or a single quadrupole mass detector (SQD or SQD2) equipped with an Acquity ultra-high performance liquid chromatography (UPLC or UPLC I-Class) manufactured by Waters Corporation.

As the conditions of the high-performance liquid chromatography, A or F of the following Table 7 and Table 8 was used. In the following Table 7 and Table 8, "TFA" means trifluoroacetic acid, "FA" means formic acid, "AA" means ammonium acetate, and "AC" means ammonium hydrogencarbonate.

**[Table 7]**

| Analysis conditions | Apparatus | Column | Column temperature | Detection wavelength (PDA) |
|---|---|---|---|---|
| A | Acquity | Ascentis Express C18 | 35°C | 210-400 nm |
| | SQD/SQD2 | 2.1 mm I.D. × 50 mm L, 2.7 µm | | |
| F | Nexera UC | Ascentis Express C18 | 35°C | 210-400 nm |
| | LCMS-2020 | 2.1 mm I.D. × 50 mm L, 2.7 µm | | |

**[Table 8]**

| Analysis conditions | Mobile phase | Time after injection (min) | Mobile phase A/B | Flow velocity (mL/min) |
|---|---|---|---|---|
| A | A) 0.1% FA/CH₃CN | 0-1.0 | 5/95 → 100/0 | 1 |
| | B) 0.1% FA/H2O | 1.0-1.4 | 100/0 | |
| F | A) 0.1% FA/CH₃CN | 0-1.5 | 5/95 → 100/0 | 1 |
| | B) 0.1% FA/H2O | 1.5-2.0 | 100/0 → 100/0 | |

Commercially available reagents were used without further purification. All the nonaqueous reactions were conducted by using commercially available anhydrous solvents. Concentration under reduced pressure or solvent distillation was carried out using a rotary evaporator.

As used herein, the term "room temperature" means a temperature from about 20°C to about 25°C.

### 1. Experimental material and method

### (1) Production of 3-amino-6-cyclopropyl-4-(7-fluoro-1H-indazol-4-yl)-1H-1,7-phenanthrolin-2-one (a compound represented by the formula (3), hereinafter, also referred to as "compound 1 ")

The compound 1 was produced in accordance with the following method.

### Compound c-1

### 8-Bromo-6-iodoquinolin-5-amine

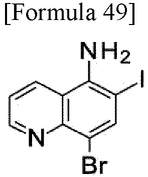

A suspension of 5-amino-8-bromoquinoline (49.7 g, 223 mmol) in acetonitrile (446 mL) in a reaction vessel was cooled to 0°C, TFA (18.02 mL, 234 mmol) was added, and then N-iodosuccinimide (52.6 g, 234 mmol) was added, and the mixture was stirred for 1.5 hours. After the reaction mixture was poured into a 3% aqueous sodium bicarbonate solution (1.25 L), a 10% aqueous sodium thiosulfate solution (250 mL) was added. The solid was collected by filtration and washed with water (650 mL) to obtain the title compound (66.78 g, yield 86%).
LCMS: m/z 349 [M+H]⁺
HPLC retention time: 0.97 min (analysis condition F)

### Compound c-2

### (E)-N'-(8-Bromo-6-iodoquinolin-5-yl)-N,N-dimethylmethaneimidamide

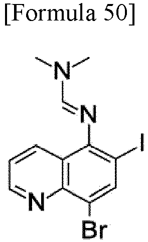

N,N-Dimethylformamidedimethylacetal (80 mL, 594 mmol) was added to a suspension of 8-bromo-6-iodoquinolin-5-amine (compound c-1, 51.86 g, 149 mmol) in EtOH (248 mL) in a reaction vessel, and the mixture was stirred under a nitrogen atmosphere at 80°C for 2.5 hours. The reaction mixture was cooled to room temperature, and ethyl acetate (1.0 L), a 10% aqueous sodium thiosulfate solution (260 mL), and brine (260 mL) were added thereto. The obtained mixture was filtered through amino silica gel (104 g), and amino silica gel was washed with ethyl acetate (260 mL). The organic layer was separated from the filtrate, and the aqueous layer was extracted with ethyl acetate (260 mL). The obtained organic layers were combined, washed with a 13% salt solution, and dried over anhydrous magnesium sulfate, the drying agent was filtered off, and then the filtrate was concentrated under reduced pressure. TBME (50 mL) and hexane (100 mL) were added to the obtained residue, the mixture was stirred at room temperature for 15 min, hexane (100 mL) was further added thereto, and the mixture was stirred for 30 min. The solid was collected by filtration and washed with hexane (500 mL) to obtain the title compound (56.08 g, yield 93%).
LCMS: m/z 404 [M+H]⁺
HPLC retention time: 0.62 min (analysis condition F)

### Compound c-3

### 4-Bromo-7-fluoro-2-(oxan-2-yl)indazole

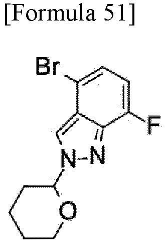

To a solution of 4-bromo-7-fluoro-1H-indazole (15 g, 69.8 mmol) in DCM (349 mL) in a reaction vessel, 3,4-dihydro-2H-pyran (11.74 g, 140 mmol) and pyridinium p-toluene sulfonate (3.51 g, 13.95 mmol) were added, and the mixture was stirred at room temperature for 2 hours. The reaction mixture was concentrated under reduced pressure, and the obtained residue was purified by silica gel column chromatography to obtain the title compound (19.4 g, yield 93%) as a colorless liquid.
LCMS: m/z 299 [M+H]⁺
HPLC retention time: 1.24 min (analysis condition F)

### Compound c-4

### (2,4,6-Trichlorophenyl) 7-fluoro-2-(oxan-2-yl)indazole-4-carboxylate

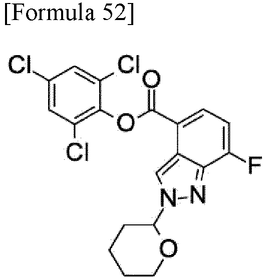

Toluene (436 mmol) was added to a reaction vessel containing a mixture of 4-bromo-7-fluoro-2-(oxan-2-yl)indazole (compound c-3, 30.0 g, 100 mmol) and triethylamine (28.0 mL, 201 mmol), and the reaction vessel was degassed under reduced pressure and subjected to nitrogen replacement. To the reaction mixture, palladium(II) acetate (0.68 g, 3.01 mmol) and 4,5-bis(diphenylphosphino)-9,9-dimethylxanthene (3.48 g, 6.02 mmol) were added, and the reaction vessel was further degassed under reduced pressure and subjected to nitrogen replacement. To the reaction mixture, a solution of 2,4,6-trichlorophenyl formate (27.1 g, 120 mmol) in toluene (65.1 mL) which was degassed under reduced pressure and subjected to nitrogen replacement was added dropwise at 80°C. The reaction mixture was stirred under a nitrogen atmosphere at 80°C for 30 min and degassed under reduced pressure to obtain a crude product of the title compound.
LCMS: m/z 443 [M+H]⁺
HPLC retention time: 1.59 min (analysis condition F)

### Compound c-5

### 7-Fluoro-2-(oxan-2-yl)indazole-4-carboxylic acid

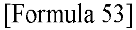

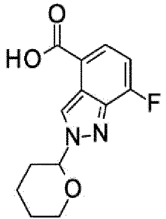

To a suspension of the crude product of (2,4,6-trichlorophenyl) 7-fluoro-2-(oxan-2-yl)indazole-4-carboxylate (compound c-4) in THF (400 mL) in a reaction vessel, a 2.5 M aqueous sodium hydroxide solution (160 mL, 400 mmol) was added, and the mixture was stirred at 60°C for 3.5 hours. The reaction mixture was cooled to room temperature and filtered using Celite. To the filtrate, a 2.5 M aqueous phosphoric acid solution (400 mmol, 160 mL) was added, and the mixture was extracted with a mixed solution of ethyl acetate and THF. The organic layer was extracted three times with an aqueous sodium carbonate solution and water, and then the obtained aqueous layer was filtered. To the filtrate, a 2.5 M aqueous phosphoric acid solution (400 mmol, 160 mL) was added, and the solid was collected by filtration to obtain the title compound (24.01 g, yield 91%) as a colorless solid.
LCMS: m/z 263 [M-H]⁻
HPLC retention time: 0.86 min (analysis condition F)

### Compound c-6

### 7-Fluoro-N-methoxy-N-methyl-2-(oxan-2-yl)indazole-4-carboxamide

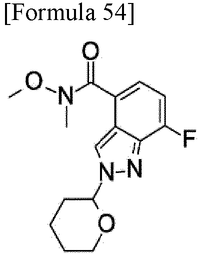

To a solution of 7-fluoro-2-(oxan-2-yl)indazole-4-carboxylic acid (compound c-5, 18.9 g, 71.6 mmol) in THF (358 mL) in a reaction vessel, N,O-dimethylhydroxylamine hydrochloride (9.77 g, 100 mmol) and triethylamine (30.9 mL, 222 mmol) were added, and the mixture was stirred at room temperature for 30 min. To the reaction mixture, HATU (35.4 g, 93 mmol) was added, and the mixture was further stirred for 24 hours. The reaction mixture was combined with that of another lot, then a saturated aqueous ammonium chloride solution was added thereto, and the mixture was extracted with ethyl acetate. The obtained organic layer was washed with a 5% aqueous sodium carbonate solution and brine. The obtained organic layer was dried over anhydrous sodium sulfate, the drying agent was filtered off, and then the obtained filtrate was concentrated under reduced pressure. The obtained residue was purified by silica gel column chromatography (hexane/ethyl acetate) to obtain the title compound (36.1 g).
LCMS: m/z 308 [M+H]⁺
HPLC retention time: 0.88 min (analysis condition F)

### Compound c-7

### (E)-N'-[8-Bromo-6-[7-fluoro-2-(oxan-2-yl)indazole-4-carbonyl]quinolin-5-yl]-N,N-dimethylmethaneimidamide

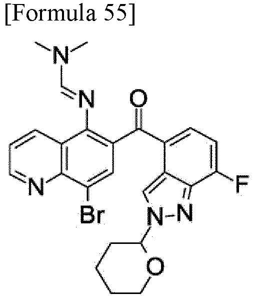

A solution of (E)-N'-(8-bromo-6-iodoquinolin-5-yl)-N,N-dimethylmethaneimidamide (c-2, 41.2 g, 102 mmol) and 7-fluoro-N-methoxy-N-methyl-2-(oxan-2-yl)indazole-4-carboxamide (compound c-6, 34.5 g, 112 mmol) in toluene (638 mL) in a reaction vessel was cooled to -40°C, a 1.3 M isopropylmagnesium chloride-lithium chloride complex solution (110 mL, 143 mmol) was added thereto, and the mixture was stirred at -40°C for 30 min and at 0°C for 3.5 hours. To the reaction mixture, a saturated aqueous ammonium chloride solution was added, and the mixture was extracted with ethyl acetate. The organic layer was washed with brine and dried over anhydrous sodium sulfate, the drying agent was filtered off, and then the filtrate was concentrated under reduced pressure. To the residue, acetonitrile (350 mL) was added, the mixture was stirred for 1 hour, water (88 mL) was then added thereto, and the mixture was further stirred for 1 hour. The solid was collected by filtration and washed with a mixed solution of acetonitrile (20 mL) and water (80 mL) to obtain the title compound (44.39 g, yield 77%) as a yellow solid.
LCMS: m/z 524 [M+H]⁺
HPLC retention time: 0.91 min (analysis condition F)

### Compound c-8

### (5-Amino-8-bromoquinolin-6-yl)-[7-fluoro-2-(oxan-2-yl)indazol-4-yl]methanone

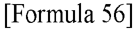

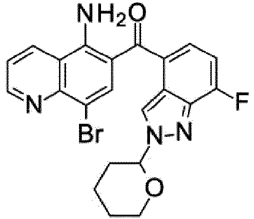

To a suspension of (E)-N'-[8-bromo-6-[7-fluoro-2-(oxan-2-yl)indazole-4-carbonyl]quinolin-5-yl]-N,N-dimethylmethaneimidamide (compound c-7, 22.65 g, 43.2 mmol) in DMSO (216 mL) in a reaction vessel, a 5 M aqueous sodium hydroxide solution (25.9 mL, 130 mmol) was added, and the mixture was stirred at room temperature for 3 hours. To the reaction mixture, a saturated aqueous ammonium chloride solution was added, and the mixture was extracted with ethyl acetate. The obtained organic layer was washed with an aqueous saturated sodium chloride solution and dried over anhydrous sodium sulfate, the drying agent was filtered off, and then the filtrate was concentrated under reduced pressure. The obtained residue was purified by silica gel column chromatography (hexane/ethyl acetate) to obtain the title compound (18.3 g, yield 90%) as a yellow solid.
LCMS: m/z 469 [M+H]⁺
HPLC retention time: 1.15 min (analysis condition F)

### Compound c-259

### (5-Amino-8-cyclopropylquinolin-6-yl)-[7-fluoro-2-(oxan-2-yl)indazol-4-yl]methanone

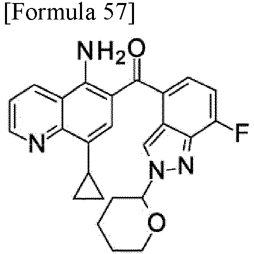

A solution of (5-amino-8-bromoquinolin-6-yl)-[7-fluoro-2-(oxan-2-yl)indazol-4-yl]methanone (compound c-8, 15 g, 32 mmol) in DMA (128 mL) in a reaction vessel was degassed under reduced pressure and subjected to nitrogen replacement, and then XPhos Pd G4 (0.825 g, 0.959 mmol) was added thereto. To the mixture, a solution of 0.5 M cyclopropylzinc bromide in THF (211 mL, 105 mmol) was added, and the mixture was stirred under a nitrogen atmosphere at room temperature for 45 min. The reaction mixture was combined with that of another lot, then a saturated aqueous ammonium chloride solution was added thereto, and the mixture was extracted with ethyl acetate. The organic layer was washed with an aqueous saturated sodium chloride solution and then dried over anhydrous sodium sulfate, the drying agent was filtered off, and then the filtrate was concentrated under reduced pressure. The obtained residue was purified by silica gel column chromatography (hexane/ethyl acetate) to obtain the title compound (20.1 g) as an orange solid.
LCMS: m/z 431 [M+H]⁺
HPLC retention time: 0.94 min (analysis condition F)

### Compound c-260

### 2-Chloro-N-[8-cyclopropyl-6-[7-fluoro-2-(oxan-2-yl)indazole-4-carbonyl]quinolin-5-yl]acetamide

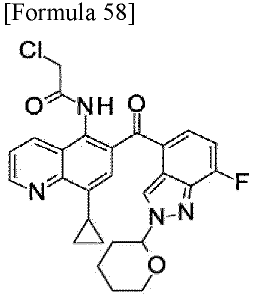

A solution of (5-amino-8-cyclopropylquinolin-6-yl)-[7-fluoro-2-(oxan-2-yl)indazol-4-yl]methanone (compound c-259, 19.52 g, 45.3 mmol) in DCM (302 mL) in a reaction vessel was cooled to 0°C, and then chloroacetyl chloride (6.14 mL, 77 mmol) and pyridine (7.31 mL, 91 mmol) were added thereto, and the mixture was stirred under a nitrogen atmosphere at 0°C for 1 hour. The reaction mixture was concentrated under reduced pressure to obtain a crude product of 2-chloro-N-[8-cyclopropyl-6-[7-fluoro-2-(oxan-2-yl)indazole-4-carbonyl]quinolin-5-yl]acetamide (compound c-260).
LCMS: m/z 507 [M+H]⁺
HPLC retention time: 1.10 min (analysis condition F)

### Compound c-261

### 6-Cyclopropyl-4-[7-fluoro-2-(oxan-2-yl)indazol-4-yl]-3-pyridin-1-ium-1-yl-1H-1,7-phenanthrolin-2-one; chloride

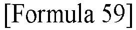

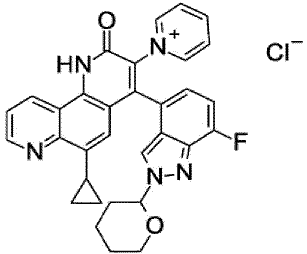

Pyridine (230 mL) was added to the crude product of the compound c-260, and the mixture was stirred at 80°C for 1 hour. The reaction mixture was cooled to room temperature to obtain a solution of 6-cyclopropyl-4-[7-fluoro-2-(oxan-2-yl)indazol-4-yl]-3-pyridin-1-ium-1-yl-1H-1,7-phenanthrolin-2-one; chloride (compound c-261) in pyridine.
LCMS: m/z 532 [M]⁺
HPLC retention time: 0.79 min (analysis condition F)

### Compound c-262

### 6-Cyclopropyl-4-(7-fluoro-1H-indazol-4-yl)-3-pyridin-1-ium-1-yl-1H-1,7-phenanthrolin-2-one; chloride

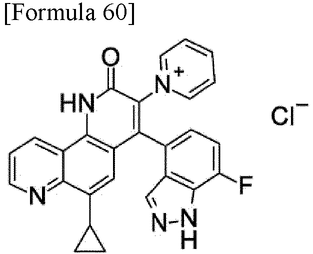

To the solution of the compound c-261 in pyridine, 12 M hydrochloric acid (38.9 mL, 453 mmol) was added, and the mixture was stirred at 80°C for 8 hours. The reaction mixture was cooled to room temperature, and acetonitrile (257 mL) was added thereto. The solid was collected by filtration and then washed three times with acetonitrile (257 mL) to obtain the title compound (compound c-262, 19.18 g, yield 87%).
LCMS: m/z 448 [M]⁺
HPLC retention time: 0.66 min (analysis condition F)

### Compound 1

### 3-Amino-6-cyclopropyl-4-(7-fluoro-1H-indazol-4-yl)-1H-1,7-phenanthrolin-2-one

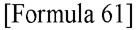

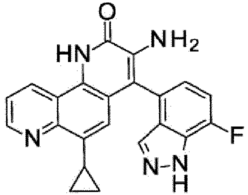

To 6-cyclopropyl-4-(7-fluoro-1H-indazol-4-yl)-3-pyridin-1-ium-1-yl-1H-1,7-phenanthrolin-2-one; chloride (compound c-262, 19.18 g, 39.6 mmol) in a reaction vessel, DMSO (189 mL) and hydrazine monohydrate (19.64 mL, 396 mmol) were added, and the mixture was heated at 80°C for 1 hour. The reaction mixture was cooled to room temperature, and water (253 mL) was added thereto. The solid was collected by filtration and washed twice with water (200 mL) to obtain the title compound (11.63 g, yield 76%).
LCMS: m/z 386 [M+H]⁺
HPLC retention time: 0.49 min (analysis condition A)

### (2) Production of compound 2

(S)-2-amino-1-(3-hydroxy-2,6-dimethylphenyl)-5,6-dimethylpyrrolo[2,3-b]pyridine-3-carboxamide (also referred to as "compound 2") was produced in accordance with the method described in Patent Literature 1.

### (3) Test for binding to ATP binding site of MYT1

The binding capacity of the compound 1 and the compound 2 to the ATP binding site of MYT1 Kinase protein was evaluated. 5X Kinase Buffer A (manufactured by Thermo Fisher Scientific Inc., PV3189) was diluted to 5 times with Milli-Q water, thereby preparing 1x Kinase Buffer. Using 1x Kinase Buffer, MYT1 protein (manufactured by Carna Biosciences, Inc.) was diluted to a concentration of 0.005 µM and Eu-Anti-GST Antibody was diluted to a concentration of 1 µM. In addition, Kinase Tracer 178 (manufactured by Thermo Fisher Scientific Inc., PV5593) was diluted to a concentration of 0.1 µM using 1x Kinase Buffer. To a 96 well-plate, 2.5 µL of the compound 1 or the compound 2 diluted with DMSO was added. Thereafter, 5 µL of a solution in which MYT1 protein and Eu-GST-Antibody diluted above were mixed in a ratio of 1:1 was added, and 2.5 µL of Kinase Tracer 178 diluted above was further added thereto. The mixture was well mixed and then incubated at room temperature for 30 minutes. Thereafter, emission of 665 nm and 615 nm generated by the excitation light of 340 nm were detected using Envision (manufactured by PerkinElmer). The proportion of Tracer bound to MYT1 protein was calculated for every condition by dividing the emission intensity of 665 nm by the emission intensity of 615 nm. The inhibition rate of the compound 1 and the compound 2 added at each concentration was calculated by setting the signal when the compound 1 or the compound 2 was not added as 100% and the signal when MYT1 protein was not added as 0%, and the calculation results of IC₅₀ were shown in Table 9. When the compound's IC₅₀ is 10 µM or less, the compound can be judged to have binding activity to the ATP binding site of MYT1 protein.

### (4) MYT1 kinase activity inhibition test

The MYT1 kinase activity inhibitory capacity of the compound 1 and the compound 2 was evaluated based on the phosphorylation level of Y15 of CDK1 protein caused by MYT1 protein, by ELISA. The kit of CycLex Wee1 Kinase Assay/Inhibitor Screening Kit Ver. 3 (manufactured by MBL, CY-1172V3) was used. Using Kinase Buffer, 10 µL of 5 nM MYT1 protein (manufactured by Carna Biosciences, Inc.) was prepared. In addition, 30 µL of 83.3 µM of ATP was prepared using Kinase Buffer. In addition, 10 µL of the compound 1 or the compound 2 was diluted to each concentration using Kinase Buffer. 30 µL of ATP was added to the plate of the kit, and thereafter, 10 µL of each compound was added thereto. Further, 10 µL of the diluted MYT1 protein solution was added thereto, and then the mixture was mixed, which was incubated at room temperature for 60 minutes. The reaction solution was removed from each well, and each well was washed using Wash Buffer. After Wash Buffer was removed, 100 µL of HRP conjugated Anti-phospho-tyrosine antibody was added to each well, which was incubated at room temperature for 60 minutes. Thereafter, the reaction solution was removed from each well, and each well was washed using Wash Buffer. After Wash Buffer was removed, 100 µL of Substrate Reagent was added to each well, which was incubated at room temperature for 8 minutes. Thereafter, 100 µL of Stop Solution (1 N sulfuric acid) was added to each well according to the manual. Absorbances at 450 nm and 590 nm were measured using Envision (manufactured by PerkinElmer). The phosphorylation level of CDK1 protein was evaluated for each well by subtracting the absorbance at 590 nm from the absorbance at 450 nm. The inhibition rate of the compound 1 and the compound 2 added at each concentration was calculated by setting the signal when the compound 1 or the compound 2 was not added as 100% and the signal when MYT1 protein was not added as 0%, and the calculation results of IC₅₀ were shown in Table 9. When the compound's IC₅₀ is 10 µM or less, the compound can be judged to exhibit inhibition of kinase activity of MYT1 protein.

**[Table 9]**

| Compound name | binding assay IC50 (µM) | ELISA IC50 (µM) |
|---|---|---|
| Compound 1 | 0.0023 | 0.00025 |
| Compound 2 | 0.0046 | 0.00013 |

### 2. Results

Table 9 showed that the compound 1 and the compound 2 both exhibit IC₅₀ of 10 µM or less in the binding assay to the ATP binding site of MYT1 and suggesting that they bind to the ATP binding site of MYT1 protein. It was also suggested that the compound 1 and the compound 2 are compounds having an inhibitory capacity on the kinase activity of MYT1 protein, that is, MYT1 inhibitors.

### (Example 3: Anticancer activity test on each anticancer agent in combination with MYT1 inhibitor)

### 1. Experimental material and method

### (1) Cell line

MDA-MB-175VII (received from ATCC), HCC38 (received from ATCC), MDA-MB-361 (received from ATCC), DU4475 (received from ATCC), Colo-824 (received from CLS Cell Lines Service), MDA-MB-157 (received from ATCC), MDA-MB-468 (received from ATCC) (all of which are breast cancer strains), HLC-1 (received from RIKEN), EKVX (received from NCI), NCI-H1395 (received from ATCC), DMS114 (received from ATCC), SW1271 (received from ATCC), NCI-H1155 (received from ATCC), Lu65 (received from JCRB, JCRB0079, established by Hirohashi, S. and Simosato, Y.), NCI-H2228 (received from ATCC), NCI-H596 (received from ATCC), NCI-H446 (received from ATCC), ChaGo-K-1 (received from ATCC) (all of which are lung cancer strains), RT-4 (received from ATCC), HT-1197 (received from ATCC), TCCSUP (received from ATCC), 5637 (received from ATCC) (all of which are bladder cancer strains), OVMANA (received from JCRB, JCRB1045, established by Gorai, I et al.), RMG-I (received from JCRB, IFO50315, established by Nozawa, S et al.), NCI:OVCAR-3 (received from ATCC) (all of which are ovarian cancer strains) were used in the evaluation of the present invention.

MDA-MB-175VII, HCC38, MDA-MB-361, HLC-1, EKVX, NCI-H1395, DMS114, SW1271, RT-4, HT-1197, OVMANA, and RMG-I are wild-type cancer cell lines having no mutation and deletion of the RB1 gene, and RB1 functions in these cell lines. DU4475, Colo-824, MDA-MB-157, MDA-MB-468, ChaGo-K-1, TCCSUP, and NIH:OVCAR-3 are cancer cell lines in which the RB 1 gene is deleted, NCI-H1155, Lu65, NCI-H596, and 5637 are cancer cell lines having a truncated deletion mutation in the RB1 gene, and NCI-H2228 and NCI-H446 are cancer cell lines having a mutation on a splicing site of the RB1 gene. A decrease in RB1 function is caused in these cell lines. The information on these cancer cell lines and gene mutations are summarized in Table 10.

**[Table 10]**

| Cancer type | Cell line | RB1 status | Detailed RB1 status |
|---|---|---|---|
| Breast cancer | MDA-MB-175 VII | proficient | No alteration¹ |
| Breast cancer | HCC38 | proficient | No alteration¹ |
| Breast cancer | MDA-MB-361 | proficient | No alteration¹ |
| Breast cancer | DU4475 | deficient | HOMDEL¹ |
| Breast cancer | Colo-824 | deficient | S350I, Deletion² |
| Breast cancer | MDA-MB-157 | deficient | HOMDEL¹ |
| Breast cancer | MDA-MB-468 | deficient | HOMDEL¹ |
| Lung cancer | HLC-1 | proficient | No alteration³ |
| Lung cancer | EKVX | proficient | No alteration¹ |
| Lung cancer | NCI-H1395 | proficient | No alteration¹ |
| Lung cancer | DMS114 | proficient | No alteration¹ |
| Lung cancer | SW1271 | proficient | No alteration¹ |
| Lung cancer | NCI-H1155 | deficient | R467*¹ |
| Lung cancer | LU65 | deficient | S82*¹ |
| Lung cancer | NCI-H2228 | deficient | X204_splice, HOMDEL¹ |
| Lung cancer | NCI-H596 | deficient | S182Ifs*3¹ |
| Lung cancer | NCI-H446 | deficient | X654_splice¹ |
| Lung cancer | ChaGo-K-1 | deficient | E837K, HOMDEL¹ |
| Bladder cancer | RT-4 | proficient | No alteration¹ |
| Bladder cancer | HT-1197 | proficient | No alteration¹ |
| Bladder cancer | TCCSUP | deficient | HOMDEL¹ |
| Bladder cancer | 5637 | deficient | Y325*³ |
| Ovarian cancer | OVMANA | proficient | No alteration¹ |
| Ovarian cancer | RMG-I | proficient | No alteration¹ |
| Ovarian cancer | NIH:OVCAR-3 | deficient | HOMDEL¹ |

| | | | |
|---|---|---|---|
| ¹ Nature. 2019 May; 569(7757): 503-508. ² Cell Model Passports (https://cellmodelpassports.sanger.ac.uk/, 2022/12/27 access) ³ Nature. 2012 Mar 28; 483(7391): 603-7. | | | |

### (2) Cytotoxicity test of each anticancer agent upon addition of MYT1 inhibitor

Pemetrexed (manufactured by Tokyo Kasei Kogyo Co., Ltd.), gemcitabine (manufactured by Nippon Kayaku Co., Ltd.), carboplatin (manufactured by SANDOZ), SN-38 (active metabolite of irinotecan, topoisomerase I inhibitor, manufactured by Tokyo Kasei Kogyo Co., Ltd.), sacituzumab govitecan (antibody-drug conjugate that contains SN-38 as a payload and recognizes Trop2, manufactured by MedChem Express), DXd (exatecan derivative for antibody-drug conjugate, topoisomerase I inhibitor, manufactured by MedChem Express), trastuzumab deruxtecan (antibody-drug conjugate that contains DXd as a payload and recognizes HER2, manufactured by DAIICHI SANKYO COMPANY, LIMITED.) were used in the evaluation.

By using Echo (manufactured by Beckman Coulter, Inc.), each concentration of pemetrexed (0.0195 µM to 5.00 µM, common ratio 2), gemcitabine (0.835 nM to 244 nM, common ratio 2), carboplatin (0.781 µM to 200 µM, common ratio 2), SN-38 (0.610 nM to 156 nM, common ratio 2), sacituzumab govitecan (SG) (0.195 nM to 50.0 nM, common ratio 2), DXd (0.195 nM to 50.0 nM, common ratio 2), or trastuzumab deruxtecan (T-DXd) (1.95 nM to 500 nM, common ratio 2) was added to each well of cell culture plates. As the control, the solvents alone which were used to dilute each anticancer agent were added to empty wells.

By adding each MYT1 inhibitor (the compound 1 and the compound 2) (4.88 nM to 5.00 µM, common ratio 2) to the cell culture plates to which each of the anticancer agents described above was added using Echo (manufactured by Beckman Coulter, Inc.), combinations of each anticancer agent at each concentration and each MYT1 inhibitor at each concentration were prepared. As the control, DMSO alone used to dilute each MYT1 inhibitor was added to empty wells.

Each cell cultured under the conditions summarized in Table 11 was seeded on the above plates by the number of cells summarized in Table 12, and incubated at 37°C. After pemetrexed, gemcitabine, carboplatin, SN-38, sacituzumab govitecan (SG), DXd, and trastuzumab deruxtecan (T-DXd) were respectively incubated for 7 days, 4 days, 4 days, 4 days, 4 or 5 days, 4 days, and 5 days, CellTiter-Glo2.0 Cell Viability Assay (manufactured by Promega Corporation) was added, and the luminescence intensity was measured using Multimode Plate Reader EnVision(R) Xcite (manufactured by PerkinElmer, Inc.), which was used in the monitoring of the number of cells. The cell survival rate is calculated by setting the signal of the control well to 100%, and the cytotoxic activity is represented by subtracting the survival rate from the value of 100%. The following evaluation of the combination effect was carried out by using the compound 1 of 156 nM and the compound 2 of 313 nM. When the survival rate under the single agent treatment conditions using the compound 1 or the compound 2 was lower than 80%, the MYT1 inhibitor concentration was reduced at a common ratio of 2, and the combination effect at a concentration at which the survival rate was 80% or more was evaluated.

**[Table 11]**

| Cell line | Culture conditions |
|---|---|
| MDA-MB-175 VII | Leibovitz's L-15 (GIBCO, 11415-064), 10% FBS (SIGMA, F2442-500 mL) |
| HCC38 | RPMI-1640 (SIGMA, R8758-500 mL), 10% FBS (CORNING, 35-076-CV), 10 mM HEPES (SIGMA, H0887-100 mL), 0.45% D-glucose (SIGMA, G8769-100 mL), 1 mM Sodium Pyruvate (GIBCO, 11360-070) |
| MDA-MB-361 | Leibovitz's L-15 (GIBCO, 11415-064), 10% FBS (SIGMA, F2442-500 mL) |
| DU4475 | RPMI 1640 (Nacalai tesque, 30264-56), 20% FBS (SIGMA, F2442-500 mL) |
| Colo-824 | RPMI 1640 (Nacalai tesque, 30264-56), 10% FBS (CORNING, 35-076-CV) |
| MDA-MB-157 | Leibovitz's L-15 (GIBCO, 11415-064), 10% FBS (SIGMA, F2442-500 mL), 0.7 g/L Sodium bicarbonate (GIBCO, 25080-094) |
| MDA-MB-468 | Leibovitz's L-15 (GIBCO, 11415-064), 10% FBS (SIGMA, F2442-500 mL), 0.7 g/L Sodium bicarbonate (GIBCO, 25080-094) |
| HLC-1 | Ham's F-12 (GIBCO, 11765-054), 10% FBS (SIGMA, F2442-500 mL) |
| EKVX | RPMI 1640 (Nacalai tesque, 30264-56), 10% FBS (SIGMA, F2442-500 mL) |
| NCI-H1395 | RPMI-1640 (SIGMA, R8758-500 mL), 10% FBS (SIGMA, F2442-500 mL), 10 mM HEPES (SIGMA, H0887-100 mL), 0.45% D-glucose (SIGMA, G8769-100 mL), 1 mM Sodium Pyruvate (GIBCO, 11360-070) |
| DMS114 | Waymouth's Medium (Thermo, 11220035), 10% FBS (SIGMA, F2442-500 mL) |
| SW1271 | Leibovitz's L-15 (GIBCO, 11415-064), 10% FBS (SIGMA, F2442-500 mL) |
| NCI-H1155 | D-MEM:Ham's F-12=1:1 (GIBCO, 11330-032), 0.02 mg/mL insulin (GIBCO, 12585-014), 0.01 mg/mL transferrin (SIGMA, T8158), 25 nM Sodium selenite (SIGMA, S9133), 50 nM hydrocortisone (SIGMA, H6909), 1 ng/mL hEGF (R&D systems, 236-EG), 0.01 mM Ethanolamine (SIGMA, E0135), 0.01 mM O-phosphorylethanolamine (SIGMA, P0503), 100 pM T3 (SIGMA, T5516), 0.5% (w/v) BSA (SIGMA, A9576), 0.5 mM Sodium pyruvate (GIBCO, 11360-070), 10 mM HEPES (SIGMA, H0887), 2 mM Glutamine (SIGMA, G6013) |
| LU65 | RPMI-1640 (Nacalai tesque, 30264-56),10% FBS (SIGMA, F2442-500 mL) |
| NCI-H2228 | RPMI-1640 (Nacalai tesque, 30264-56),10% FBS (SIGMA, F2442-500 mL), 10 mM HEPES (SIGMA, H0887-100 mL), 0.45% D-glucose (SIGMA, G8769-100 mL), 1 mM Sodium Pyruvate (GIBCO, 11360-070) |
| NCI-H596 | RPMI-1640 (SIGMA, R8758-500 mL), 10% FBS (SIGMA, F2442-500 mL) |
| NCI-H446 | RPMI-1640 (Nacalai tesque, 30264-56), 10% FBS (CORNING, 35-076-CV) |
| ChaGo-K-1 | RPMI-1640 (Nacalai tesque, 30264-56),10% FBS (SIGMA, F2442-500 mL), 10 mM HEPES (SIGMA, H0887-100 mL), 0.45% D-glucose (SIGMA, G8769-100 mL), 1 mM Sodium Pyruvate (GIBCO, 11360-070) |
| RT-4 | McCoy's5A (SIGMA, M8403-500 mL), 10% FBS (SIGMA, F2442-500 mL), 1.5 mM L-Glutamine (SIGMA, G7513) |
| HT-1197 | EMEM (nacalai tesque, 21442-25), 10% FBS (SIGMA, F2442-500 mL), 0.1 mM NEAA (GIBCO, 11140-050), 1 mM Sodium pyruvate (GIBCO, 11360-070) |
| TCCSUP | RPMI-1640 (SIGMA, R8758-500 mL), 10% FBS (SIGMA, F2442-500 mL), 10 mM HEPES (SIGMA, H0887-100 mL), 0.45% D-glucose (SIGMA, G8769-100 mL), 1 mM Sodium Pyruvate (GIBCO, 11360-070) |
| 5637 | RPMI 1640 (Nacalai tesque, 30264-56), 10% FBS (SIGMA, F2442-500 mL) |
| OVMANA | RPMI 1640 (Nacalai tesque, 30264-56), 10% FBS (SIGMA, F2442-500 mL) |
| RMG-I | Ham's F-12 (GIBCO, 11765-054), 10% FBS (SIGMA, F2442-500 mL) |
| NIH:OVCAR-3 | RPMI-1640 (nacalai tesque, 30264-56), 20% FBS (SIGMA, F2442-500 mL), 10 mM HEPES (SIGMA, H0887-100 mL), 0.45% D-glucose (SIGMA, G8769-100 mL), 1 mM Sodium Pyruvate (GIBCO, 11360-070), 0.01 mg/mL insulin (GIBCO, 12585014) |

**[Table 12]**

| Chemotherapeutic agent | Cancer type | Cell line | Number of cells (cells/well) |
|---|---|---|---|
| Pemetrexed | Lung cancer | HLC-1 | 500 |
| | | EKVX | 500 |
| | | NCI-H1395 | 1000 |
| | | NCI-H1155 | 700 |
| | | LU65 | 1000 |
| | | NCI-H2228 | 2000 |
| Gemcitabine | Breast cancer | MDA-MB-175 VII | 1800 |
| | | DU4475 | 4000 |
| | | Colo-824 | 2000 |
| | | MDA-MB-157 | 2000 |
| | Lung cancer | NCI-H1395 | 2000 |
| | | EKVX | 1000 |
| | | NCI-H596 | 1000 |
| | | NCI-H1155 | 1000 |
| | | LU65 | 2000 |
| | Bladder cancer | RT-4 | 4000 |
| | | TCCSUP | 1000 |
| | | 5637 | 1000 |
| | Ovarian cancer | OVMANA | 2000 |
| | | NIH:OVCAR-3 | 2000 |
| Carboplatin | Breast cancer | HCC38 | 2000 |
| | | Colo-824 | 2000 |
| | | MDA-MB-157 | 500 |
| | Lung cancer | EKVX | 1000 |
| | | NCI-H446 | 3000 |
| | Bladder cancer | HT-1197 | 2000 |
| | | 5637 | 1000 |
| | Ovarian cancer | OVMANA | 2000 |
| | | RMG-I | 2000 |
| | | NIH:OVCAR-3 | 2000 |
| SN-38 | Breast cancer | HCC38 | 2000 |
| | | Colo-824 | 2000 |
| | | MDA-MB-157 | 2000 |
| | Lung cancer | HLC-1 | 1000 |
| | | EKVX | 1000 |
| | | DMS114 | 4000 |
| | | SW1271 | 2000 |
| | | ChaGo-K-1 | 1000 |
| | | NCI-H446 | 4000 |
| | Ovarian cancer | OVMANA | 2000 |
| | | RMG-I | 2000 |
| | | NIH:OVCAR-3 | 3000 |
| Sacituzumab govitecan | Breast cancer | HCC38 | 2000 |
| | | Colo-824 | 2000 |
| | | MDA-MB-157 | 500 |
| | Lung cancer | HLC-1 | 1000 |
| | | EKVX | 1000 |
| | | SW1271 | 1500 |
| | | ChaGo-K-1 | 1000 |
| DXd | Breast cancer | MDA-MB-361 | 4000 |
| | | HCC38 | 2000 |
| | | Colo-824 | 2000 |
| | Lung cancer | NCI-H1395 | 3000 |
| | | SW1271 | 2000 |
| | | NCI-H446 | 4000 |
| | | ChaGo-K-1 | 1000 |
| | Ovarian cancer | RMG-I | 2000 |
| | | NIH:OVCAR-3 | 3000 |
| Trastuzumab deruxtecan | Breast cancer | HCC38 | 2000 |
| | | MDA-MB-361 | 4000 |
| | | MDA-MB-175 VII | 3000 |
| | | DU4475 | 3000 |
| | | MDA-MB-157 | 500 |
| | | MDA-MB-468 | 2000 |

Bliss score based on the Bliss independence, which is an index of the combination effect in various combinations of the chemotherapeutic agent and the MYT1 inhibitor, was calculated based on the cytotoxic activity of each chemotherapeutic agent at each concentration in the MYT1 inhibitor-free well and the cytotoxic activity of each MYT1 inhibitor at each concentration in the chemotherapeutic agent-free well (see Non Patent Literature 9). In addition, HSA score, which is an index of the combination effect different from the Bliss independence, was calculated (see Non Patent Literature 9). The maximum value of Bliss score, maximum value of HSA score, and the concentration of the MYT1 inhibitor were summarized in Table 13. When the maximum value was lower than 0, each score was denoted as 0.00.

**[Table 13]**

| Chemotherapeutic agent | Cancer type | Cell line | RB1 status | Com pound 1 | | | Com pound 2 | | |
|---|---|---|---|---|---|---|---|---|---|
| | | | | MYT1 inhibitor concentration | Bliss score | HSA score | MYT1 inhibitor concentration | Bliss score | HSA score |
| Pemetrexed | Lung cancer | HLC-1 | proficient | 156 nM | 14.5 | 13.2 | 313 nM | 2.41 | 0.775 |
| | | EKVX | proficient | 156 nM | 29.4 | 26.9 | 313 nM | 29.3 | 26.8 |
| | | NCI-H1395 | proficient | 156 nM | 8.05 | 2.74 | 313 nM | 26.8 | 10.4 |
| | | NCI-H1155 | deficient | 156 nM | 79.2 | 81.1 | 313 nM | 69.1 | 72.9 |
| | | LU65 | deficient | 156 nM | 81.8 | 78.1 | 313 nM | 60.0 | 65.5 |
| | | NCI-H2228 | deficient | 39.1 nM | 43.6 | 53.1 | 156 nM | 50.9 | 62.0 |
| Gemcitabine | Breast cancer | MDA-MB-175VII | proficient | 156 nM | 33.1 | 21.0 | 313 nM | 28.9 | 17.1 |
| | | DU4475 | deficient | 156 nM | 64.2 | 64.2 | 313 nM | 61.8 | 73.4 |
| | | Colo-824 | deficient | 156 nM | 92.2 | 86.5 | 313 nM | 80.6 | 78.2 |
| | | MDA-MB-157 | deficient | 156 nM | 67.2 | 74.9 | 313 nM | 53.0 | 57.8 |
| | Lung cancer | NCI-H1395 | proficient | 156 nM | 16.2 | 3.94 | 313 nM | 9.12 | 0.00 |
| | | EKVX | proficient | 156 nM | 16.2 | 7.70 | 313 nM | 20.5 | 10.1 |
| | | NCI-H596 | deficient | 156 nM | 39.3 | 42.9 | 313 nM | 38.7 | 42.9 |
| | | NCI-H1155 | deficient | 156 nM | 49.1 | 47.7 | 313 nM | 51.6 | 56.3 |
| | | LU65 | deficient | 156 nM | 73.3 | 66.8 | 313 nM | 79.2 | 64.7 |
| | Bladder cancer | RT-4 | proficient | 156 nM | 2.73 | 1.81 | 313 nM | 14.0 | 9.25 |
| | | TCCSUP | deficient | 156 nM | 45.1 | 56.1 | 313 nM | 41.2 | 47.1 |
| | | 5637 | deficient | 156 nM | 63.0 | 66.4 | 313 nM | 63.8 | 72.7 |
| | Ovarian cancer | OVMANA | proficient | 156 nM | 14.0 | 16.9 | 313 nM | 3.92 | 15.6 |
| | | NIH:OVCAR-3 | deficient | 156 nM | 48.3 | 57.2 | 313 nM | 54.9 | 60.3 |
| Carboplatin | Breast cancer | HCC38 | proficient | 156 nM | 29.6 | 30.6 | 313 nM | 29.3 | 31.6 |
| | | Colo-824 | deficient | 156 nM | 87.4 | 84.8 | 313 nM | 53.0 | 59.0 |
| | | MDA-MB-157 | deficient | 78.3 nM | 60.1 | 66.3 | 78.3 nM | 49.8 | 63.4 |
| | Lung cancer | EKVX | proficient | 156 nM | 18.1 | 6.95 | 313 nM | 11.6 | 7.55 |
| | | NCI-H446 | deficient | 156 nM | 55.5 | 57.7 | 313 nM | 51.3 | 61.5 |
| | Bladder cancer | HT-1197 | proficient | 156 nM | 8.12 | 6.08 | 313 nM | 6.68 | 2.46 |
| | | 5637 | deficient | 156 nM | 33.2 | 50.1 | 156 nM | 33.2 | 43.5 |
| | Ovarian cancer | OVMANA | proficient | 156 nM | 5.95 | 1.89 | 313 nM | 4.34 | 11.1 |
| | | RMG-I | proficient | 156 nM | 7.84 | 12.20 | 313 nM | 11.2 | 16.8 |
| | | NIH:OVCAR-3 | deficient | 156 nM | 55.2 | 62.0 | 156 nM | 49.8 | 62.8 |
| SN-38 | Breast cancer | HCC38 | proficient | 156 nM | 10.6 | 16.0 | 313 nM | 3.76 | 15.6 |
| | | Colo-824 | deficient | 156 nM | 65.8 | 72.7 | 313 nM | 55.5 | 63.1 |
| | | MDA-MB-157 | deficient | 156 nM | 81.7 | 83.0 | 313 nM | 70.5 | 71.9 |
| | Lung cancer | HLC-1 | proficient | 156 nM | 7.36 | 8.45 | 313 nM | 6.16 | 6.88 |
| | | EKVX | proficient | 156 nM | 22.5 | 11.5 | 313 nM | 18.9 | 9.03 |
| | | DMS114 | proficient | 156 nM | 18.8 | 17.7 | 313 nM | 19.9 | 19.1 |
| | | SW1271 | proficient | 156 nM | 17.7 | 18.0 | 313 nM | 12.1 | 19.1 |
| | | ChaGo-K-1 | deficient | 156 nM | 72.0 | 71.5 | 313 nM | 61.7 | 69.4 |
| | | NCI-H446 | deficient | 156 nM | 54.8 | 51.1 | 313 nM | 65.4 | 60.3 |
| | Ovarian cancer | OVMANA | proficient | 156 nM | 8.53 | 4.81 | 313 nM | 6.95 | 11.1 |
| | | RMG-I | proficient | 156 nM | 19.0 | 19.4 | 313 nM | 9.07 | 12.8 |
| | | NIH:OVCAR-3 | deficient | 156 nM | 70.3 | 74.1 | 313 nM | 66.5 | 77.5 |
| Sacituzumab govitecan | Breast cancer | HCC38 | proficient | 156 nM | 11.9 | 13.0 | 313 nM | 13.6 | 19.4 |
| | | Colo-824 | deficient | 156 nM | 78.7 | 66.7 | 313 nM | 40.5 | 42.8 |
| | | MDA-MB-157 | deficient | 39.1 nM | 60.3 | 71.3 | 39.1 nM | 88.9 | 76.9 |
| | Lung cancer | HLC-1 | proficient | 156 nM | 16.8 | 16.7 | 313 nM | 13.7 | 16.0 |
| | | EKVX | proficient | 156 nM | 4.84 | 22.9 | 313 nM | 1.42 | 16.9 |
| | | SW1271 | proficient | 156 nM | 11.5 | 16.0 | 313 nM | 1.17 | 4.51 |
| | | ChaGo-K-1 | deficient | 156 nM | 42.8 | 49.3 | 313 nM | 37.0 | 51.6 |
| DXd | Breast cancer | MDA-MB-361 | proficient | 156 nM | 12.0 | 15.5 | 313 nM | 2.51 | 4.03 |
| | | HCC38 | proficient | 156 nM | 5.06 | 13.8 | 313 nM | 10.8 | 10.9 |
| | | Colo-824 | deficient | 156 nM | 65.7 | 63.7 | 313 nM | 73.2 | 75.7 |
| | Lung cancer | NCI-H1395 | proficient | 156 nM | 5.14 | 0.00 | 313 nM | 6.52 | 0.00 |
| | | SW1271 | proficient | 156 nM | 13.1 | 20.2 | 313 nM | 5.86 | 16.2 |
| | | NCI-H446 | deficient | 156 nM | 48.3 | 49.3 | 313 nM | 52.4 | 50.4 |
| | | ChaGo-K-1 | deficient | 156 nM | 51.8 | 51.9 | 313 nM | 56.7 | 62.6 |
| | Ovarian cancer | RMG-I | proficient | 156 nM | 9.2 | 9.1 | 313 nM | 0.00 | 10.1 |
| | | NIH:OVCAR-3 | deficient | 156 nM | 49.6 | 58.4 | 313 nM | 45.3 | 59.7 |
| Trastuzumab deruxtecan | Breast cancer | HCC38 | proficient | 156 nM | 5.22 | 12.5 | 313 nM | 10.9 | 19.2 |
| | | MDA-MB-361 | proficient | 156 nM | 3.70 | 5.58 | 313 nM | 2.79 | 8.12 |
| | | MDA-MB-175VII | proficient | 156 nM | 0.00 | 0.00 | 313 nM | 3.73 | 0.00 |
| | | DU4475 | deficient | 156 nM | 46.3 | 50.7 | 313 nM | 32.8 | 38.1 |
| | | MDA-MB-157 | deficient | 39.1 nM | 55.2 | 58.9 | 78.3 nM | 37.1 | 43.1 |
| | | MDA-MB-468 | deficient | 156 nM | 59.1 | 59.1 | 313 nM | 70.9 | 68.4 |

In Figure 6 to Figure 12, the maximum value of Bliss score and the maximum value of HSA score were plotted separately for the cell line in which the RB 1 gene is wild type and RB1 functions and the cell line whose RB 1 gene has a mutation or deletion and in which a decrease in RB1 function is caused, and Student's t-test was carried out to calculate p values. Based on the p values, asterisks were described in each Figure (*: p < 0.05, **: p < 0.01, ***: p < 0.001, ****: p < 0.0001).

### 2. Results

### (1) Pemetrexed

As shown in Table 13 and Figure 6, it was confirmed that Bliss score and HSA score of the cytotoxic activity caused by pemetrexed upon MYT1 inhibitor treatment with the compound 1 or the compound 2 are significantly high in the lung cancer cell line in which an RB 1 gene mutation or deletion is observed and a decrease in RB1 function is caused, as compared with the cancer cell line in which neither RB 1 gene mutation nor deletion is observed and RB 1 functions.

These results demonstrated that the cytotoxic activity of the chemotherapeutic agent such as pemetrexed can be effectively enhanced by administering the MYT1 inhibitor to cancer such as lung cancer in which an RB 1 gene mutation or deletion is observed and a decrease in RB1 function is caused.

### (2) Gemcitabine

As shown in Table 13 and Figure 7, it was confirmed that Bliss score and HSA score of the cytotoxic activity caused by gemcitabine upon MYT1 inhibitor treatment with the compound 1 or the compound 2 are significantly high in the lung cancer, breast cancer, bladder cancer, and ovarian cancer cell lines in which an RB1 gene mutation or deletion is observed and a decrease in RB 1 function is caused, as compared with the cancer cell line in which neither RB 1 gene mutation nor deletion is observed and RB1 functions.

These results demonstrated that the cytotoxic activity of the chemotherapeutic agent such as gemcitabine can be effectively enhanced by administering the MYT1 inhibitor to cancer such as lung cancer, breast cancer, bladder cancer, and ovarian cancer in which an RB 1 gene mutation or deletion is observed and a decrease in RB1 function is caused.

### (3) Carboplatin

As shown in Table 13 and Figure 8, it was confirmed that Bliss score and HSA score of the cytotoxic activity caused by carboplatin upon MYT1 inhibitor treatment with the compound 1 or the compound 2 are significantly high in the lung cancer, breast cancer, bladder cancer, and ovarian cancer cell lines in which an RB1 gene mutation or deletion is observed and a decrease in RB 1 function is caused, as compared with the cancer cell line in which neither RB 1 gene mutation nor deletion is observed and RB1 functions.

These results demonstrated that the cytotoxic activity of the chemotherapeutic agent such as carboplatin can be effectively enhanced by administering the MYT1 inhibitor to cancer such as lung cancer, breast cancer, bladder cancer, and ovarian cancer in which an RB 1 gene mutation or deletion is observed and a decrease in RB1 function is caused.

### (4) SN-38

As shown in Table 13 and Figure 9, it was confirmed that Bliss score and HSA score of the cytotoxic activity caused by SN-38 upon MYT1 inhibitor treatment with the compound 1 or the compound 2 are significantly high in the lung cancer, breast cancer, and ovarian cancer cell lines in which an RB 1 gene mutation or deletion is observed and a decrease in RB 1 function is caused, as compared with the cancer cell line in which neither RB 1 gene mutation nor deletion is observed and RB 1 functions.

These results demonstrated that the cytotoxic activity of the chemotherapeutic agent such as SN-38 can be effectively enhanced by administering the MYT1 inhibitor to cancer such as lung cancer, breast cancer, and ovarian cancer in which an RB 1 gene mutation or deletion is observed and a decrease in RB1 function is caused.

### (5) Sacituzumab govitecan (SG)

As shown in Table 13 and Figure 10, it was confirmed that Bliss score and HSA score of the cytotoxic activity caused by sacituzumab govitecan (SG) upon MYT1 inhibitor treatment with the compound 1 or the compound 2 are significantly high in the lung cancer and breast cancer cell lines in which an RB 1 gene mutation or deletion is observed and a decrease in RB1 function is caused, as compared with the cancer cell line in which neither RB 1 gene mutation nor deletion is observed and RB 1 functions.

These results demonstrated that the cytotoxic activity by the antibody-drug conjugate using a chemotherapeutic agent such as SN-38 as a payload can be effectively enhanced by administering the MYT1 inhibitor to cancer such as lung cancer and breast cancer in which an RB1 gene mutation or deletion is observed and a decrease in RB1 function is caused.

### (6) DXd

As shown in Table 13 and Figure 11, it was confirmed that Bliss score and HSA score of the cytotoxic activity caused by DXd upon MYT1 inhibitor treatment with the compound 1 or the compound 2 are significantly high in the lung cancer, breast cancer, and ovarian cancer cell lines in which an RB1 gene mutation or deletion is observed and a decrease in RB1 function is caused, as compared with the cancer cell line in which neither RB1 gene mutation nor deletion is observed and RB1 functions.

These results demonstrated that the cytotoxic activity by the chemotherapeutic agent such as DXd can be effectively enhanced by administering the MYT1 inhibitor to cancer such as lung cancer, breast cancer, and ovarian cancer in which an RB1 gene mutation or deletion is observed and a decrease in RB1 function is caused.

### (7) Trastuzumab deruxtecan (T-DXd)

As shown in Table 13 and Figure 12, it was confirmed that Bliss score and HSA score of the cytotoxic activity caused by trastuzumab deruxtecan (T-DXd) upon MYT1 inhibitor treatment with the compound 1 or the compound 2 are significantly high in the breast cancer cell line in which an RB1 gene mutation or deletion is observed and a decrease in RB1 function is caused, as compared with the cancer cell line in which neither RB1 gene mutation nor deletion is observed and RB1 functions.

These results demonstrated that the cytotoxic activity by the antibody-drug conjugate using a chemotherapeutic agent such as DXd as a payload can be effectively enhanced by administering the MYT1 inhibitor to cancer such as breast cancer in which an RB1 gene mutation or deletion is observed and a decrease in RB1 function is caused.

### (Example 4: Anticancer activity test using MYT1 inhibitor in combination with gemcitabine in in vivo)

### 1. Experimental material and method

### (1) Drug efficacy test on DU4475 tumor-bearing mouse using compound 1 and gemcitabine in combination

To examine the combination effect of the MYT1 inhibitor and the anticancer agent on cancer with a decrease in RB1 function in in vivo, gemcitabine alone, the compound 1 alone, or a combination of gemcitabine and the compound 1 was administered to mice implanted with DU4475. After DU4475 was cultured in vitro, cells were collected on the day of implantation in mice, and the cells were suspended in a Matrigel (manufactured by Corning Inc., product code: 356234) solution diluted with a Hanks' Balanced Salt solution (manufactured by Sigma-Aldrich, product code: H9269) (final concentration: 50%) at 2.5 × 10⁷ cells/mL. 0.2 mL of the prepared cell suspension was implanted into the groin of each BALB/c-nu/nu mouse (CAnN.Cg-Foxn1<nu>/CrlCrlj, manufactured by Charles River Laboratories).

Then, to the mice (DU4475 tumor-bearing mice) in which the engraftment of the tumor (cancer cells) was confirmed, the compound 1 suspended in a mixed solution of 10% DMSO (manufactured by Wako Pure Chemical Industries, Ltd., product code: 043-07216), 10% Cremophor (manufactured by Sigma-Aldrich, product code: C5135), 15% polyethylene glycol 400 (manufactured by Wako Pure Chemical Industries, Ltd., product code: 161-09065), and 15% hydroxypropyl-β-cyclodextrin (manufactured by NIHON SHOKUHIN KAKO CO., LTD., product code: 7585-39-9) was orally administered at 100 mg/kg once daily for 12 days, or gemcitabine (manufactured by Nippon Kayaku Co., Ltd.) diluted with a physiological saline was intraperitoneally administered at 60 mg/kg once every four days, twice in total, or a combination administration thereof was carried out.

Administration of each drug was started at day 7 after implantation, and the tumor volume was measured until day 15. The tumor volume was calculated by a least-squares method by measuring the minor axis and the major axis of the tumor using an electronic caliper (manufactured by Mitutoyo Corporation, product code: CD-15AX). Also, for the DU4475 tumor-bearing mouse to which no drug was administered (drug non-administration group), the tumor volume was measured.

The change of tumor volume with time after implantation of DU4475 in the drug non-administration group, the compound 1 single administration group, the gemcitabine single administration group, and the combination group is shown in Figure 13. The two-tailed Student's t-test was carried out on the tumor volume of each group at day 15 after implantation, and p values (significant level: 5%) were calculated. First, it was compared whether there was a difference in the tumor volume between the drug non-administration group and the gemcitabine single administration group. Next, it was compared whether there was a difference in the tumor volume between the compound 1 single administration group and the combination administration group. When a significant difference was recognized, it was also compared whether there was a difference in the tumor volume between the gemcitabine single administration group and the combination administration group. Based on the p values, asterisks were described in each Figure (n.s.: p ≥ 0.05, *: p < 0.05, **: p < 0.01, ***: p < 0.001, ****: p < 0.0001).

### (2) Drug efficacy test on NIH:OVCAR-3 tumor-bearing mouse using compound 1 and gemcitabine in combination

To examine the combination effect of the MYT1 inhibitor and the anticancer agent on cancer with a decrease in RB1 function in in vivo, gemcitabine alone, the compound 1 alone, or a combination of gemcitabine and the compound 1 was administered to mice implanted with NIH:OVCAR-3. After NIH:OVCAR-3 was cultured in vitro, cells were collected on the day of implantation in mice using a 2.5 g/L-Tripsin/1 mmol-EDTA Solution (manufactured by NACALAI TESQUE, INC., product code: 35554-64), and the cells were suspended in a Matrigel (manufactured by Corning Inc., product code: 356234) solution diluted with a Hanks' Balanced Salt solution (manufactured by Sigma-Aldrich, product code: H9269) (final concentration: 50%) at 2.5 × 10⁷ cells/mL. 0.2 mL of the prepared cell suspension was implanted into the groin of each BALB/c-nu/nu mouse (CAnN.Cg-Foxn1<nu>/CrlCrlj, Charles River Laboratories).

Then, to the mice (NIH: OVCAR-3 tumor-bearing mice) in which the engraftment of the tumor (cancer cells) was confirmed, the compound 1 suspended in a mixed solution of 10% DMSO (manufactured by Wako Pure Chemical Industries, Ltd., product code: 043-07216), 10% Cremophor (manufactured by Sigma-Aldrich, product code: C5135), 15% polyethylene glycol 400 (manufactured by Wako Pure Chemical Industries, Ltd., product code: 161-09065), and 15% hydroxypropyl-β-cyclodextrin (manufactured by NIHON SHOKUHIN KAKO CO., LTD., product code: 7585-39-9) was orally administered at 100 mg/kg once daily for 14 days, or gemcitabine (manufactured by Nippon Kayaku Co., Ltd.) diluted with a physiological saline was intraperitoneally administered at 60 mg/kg once every seven days, twice in total, or a combination administration thereof was carried out.

Administration of each drug was started at day 38 after implantation, and the tumor volume was measured in all the groups from day 38 to 52. The tumor volume was calculated by a least-squares method by measuring the minor axis and the major axis of the tumor using an electronic caliper (manufactured by Mitutoyo Corporation, product code: CD-15AX). Also, for the NIH:OVCAR-3 tumor-bearing mouse to which no drug was administered (drug non-administration group), the tumor volume was measured.

The change of tumor volume with time after implantation of NIH:OVCAR-3 in the drug non-administration group, the compound 1 single administration group, the gemcitabine single administration group, and the combination administration group is shown in Figure 14.

The two-tailed Student's t-test was carried out on the tumor volume of each group at day 52 after implantation, and P values (significant level: 5%) were calculated. First, it was compared whether there was a difference in the tumor volume between the drug non-administration group and the gemcitabine single administration group. Next, it was compared whether there was a difference in the tumor volume between the compound 1 single administration group and the combination administration group. When a significant difference was recognized, it was also compared whether there was a difference in the tumor volume between the gemcitabine single administration group and the combination administration group. Based on the p values, asterisks were described in each Figure (n.s.: p ≥ 0.05, *: p < 0.05, **: p < 0.01, ***: p < 0.001, ****: p < 0.0001).

### 2. Results

As shown in Figure 13, in the in vivo experiment using DU4475 which is a breast cancer strain with a decrease in RB1 function, the gemcitabine single administration group does not have a significant difference in the tumor volume as compared with that of the drug non-administration group, and it is suggested that the DU4475 model is a model on which gemcitabine administration does not exhibit significant drug efficacy. The combination administration group had a significantly small tumor volume as compared with that of the compound 1 single administration group. In addition, the combination administration group had a significantly small tumor volume as compared with that of the gemcitabine single administration group.

This suggests that a significantly high combination antitumor effect is exerted by using the compound 1 and gemcitabine in combination in a model on which gemcitabine does not exhibit drug efficacy. As shown in Figure 14, in the in vivo experiment using the NIH:OVCAR-3 cell which is an ovarian cancer strain with a decrease in RB1 function, the gemcitabine single administration group had a significantly small tumor volume as compared with that of the drug non-administration group. This suggests that the NIH:OVCAR-3 cell is a model on which gemcitabine administration exhibits drug efficacy. The combination administration group had a significantly small tumor volume as compared with that of the compound 1 single administration group. In addition, the combination administration group had a significantly small tumor volume as compared with that of the gemcitabine single administration group. This suggests that a significantly high combination antitumor effect is exerted by using the compound 1 and gemcitabine in combination in a model on which gemcitabine exhibits drug efficacy.

### (Example 5: Cytotoxicity test of gemcitabine upon addition of MYT1 inhibitor in cancer cell line with positive expression of hypophosphorylated RB1 protein and cancer cell line with positive expression of hyperphosphorylated RB1 protein)

### 1. Experimental material and method

### (1) Cell line

RMG-I (ovarian cancer strain, received from JCRB, IFO50315, established by Nozawa, S et al.), OVTOKO (ovarian cancer strain, received from JCRB, JCRB1048, established by Gorai, I et al.), OVMANA (ovarian cancer strain, received from JCRB, JCRB1045, established by Gorai, I et al.), ES-2 (ovarian cancer strain, received from ATCC), JHOC-5 (ovarian cancer strain, received from RIKEN), PA-1 (ovarian cancer strain, received from ATCC), MDA-MB-175VII (breast cancer strain, received from ATCC), NCI-H1395 (lung cancer strain, received from ATCC), RT-4 (bladder cancer strain, received from ATCC) were used in the evaluation.

RMG-I, MDA-MB-175VII, NCI-H1395, OVTOKO, OVMANA, RT-4, ES-2, JHOC-5, PA-1 are RB1 wild-type cancer cell lines having no mutation and deletion of RB1 gene, and RB1 is expressed in these cell lines.

RMG-I is a CCNE1-amplified cell line, in which the function of CCNE1 is enhanced. MDA-MB-175VII, NCI-H1395, OVTOKO, OVMANA, RT-4, ES-2, JHOC-5, and PA-1 are RB1 wild-type cancer cell lines having no mutation and amplification of CCNE1 gene, and the function of CCNE1 is not enhanced in these cell lines.

RMG-I, MDA-MB-175VII, NCI-H1395, OVTOKO, OVMANA, RT-4, ES-2, JHOC-5, and PA-1 are wild-type cancer cell lines having no mutation and deletion of FBXW7 gene, and FBXW7 functions in these cell lines.

OVTOKO is a cell line having an R183G mutation of PPP2R1A gene, and according to Non Patent Literature 11, is a cancer cell line in which a decrease in PPP2R1A function is caused. RMG-I, MDA-MB-175VII, NCI-H1395, OVMANA, RT-4, ES-2, JHOC-5, and PA-1 are PPP2R1A wild-type cancer cell lines having no mutation and deletion of PPP2R1A gene, and PPP2R1A functions in these cell lines.

The information on these cancer cell lines and gene mutations are summarized in Table 14.

**[Table 14]**

| Cell line | Cancer type | RB1 status | CCNE1 status | FBXW7 status | PPP2R1A status |
|---|---|---|---|---|---|
| RMGI | Ovarian cancer | No alteration*¹ | AMP*¹ | No alteration*¹ | No alteration*¹ |
| MDA-MB-175VII | Breast cancer | No alteration*¹ | No alteration*¹ | No alteration*¹ | No alteration*¹ |
| NCI-H1395 | Lung cancer | No alteration*¹ | No alteration*¹ | No alteration*¹ | No alteration*¹ |
| OVTOKO | Ovarian cancer | No alteration*¹ | No alteration*¹ | No alteration*¹ | R183G*¹ |
| OVMANA | Ovarian cancer | No alteration*¹ | No alteration*¹ | No alteration*¹ | No alteration*¹ |
| RT-4 | Bladder cancer | No alteration*¹ | No alteration*¹ | No alteration*¹ | No alteration*¹ |
| ES-2 | Ovarian cancer | No alteration*² | No alteration*² | No alteration*² | No alteration*² |
| JHOC-5 | Ovarian cancer | No alteration*¹ | No alteration*¹ | No alteration*¹ | No alteration*¹ |
| PA-1 | Ovarian cancer | No alteration*^{1, 3} | No alteration*^{1, 3} | No alteration*^{1, 3} | No alteration*^{1, 3} |

| | | | | | |
|---|---|---|---|---|---|
| 1: Nature 2019 May;569(7757):503-508. 2: Nature 2012 Mar 28;483(7391):603-7. 3: DepMap portal Copy Number Public 23Q2 (https://depmap.org/portal/) | | | | | |

### (2) Evaluation of phosphorylation level of RB1 protein of each cancer cell line

One tablet of PhosSTOP^{™} (manufactured by Roche, 4906845001) was dissolved in 1 mL of MilliQ to prepare a 10x Phosphatase inhibitor solution. One tablet of cOmplete^{™} protease inhibitor cocktail (manufactured by Roche, 11697498001) was dissolved in 1 mL of MilliQ to prepare a 25x Protease inhibitor solution. Using 10x Cell Lysis Buffer (manufactured by Cell Signaling Technology, Inc: #9803), the 10x Phosphatase inhibitor solution, the 25x Protease inhibitor solution, and MilliQ, a buffer for dissolving cells (1x Cell Lysis Buffer, 1x Phosphatase inhibitor solution, and 1x Protease inhibitor solution) was prepared and ice-cooled.

Each cancer cell line was cultured under the conditions described in Table 15, and the cell surface was washed with PBS (phosphate buffered saline, manufactured by Nacalai tesque, Inc.: 14249-24). Then, cells were collected with a scraper, and then suspended with a cell lysis buffer, and after suspension, the mixture was incubated on ice for 15 minutes. Thereafter, centrifugation was carried out at 15000 rpm and 4°C for 10 minutes to collect a supernatant. For the collected supernatant, the protein concentration was quantitatively determined with a Direct Detect^{™} spectrometer (manufactured by Merck). Using protein extracts of each cell line and a sample buffer solution (for SDS-PAGE, 6x concentrated, containing a reducing agent) (manufactured by Nacalai tesque, Inc.: 09499-14) and MilliQ, samples for Western blotting of each cell line (protein final concentration of 1 µg/µL or 0.5 µg/ µL, 1x sample buffer solution) were prepared.

**[Table 15]**

| Cell line | Culture conditions |
|---|---|
| RMG-I | Ham's F-12 (GIBCO, 11765-054), 10% FBS (SIGMA, F2442-500 mL) |
| MDA-MB-175 VII | Leibovitz's L-15 (GIBCO, 11415-064), 10% FBS (SIGMA, F2442-500 mL) |
| NCI-H1395 | RPMI-1640 (SIGMA, R8758-500 mL), 10% FBS (SIGMA, F2442-500 mL), 10 mM HEPES (SIGMA, H0887-100 mL), 0.45% D-glucose (SIGMA, G8769-100 mL), 1 mM Sodium Pyruvate (GIBCO, 11360-070) |
| OVTOKO | RPMI-1640 (SIGMA, R8758-500 mL), 10% FBS (SIGMA, F2442-500 mL) |
| OVMANA | RPMI-1640 (SIGMA, R8758-500 mL), 10% FBS (SIGMA, F2442-500 mL) |
| RT-4 | McCoy's5A (SIGMA, M8403-500 mL), 10% FBS (SIGMA, F2442-500 mL), 1.5 mM L-Glutamine (SIGMA, G7513) |
| ES-2 | McCoy's5A (SIGMA, M8403-500 mL), 10% FBS (SIGMA, F2442-500 mL), 1.5 mM L-Glutamine (SIGMA, G7513) |
| JHOC-5 | D-MEM:Ham's F-12=1:1 (GIBCO, 11330-032), 10% FBS (SIGMA, F2442-500 mL), 0.1 mM NEAA (GIBCO, 11140-050) |
| PA-1 | EMEM (nacalai tesque, 21442-25), 10% FBS (SIGMA, F2442-500 mL), 0.1 mM NEAA (GIBCO, 11140-050), 1 mM Sodium pyruvate (GIBCO, 11360-070) |

Each sample was applied to SuperSep^{™} Ace, 5-20%, 17 well (manufactured by FUJIFILM Wako Pure Chemical Corporation: #194-15021) so that the amount of protein was equal to each other, and SDS-PAGE was carried out with SDS-PAGE buffer, pH 8.5 (manufactured by FUJIFILM Wako Pure Chemical Corporation: #192-16801). Thereafter, each sample was transferred to Trans-Blot Turbo Transfer Pack PVDF (mini) (manufactured by Bio-Rad Laboratories, Inc.: #1704156), and blocking was carried out at room temperature for 30 minutes using Bullet Blocking One (manufactured by Nacalai tesque, Inc.: 13779-01). The primary antibody was diluted using Signal Enhancer HIKARI for Western Blotting and ELISA Solution A (manufactured by Nacalai tesque, Inc.: 02272-74), the secondary antibody was diluted using Signal Enhancer HIKARI for Western Blotting and ELISA Solution B (manufactured by Nacalai tesque, Inc.: 02297-64), and blotting was sequentially carried out. The antibody used and dilution rate are shown in Table 16.

**[Table 16]**

| Detection protein or phosphorylation | Primary antibody name (manufacturer name, catalog number) | Primary antibody dilution rate | Secondary antibody | Secondary antibody dilution rate |
|---|---|---|---|---|
| RB1 T826 | Anti-Rb (phospho T826) antibody [EPR5351] manufactured by Abcam plc, ab133446) | 1000 | Anti-rabbit IgG, HRP-linked Antibody (manufactured by Cell signaling Technoogy, Inc, #7074) | 10000 |
| RB1 T821 | Anti-Rb (phospho T821) antibody manufactured by Abcam plc, ab4787) | 250 | Anti-rabbit IgG, HRP-linked Antibody (manufactured by Cell signaling Technoogy, Inc, #7074) | 10000 |
| RB1 S807/S811 | Phospho-Rb (Ser807/811) (D20B12) XP^{®} Rabbit mAb (manufactured by Cell signaling Technology, Inc, #8516) | 500 | Anti-rabbit IgG, HRP-linked Antibody (manufactured by Cell signaling Technoogy, Inc, #7074) | 10000 |
| RB1 S795 | Phospho-Rb (Ser795) Antibody (manufactured by Cell signaling Technology, Inc, #9301) | 250 | Anti-rabbit IgG, HRP-linked Antibody (manufactured by Cell signaling Technoogy, Inc, #7074) | 10000 |
| RB1 S788 | Anti-Rb (phospho S788) antibody [6HCLC] manufactured by Abcam plc, ab277775) | 250 | Anti-rabbit IgG, HRP-linked Antibody (manufactured by Cell signaling Technoogy, Inc, #7074) | 10000 |
| RB1 S780 | Phospho-Rb (Ser780) Antibody (manufactured by Cell signaling Technology, Inc, #9307) | 250 | Anti-rabbit IgG, HRP-linked Antibody (manufactured by Cell signaling Technoogy, Inc, #7074) | 10000 |
| RB1 T373 | Anti-Rb (phospho T373) antibody [EP821Y] manufactured by Abcam plc, ab52975) | 1000 | Anti-rabbit IgG, HRP-linked Antibody (manufactured by Cell signaling Technoogy, Inc, #7074) | 10000 |
| RB1 T356 | Anti-Rb (phospho T356) antibody [EPR2153AY] manufactured by Abcam plc, ab76298) | 250 | Anti-rabbit IgG, HRP-linked Antibody (manufactured by Cell signaling Technoogy, Inc, #7074) | 10000 |
| RB1 T252 | Anti-Rb (phospho T252) antibody [EPR17733] manufactured by Abcam plc, ab184797) | 250 | Anti-rabbit IgG, HRP-linked Antibody (manufactured by Cell signaling Technoogy, Inc, #7074) | 10000 |
| RB1 total | BD Pharmingen^{™} Purified Mouse Anti-Human Retinoblastoma Protein (manufactured by BD Biosciences, 554136) | 250 | Anti-mouse IgG, HRP-linked Antibody (manufactured by Cell signaling Technoogy, Inc, #7076) | 10000 |
| GAPDH | GAPDH (14C10) Rabbit mAb (manufactured by Cell signaling Technology, Inc, #2118) | 4000 | Anti-rabbit IgG, HRP-linked Antibody (manufactured by Cell signaling Technoogy, Inc, #7074) | 20000 |

For the detection, Pierce^{™} ECL Plus Western Blotting Substrate (manufactured by Thermo Fischer Scientific Inc.: 32132) was used. Figure 15 is the results of blotting obtained by using ChemiDoc Touch MP (manufactured by Bio-Rad Laboratories, Inc.). The band intensity detected by Western blotting was quantitatively determined by using ImageLab (manufactured by Bio-Rad Laboratories, Inc.), values (relative values) of each band intensity relative to the quantitative value of GAPDH (housekeeping gene) were calculated, and relative values (phosphorylation level) of each cell line when the numerical value of the cell line exhibiting the lowest value was set to 1 were shown in Table 17.

It was found from Table 17 that the phosphorylation level of T826, T821, S807 and/or S811, S780, T373, or T356 of RB1 protein in 3 cell lines, ES-2, JHOC-5, and PA-1 is high as compared with that in any cell line among 6 cell lines, RMG-I, MDA-MB-175VII, NCI-H1395, OVTOKO, OVMANA, and RT-4. Generally, when the phosphorylation level of an amino acid residue is high, the phosphorylated amino acid residue can be determined to be highly expressed. Therefore, 3 cell lines, ES-2, JHOC-5, and PA-1 were determined to be cell lines with positive expression of hyperphosphorylated RB1 protein. In addition, 2 cell lines, RMG-I and MDA-MB-175VII were found to have low phosphorylation levels of the amino acid residues other than S795, S788, and T252, as compared with those in 3 cell lines, ES-2, JHOC-5, and PA-1. Further, 4 cell lines, NCI-H1395, OVTOKO, OVMANA, and RT-4 were found to have low phosphorylation levels in all amino acid residues measured, as compared with those in 3 cell lines, ES-2, JHOC-5, and PA-1. Hereinafter, these 6 cell lines, RMG-I, MDA-MB-175VII, NCI-H1395, OVTOKO, OVMANA, and RT-4 were determined to be the group "with positive expression of hypophosphorylated RB 1 protein" and analyzed.

**[Table 17]**

| Cell line | T826 | T821 | S807/S811 | S795 | S788 | S780 | T373 | T356 | T252 |
|---|---|---|---|---|---|---|---|---|---|
| RMG-I | 4.94 | 8.19 | 6.30 | 9.79 | 34.15 | 2.72 | 6.02 | 6.37 | 9.53 |
| MDA-MB-175 VII | 3.93 | 3.36 | 3.97 | 5.27 | 19.75 | 1.69 | 2.73 | 2.97 | 3.21 |
| NCI-H1395 | 1.02 | 2.58 | 3.19 | 3.90 | 12.13 | 1.39 | 1.54 | 3.54 | 4.03 |
| OVTOKO | 1.95 | 2.99 | 4.20 | 4.04 | 3.51 | 2.83 | 3.82 | 2.35 | 1.75 |
| OVMANA | 1.10 | 2.68 | 2.03 | 1.82 | 1.82 | 1.00 | 1.00 | 1.79 | 1.14 |
| RT-4 | 1.00 | 1.00 | 1.00 | 1.00 | 1.00 | 1.12 | 1.48 | 1.00 | 1.00 |
| ES-2 | 25.86 | 21.63 | 13.84 | 10.04 | 28.01 | 9.87 | 13.87 | 28.35 | 8.62 |
| JHOC-5 | 29.22 | 21.77 | 14.11 | 9.23 | 25.66 | 10.69 | 11.50 | 24.72 | 12.59 |
| PA-1 | 24.50 | 12.61 | 9.14 | 5.30 | 18.18 | 6.19 | 7.94 | 10.77 | 8.20 |

### (3) Analysis of expression level of E2F3 gene in ovarian cancer cell line

Expression Public 23Q2 was downloaded from DepMap portal (URL: https://depmap.org/portal/ [searched on July 14, 2023]), and the expression level of the E2F3 gene in each cell line was shown in Table 18. The expression level of the E2F3 gene in each cell line which was determined to be the cell line with positive expression of hypophosphorylated RB1 protein or cell line with positive expression of hyperphosphorylated RB1 protein was plotted, Student's t-test was carried out to calculate p values, and each expression level of the E2F3 gene was described in Figure 16 based on the values (*: p < 0.05, **: p < 0.01).

**[Table 18]**

| Cell line | E2F3 log2 (TPM+1) |
|---|---|
| RMGI | 2.68 |
| OVTOKO | 2.54 |
| OVMANA | 2.33 |
| ES-2 | 3.48 |
| JHOC-5 | 4.11 |
| PA-1 | 4.01 |

### (4) Cytotoxicity test of gemcitabine upon addition of MYT1 inhibitor

Gemcitabine (manufactured by Nippon Kayaku Co., Ltd.) was used in the evaluation. By using Echo (manufactured by Beckman Coulter, Inc.), each concentration of gemcitabine (0.835 nM to 214 nM, common ratio 2) was added to each well of a cell culture plate. As the control, the solvents alone which were used to dilute each anticancer agent were added to empty wells. By using Echo (manufactured by Beckman Coulter, Inc.), the MYT1 inhibitor (compound 1 or compound 2, 4.88 nM to 5.00 µM, common ratio 2) was also added to the above cell culture plate to which gemcitabine was added to prepare combinations of each anticancer agent at each concentration and each MYT1 inhibitor at each concentration. As the control, DMSO alone used to dilute each MYT1 inhibitor was added to an empty well.

Each cell cultured under the conditions summarized in Table 15 was seeded on the above plate by the number of cells summarized in Table 19, and incubated at 37°C. After incubation for 4 days, CellTiter-Glo 2.0 Cell Viability Assay (manufactured by Promega Corporation) was added to the culture solutions, and the luminescence intensity was measured using Multimode Plate Reader EnVision (R) Xcite (manufactured by PerkinElmer, Inc.), which was used in the monitoring of the number of cells. The cell survival rate is calculated by setting the signal of the control well to 100%, and the cytotoxic activity is represented by subtracting the survival rate from the value of 100%. Setting the compound 1 to 156 nM and the compound 2 to 313 nM, the combination effect was evaluated as follows. When the survival rate under the single agent treatment conditions using the compound 1 or the compound 2 was lower than 75%, the MYT1 inhibitor concentration was reduced at a common ratio of 2, and the combination effect at a concentration at which the survival rate was 75% or more was evaluated.

**[Table 19]**

| Cell line | Number of cells (cells/well) | Culture period |
|---|---|---|
| RMG-I | 2000 | 4 days |
| MDA-MB-175 VII | 1800 | 4 days |
| NCI-H1395 | 2000 | 4 days |
| OVTOKO | 1000 | 4 days |
| OVMANA | 2000 | 4 days |
| RT-4 | 4000 | 4 days |
| ES-2 | 750 | 4 days |
| JHOC-5 | 500 | 4 days |
| PA-1 | 500 | 4 days |

Bliss score based on the Bliss independence, which is an index of the combination effect in various combinations of gemcitabine and the MYT1 inhibitor, was calculated based on the cytotoxic activity of gemcitabine at each concentration in the MYT1 inhibitor-free well and the cytotoxic activity of each MYT1 inhibitor at each concentration in the gemcitabine-free well (see Non Patent Literature 9). In addition, as an index of the combination effect different from the Bliss independence, HSA score was calculated (see Non Patent Literature 9). The maximum value of Bliss score, the maximum value of HSA score, and the concentration of the MYT1 inhibitor were summarized in Table 20. When the maximum value was lower than 0, each score was denoted as 0.00. Each score was calculated to three significant figures.

**[Table 20]**

| Cell line | Compound 1 | | | Compound 2 | | |
|---|---|---|---|---|---|---|
| | Evaluated MYT1 inhibitor concentration | Bliss score | HSA score | Evaluated MYT1 inhibitor concentration | Bliss score | HSA score |
| RMG-I | 156 nM | 8.66 | 8.50 | 313 nM | 21.8 | 31.2 |
| MDA-MB-175VII | 156 nM | 33.1 | 21.0 | 313 nM | 28.9 | 17.1 |
| NCI-H1395 | 156 nM | 16.2 | 3.94 | 313 nM | 9.12 | 0.00 |
| OVTOKO | 156 nM | 31.4 | 29.0 | 313 nM | 20.8 | 24.2 |
| OVMANA | 156 nM | 14.0 | 16.9 | 313 nM | 3.92 | 15.6 |
| RT-4 | 156 nM | 2.73 | 1.81 | 313 nM | 14.0 | 9.25 |
| ES-2 | 78.1 nM | 73.2 | 65.3 | 39.1 nM | 59.1 | 62.0 |
| JHOC-5 | 156 nM | 59.5 | 62.3 | 313 nM | 48.9 | 58.5 |
| PA-1 | 156 nM | 36.1 | 36.9 | 313 nM | 39.3 | 56.2 |

The maximum value of Bliss score and the maximum value of HSA score in each cell line which was determined to be the cell line with positive expression of hypophosphorylated RB1 protein or the cell line with positive expression of hyperphosphorylated RB1 protein were plotted, and Student's t-test was carried out to calculate p values. The results are shown in Figure 17. Based on the p values, asterisks were described in Figure 17 (n.s.: p ≥ 0.05, *: p < 0.05, **: p < 0.01, ***: p < 0.001, ****: p < 0.0001).

### 2. Results

It was suggested from Table 14 that the expression of hyperphosphorylated RB1 protein does not depend on the gene mutation, amplification or deletion of RB1, CCNE1, FBXW7, and PPP2R1A.

RB1 protein plays a role as the G1/S checkpoint by suppressing the activity of E2F protein which is a transcription factor for promoting the transition from the G1 stage to the S stage, and is known to cause a decrease in function for suppressing E2F protein by hyperphosphorylation (Non Patent Literature 12).

The E2F gene is included in the target of the E2F transcription factor, and it is known that there is positive feedback (Non Patent Literature 13). This indicates that the expression level of the E2F gene is an index of the transition to the S stage and the decrease in function of RB1 protein.

Table 19 and Figure 16 suggested that the decrease in function of RB 1 protein is caused in the ovarian cancer cell lines with positive expression of hyperphosphorylated RB1 protein, because the expression level of the E2F3 gene is significantly high in the ovarian cancer cell lines with positive expression of hyperphosphorylated RB 1 protein, as compared with that in the ovarian cancer cell lines with positive expression of hypophosphorylated RB1 protein.

As shown in Table 20 and Figure 17, it was confirmed that the Bliss score and the HSA score of the cytotoxic activity caused by gemcitabine upon treatment with the MYT1 inhibitor of the compound 1 or the compound 2 is significantly high in the cancer cell lines in which the expression of hyperphosphorylated RB 1 protein was confirmed, as compared with the cancer cell lines with positive expression of hypophosphorylated RB1 protein.

These results demonstrated that a decrease in function of RB1 protein is caused in the cancers determined to be cancers with positive expression of hyperphosphorylated RB1 protein, and by administering the MYT1 inhibitor to these cancers, the cytotoxic activity of the chemotherapeutic agent such as gemcitabine can be synergistically enhanced.

### (Example 6: Anticancer activity test on cancer with positive expression of hyperphosphorylated RB1 protein by combination administration of gemcitabine and MYT1 inhibitor in in vivo)

### 1. Experimental material and method

### (1) Drug efficacy test on ES-2 tumor-bearing mouse using compound 1 and gemcitabine in combination

To examine the combination effect of the MYT1 inhibitor and the anticancer agent on the cancer with positive expression of hyperphosphorylated RB 1 protein in in vivo, mice implanted with ES-2 (a cell line determined to be a cell line with positive expression of hyperphosphorylated RB1 protein, as described above) were divided into 4 groups, gemcitabine alone, the compound 1 alone, or a combination of gemcitabine and the compound 1 was administered to mice in each of three groups. No drug was administered to the remaining group (drug non-administration group). After ES-2 was cultured in vitro, cells were collected on the day of implantation in mice, and the cells were suspended in a Matrigel (manufactured by Corning Inc., product code: 356234) solution diluted with a Hanks' Balanced Salt solution (manufactured by Sigma-Aldrich, product code: H9269) (final concentration: 50%) at 5 × 10⁶ cells/mL. 0.2 mL of the prepared cell suspension was implanted into the groin of each BALB/c-nu/nu mouse (CAnN.Cg-Foxn1<nu>/CrlCrlj manufactured by Charles River Laboratories).

Then, 10% DMSO (manufactured by Wako Pure Chemical Industries, Ltd., product code: 043-07216), 10% Cremophor (manufactured by Sigma-Aldrich, product code: C5135), 15% polyethylene glycol 400 (manufactured by Wako Pure Chemical Industries, Ltd., product code: 161-09065), and 15% hydroxypropyl-β-cyclodextrin (manufactured by NIHON SHOKUHIN KAKO CO., LTD., product code: 7585-39-9) were mixed to prepare a mixed solution. To the mice (ES-2 tumor-bearing mice) in which the engraftment of the tumor (cancer cells) was confirmed, the compound 1 suspended in the above mixed solution was orally administered at 30 mg/kg once daily for 14 days, or a gemcitabine solution (manufactured by Nippon Kayaku Co., Ltd.) diluted with a physiological saline was intraperitoneally administered at 60 mg/kg once every seven days, twice in total, or a combination administration thereof was carried out. In the combination administration group, the compound 1 suspended in the mixed solution was orally administered and a gemcitabine solution diluted with a physiological saline was intraperitoneally administered, in the same manner as above.

Administration of each drug was started at day 11 after implantation, and the tumor volume was measured until day 25. The tumor volume was calculated by a least-squares method by measuring the minor axis and the major axis of the tumor using an electronic caliper (manufactured by Mitutoyo Corporation, product code: CD-15AX). Also, for the ES-2 tumor-bearing mouse to which no agent was administered (drug non-administration group), the tumor volume was measured. At the time when an individual whose tumor volume reached 2,000 mm³ or more was generated in each group, the administration to the mice in the group was stopped, and the mice were euthanized.

The change of tumor volume with time after implantation of ES-2 in the drug non-administration group, the compound 1 single administration group, the gemcitabine single administration group, and the combination administration group is shown in Figure 18A. The two-tailed Student's t-test was carried out at day 18 after implantation (the final day of administration of the compound 1 single administration group) whether there was a difference in the tumor volume between the compound 1 single administration group and the combination administration group, and p values (significant level: 5%) were calculated. When a significant difference was recognized, the two-tailed Student's t-test was carried out at day 25 after implantation (the next day of the final day of administration of the gemcitabine single administration group) whether there was a difference in the tumor volume between the gemcitabine single administration group and the combination administration group, and p values (significant level: 5%) were calculated. Based on the p values, asterisks were described in Figure 18 (n.s.: p ≥ 0.05, *: p < 0.05, **: p < 0.01, ***: p < 0.001, ****: p < 0.0001).

### (2) Drug efficacy test on ES-2 tumor-bearing mouse using compound 2 and gemcitabine in combination

To examine the combination effect of the MYT1 inhibitor and the anticancer agent on the cancer with positive expression of hyperphosphorylated RB1 protein (a cell line determined to be a cell line with positive expression of hyperphosphorylated RB1 protein, as described above) in in vivo, gemcitabine alone, the compound 2 alone, or a combination of gemcitabine and the compound 2 was administered to mice implanted with ES-2. After ES-2 was cultured in vitro, cells were collected on the day of implantation in mice, and the cells were suspended with a Hanks' Balanced Salt solution (manufactured by Sigma-Aldrich, product code: H9269) at 5 × 10⁶ cells/mL. 0.2 mL of the prepared cell suspension was implanted into the groin of each BALB/c-nu/nu mouse (CAnN.Cg-Foxn1<nu>/CrlCrlj manufactured by Charles River Laboratories).

Then, 10% DMSO (manufactured by Wako Pure Chemical Industries, Ltd., product code: 043-07216), 10% Cremophor (manufactured by Sigma-Aldrich, product code: C5135), 15% polyethylene glycol 400 (manufactured by Wako Pure Chemical Industries, Ltd., product code: 161-09065), and 15% hydroxypropyl-β-cyclodextrin (manufactured by NIHON SHOKUHIN KAKO CO., LTD., product code: 7585-39-9) were mixed to prepare a mixed solution. The compound 2 suspended in the above mixed solution was orally administered at 10 mg/kg once daily for 14 days, or a gemcitabine solution (manufactured by Nippon Kayaku Co., Ltd.) diluted with a physiological saline was intraperitoneally administered at 60 mg/kg once every seven days, twice in total, or a combination administration thereof was carried out. In the combination administration group, the compound 1 suspended in the mixed solution was orally administered and a gemcitabine solution diluted with a physiological saline was intraperitoneally administered, in the same manner as above.

Administration of each drug was started at day 10 after implantation, and the tumor volume was measured until day 24. The tumor volume was calculated by a least-squares method by measuring the minor axis and the major axis of the tumor using an electronic caliper (manufactured by Mitutoyo Corporation, product code: CD-15AX). Also, for the ES-2 tumor-bearing mouse to which no agent was administered (drug non-administration group), the tumor volume was measured. At the time when an individual whose tumor volume reached 1,400 mm³ or more was generated in each group, the administration to the mice in the group was stopped, and the mice were euthanized.

The change of tumor volume with time after implantation of ES-2 in the drug non-administration group, the compound 2 single administration group, the gemcitabine single administration group, and the combination administration group is shown in Figure 18B. The two-tailed Student's t-test was carried out at day 17 after implantation (the final day of administration of the compound 2 single administration group) whether there was a difference in the tumor volume between the compound 2 single administration group and the combination administration group, and p values (significant level: 5%) were calculated. When a significant difference was recognized, the two-tailed Student's t-test was carried out at day 24 after implantation (the next day of the final day of administration of the gemcitabine single administration group) whether there was a difference in the tumor volume between the gemcitabine single administration group and the combination administration group, and p values (significant level: 5%) were calculated. Based on the p values, asterisks were described in Figure 18 (n.s.: p ≥ 0.05, *:p < 0.05, **: p < 0.01, ***: p < 0.001, ****: p < 0.0001).

### 2. Results

As shown in Figure 18A and 18B, in the antitumor test using ES-2 which is a cancer cell line with positive expression of hyperphosphorylated RB1 protein in in vivo, the combination administration group had a significantly small tumor volume as compared with those of the compound 1 single administration group and the compound 2 single administration group. In addition, the combination administration group had a significantly small tumor volume as compared with that of the gemcitabine single administration group in all the tests. This demonstrates that a synergistic therapeutic effect is expected in the cancer with positive expression of hyperphosphorylated RB1 protein by using the MYT1 inhibitor and gemcitabine in combination.

This suggests that a synergistic therapeutic effect is expected in a cancer patient in which positive expression of hyperphosphorylated RB1 protein is detected by administrating the MYT1 inhibitor and the chemotherapeutic agent in combination.

### [Acknowledgements]

We appreciate NCI for providing us cell lines, EKVX and HOP-62 used in Examples. We appreciate Drs. Gazdar and Minna formerly of NCI, NIH and Public Health Service for providing us cell lines, NCI-H596, NCI-H661, NCI-H322, NCI-H1993, NCI-H441, NCI-H1666, NCI-H2122, NCI-H2110, NCI-H358, NCI-H460, NCI-H1395, NCI-H292, NCI-H1155, NCI-H2228, and NCI-H446, used in Examples.

[Sequence List]

## Claims

1. A pharmaceutical composition comprising, in combination with a chemotherapeutic agent, an MYT1 inhibitor as an active ingredient for treatment or prevention of cancer of a cancer patient in which RB1 gene mutation positivity, a decrease in expression of an RB1 gene or protein, or positive expression of hyperphosphorylated RB 1 protein is detected.

2. A pharmaceutical composition comprising, in combination with an MYT1 inhibitor, a chemotherapeutic agent as an active ingredient for treatment or prevention of cancer of a cancer patient in which RB1 gene mutation positivity, a decrease in expression of an RB1 gene or protein, or positive expression of hyperphosphorylated RB 1 protein is detected.

3. The pharmaceutical composition according to claim 1 or 2, wherein the RB1 gene mutation comprises a mutation causing insertion, substitution, deletion, and/or addition of at least one amino acid residue to wild-type RB1 protein.

4. The pharmaceutical composition according to any one of claims 1 to 3, wherein the RB1 gene mutation is a nonsense mutation, a frameshift mutation, a splice site mutation, or a homozygous or heterozygous deletion.

5. The pharmaceutical composition according to any one of claims 1 to 4, wherein the RB1 gene mutation is a mutation decreasing a function of RB1.

6. The pharmaceutical composition according to any one of claims 1 to 5, wherein the MYT1 inhibitor is at least one selected from the group consisting of a low molecular compound, a polypeptide, and a polynucleotide.

7. The pharmaceutical composition according to any one of claims 1 to 6, wherein the chemotherapeutic agent is at least one selected from the group consisting of an antimetabolite, an anticancer antibiotic, a mitosis inhibitor, a topoisomerase inhibitor, a platinating agent, an alkylating agent, and an antibody-drug conjugate.

8. The pharmaceutical composition according to any one of claims 1 to 7, wherein the MYT1 inhibitor and the chemotherapeutic agent are simultaneously or separately administered.

9. The pharmaceutical composition according to any one of claims 1 to 7, wherein the MYT1 inhibitor and the chemotherapeutic agent are administered as a combination drug.

10. A method for treating or preventing cancer of a cancer patient in which RB 1 gene mutation positivity, a decrease in expression of an RB 1 gene or protein, or positive expression of hyperphosphorylated RB 1 protein is detected,
the method comprising administering a combination of a chemotherapeutic agent and an MYT1 inhibitor to the cancer patient.

11. A method for suppressing growth of cancer cells in a cancer patient in which RB 1 gene mutation positivity, a decrease in expression of an RB 1 gene or protein, or positive expression of hyperphosphorylated RB 1 protein is detected,
the method comprising bringing an MYT1 inhibitor and a chemotherapeutic agent into contact with the cancer cells.

12. A method for improving responsiveness to cancer treatment with a chemotherapeutic agent, the method comprising:
administering an MYT1 inhibitor together with the chemotherapeutic agent to a cancer patient,
wherein the cancer is a cancer of the cancer patient in which RB 1 gene mutation positivity, a decrease in expression of an RB 1 gene or protein, or positive expression of hyperphosphorylated RB 1 protein is detected.

13. A method for predicting responsiveness to treatment of cancer with a combination of an MYT1 inhibitor and a chemotherapeutic agent, the method comprising:
detecting or allowing a third person to detect the presence or absence of an RB 1 gene mutation, the presence or absence of a decrease in expression of an RB 1 gene or protein, or the presence or absence of the hyperphosphorylated RB 1 protein, in a cancer patient-derived biological sample, and
determining the patient as having responsiveness to the treatment of cancer with the combination of the MYT1 inhibitor and the chemotherapeutic agent, when the RB1 gene mutation is positive, the expression of the RB 1 gene or protein is decreased, or the expression of the hyperphosphorylated RB 1 protein is positive.

14. A method for selecting a cancer patient to which administration of a combination of an MYT1 inhibitor and a chemotherapeutic agent is more effective, the method comprising:
detecting or allowing a third person to detect the presence or absence of an RB 1 gene mutation, the presence or absence of a decrease in expression of an RB 1 gene or protein, or the presence or absence of the hyperphosphorylated RB 1 protein, in a cancer patient-derived biological sample, and
determining the cancer patient as a cancer patient to which administration of the combination of the MYT1 inhibitor and the chemotherapeutic agent is more effective, based on the presence of the mutation, the decrease in expression, or the positivity of the expression of the hyperphosphorylated RB 1 protein.

15. A method for screening a compound effective for treatment or prevention of cancer in a cancer patient in which RB 1 gene mutation positivity, a decrease in expression of an RB 1 gene or protein, or positive expression of hyperphosphorylated RB 1 protein is detected, the method comprising:
measuring MYT1 inhibitory activity of a candidate compound; and
selecting a candidate compound having the MYT1 inhibitory activity as a compound effective for treatment of cancer.
